# EUROPEAN PATENT APPLICATION

(11) **EP 1 997 805 A1**
(43) Date of publication of application: **03.12.2008**
(21) Application number: 07290683.7
(22) Date of filing: 01.06.2007
(51) Int. Cl.: C07D 211/16, C07D 211/58, C07D 235/26, C07D 401/04, C07D 401/06, C07D 401/12, C07D 401/14, C07D 403/04, C07D 403/06, C07D 409/14, C07D 413/12, C07D 417/14, C07D 471/04, C07D 487/06, C07D 495/04, A61K 31/4412, A61K 31/4166, A61K 31/4188, A61P 33/02, A61P 33/06

(54) **Compounds with antiparasitic activity, applications thereof to the treatment of infectious diseases caused by apicomplexans**

(71) Applicant: Commissariat à l'Energie Atomique, 75015 Paris (FR)
(72) Inventor: Deligny, Michael, 14000 Caen (FR); Saidani, Nadia, 34000 Montpellier (FR); Bonneau, Anne-Laure, 75016 Paris (FR); Botte, Cyrille, 38100 Grenoble (FR); Hardre, Hélène, 38300 Châteauvillain (FR); Rousseau, Bernard, 92300 Levallois-Perret (FR); Vial, Henri, 34080 Montpellier (FR); Mercier, Corinne, 38070 La Tronche (FR); Lopez, Roman, 92130 Issy les Moulineaux (FR); Marechal, Eric, 38000 Grenoble (FR)
(74) Representative: Goulard, Sophie

(57) **Abstract**

The Invention relates to compounds having an antiparasitic activity, and to their use as a drug, in particular as a drug for the prevention and/or treatment of parasitic diseases caused by apicomplexans. The invention also relates to pharmaceutical compositions containing those compounds.

## Description

The Invention relates to compounds having an antiparasitic activity, and to their use as a drug, in particular as a drug for the prevention and/or treatment of parasitic diseases caused by apicomplexans. The invention also relates to pharmaceutical compositions containing those compounds.

The Apicomplexa phylum constitutes a particularly diverse group of unicellular protists. Besides members of the Colpodellida sub-group, all apicomplexans are obligate intracellular parasites, infecting virtually all animals from mollusks to mammals. They were originally classified as protozoa (> 4000 species). Amongst them, the malarial parasite *Plasmodium* sp., represents one of the most important threats for humankind, with 300 to 500 million estimated clinical cases and 1.5 to 3 million deaths each year, most of them African children. More benign, but widely spread is *Toxoplasma gondii,* a parasite causing congenital neurological birth defects and, along with *Cryptosporidium,* being the most reported opportunistic infection associated with immunosuppressive conditions, including AIDS. Other parasites in this phylum are of veterinary importance including pathogens of cattle and chicken, *Theileria* and *Eimeria.*

The Apicomplexa phylum contains several thousands of unicellular parasites among which:
- *Plasmodium falciparum,* the major malaria causative agent, as well as other parasites of the same genus (the three other *Plasmodium* species that cause human malaria: *P. vivax, P. ovale, P. malariae;* the *Plasmodium* species known to infect other vertebrates: *P. yoelii, P. berghei, P. chabaudi, P. vivax, P. gallinaceum, P. knowlesi, P. reichenowi,* etc.);
- *Babesia microti,* the causative agent of human babesiosis, as well as all other parasites that cause babiesosis in mammals (e.g. *B. divergens, B. bigemina, B. major, B. bovis* (bovine babesiosis), *B. canis, B. gibsoni* (canine babesiosis), *B. equi, B. caballi* (equine babesiosis), etc.);
- *Toxoplasma gondii,* the causative agent of toxoplasmosis in humans and other mammals;
- *Neospora caninum,* the causative agent of neosporosis;
- *Cryptosporidium hominis,* the causative agent of human cryptosporidiosis, as well as other parasites of the same genus (*C. parvum,* etc.);
- *Theileria annulata,* the causative agent of theileriosis, as well as other parasites of the same genus (*T. parva,* etc.)
- *Sarcocystis neurona, Eimeria tenella, Gregarina niphandrodes,* as well as any other parasites of the apicomplexa phylum causing infectious diseases in metazoans.

All apicomplexans share a polarized cellular organization schema, with a basal pole and an apical pole. Membrane compartmentalization exhibits some specific features as compared to other eukaryotic cells such as those of yeasts or mammals. The cell is polarized and exhibits a set of subcellular structures called the apical complex that contains a hollow truncated cone, the conoid, and three types of vesicular secretory organelles, namely rhoptries, micronemes and dense granules. During host cell invasion, the apical pole is the first to enter the cell. Membrane compartments include those known to be connected through the endomembrane flow in all eukaryotic cells, i.e. the Golgi, endoplasmic reticulum, nuclear envelope and plasma membrane. A peripheral inner membrane complex is applied to the plasma membrane surface. Semi autonomous organelles include a mitochondria surrounded by two membranes, and the apicoplast (absent in some genera such as *Cryptosporidium*) surrounded by four membranes.

The apicomplexan parasites are transmitted by different vectors in complex life cycles. For instance, and like all other malaria parasites of the *Plasmodium* genus, *Plasmodium falciparum* spreads by infecting successively two types of hosts: humans and female Anopheles mosquitoes. In humans, the parasites grow and multiply first in the liver cells and then in the red cells of the blood. In the blood, successive broods of parasites grow inside the red cells and destroy them, releasing daughter parasites ("merozoites") that continue the cycle by invading other red cells. The blood stage parasites are those that cause the symptoms of malaria. When certain forms of blood stage parasites ("gametocytes") are picked up by a female Anopheles mosquito during a blood meal, they start another, different cycle of growth and multiplication in the mosquito. After 10-18 days, the parasites are found (as "sporozoites") in the mosquito's salivary glands. When the Anopheles mosquito takes a blood meal on another human, the sporozoites are injected with the insect's saliva and start another human infection.

Chemotherapies are needed to fight against apicomplexan-caused diseases. These therapies generally differ with the type of parasite responsible of the infection.

In the case of malaria, this life-threatening disease affects half a billion humans in underdeveloped and developing countries. Its global heartland is Africa, with an appalling death toll of I to 2 million people every year (WHO World Malaria Report, Geneva, World Health Organization, WHO/UNICEF; 2005). Endemic malaria ranges from a permanent incidence in sub-Saharan and equatorial Africa, to a seasonal but recently escalating prevalence in Southern Africa Grover-Kopec et al., Malar J. 2006, 5:38). Malaria was eradicated from temperate regions following concerted preventative sanitary actions and after important insecticide campaigns and systematic treatments with available drugs, i.e. quinine and chloroquine. The prophylactic programs of the 1950's and 1960's, essentially based on insecticide and drug treatments, failed to control malaria in subtropical areas. Resistance to chloroquine spread rapidly. Subsequent attempts to achieve progress in malaria prophylaxis have been characterized by the failure of vaccine development, withdrawal of some insecticides because of toxicity and negative environmental impact, the alarming spread of mosquito resistance to insecticides and of resistance of *Plasmodium* to the very few drugs that have been developed.

In an effort to improve the success rate of drug development, several approaches are being investigated. Most of the strategies are based on re-design of existing drugs, for example artemisinin, an antiplasmodial molecule from *Artemisia annua* comprising an endoperoxide moiety, and derivatives thereof such as Artesunate, which can be produced efficiently and cheaply (Renslo A. R., McKerrow J. H., Nature Chem. Biol., 2006, 2, 701-710).

Others choose to only keep the endoperoxyde moiety of artemisinin, leading to the discovery of synthetic RBx 11160 (OZ277) which mimics the chemical and biological properties of this molecule and is already in clinical trials as antimalaria drug used with piperaquine (Vennerstrom, J.L. et al., Nature, 2004, 430, 900-904).

The novel use of combination of older drugs is also under investigation: Lapdap^{®} (Chloroproguanil-dapsone) or Lapdap^{®}-artesunate are based on combinations of chloroproguanil (CPG) and dapsone (DDS) and was launched in 2003 as an effective and cheap antimalarial (Mutabingwa T, et al., Lancet, 2001, 358, 1218-1223).

Another well known drug is the quinolines family. Work on this old drug eventually succeeded to introduce antiplasmodial compounds such as quinoline and isoquine derivatives (Madrid, P.B. et al., J. J. Med. Chem. 2007, 50, 889-896). Very recently astemizole, a known antihistaminic drug was shown to have a similar activity toward plasmodium than artemisinin and other derivatives (Chong, C.R., et al., Nat. Chem. Biol., 2006, 2, 415-416).

Current efforts focus therefore on chemotherapy using multiple therapies including artemisinin. However, the scientific community is worried that plans for the extensive use of artemisinin might be ruined by emergence of the parasitic resistance it will almost certainly trigger, sooner or later. Given the small number of available drugs and the resistance they have already induced, discovery of new targets and of new drugs remains a key priority.

In the case of toxoplasmosis, *Toxoplasma gondii* has a broad spectrum of hosts and can virtually infect any mammal. It is well established that the final hosts are cats where sexual reproduction occurs. Transmission to humans occurs most often by infected food, or by direct or indirect contacts with cats. Severity of toxoplasmosis is very variable. In healthy human adults, infection is usually not visible besides serum analyses. Most healthy people don't require any treatment. In infected patients, the parasite remains as cysts that can reactivate a toxoplasmosis in case of immune deficiencies. Treatments are critically needed in the case of pregnant women and babies, people with HIV/AIDS and patient treated for cancer by chemotherapies. Most widely used treatments include pyrimethamine and sulfadiazine

Because these drugs can have serious side effects for both women and babies, they are normally not used during pregnancy. Drug treatment can lessen the severity of congenital toxoplasmosis, but it will not undo any damage that has already occurred. Pyrimethamine is an antimalarial medication also used to treat toxoplasmosis, and acts as a folic acid antagonist. It may prevent the body from absorbing the important B vitamin folate (folic acid, vitamin B-9), especially when taking high doses over a long period of time. Other potential side effects of pyrimethamine include bone marrow suppression and liver toxicity. Sulfadiazine is an antibiotic used in combination with pyrimethamine to treat toxoplasmosis. Possible side effects include nausea, vomiting and diarrhea. Pyrimethamine can also be supplied in conjunction with clindamycin, an antibiotic that can sometimes cause severe diarrhea. Patients suffering from AIDS need to take these medications for life, although in some cases, toxoplasmosis therapies can be stopped if CD4 count remains very high for at least three to six months. Side effects of most drugs can be more severe in people with HIV/AIDS. Toxoplasmosis is also an important veterinary infection in ovines, bovines, caprines and pigs, and is responsible for spontaneous abortion. Efficient drugs with low side effects, acting on cyst forms and usable both for human therapies and preventions in cattle farms are therefore highly needed.

The search for effective therapeutics for toxoplasmosis received much less interest and only a few examples are reported. The main target is dihydrofolate reductase (DHFR) and some inhibitors with high ligand efficiency were reported; for example starting from a QSAR analysis some authors obtained the synthesis of new 6-fluoroquinolones as potential active against *T. gondii* (Anquetin G., et al., Eur. J. Med. Chem., 2006, 41, 1478-1493.

Very recently others reported the SAR studies based on trimethoprim and methotrexate analogues. Starting from this 1960-drug they obtained high *in vitro* inhibitory activities toward a homology model of DHFR.

In the case of babesiosis, infection is similar to malaria. Transmission of *Babesia* parasites is due to bites by ticks. Wild animals (rodents and bovids) are final or intermediary parasitic reservoirs. Some *Babesia,* such as B. *microti* and *B. divergens* can infect humans. Babesiosis is reported in various domestic species, including bovids, equids, and dogs with a large spectrum of symptom, from non-severe cases to lethality. Untreated canine babesiosis is always lethal. Besides a partially efficient vaccine, no drug treatment is currently available to fight against all *Babesia* species. Human treatments include the use of quinine combined with clindamycin, or hydroxychloroquine. For veterinary purposes, bovine and equine babesiosis caused by only two species can be mainly treated, using imidocarb dipropionate.

In the case of neosporosis, data are fragmentary and poor. *Neospora* is a parasite closely related to *Toxoplasma,* capable of infecting virtually any mammal. Dog is the final host were sexual reproduction occurs. Cattles can be infected due to proximity with infected wild animals. Drugs used to treat toxoplasmosis are used to treat neosporosis.

In the case of coccidiosis, tens of species of the *Eimeria* genus can cause infections of the gastro-intestinal system of birds and mammals. Coccidiosis is a very frequent plague in bovids, ovids, birds, rodents, etc. Massive poultry breeding requires prophylaxis against avian coccidiosis, particularly against *Eimeria necatrix* or *E. tenella* that cause severe infections. Drug treatments include the prescription of antibiotics or non-antibiotic molecules such as decoquinate, a molecule of the hydroxyquinoline family.

New molecules are therefore needed for some specific infections such as malaria or numerous babesioses, or to treat some parasitic stages that are currently resistant to drugs, such as toxoplasmic cysts. Molecules having an effect on numerous apicomplexan species would be benefic for both medical and veterinary purposes.

The inventors have developed the subject of the invention in order to remedy these problems.

A first subject of the Invention is therefore compounds of general formula (I) below: 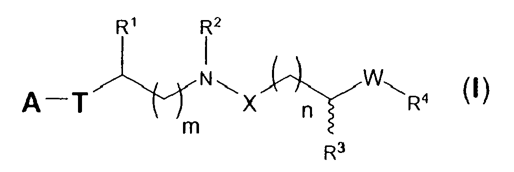 or a pharmaceutically acceptable salt thereof, wherein:
■ T is selected from the group consisting of -CH₂-, -CH₂CH₂-, -O-, -N-, a simple bond and a double bond;
■ m and n, independently, are an integer equal to 0, 1, 2, 3 or 4;
■ R¹ and R² simultaneously represent an hydrogen atom or R¹ and R² form together an alkyl chain having from 2 or 3 carbon atoms with possibly one insaturation;
■ R³ represents hydrogen or a group selected among alkyl, alkenyl, cycloalkyl, aryl, arylalkyl, heteroalkyl, heteroaryl and heteroarylalkyl groups wherein said groups defined for R³ are optionnaly substituted with one or more groups independently selected from halogen, trifluoromethyl, difluoromethyl, azido, alkyl, alkoxy, cyano; nitro and carbethoxy;
■ R⁴ represents hydrogen or a group selected among alkyl, alkenyl, cycloalkyl, aryl, arylalkyl, heteroaryl, heteroarylalkyl and heteroalkyl, wherein said groups defined for R⁴ are optionnaly substituted with one or more groups independently selected from halogen, trifluoromethyl, difluoromethyl, azido, alkyl, alkoxy, cyano, nitro, carboxy and carbethoxy;
■ X represents a single bond, -CH₂- or a functional group chosen among the groups of formulas (X-1) to (X-6) below: 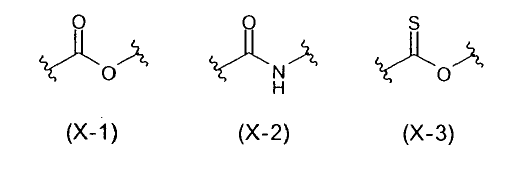 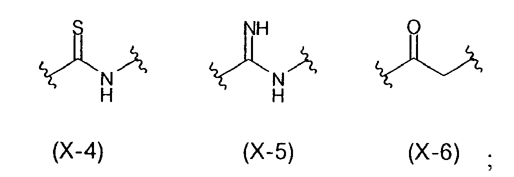
■ A represents a cyclic moiety selected from the group consisting of:
   i) a moiety of formula (A-1) below: 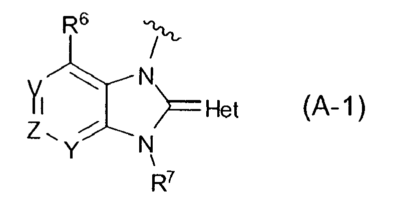 in which:
      - V, Y and Z, identical or different, represent -CH-, C-R⁶ or N, it being understood that V can also form, together with R⁶ or Z, a fused aryl or heteroaryl ring depending of the nature of R⁶ or Z;
      - Het represents an oxygen atom or a sulphur atom;
      - R⁶ represents hydrogen, halogen, a group selected from the group consisting in alkoxy, alkyl, alkenyl, alkynyl, cycloalkyl, aryl, arylalkyl, heteroaryl and heteroalkyl, wherein said alkoxy, alkyl, alkenyl, alkynyl cycloalkyl, aryl, arylalkyl, heteroaryl and heteroalkyl groups are optionnaly substituted with one or more groups independently selected from halogen, trifluoromethyl, difluoromethyl, azido, alkyl, alkoxy, cyano and nitro;
      - R⁷ represents a hydrogen atom, a linear or branched alkyl or alkenyl group; a heteroaryl group, a cycloalkyl group, an aryl group and an arylalkyl group, wherein said groups are optionnaly substituted with one or more groups independently selected from halogen, trifluoromethyl, difluoromethyl, azido, alkyl, alkoxy, cyano, nitro; a SO₂R⁸, a COR⁸ group or a COOR⁸ group wherein R⁸ represents a group chosen among alkyl, alkenyl, cycloalkyl, aryl, heteroaryl, arylalkyl and heteroalkyl, wherein said groups mentioned for R⁸ are optionnaly substituted with one or more groups independently selected from halogen, trifluoromethyl, difluoromethyl, azido, alkyl, alkoxy, cyano, nitro, carboxy and carbethoxy; a group of formula-alkylC(O)R⁹ in which R⁹ represents hydrogen or an alkoxy group; a group of formula alkyl-NHR¹⁰ in which R¹⁰ represents an alkoxycarbonyl group or a biotine moiety; R⁷ may also form, together with Y when Y is a carbon atom, a saturated heterocarbonated ring comprising 3 or 4 carbon atoms in addition to the carbon atom of Y, said ring being optionally substituted with an oxo functional group;
   ii) a moiety of formula (A-2) below: 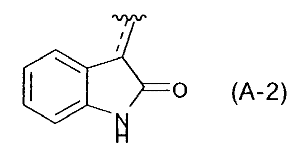 in which the dashed line represents an optional double bond;
   iii) a moiety of formula (A-3) below: 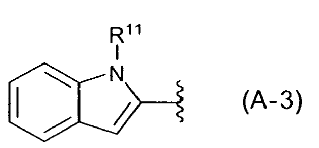 in which R¹¹ represents an ethylformate group or an alkylaryl group;
   iv) a moiety of formula (A-4) below: 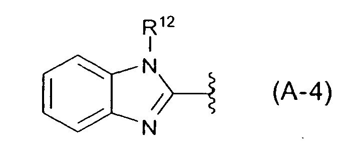 in which R¹² represents a hydrogen atom, an alkyl radical or a group of formula -NC(O)-OCH₂-CH(CH₃)-N-(Bz)₂ in which Bz stands for benzyl;
   v) a moiety of formula (A-5) below: 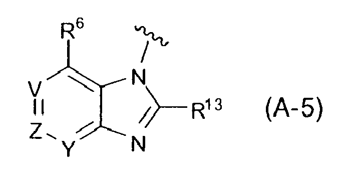 in which:
      - V, Y, Z and R⁶ are as defined for the moeity of formula (A-1) above;
      - R¹³ represents a hydrogen atom or a group chosen among alkoxy, alkyl, alkenyl, aryl, arylalkyl, heteroaryl and heteroalkyl groups, wherein said groups defined for R¹³ are optionnaly substituted with one or more groups independently selected from halogen, trifluoromethyl, difluoromethyl, azido, alkyl, alkoxy, cyano and nitro;
   vi) a moiety of formula (A-6) below: 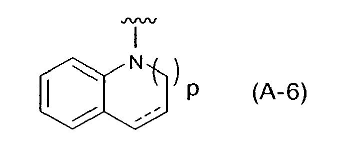 in which:
      - p is an integer equal to 0, 1 or 2; and
      - the dashed line represents an optional double bond;
   vii) a moiety of formulas (A-7) or (A-7') below: 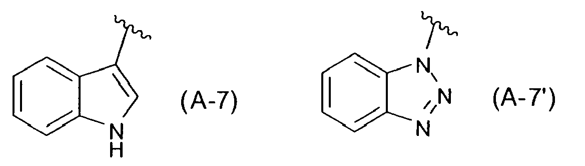
   viii) a moiety of formula (A-8) below: 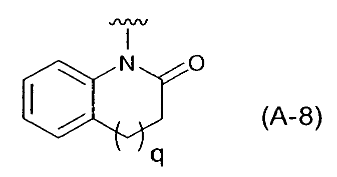 in which q is an integer equal to 0, 1 or 2;
   ix) a moiety of formula (A-9) below: 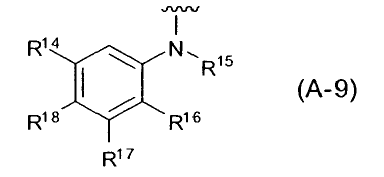 in which:
      - R¹⁴, R¹⁶, R¹⁷ and R¹⁸, which may be identical or different, represent a hydrogen atom, a halogen atom, or a group selected among alkyl, nitro, alkoxy, aminoalkyl, aryl and heteroaryl groups;
      - R¹⁵ represents a hydrogen atom, an alkyl group, an alkenyl group, an aryl group, a heteroaryl group or a group selected from the groups having the following formulas -COOR¹⁹, -C(O)NR¹⁹R²⁰, -C(S)NR¹⁹R²⁰, -C(O)NHR¹⁹, -C(O)R¹⁹,
      - SO₂R¹⁹ in which R¹⁹ and R²⁰, which may be identical or different, represent a hydrogen atom, an alkoxy group, an amino group, an alkyl group, an alkenyl group, an aryl group and a heteroaryl group; and
   x) a moiety of formula (A-10) or (A-11) below: 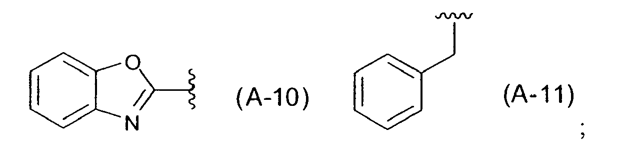
■ W represents C-R²¹, N-R²¹ or O-R²¹ wherein R²¹ represents a group selected among alkyl, alkenyl, cycloalkyl, aryl, arylalkyl heteroaryl and heteroalkyl, wherein said groups defined for R²¹ are optionnaly substituted with one or more groups independently selected from halogen, trifluoromethyl, difluoromethyl, azido, alkyl, alkoxy, cyano, nitro, carboxy and carbethoxy; W may also represents a moiety having the formula W-1 below: 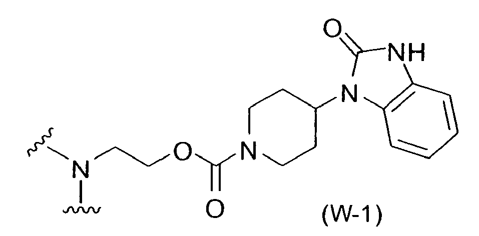 or a N-containing ring selected among moieties having the formulas W-2, W-3 and W-4 below:

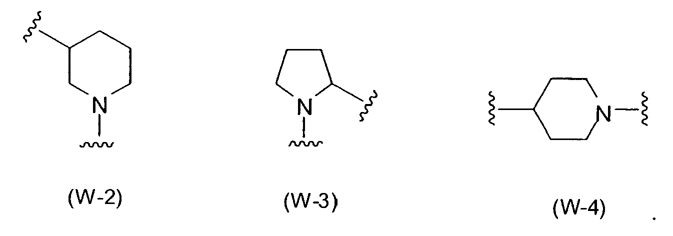

According to a preferred embodiment of the present Invention, R¹ and R², together with the carbon atom (for R¹), the nitrogen atom (for R²) and the chain bonding the carbon atom bearing R¹ and the nitrogen atom bearing R² (i.e.: -(CH₂)ₘ-) form a moiety selected from the group consisting of the following moieties: 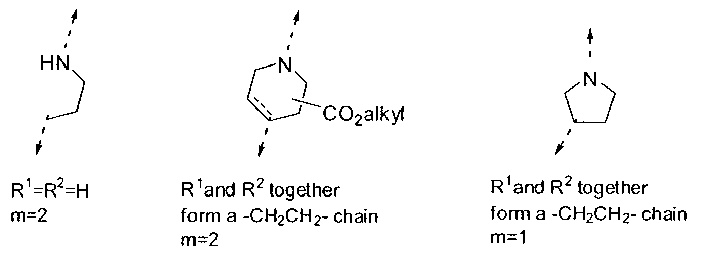 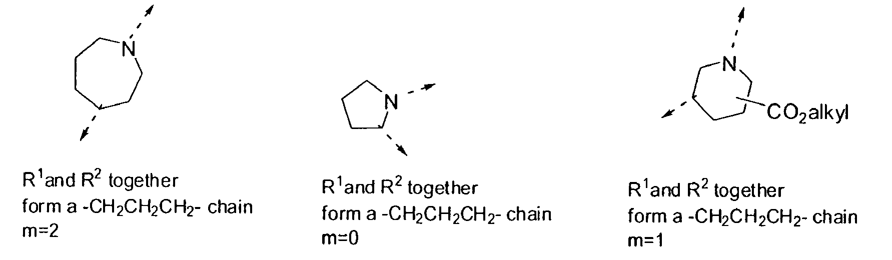 wherein the dashed arrows represent the attachment point of these moieties to T and X respectively *via* a covalent bond.

In the sense of the present Invention, linear and branched alkyl groups (main or auxiliary) mentioned in the present specification are chosen among (C₁-C₄)alkyl groups such as methyl, ethyl, n-propyl, isopropyl, n-butyl, *tert*-butyl and isobutyl radicals; linear and branched alkoxy groups (main or auxiliary) mentioned in the present specification are chosen among (C₁-C₄)alkoxy groups such as methyloxy, ethyloxy, n-propyloxy, iso-propyloxy, n-butyloxy, *tert*-butyloxy and isobutyloxy radicals.

Also in the sense of the present Invention, halogen atoms are chosen among bromine, chlorine, fluorine and iodine.

According to the Invention, aryl and heteroaryl groups refer to any functional group or substituent derived from at least one simple aromatic ring; an aromatic ring corresponding to any planar cyclic compound having a delocalized π system in which each atom of the ring comprises a p-orbital, said p-orbitals overlapping themselves. Among heteroaryl groups, one can mention furan, pyridine, pyrrole, thiophene, imidazole, pyrazole, oxazole, isoxazole, thiazole, benzene, pyridine, pyrazine, pyrimidine, pyridazine, benzylcyclobutene, pentalene, benzofurane, isobenzofurane, indole, isoindole, benzothiophene, benzo[c]thiophene, benzimidazole, indazole, benzoxazole, benzisoxazole, benzothiazole, naphthalene, quinoline, isoquinoline, quinoxaline, quinazoline, cinnoline, purine, anthracene and acridine.

A large number of the compounds of formula (I) as above-defined possess an asymmetric carbon. In this case, they can have the (R) or the (S) absolute configuration. The present Invention encompasses both configurations and mixtures thereof, in particular racemate mixtures.

As particular compounds of formula (I) above, one can mention:
- (S)-2-(dibenzylamino)propyl 4-(1,2-dihydro-2-oxobenzo[d]imidazol-3-yl)piperidine-1-carboxylate;
- (S)-2-(dibenzylamino)-3-phenylpropyl 4-(1,2-dihydro-2-oxobenzo[d]imidazol-3-yl)piperidine-1-carboxylate;
- 2-(dibenzylamino)ethyl 4-(1,2-dihydro-2-oxobenzo[d]imidazol-3-yl)piperidine-1-carboxylate;
- 3-(dibenzylamino)propyl 4-(1,2-dihydro-2-oxobenzo[d]imidazol-3-yl)piperidine-1-carboxylate;
- 2-(N-benzyl-N-methylamino)ethyl 4-(1,2-dihydro-2-oxobenzo[d]imidazol-3-yl)piperidine-1-carboxylate;
- (S)-2-(dibenzylamino)propyl 5,6-dihydro-4-(1,2-dihydro-2-oxobenzo[d]imidazol-3-yl)pyridine-1 (2H)-carboxylate;
- (S)-2-(dibenzylamino)propyl 4-benzylpiperidine-1-carboxylate;
- 2-(benzyloxy)ethyl 4-(1,2-dihydro-2-oxobenzo[d]imidazol-3-yl)piperidine-1-carboxylate;
- (S)-2-(dibenzylamino)propyl 4-(1,2-dlhydro-2-thioxobenzo[d]imidazol-3-yl)piperidine-1-carboxylate;
- Ethyl 2-(1-(((S)-2-(dibenzylamino)propoxy)carbonyl)piperidin-4-yloxy)-1H-indole-1-carboxylate;
- (S)-2-(dibenzylamino)propyl 4-(2-oxobenzo[d]oxazol-3(2H)-yl)piperidine-1-carboxylate;
- (S)-2-(dibenzylamino)propyl 4-(1H-benzo[d]imidazol-1-yl)piperidine-1-carboxylate;
- (S)-2-(dibenzylamino)propyl 4-(1-methyl-1H-benzo[d]imidazol-2-yloxy)piperidine-1-carboxylate;
- (S)-2-(dibenzylamino)propyl 3-(1,2-dihydro-2-oxobenzo[d]imidazol-3-yl) pyrrolidine-1-carboxylate;
- *tert*-butyl 3-(1-(((S)-2-(dibenzylamino)propoxy)carbonyl) piperidin-4-yl)-2,3-dihydro-2-oxobenzo[d]imidazole-1-carboxylate;
- 1-(1-(3-(dibenzylamino)propyl)piperidin-4-yl)-1H-benzo[d]-imidazol-2(3H)-one;
- (S)-2-(dibenzylamino)propyl 4-(1,2-dihydro-1-methyl-2-oxobenzo-[d]imidazol-3-yl)piperidine-1-carboxylate;
- 1-(1-(2-(dibenzylamino)ethyl)piperidin-4-yl)-1H-benzo[d]imidazol-2(3H)-one;
- (S)-2-(dibenzylamino)propyl 4-(1H-benzo[d][1,2,3]triazol-1-yl)piperidine-1-carboxylate;
- ]-(1-((S)-2-(dibenzylamino)-3-phenylpropyl)piperodin-4-yl)-1H-benzo[d]imidazol-2(3H)-one;
- 1-(1-((S)-2-(dibenzylamino)propyl)plperidin-4-yl)-1H-benzo[d]imidazol-2(3H)-one;
- O-2-(dibenzylamino)ethyl 4-(1,2-dihydro-2-oxobenzo[d]imidazol-3-yl)piperidine-1-carbothioate;
- (S)-2-(dibenzylamino)propyl 4-(2-oxolndolin-3-ylidene)piperidine-1-carboxylate;
- O-2-(benzyloxy)ethyl 4-(1,2-dihydro-2-oxobenzo[d]imidazol-3-yl)piperidine-1-carbothioate;
- O-(S)-2-(dibenzylamino)propyl 4-(1,2-dihydro-2-oxobenzo[d]imidazol-3-yl)piperidine-1-carbothioate;
- (S)-2-(dibenzylamino)propyl 4-((1,2-dihydro-2-oxobenzo[d]imidazol-3-yl)methyl)piperidine-1-carboxylate;
- (S)-2-(dibenzylamino)propyl 4-(2-(trifluoromethyl)-1H-benzo[d]imidazol-1-yl)piperidine-1-carboxylate;
- (S)-2-(dibenzylamino)propyl 3-(1,2-dihydro-2-oxobenzo[d]imidazol-3-yl)propylcarbamate;
- (S)-2-(dibenzylamino)propyl 4-(2,3-dihydro-2-oxoimidazo[4,5-b]pyridin-1-yl)piperidine-1-carboxylate;
- 1-benzylpiperidin-3-yl 4-(1,2-dihydro-2-oxobenzo[d]imidazol-3-yl)piperidine-1-carboxylate;
- (S)-2-(dibenzylamino)propyl 3-(1,2-dihydro-2-oxobenzo[d]imidazol-3-yl)piperidine-1-carboxylate;
- (S)-2-(dibenzylamino)propyl 4-(2-oxoindolin-3-yl)piperidine-1-carboxylate;
- (S)-2-(dibenzylamino)propyl 4-(1-benzyl-1,2-dihydro-2-oxobenzo[d]imidazol-3-yl)piperidine-1-carboxylate;
- (S)-2-(dibenzylamino)propyl 4-(1-(cyclohexylmethyl)-1,2-dihydro-2-oxobenzo[d]imidazol-3-yl)piperidine-1-carboxylate;
- (S)-2-(dibenzylamino)propyl 4-(2-oxo-3-methylsulfonyl-2,3-dihydro-1H-benzo[d]imidazol-1-yl)piperidine-1-carboxylate;
- (S)-2-(dibenzylamino)propyl 4-(1H-indol-1-yl)piperidine-1-carboxylate;
- (S)-2-(dibenzylamino)propyl 4-(2-methyl-1H-benzo[d]imidazot-1-yl)piperidine-1-carboxylate;
- 1-(S)-2-(dibenzylamino)propyl 3-ethyl 4-(1,2-dihydro-2-oxobenzo[d]imidazol-3-yl)piperidine-1,3-dicarboxylate;
- (S)-2-(dibenzylamino)propyl 4-(2-oxo-3-phenylsulfonyl-2,3-dihydro-1H-benzo[d]imidazol-1-yl)piperidine-1-carboxylate;
- (S)-2-(dibenzylamino)propyl 4-(1,2-dihydro-2-oxo-1-(benzoyl)benzo[d]imidazol-3-yl)piperidine-1-carboxylate;
- (S)-2-(dibenzylamino)propyl 4-(indolin-1-yl)piperidine-1-carboxylate;
- (S)-2-(dibenzylamino)propyl 4-(6-chloro-1,2-dihydro-2-oxobenzo[d]imidazol-3-yl)plperldine-1-carboxylate;
- (S)-2-(dibenzylamino)propyl 4-(3,4-dihydroquinolin-1(2H)-yl)piperidine-1-carboxylate;
- (S)-2-(dibenzylamino)propyl 4-(1,2-dihydro-2-oxobenzo[d]imidazol-3-yl)azepane-1-carboxylate;
- 2-(diethylamino)ethyl 4-(1,2-dihydro-2-oxobenzo[d]imidazol-3-yl)piperidine-1-carboxylate;
- (S)-2-(dibenzylamino)propyl 4-(1,2-dihydro-2-oxo-1-(pivaloyl)benzo[d]imidazol-3-yl)piperidine-1-carboxylate;
- 2-(diisopropylamino)ethyl 4-(1,2-dihydro-2-oxobenzo[d]imidazol-3-yl)piperidine-1-carboxylate;
- 2-(1-benzylpiperidin-4-yl)ethyl 4-(1,2-dihydro-2-oxobenzo[d]imidazol-3-yl)piperidine-1-carboxylate;
- 2-(ethyl(phenyl)amino)ethyl 4-(2-oxo-2,3-dihydro-1H-benzo[d]imidazol-1-yl)piperidine-1-carboxylate;
- ((S)-1-benzylpyrrolidin-2-yl)methyl 4-(1,2-dihydro-2-oxobenzo[d]imidazol-3-yl)piperidine-1-carboxylate;
- 2-(dimethylamino)ethyl 4-(1,2-dihydro-2-oxobenzo[d]imidazol-3-yl)piperidine-1-carboxylate;
- 3,3-diphenylpropyl 4-(1,2-dihydro-2-oxobenzo[d]imidazol-3-yl)piperidine-1-carboxylate;
- (S)-2-(dibenzylamino)propyl 2-((1,2-dihydro-2-oxobenzo[d]imidazol-3-yl)methyl)pyrrolidine-1-carboxylate;
- (S)-2-(dibenzylamino)propyl 4-(2-(1,2-dihydro-2-oxobenzo[d]imidazol-3-yl)ethyl)piperidine-1-carboxylate;
- (S)-2-(dibenzylamino)propyl 4-(1,2-dihydro-2-oxonaphtho[2,3-d]imidazol-3-yl)piperidine-1-carboxylate;
- (S)-2-(dibenzylamino)propyl 4-(1H-indol-3-yl)piperidine-1-carboxylate;
- (S)-2-(dibenzylamino)propyl 4-(N-phenylpropionamido)piperidine-1-carboxylate;
- (S)-2-(dibenzylamino)propyl 4-(1,2-dihydro-2-oxo-1-phenylbenzo[d]imidazol-3-yl)piperidine-1-carboxylate;
- 1-(1-(4-(dibenzylamino)butyl)piperidin-4-yl)-1H-benzo[d]imidazol-2(3H)-one;
- (S)-2-(dibenzylamino)-3-phenylpropyl 4-(1,2-dihydro-1-methyl-2-oxobenzo[d]imidazol-3-yl)piperidine-1-carboxylate;
- (S)-2-(dibenzylamino)-3-phenylpropyl 4-(1,2-dihydro-3-phenylsulfonyl-2-oxobenzo[d]imidazol-1-yl)piperidine-1-carboxylate;
- N-((S)-2-(dibenzylamino)propyl)-4-(1,2-dihydro-2-oxobenzo[d]imidazol-3-yl)piperidine-1-carboxamide;
- N-((S)-2-(dibenzylamino)propyl)-4-( 1,2-dihydro-2-oxobenzo[d]imidazol-3-yl)piperidine-1-carbothioamide;
- (S)-2-(dibenzylamino)propyl 4-(2-oxoindolin-1-yl)piperidine-1-carboxylate;
- (S)-2-(dibenzylamino)propyl 4-(3-(4-ethoxy-4-oxobutyl)-2-oxo-2,3-dihydro-1H-benzo[d]imidazo)-1-yl)pipendine-1-carboxylate;
- (S)-4-(3-(1-((2-(dibenzylamino)propoxy)carbonyl)piperidin-4-yl)-2-oxo-2,3-dihydro-1H-benzo[d]imidazol-1-yl)butanoic acid;
- 2,2-diphenylethyl 4-(1,2-dihydro-2-oxobenzo[d]imidazol-3-yl)piperidine-1-carboxylate;
- (S)-2-(dibenzylamino)propyl 4-(2-oxo-3-(4-propylphenylsulfonyl)-2,3-dihydro-1H-benzo[d]imidazol-1-yl)piperidine-1-carboxylate;
- (S)-2-(dibenzylamino)propyl 4-(3-(4-fluorophenylsulfonyl)-2-oxo-2,3-dihydro-1H-benzo[d]imidazol-yl)piperidine-1-carboxytate;
- (S)-2-(dibenzylamino)propyl 4-(3-(4-methoxyphenylsulfonyl)-2-oxo-2,3-dihydro-1H-benzo[d]imidazol-1-yl)piperidine-1-carboxylate;
- (S)-2-(dibenzylamino)propyl 4-(2-oxo-3-(4-(trifluoromethyl)phenylsulfonyl)-2,3-dihydro- 1H-benzo[d]imidazol-1-yl)piperidine-1-carboxylate;
- (S)-2-(dibenzylamino)propyl 4-(2-oxo-3-(thiophen-2-ylsulfonyl)-2,3-dihydro-1H-benzo[d]imidazol-1-yl)piperidine-1-carboxylate;
- (S)-2-(dibenzylamino)propyl 4-(2-oxo-3-(quinolin-8-ylsulfonyl)-2,3-dihydro-1H-benzo[d]imidazol-1-yl)piperidine-1-carboxylate;
- (S)-2-(dibenzylamino)propyl 4-(3-(naphthalen-1-ylsulfonyl)-2-oxo-2,3-dihydro-1H-benzo[d]imidazol-1-yl)piperidine-1-carboxylate;
- (S)-2-(dibenzylamino)propyl 2-(1-(((S)-2-(dibenzylamino)propoxy)carbonyl)piperidin-4-ylamino)-1H-benzo[d]imidazole-1-carboxylate;
- (S)-2-(dibenzylamino)propyl 4-(1H-benzo[d]imidazol-2-ylamino)piperidine-1-carboxylate;
- (S)-2-(dibenzylamino)propyl 4-(phenylamino)piperidine-1-carboxylate;
- (S)-2-(dibenzylamino)propyl 4-(4-methoxyphenylamino) piperidine-1-carboxylate;
- (S)-2-(dibenzylamino)propyl 4-(2,4-dimethoxyphenylamino) piperidine-1-carboxylate;
- (S)-2-(dibenzylamino)propyl 4-(3,4,5-trimethoxyphenylamino) piperidine-1-carboxylate;
- (S)-2-(dibenzylamino)propyl 4-(4-iodophenylamino)piperidine-1-carboxylate;
- 2-(N-(4-nitrobenzyl)-N-benzylamino)ethyl 4-(1,2-dihydro-2-oxobenzo[d]imidazol-3-yl)piperidine-1-carboxylate;
- (S)-2-(dibenzylamino)propyl 4-(2-oxo-3-(4-acetamidyl-phenylsulfonyl)-2,3-dihydro-1H-benzo[d]imidazol-1-yl)piperidine-1-carboxylate;
- (S)-2-(dibenzylamino)-3-phenylpropyl 4-(1-benzyl-1,2-dihydro-2-oxobenzo[d]imidazol-3-yl)piperidine-1-carboxylate;
- N-((S)-2-(dibenzylamino)propyl)-4-(1,2-dihydro-2-oxobenzo[d]imidazol-3-yl)piperidine-1-carboxamidine;
- (S)-2-(dibenzylamino)propyl 4-(1,3-diphenylureido)piperidine-1-carboxylate;
- (S)-2-(dibenzylamino)propyl 4-(N-phenylbenzamido)plperldine-1-carboxylate;
- (S)-2-(dibenzylamino)propyl 4-(N-phenylphenylsulfonamido) piperidine-1-carboxylate;
- (S)-2-(dibenzylamino)propyl 4-(3-fluorophenylamino)piperidine-1-carboxylate;
- (S)-2-(dibenzylamino)propyl 4-(3,4-dichlorophenylamino) piperidine-1-carboxylate;
- (S)-2-(dibenzylamino)propyl 4-(2-aminophenylamino)piperidine-1-carboxylate;
- (S)-2-(dibenzylamino)propyl 4-(1-acetyl-1,2-dihydro-2-oxobenzo[d]lmidazol-3-yl)piperidine-1-carboxylate;
- (S)(1-((2-(dibenzylamino)propoxy)carbonyl)piperidin-4-yl)-5,6-dihydro-imidazo[4,5, 1,j,k][ 1 ]benzazepine-2,7(1H,4H)-dione;
- 2-benzylamino bis [ethyl 4-(1,2-dihydro-2-oxobenzo[d]imidazol-3-yl)plpefidine-1-carboxylate;
- (S)-2-(dibenzylamino)propyl 4-(3-(2-tert-butyloxycarbonylaminoethyl)-2-oxo-2,3-dihydro-1H-benzo[d]imidazol-1-yl)piperidine-1-carboxylate;
- (S)-2-(dibenzylamino)-3-phenylpropyl 4-(3-(2-tert-butyloxy carbonylaminoethyl)-2-oxo-2,3-dihydro-1H-benzo[d]imidazol-1-yl)piperidine-1-carboxylate;
- (S)-2-(dibenzylamino)-3-phenylpropyl 4-(3-(2 aminoethyl)-2-oxo-2,3-dihydro-1H-benzo[d]imidazol-1-yl)pipehdine-1-carboxytate D-biotin amid;
- (S)-2-(dibenzylamino)propyl 3-(1,2-dihydro-2-oxobenzo[d]imidazol-3-yl)pyrrolidine-1-carboxylate;
- 1-[ 1-(4-dibenzylamino-butyryl)-piperidin-4-yl]-1,3-dihydro-benzolmidazol-2-one; and pharmaceutically acceptable salts thereof.

The pharmaceutically acceptable salts of the compounds of formula (I) can be acid addition salts formed with pharmaceutically acceptable acids. Such a definition encompasses hydrochloride, hydrobromide, sulphate or bisulphate, phosphate or hydrogenophosphate, acetate, benzoate, succinate, fumarate, maleate, lactate, citrate, tartrate, gluconate, methanesulphonate, benzene-sulphonate, paratoluene-sulphonate salts of compounds of formula (I).

Two different protocols can be employed for the preparation of all compounds of the above defined formula (I):
- a classical solution phase synthesis and
- a solid phase synthesis on solid support.

Firstly, in the case of classical solution phase synthesis, all compounds of formula (I) according to the present Invention can be prepared using state of the art methodologies which are briefly described here. Final compounds belong to general structure I which was decomposed in 3 substructures A, B, and C with a link ("X") between the substructures B and C as depicted in the following formula: 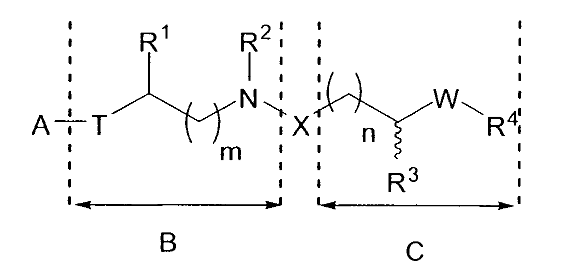 in which A, T, R¹, R², R³, R⁴, m, n, X and W can have the same significations than in formula (I) above.

Several strategies can be employed which all include a link procedure. The linkage takes place using either synthetic A-B or some commercially available A-B as building blocks but also B blocks for a later introduction of the A aryl/heteroaryl moiety. Then, scheme 1 bellow illustrates the linking procedures between AB and C or between B [(II-a) or (II-b)] and C: 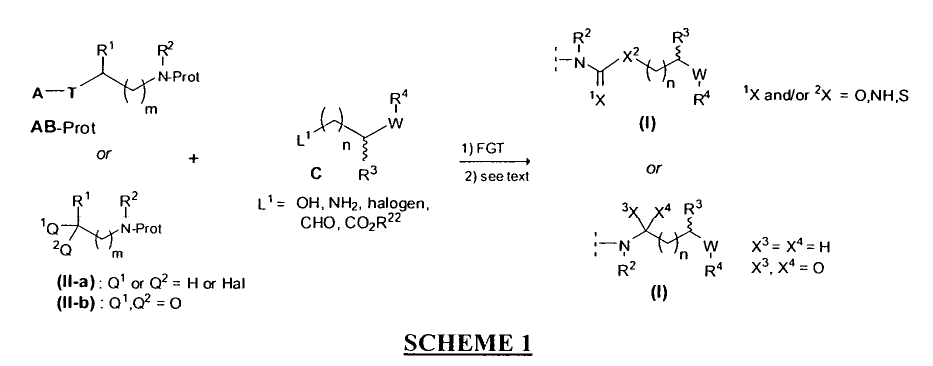 in which A, T, R¹, R², R³, R⁴, m, n, W can have the same significations than in formula (I) as defined above; Prot represents a classical protective group, L¹ represents OH, NH₂, halogen, CHO or COOR²² whereas R²² represents hydrogen, halogen or alkyl, X¹ and X², which may be identical or different, represent O, NH or S and X³ and X⁴, which are identical, represent H or O. Protecting groups are well known from the one skilled in the art and are described for example in *"*Protective groups in organic synthesis", T.W. GREENE et al., 2nd edition, Wiley Interscience, 1991. As examples of these protecting groups, one can mention benzyl; benzyloxycarbonyle (Z); trifluoroacetyle (TFA); *tert*-butyloxycarbonyle (Boc); trimethylsilylethoxycarbonyle (Teoc) and fluorenylmethyloxycarbonyle (Fmoc) groups. These protecting groups can be removed *via* hydrogenation or acidic treatment (for example with trifluoroacetic acid: TFA) using the standard corresponding protocols.

Intermediary compounds of formula C in which L¹ = NH₂ are either commercially available or prepared from the corresponding alcohol using phtalimide followed by hydrazine deprotection as described for example in the article by Berger, Y., et al., J. Med. Chem., 2005, 48, 483-498. In the case where compounds of formula C are non commercial aminoalcohols (L¹=OH, W = N-R²¹), they can be prepared by amine alkylation using the convenient halide in the presence of potassium carbonate as described for example by Le Bihan, G. et al., J. Med. Chem., 1999, 42, 1587-1603, or functional group transformation (FGT) starting from commercially available aminoacids. FGT also means for protection/deprotection sequences.

For alkyl chain length (n) variations, compounds can be obtained after addition of dialkylamines on different cyclic anhydrides followed by standard hydride reduction or alkylations using the convenient halogeno alcohols. Aldehydes can be obtained by diisobutylaluminium hydride reduction of the corresponding methylesters. Acids and derivatives may be obtained after esters hydrolysis using hydrogen chloride in methanol for hydrolysis. The esters precursors are prepared by nucleophilic displacement with a corresponding reactive H-WR⁴. When L¹ = halogen, compounds can be prepared from the corresponding alcohols by halogenation for example using triphenylphosphin with carbon tetrabromide according to the methods classically used by the one skilled in the art.

The link procedure between A-B and C or between intermediate of formula (II) and C depends on the nature of L¹. When L¹ = OH or NH₂, carbamates or ureas can be classically prepared using treatment of alcohol or amines with carbonyldiimidazol (CDI) as described for example by Matsuno, K. et al., J. Med. Chem., 2002, 45, 3057-3066, followed by nucleophilic displacement with the corresponding amines. For thiocarbamates preparation, sodium salt of the corresponding alcohols (L¹ = OH) are first treated with carbondisulfide in hot methanol followed by methylation with methyl iodide. The corresponding dithiocarbonates obtained are then added to the different (II-a)/(II-b) or A-B moieties. For thioureas preparation (L¹ = NH₂), amines are added to carbondisulfide in acetonitrile generating the corresponding thiols which are activated by 1-(3-dimethylaminopropyl)-3-ethylcarbodiimide hydrochloride (EDCI) before addition of the (II-a)/(II-b) or AB moieties in order to afford thioureas.

When L¹ = NH₂, guanidines can be obtained by successive addition of the two corresponding amines on diimidazolmethanimine prepared in a mixture tetrahydrofurane/dimethylformamide (THF/DMF) as described for example in the article by Wu, Y.-Q. et al., J. Org. Chem., 2002, 67, 7553-7556.

Other compounds can be obtained either by amines alkylation of the corresponding halides using potassium carbonate with catalytic amounts of potassium iodide in hot DMF or reductive amination of the corresponding aldehydes using sodium triacetoxy borohydride in dichloroethane (DCE).

Amides are obtained by nucleophilic addition of the (II-a)/(II-b) or A-B moieties on acyl chlorides using triethylamine (TEA) in DCM (dichloromethane). Acyl chlorides are conveniently prepared from the corresponding acids by thionyl chloride treatment.

The linking method between A and BC and A and B are represented on the following Schemes 2 and 3: 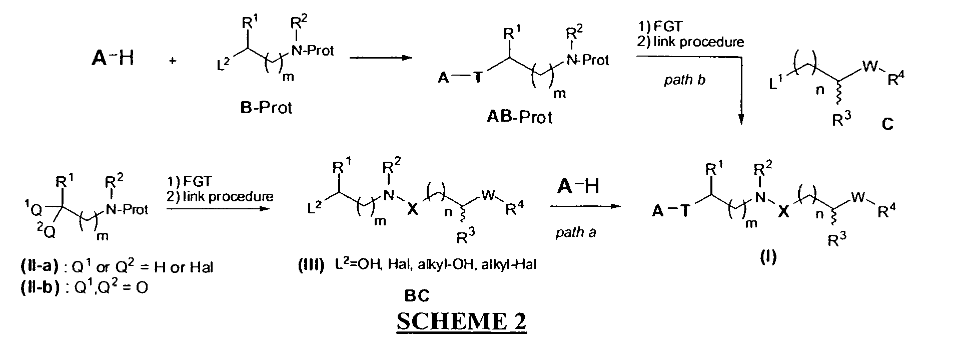 in which in which A, T, R¹, R², R³, R⁴, m, n, X and W have the same significations as in formula (I) as defined above; Prot represents a classical protective group as defined in scheme 1, L² represents OH, halogen, hydroxyalkyl or halogenoalkyl.

Different aryl/heteroaryl moieties A-H can be attached to the corresponding alcohols (B-C type, path a) using the method according to Mitsunobu O. (Synthesis, 1981, 1-28) which is a well established method for the preparation of amines from alcohols, or using standard alkylation procedures. In the case when part C is further connected (path b), protective groups, such a those mentioned before for scheme 1, are used. Deprotection and link procedures are employed as previously described affording final compounds.

In some cases alcohols can be obtained from the corresponding ketones using sodium borohydride reduction.

Connexions between A and BC or between A and B can also be performed by a reductive amination process as described on Scheme 3 below: 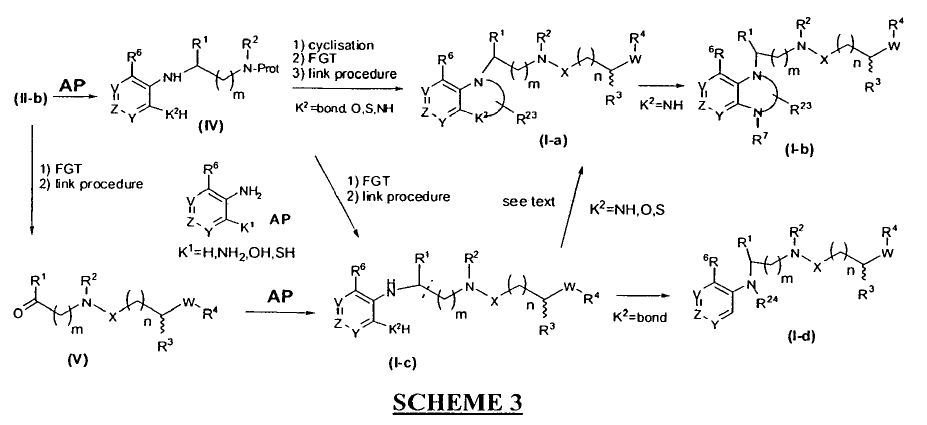 in which V, Z, Y, R¹, R², R³, R⁴, R⁶, R⁷, m, n, X, W can have the same significations than those recited for formula (I) and sub-formula (A-1) as defined above; Prot represents a classical protective group as defined in scheme 1, K¹ represents H, OH, SH or NH₂, K² represents a bond or O, S atoms or a NH group, R²³ represents C(=Het)-, C(=Het)CH₂-, C(=Het)C(=Het), C(=Het)CH₂CH₂-; C=C-R¹³-, Het and R¹³ having the same signification as previously mentioned for (I) and subformula (A-5).

According to this scheme, final compounds can be obtained upon two interconnected pathways starting from ketone of formula (II-b):
- by connecting the A ring precursor (AP) to (II-b) by reductive amination, leading to compound (IV) which undergoes cyclisation and link procedure; or
- by performing first the link procedure leading to compound of formula (V) following by (AP) connection and further transformations.

In the case of aniline derivatives, the secondary amines obtained (I-c) are converted into compounds (I-d) where R²⁴ represents a hydrogen atom, an alkyl group, an alkenyl group, an aryl group, a heteroaryl group or a group selected from the groups of the following formulas -COOR²⁵, -C(O)NHR²⁵, -C(O)R²⁵, -C(S)R²⁵, -SO₂R²⁵ in which R²⁵ represents a hydrogen atom, an alkyl group, an alkenyl group, an aryl or heteroaryl groups; wherein said aryl, heteroaryl are optionnaly substituted with one or more groups independently selected from halogen, trifluoromethyl, difluoromethyl, azido, alkyl, alkoxy, cyano, nitro and carboalkoxy.

The conversion can be performed using different electrophiles such as isocyanates, acyls chlorides, sulfonyl chlorides in the presence of triethylamine in dichloromethane (DCM).

In the case of compounds of formula (I-c) and (IV), the corresponding mono alkylated compounds are cycled using carbonyldimidazol (CDI) or others reagents such as glyoxal, glyoxylates, pyruvates, halogenoalkyl acyl halides or carboxylic acids in order to obtain the fused new heterocyclic structures bearing R²³ as defined before.

In the case of some dihydro aromatic heterocyclic moieties, oxidation in order to get the full aromatic structure can be performed using 2,3-dichloro-5,6-dicyano-1,4-benzoquinone (DDQ) as for example described in the article by Zhang, X. et al., Bioorg. Med. Chem. Lett., 2004, 14, 6011-6016.

The precedent methodology is also used with different cyclic aminoketones (V) already linked to part C as previously described. In some cases, when a carbonyl group adjacent to the keto function (CO₂alkyl) is present, supported cyanoborohydride can be used with acetic acid in methanol instead of the sodium triacetoxyborydride procedure as described for example in the article by Ley, S.V et al., J. Chem. Soc., Perkin Trans 1, 1998, 1, 2239-2241. In some cases when bifunctional reagents are used for alkylation *(vide infra*) of molecules of type (I-c), the corresponding adducts are cycled affording new heterocyclic structures A.

Sodium salts of molecules of formula (I-a) in which K² = NH, obtained upon NaH treatment are treated with different electrophiles such as acyl chlorides, sulfonyl chlorides, alkyl halides according to methods well known in the art. Aryl substitution was also performed using copper catalysed coupling reactions according to a method as described for example in the article by Lam, P. Y. S. et al., Tetrahedron Lett., 2001, 42, 3415-3418.

In the case of alkylations with alkyl halides reactions take usually place in DMF or THF with a catalytic amount of potassium iodide.

In the case of alkyl group with carbonyl function, acyl activated compounds are obtained by the corresponding esters hydrolysis with sodium hydroxide and are treated upon Friedel et Crafts conditions in order to obtain cyclic compounds (Salaski, E.J., Tetrahedron Lett., 1995, 36, 1387-1390).

Compounds of type (I-a) are functionalized *via* alkylamino chain linker and eventually connected to another carboxylic acid moiety *via* classical peptide coupling using O-benzotriazole-N,N,N',N'-tetramethyl-uronium-hexafluorophosphate (HBTU). In the case of benzimidazolones, sodium salts generated with NaH are used in DMF for the last stage alkylation or acylation reactions.

The corresponding thioureas are also obtained exclusively from some A rings using Lawesson reagent as described for example in the article by Zhang, P. et al., Bioorg. Med. Chem. Lett., 2001, 11, 2747.

Other AB fragments can be obtained using diverse methods: for example condensation of indolinone sodium salt with N-protected amino ketones of the formula (II-b) type afforded insaturated compounds which can also be reduced by hydrogenation in acetic acid dichloromethane. After deprotection, the same procedure as those detailed above can be used to link the different moieties.

Benzimidazoles (compounds of formula (I) in which A is a moiety of formula (A-4) or (A-5) as above-defined and T is -O- or -NH-) can be prepared using classical methods starting either from the corresponding diaminoarylic precursors AP which reacted with a carbonyl derivative of formula (II-b) as above-defined, or starting from chlorobenzimidazole by nucleophilic substitution with different amines according to the well known in the art methods.

In the case of protected amines, deprotection was followed by link procedure in order to connect to C fragment. Cyclisations were performed heating with carboxylic acids according to well know methods.

Secondly, it is also possible to synthesise compounds of formula (I) according to the present invention, using solid support library synthesis

Solid support parallel synthesis uses an adaptation to library production of the triazene linker strategy developed by Bräse, S. et al., Angew. Chem. Int. Ed., 1998, 37, 3413-3415 and Bräse, S., Acc. Chem. Res., 2004, 37, 8005-8016. This methodology allows the production of diazonium salts upon cleavage. The known properties of theses salts allow the possibility of a traceless synthesis *via* reduction or functionalisation. This methodology is used for the complete construction of the final compounds of formula (I) as previously defined and can be schematically represented on the following schemes 4 and 5: 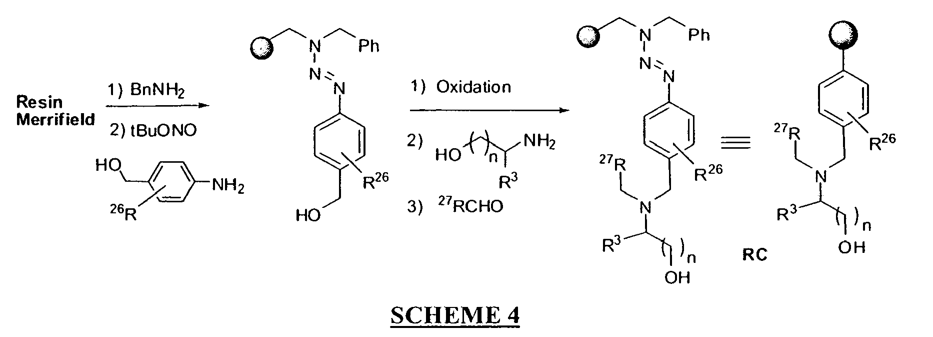 in which R³ and n have the same definition as in general formula (I), R²⁶ represents hydrogen, halogen, or an alkoxy or alkyl group, R²⁷ represents a group selected among alkyl, alkenyl, cycloalkyl, aryl, heteroaryl, arylalkyl and heteroarylalkyl groups wherein said groups defined for R²⁷ are optionnaly substituted with one or more groups independently selected from halogen, trifluoromethyl, difluoromethyl, azido, alkyl, alkoxy, cyano, nitro and carbethoxy.

Starting from Merrifield resin, benzylamin resin can be obtained using the reported procedure and triazene are formed by action of sodium tert-butyl nitrite, Lewis acid and the corresponding anilines such as 4-hydroxymethyl aniline (R²⁶=H). Oxidation to aldehyde takes place using ioded-based reagents such as 1-hydroxy-1,2-benziodoxol-3(1H)-one 1-oxide (IBX) or 1,1,1-triacetoxy-1,1-dihydro-1,2-benziodoxol-3(1H)-one (Dess Martin periodinane). First element of diversity is introduced *via* the reductive amination using the selected aminoalcohols bearing R³ group. Second element of diversity is introduced via a second reductive amination using the selected aldehydes.

Starting from those supported aminoalcohols, two synthetic pathways (Paths a and b) can be used depending of linker stability toward further reactions employed according to Scheme 5 below: 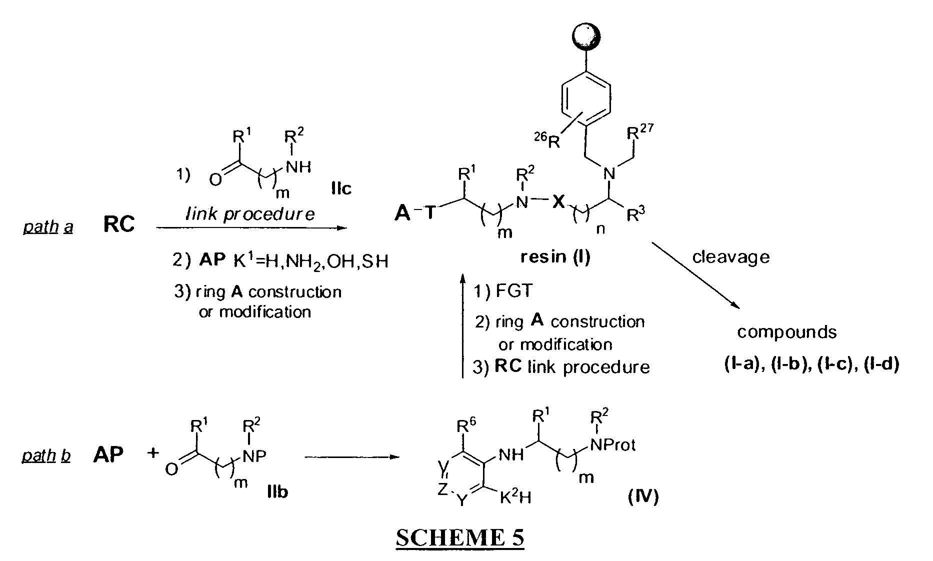 in which A, T, R¹, R², R⁶, m, n, V, Z, Y and X have the same meanings as those defined for formula (I), K¹ and K² have the same meanings as those defined in Scheme 3 and Prot has the same signification as those defined for scheme 2,.

According to Path a, (see Scheme 5), compounds of formula (I) are obtained in a linear « all supported » way: i.e. starting from the aminoalcohols. Resins R-C are first connected using the link procedure as described above with aminoketones (II-c), followed by reductive amination with precursor AP and A ring functionalisation/building using adapted versions of already discussed methodologies afforded supported final compounds Resin (I).

According to Path b, compounds of formula (I) are obtained using a « catch & release » methodology allowing a convergent synthesis : first molecules of formula (IV) are prepared without any purification in solution phase as described before and functionnalized or cyclized prior to the connection to the resin R-C with the link procedure affording Resin (I).

Cleavages of compounds (I) from the resin were performed on Resin (I) with treatments using an acid reagent such as TFA or trichlorosilane (HSiCl₃) with an adapted procedure of the literature and afforded compounds of formula (I), i.e. of formulas (I-a) to (I-d) as above described.

All the reactions used to obtain compounds of formula (I) are classically used in organic chemistry and are well known from the one skilled in the art.

After the synthesis, compounds of formula (I) according to the present invention can be recovered and purified according to methods also classically used in the art.

As it will be demonstrated in the examples illustrating the Invention, compounds of formula (I) according to the present Invention, (including pharmaceutically acceptable salts thereof), have a potent activity on the inhibition of the proliferation of apicomplexan parasites. Compounds of formula (I) then make possible to prevent and/or treat parasitic diseases involving apicomplexan parasites, when they are administered alone or in combination with usual antiparasitic drugs.

Another subject matter of the invention is therefore the compounds of formula (I) for a use as a drug, in particular as a drug for the prevention and/or the treatment of parasitic diseases involving apicomplexan parasites. According to a very preferred embodiment of the invention, the drug is for the prevention and/or the treatment of malaria or toxoplasmosis.

When the drug is intended for the prevention and/or treatment of malaria, compounds of formula (I) are preferably selected in the group consisting of:
- (S)-2-(dibenzylamino)propyl 4-(1H-benzo[d]imidazol-2-ylamino)piperidine-1-carboxylate;
- (S)-2-(dibenzylamino)propyl 4-(2-oxo-3-phenylsulfonyl-2,3-dihydro-1H-benzo[d]imidazol-1-yl)piperidine-1-carboxylate;
- (S)-2-(dibenzylamino)propyl 4-(3-(4-fluorophenylsulfonyl)-2-oxo-2,3-dihydro-1H-benzo[d]imidazol-1-yl)piperidine-1-carboxylate;
- (S)-2-(dibenzylamino)propyl 4-(3-(4-methoxyphenylsulfonyl)-2-oxo-2,3-dihydro-1H-benzo[d]imidazol-1-yl)piperidine-1-carboxylate;
- (S)-2-(dibenzylamino)propyl 4-(2-oxo-3-(4-(trifluoromethyl)phenylsulfonyl)-2,3-dihydro-1H-benzo[d]imidazol-1-yl)piperidine-1-carboxylate;
- (S)-2-(dibenzylamino)-3-phenylpropyl 4-(1,2-dlhydro-1-methyl-2-oxobenzo[d]imidazol-3-yl)piperidine-1-carboxylate;
- (S)-2-(dibenzylamino)propyl 4-(2-oxo-3-(thiophen-2-ylsulfonyl)-2,3-dihydro-1 H-benzo[d]imidazo)-1-yl)piperidine-1-carboxylate;
- N-((S)-2-(dibenzylamino)propyl)-4-(1,2-dihydro-2-oxobenzo[d]imidazol-3-yl)piperidine-1-carboxamidine;
- (S)-2-(dibenzylamino)propyl 4-(N-phenylplienylsulfonamido)piperidine-1-carboxylate;
- (S)-2-(dibenzylamino)propyl 4-(3-(4-ethoxy-4-oxobutyl)-2-oxo-2,3-dihydro-1 H-benzo[d]imidazo)-1-yl)piperidine-1-carboxylate;
- (S)-2-(dibenzylamino)propyl 4-(1-benzyl-1,2-dihydro-2-oxobenzo[d]lmldazol-3-yl)piperidine-1-carboxylate;
- (S)-2-(dibenzylamino)propyl 4-(2-oxo-3-(quinolin-8-ylsulfonyl)-2,3-dihydro-1 1H-benzo[d]imidazol-1-yl)piperidine-1-carboxylate;
- tert-butyl 3-(1-(((S)-2-(dibenzylamino)propoxy)carbonyl)piperidin-4-yl)-2,3-dihydro-2-oxobenzo[d]imidazole-1-carboxylate;
- (S)-2-(dibenzylamino)propyl 4-(2-(trifluoromethyl)-1H-benzo[d]imidazol-1-yl)piperidine-1-carboxylate;
- (S)-2-(dibenzylamino)-3-phenylpropyl 4-(1-benzyl-1,2-dihydro-2-oxobenzo[d]imidazol-3-yl)plperldine-1-carboxylate;
- (S)-2-(dibenzylamino)propyl 4-(3-(naphthalen-1-ylsulfonyl)-2-oxo-2,3-dihydro- H-benzo[d]imidazot-1-yl)piperidine-1-carboxylate;
- (S)-2-(dibenzylamino)propyl 4-(1-(cyclohexylmethyl)- 1,2-dlhydro-2-oxobenzo[d]imidazol-3-yl)piperldine-1-carboxylate;
- (S)-2-(dibenzylamino)propyl 4-(1,2-dihydro-2-thioxobenzo[d]imidazol-3-yl)piperidine-1-carboxylate;
- 1-(1-(3-(dibenzylamino)propyl)piperidin-4-yl)-1H-benzo[d]imidazol-2(3H)-one;
- (S)-2-(dibenzylamino)-3-phenylpropyl 4-(1,2-dihydro-2-oxobenzo[d]imidazol-3-yl)piperidine-1-carboxylate;
- (S)-2-(dibenzylamino)propyl 4-(N-phenylbenzamido)piperidine-1-carboxylate;
- 1-(S)-2-(dibenzylamino)propyl 3-ethyl 4-(1,2-dihydro-2-oxobenzo[d]imidazol-3-yl)piperidine-1,3-dicarboxylate;
- (S)-2-(dibenzylamino)propyl 4-(1,2-dihydro-1-methyl-2-oxobenzo[d]imidazol-3-yl)piperidine-1-carboxylate;
- (S)-2-(dibenzylamino)propyl 4-(2-oxo-3-(4-acetamidyl phenylsulfonyl)-2,3-dihydro-1H-benzo[d]imidazol-1-yl)piperidine-1-carboxylate;
- 1-(1-(2-(dibenzylamino)ethyl)piperidin-4-yl)-1H-benzo[d]imidazol-2(3H)-one;
- (S)-2-(dibenzylamino)propyl 4-(2-oxo-3-methylsulfonyl-2,3-dihydro-1H-benzo[d]imidazol-1-yl)piperidine-1-carboxylate;
- (S)-2-(dibenzylamino)propyl 4-(1H-benzo[d]imidazol-1-yl)piperidine-1-carboxylate;
- (S)-2-(dibenzylamino)-3-phenylpropyl 4-(1,2-dihydro-3-phenylsulfonyl-2-oxobenzo[d]imidazol-1-yl)piperidine-1-carboxylate;
- 2-benzylamino bis [ethyl 4-(1,2-dihydro-2-oxobenzo[d]imidazol-3-yl)piperidine-1-carboxylate;
- (S)-2-(dibenzylamino)propyl 4-(3-(2-tert-butyloxy carbonylaminoethyl)-2-oxo-2,3-dihydro-1H-benzo[d]imidazol-1-yl)piperidine-1-carboxylate;
- (S)-2-(dibenzylamino)-3-phenylpropyl 4-(3-(2-tert-butyloxy carbonylaminoethyl)-2-oxo-2,3-dihydro-1 I H-benzo[d]imidazol- I -yl)pipen'dine- I - carboxylate;
- (S)-2-(dibenzylamino)-3-phenylpropyl 4-(3-(2 aminoethyl)-2-oxo-2,3-dihydro-1H-benzo[d]imidazol-1-yl)piperidine-1-carboxylate D-biotin amid and pharmaceutically acceptable salts thereof.

When the drug is intended for the prevention and/or treatment of toxoplasmose, compounds of formula (I) are preferably selected in the group consisting of:
- O-2-(dibenzylamino)ethyl 4-(1,2-dihydro-2-oxobenzo[d]imidazol-3-yl)piperidine-1-carbothioate;
- (S)-2-(dibenzylamino)propyl 4-(1,2-dihydro-1-methyl-2-oxobenzo[d]imidazol-3-yl)piperidine-1-carboxylate;
- (S)-2-(dibenzylamino)propyl 4-(1H-indol-1-yl)pipcridine-1-carboxylate;
- (S)-2-(dibenzylamino)propyl 3-(1,2-dihydro-2-oxobenzo[d]imidazol-3-yl)propylcarbamate;
   1-benzylpiperidin-3-yl 4-(1,2-dihydro-2-oxobenzo[d]imidazol-3-yl)piperidine-1-carboxylate;
- 2-(benzyloxy)ethyl 4-(1,2-dihydro-2-oxobenzo[d]imidazol-3-yl)piperidine-1-carboxylate;
- (S)-2-(dibenzylamino)propyl 4-(1,2-dihydro-2-oxonaphtho[2,3-d)imidazol-3-yl)piperidine-l-carboxylate;
- 1-(1-(3-(dibenzylamino)propyl)piperidin-4-yl)-1 H-benzo[d]imidazol-2(3H)-one;
- (S)-2-(dibenzylamino)propyl 4-(2-oxobenzo[d]oxazol-3(2H)-yl)piperidine-1-carboxylate;
- (S)-2-(dibenzylamino)-3-phenylpropyl 4-(1,2-dihydro-1-methyl-2-oxobenzo[d]imidazol-3-yl)piperidine-1-carboxylate;
- (S)-2-(dibenzylamino)propyl 4-(1,2-dihydro-2-oxo-1-(benzoyl)benzo[d]imidazol-3-yl)piperidine-1-carboxylate;
- 2-(1-benzylpiperidin-4-yl)ethyl 4-(1,2-dihydro-2-oxobenzo[d]imidazol-3-yl)piperldine-1-carboxylate;
- (S)-2-(dibenzylamino)propyl 4-(1H-benzo[d]imidazol-2-ylamino)piperidine-1-carboxylate;
- (S)-2-(dibenzylamino)propyl 4-(1-acetyl-1,2-dihydro-2-oxobenzo[d]imidazol-3-yl)piperidine-1-carboxylate;
- (S)-2-(dibenzylamino)propyl 4-(6-chloro-1,2-dihydro-2-oxobenzo[d]imidazol-3-yl)piperidine-1-carboxylate;
- O-2-(benzyloxy)ethyl 4-(1,2-dihydro-2-oxobenzo[d]imidazol-3-yl)piperidine-1-carbothioate;
- O-(S)-2-(dibenzylamino)propyl 4-(1,2-dihydro-2-oxobenzo[d]imidazol-3-yl)piperidine-1-carbothioate;
- 3,3-diphenylpropyl 4-(1,2-dihydro-2-oxobenzo[d]imidazol-3-yl)piperidine-1-carboxylate;
- (S)-2-(dibenzylamino)propyl 4-(N-pheny)propionamido)pipehdine-1-carboxylate;
- (S)-2-(dibenzylamino)propyl 4-(2-oxoindolin-3-ylidene)piperidine-1-carboxylate;
- 1-(1-(4-(dibenzylamino)butyl)piperidin-4-yl)-1H-benzo[d]imidazol-2(3H)-one;
- (S)-2-(dibenzylamino)propyl 4-(3-(4-fluorophenylsulfonyl)-2-oxo-2,3-dihydro-1H-benzo[d]imidazol-1-yl)piperidine-1-carboxylate;
- (S)-2-(dibenzylamino)propyl 4-( 1-(cyclohexylmethyl)-1,2-dihydro-2-oxobenzo[d]imidazol-3-yl)plperidine-1-carboxylate;
- (S)-2-(dibenzylamino)propyl 4-(2-oxo-3-phenylsulfonyl-2,3-dihydro-1H-benzo[d]imidazot-1-yl)piperidine-1-carboxytate;
- (S)-4-(3-(1-((2-(dibenzylamino)propoxy)carbonyl)piperidin-4-yl)-2-oxo-2,3-dihydro-1H-benzo[d]imidazol-1-yl)butanoic acid;
- (S)-2-(dibenzylamino)propyl 4-(3-(4-methoxyphenylsulfonyl)-2-oxo-2,3-dihydro-1H-benzo[d]imidazol-1-yl)piperidine-1-carboxylate;
- (S)-2-(dibenzylamino)propyl 4-(N-phenylphenylsulfonamido)piperidine-1-carboxylate;
- (S)-2-(dibenzylamino)propyl 4-(1-benzyl-1,2-dihydro-2-oxobenzo[d]imidazol-3-yl)piperidine-1-carboxylate;
- 2-(ethyl(phenyl)amino)ethyl 4-(2-oxo-2,3-dihydro-1H-benzo[d]imidazol-1-yl)piperidine-1-carboxylate;
- (S)-2-(dibenzylamino)-3-phenylpropyl 4-(1-benzyl-1,2-dihydro-2-oxobenzo[d]imidazol-3-yl)piperidine-1-carboxylate;
- (S)-2-(dibenzylamino)propyl 4-(1H-indol-3-yl)piperidine-1-carboxylate;
- (S)-2-(dibenzylamino)propyl 4-(2-oxo-3-methylsulfonyl-2,3-dihydro-1H-bcnzo[d]imidazol-1-yl)piperidine-1-carboxylate;
- (S)-2-(dibenzylamino)propyl 4-(indolin-1-yl)piperidine-1-carboxylate;
- 2-benzylamino bis [ethyl 4-(1,2-dihydro-2-oxobenzo[d]imidazol-3-yl)piperidine-1-carboxylate and pharmaceutically acceptable salts thereof.

Finally, a subject matter of the invention is also a pharmaceutical composition comprising, as an active principle, at least one compound of formula (I) (as a free base or in the form of a pharmaceutically acceptable salt), and at least one pharmaceutically acceptable excipient.

The pharmaceutical composition of the present invention may be administered by any suitable route, for example, by oral, buccal, inhalation, sublingual, nasal, percutaneous, i.e., transdermal, or parenteral (including intravenous, intramuscular, subcutaneous and intracoronary) administration. Therefore, the pharmaceutical composition of the invention can be provided in various forms, such as in the form of hard gelatin capsules, of capsules, of compressed tablets, of suspensions to be taken orally, of lozenges or of injectable solutions or in any other form appropriate to the method of administration.

According to a preferred embodiment of the invention, the pharmaceutical composition is for an oral administration.

The pharmaceutical composition according to the invention includes those wherein the at least one compound of the above-defined formula (I) is administered in an effective amount to achieve its intended purpose. Determination of the effective amounts is well within the capability of those skilled in the art.

A "therapeutically effective dose" refers to that amount of the compounds of formula (I) that results in achieving the desired effect. It has to be noted that when the compound of formula (I) is in the form of a pharmaceutically acceptable salt, this amount is calculated on the basis of the mass of said compound of formula (I) in the form of its free base. Toxicity and therapeutic efficacy of such compounds can be easily determined by standard pharmaceutical procedures in cell cultures or experimental animals, e.g., for determining the LD₅₀ (the dose lethal to 50% of the population) end the ED₅₀ (the dose therapeutically effective in 50% of the population). The dose ratio between toxic and therapeutic effects is the therapeutic index, which is expressed as the ratio between LD₅₀ and ED₅₀ compounds of formula (I) which exhibit high therapeutic indices are of course preferred. The data obtained from such data can be used in formulating range of dosage for use in humans. The dosage of compounds of formula (I) preferably lies within a range of circulating concentrations that include the ED₅₀ with little or no toxicity. The dosage can vary within this range depending upon the dosage form employed, and the route of administration.

The exact formulation, route of administration, and dosage can be chosen by the individual physician in view of the patient's conditions. Dosage amount and interval of administration can be adjusted individually to provide plasma levels of the compound of formula (I) which are sufficient to maintain the preventive or therapeutic effects.

The amount of pharmaceutical composition administered will therefore depend on the subject being treated, on the subject's weight, the severity of the affliction and the manner of administration.

For human use, the compounds of formula (I) can be administered alone, but they are preferably administered in admixture with at least one pharmaceutically acceptable carrier, the nature of which will depend on the intended route of administration and the presentation form. Pharmaceutical composition for use according to the present invention thus can be formulated in a conventional manner using one or more physiologically acceptable carriers comprising one or more excipients and auxiliaries that facilitate processing of the compounds of formula (I) into preparations which can be used pharmaceutically. Amongst the excipients and auxiliaries which can be used in the pharmaceutical composition according to the invention, one can mention antiagglomerating agents, antioxidants, preservatives agents, dyes, vitamins, inorganic salts, taste-modifying agents, smoothing agents, coating agents, isolating agents, stabilizing agents, wetting agents, anti-caking agents, dispersing agents, emulsifying agents, aromas, penetrating agents, solubilizing agents, etc..., mixtures thereof and generally any excipient conventionally used in the pharmaceutical industry.

By way of example, when the pharmaceutical composition is administered orally, the carrier may comprise one or several excipients such as talc, lactose, starch or modified starches, cellulose or cellulose derivatives, polyethylene glycols, acrylic acid polymers, gelatin, magnesium stearate, animal or vegetal fats of natural or synthetic origin, paraffin derivatives, glycols, etc....

In addition to the at least one compound of formula (I), the pharmaceutical composition may also comprises one or more additional usually used antiparasitic active principle, such as for example an anti-malarial drugs (as example chloroquine, quinacrine, primaquine and artemisinine).

For general information about the formulation and administration of pharmaceutical compositions, one can obviously refer to the book "Remington's Pharmaceutical Sciences", last edition. Of course, a person skilled in the art will take care on this occasion that the excipient(s) and/or auxiliary(ies) optionally used are compatible with the intrinsic properties attached to the pharmaceutical composition in accordance with the invention.

These pharmaceutical compositions can be manufactured in a conventional manner, e.g., by conventional mixing, dissolving, granulating, drageemaking, emulsifying, encapsulating, entrapping, or lyophilizing processes. Proper formulation is dependent upon the route of administration chosen.

Besides the arrangements above, the invention also comprises other arrangements which will emerge from the following description, which refers to examples of preparation of compounds of formula (I) according to the invention and to *in vitro* demonstration of the antiparasitic activity of compounds of formula (I).

It should be clearly understood, however, that these examples are given only by way of illustration of the subject of the invention, of which they in no way constitute a limitation.

### EXAMPLES

### EXAMPLE 1: GENERAL PROTOCOL AND SYNTHESIS OF DIFFERENT COMPOUNDS OF FORMULA (I)

### General procedure:

Commercial reagents were purchased from Aldrich and were used as received without additional purification. All reactions were carried out under nitrogen with dry, freshly distilled solvents and oven- or flame-dried glassware.

For solid phase synthesis, the moderate-scale reactions (100-500 mg of resin) were carried out using flasks fitted with a frit at the bottom and a stopper. All solid phase reactions were agitated by variable speed orbital mixer. Merrifield resin was obtained from Aldrich (I00-200 mesh; 1% cross-linked; loading: 1.97 mmol/g).

Chromatography was carried out on Merck silica gel 60 (particle size 230-400 mesh). Thin layer chromatography (TLC) was performed on a 0.2 mm precoated plates of silica gel 60F-264 (Merck). Visualization was made with ultraviolet light or iodide or phosphomolibdic acid spray. Preparative TLC was performed on a 1.0 mm precoated plates of silica gel 60F-264 (Whatman). Visualization was made with ultraviolet light.

¹H and ¹³C NMR spectra were recorded on a Brucker Avance 400 with a BBO probe.

IR spectra of resin materials in KBr tablets were taken with a Perkin Elmer 2000 FTIR.

### Analytical Method for LC/MS:

Column: Xbridge C18 3-5 µM, 4.6 mm x 100 mm
Flow rate: 1.0 mL/min
Detector: Photodiode Array Detector Waters 2996: UV (200-400 nm), PL-ELS 1000, MS ZQ 2000
Injection volume: 1 µL using Autosampler: Waters 2767
Method: 95% A, 5% B to 0% A, 100% B with 8 minutes gradient then 5 minutes hold
A: 100% water, 0.1% formic acid
B: 100% acetonitrile

### 1) Mitsunobu procedure:

### a. General procedure for primary alcohol:

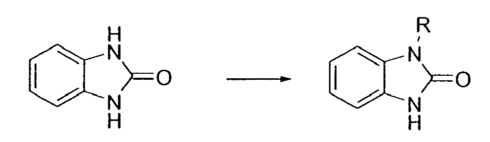

To a stirred suspension of 1H-benzo[d]imidazol-2(3H)-one (1 equiv.), appropriate alcohol (2 equiv.) and PPh₃ (1.5 equiv.) in THF (22 mL/mmol) cooled to 0°C under N₂ was added dropwise DEAD (1.5 equiv.). The resulting mixture was allowed to warm to room temperature and stirred for 16h. The solvent was evaporated. The residue was purified by appropriate method.

### Preparation of tert-butyl 4-((1,2-dihydro-2-oxobenzo[d]imidazol-3-yl)methyl)piperidine- I -carboxylate (Intermediate):

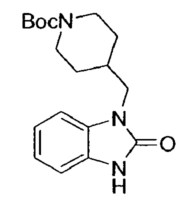

From tert-butyl 4-(hydroxymethyl)piperidine-1-carboxylate
Flash chromatography on silica gel (methylene chloride/acetone 8/2)
Colorless oil (60%).
LC/MS (ES⁺) *m*/*z* 332.1 (M+H)⁺

### Preparation of tert-butyl 4-(2-(1,2-dihydro-2-oxobenzofdlimidazol-3-yl)ethyl)piperidine-1-carboxylate (Intermediate):

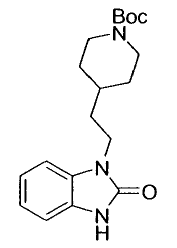

From tert-butyl 4-(2-hydroxyethyl)piperidine-1-carboxylate
Flash chromatography on silica gel (cyclohexane/ethyl acetate 5/5)
Colorless oil (50%).
LC/MS(ES⁺) *m*/*z* 246.1 ((M-Boc)+H)⁺

### Preparation of 1-((1-benzylpyrrolidin-2-yl)methyl)-1H-benzo[d]imidazol-2(3H)-one (Intermediate):

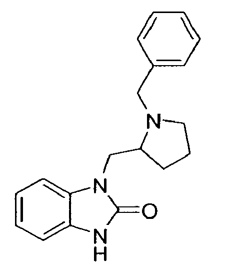

From S-(1-benzylpyrrolidin-2-yl)methanol
Flash chromatography on silica gel (cyclohexane/ethyl acetate 5/5)
Colorless oil (35%).
LC/MS (ES⁺) *m*/*z* 308.2 (M+H)⁺

### b. General procedure for secondary alcohol:

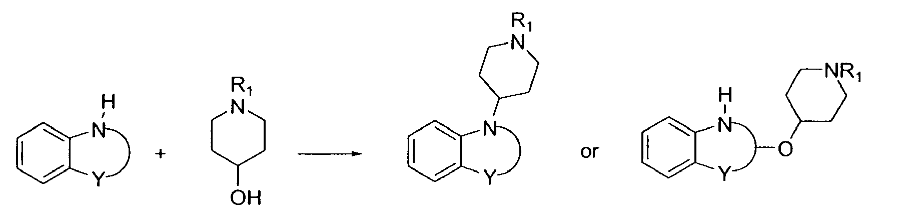

To a stirred suspension of appropriate heterocycle (0.37 mmol, 2 equiv.), (S)-2-(dibenzylamino)propyl 4-hydroxypiperidine-1-carboxylate (70 mg, 0.18 mmol) and PPh₃ (72 mg, 0.27 mmol) in THF (4 mL) cooled to 0°C under N₂ was added dropwise DEAD (43 µl, 0.27 mmol). The resulting mixture was allowed to warm to room temperature and stirred overnight. The solvent was evaporated. The residue was purified by appropriate method.

### Preparation of ethyl 2-(1-(((S)-2-(dibenzylamino)propoxy)carbonyl)piperidin-4-yloxy)-1H-indole-1-carboxylate (Compound n°1):

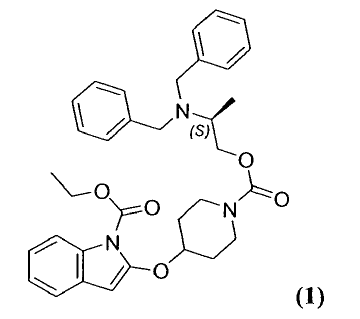

From indolinone
Flash chromatography on silica gel (cyclohexane/ethyl acetate 9/1)
Colorless oil (13%).
¹H NMR (400 MHz, CDCl₃) δ 8.01 (dd, J = 8.8, 4.0 Hz, 1H, Ar), 7.37 (m, 4H, Ar), 7.29 (m, 4H, Ar), 7.18 (m, 5H, Ar), 5.69 (s, 1H, Ar), 4.60 (m, 1H, *CH*CH₂CH₂Nₚᵢₚ), 4.49 (q, J = 7.2 Hz, 2H, O*CH₂*CH₃), 4.25 (dd, J = 7.6, 11.0 Hz, 1H, *CH₂*O)*,* 4.04 (dd, *J =* 5.6, 11.0 Hz, 1H, *CH₂*O)*,* 3.75 (d, *J* = 13.8 Hz, 2H, N*CH₂*Ph), 3.66 (m, 4H, CHCH₂*CH₂*Nₚᵢₚ), 3.56 (d, J = 13.8 Hz, 2H, N*CH₂*Ph)*,* 3.12 (m, 1H, *CH*Me), 1.99 (m, 4H, CH*CH₂*CH₂Nₚᵢₚ), 1.47 (t, *J* = 7.2 Hz, 3H, OCH₂*CH₃*), 1.09 (d, *J* = 6.8 Hz, 3H, Me).
¹³C NMR (100 MHz, CDCl₃) δ 155.3 (C=O), 150.8 (C=Oₑₛₜₑᵣ), 140.2 (C_{Bn}), 131.6 (C_{Ar}), 128.5 (CH_{Bn}), 128.2 (C_{Ar}), 128.1 (CH_{Bn}), 126.8 (CH_{Bn}), 123.1 (CH_{Ar}), 122.1 (CH_{Ar}), 118.7 (CH_{Ar}), 114.8 (CH_{Ar}), 84.2 (*CH*CH₂CH₂Nₚᵢₚ), 67.4 (*CH₂*O), 62.9 (O*CH₂*CH₃), 53.6 (N*CH₂*Ph), 51.8 (*CH*Me), 40.1 (CHCH₂*CH₂*Nₚᵢₚ), 29.6 (CH*CH₂*CH₂Nₚᵢₚ), 14.4 (OCH₂*CH₃*), 11.2 (Me).
LC/MS (ES⁺) *m*/*z* 570.2 (M+H)⁺

### Preparation of (S)-2-(dibenzylamino)propyl 4-(2-oxobenzo(dioxazol-3(2H)-yl)piperidine-1-carboxylate (Compound n°2):

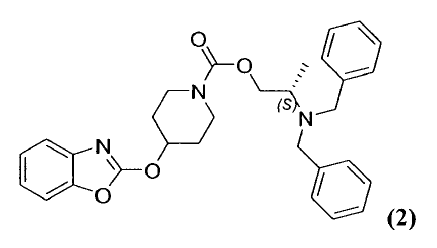

From benzoxazolone
Colorless oil (4%).
LCMS preparative
¹H NMR (400 MHz, CDCl₃, ppm) δ 7.49 (d, *J* = 7.6 Hz, 1H, *Ph*), 7.37 (d, *J* = 7.4 Hz, 4H, *Ph*), 7.28 (dd, J = 12.7, 4.9 Hz, 6H, *Ph*), 7.21 (m, 3H, *Ph*), 5.26 (m, 1H, *CH*CH₂CH₂N), 4.24 (dd, *J* = 11.0, 7.6 Hz, 1H, O*CH₂*), 4.03 (dd, *J* = 11.0, 5.6 Hz, 1H, O*CH₂*), 3.83 (m, 2H, CHCH₂*CH₂*N), 3.75 (d, *J* = 13.9 Hz, 2H, N*CH₂*Ph), 3.55 (d, *J* = 13.9 Hz, 2H, N*CH₂*Ph), 3.48 (m, 2H, CHCH₂*CH₂*N), 3.13 (dd, *J* = 13.0, 6.8 Hz, 1H, *CH*CH₃), 2.12 (m, 2H, CH*CH₂*CH₂N), 1.96 (m, 2H, CH*CH₂*CH₂N), 1.09 (d, *J* = 6.8 Hz, 3H, CH*CH₃*)
¹³C NMR (100 MHz, CDCl₃, ppm) δ 162.6 (C=O), 155.2 (C=O), 148.2, 140.9, 140.2, 128.5, 128.1, 126.8, 124.2, 122.8, 117.8, 109.7 (CPh), 77.8 (*CH*CH₂CH₂N), 66.7 (CH₂O), 53.6 (NCH₂Ph), 51.8 (*CH*CH₃), 40.6 (CHCH₂*CH₂*N), 30.3 (CH*CH₂*CH₂N), 11,1 (CH*CH₃*)
LC/MS (ES⁺) m/z 500.1 (M+H)⁺

### Preparation of (S)-2-(dibenzylamino)propyl 4-(1H-benzo[d]imidazol-1-yl)piperidine-1-carboxylate (Compound n°3):

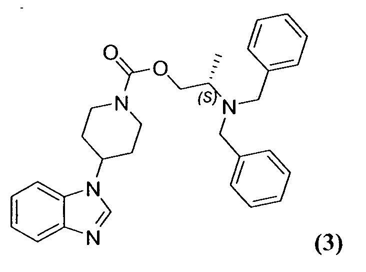

From benzimidazole
Colorless oil (65%).
preparative LCMS
LC/MS (ES+) m/z 483.1 (M+H)+

### Preparation of (S)-2-(dibenzylamino)propyl 4-(1H-benzo[d][1,2,3]triazol-1-yl)piperidine-1-carboxylate(Compound n°4):

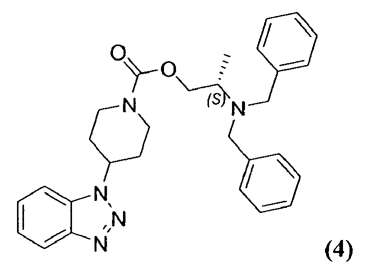

From benzotriazole
Colorless oil (65%).
preparative LCMS
LC/MS (ES+) m/z 484.2 (M+H)+

### Preparation of (S)-2-(dibenzylamino)propyl 4-(1-methyl-1H-benzo[d]imidazol-2-yloxy)piperidine-1-carboxylate(Compound n°5):

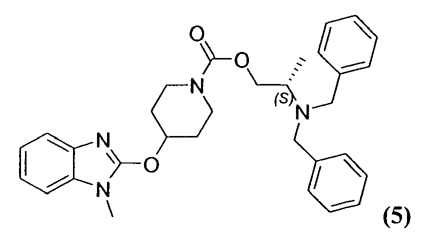

From 1-methylbenzimidazolone
Colorless oil (23%).
Flash chromatography on silica gel (cyclohexane/ ethyl acetate 1/1)
¹H NMR (400 MHz, CDCl₃, ppm) δ 7.55 (m, 1H, *Ph*)*,* 7.38 (d, *J* = 7.2 Hz, 4H, *Ph*)*,* 7.28 (dd, J = 12.7, 5.1 Hz, 4H, *Ph*)*,* 7.19 (m, 5H, *Ph*)*,* 5.34 (m, 1H, *CH*CH₂CH₂N), 4.25 (dd, *J* = 11.1, 7.5 Hz, 1 H, O*CH₂*)*,* 4.04 (dd, *J* = 11.1, 5.6 Hz, 1H, O*CH₂*)*,* 3.85 (m, 2H, CHCH₂*CH₂*N), 3.75 (d, *J* = 13.9 Hz, 2H, N*CH₂*Ph), 3.55 (d, *J* = 13.9 Hz, 5H, N*CH₂*Ph, N*CH₃*)*,* 3.42 (m, 2H, CHCH₂*CH₂*N), 3.13 (sext., *J* = 6.8 Hz, 1H, *CH*CH₃), 2.14 (m, 2H, CH*CH₂*CH₂N), 1.89 (ddd, *J* = 16.6, 8.2, 4.1 Hz, 2H, CH*CH₂*CH₂N), 1.08 (d, J = 6.8 Hz, 3H, CH*CH₃*)
¹³C NMR (100 MHz, CDCl₃, ppm) δ 156.4, 155.3 (C=O), 140.2, 139.9, 134.0, 128.5, 128.1, 126.7, 121.5, 120.8, 117.5, 107.9 (C_{Ph}), 77.2 (*CH*CH₂CH₂N), 66.7 (CH₂O), 53.6 (NCH₂Ph), 51.8 (*CH*CH₃), 41.0 (CHCH₂*CH₂*N), 30.7 (CH*CH*₂CH₂N), 28.0 (NCH₃), 11,1 (CH*CH₃*)
LC/MS (ES+) m/z 514.2 (M+H)+

### 2) seven-member cycle preparation:

### Preparation of azepan-4-one hydrochloride (Intermediate):

To a stirred solution of N-carbethoxy-4-piperidone (600 mg, 3.00 mmol) in anhydrous Et₂O (1.5 mL) were simultaneously added solutions of BF₃.Et₂O (380 µL, 3.00 mmol) and ethyl diazoacetate (412 µL, 3.92 mmol), each in anhydrous Et₂O (0.4 mL) at -35°C under a nitrogen atmosphere. The reaction mixture was stirred for 1h30 at -35°C and allowed to warm to room temperature. The solution was washed with 30% aqueous K₂CO₃ solution (3 mL) and the organic phase was extracted with ethyl acetate (3x5 mL). The organic layers were dried over anhydrous Na₂SO₄, filtered and concentrated under vacuum. The crude material was refluxed in 4N aqueous HCl (13 mL) for 6h and the solvent was removed on *vacuo* to afford a pale yellow oil (400 mg, 88%): ¹H NMR (400 MHz, CDCl₃) δ 3.04 (m, 4H, *CH₂*NH)*,* 2.59 (m, 4H, *CH₂*CO)*,* 1.86 (m, 1H, NH), 1.75 (m, 2H, *CH₂*CH₂NH).

### Preparation of (S)-2-(dibenzylamino)propyl 4-oxoazenane-1-carboxylate (Intermediate):

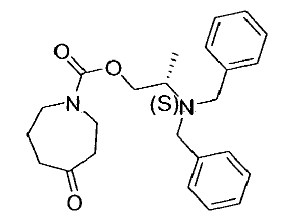

To a stirred solution of the (S)-2-(dibenzylamino)propan-1-ol (285 mg, 1.20 mmol) in THF (6 mL) at 0°C was added CDI (213 mg, 1.30 mmol) under N₂ atmosphere. The reaction mixture was stirred for 2h at room temperature. The azepan-4-one hydrochloride (140 mg, 1.12 mmol) in DMF (4 mL) and triethylamine (568 µL, 4.00 mmol) were added and the reaction mixture was heated at 50°C for 72 h. The solvent was removed on *vacuo* and the crude material was purified by flash chromatography (silica gel, cyclohexane/ethyl acetate 5/5) to afford a pale yellow oil (20 mg, 4%).
¹H NMR (400 MHz, CDCl₃) δ 7.35 (d, *J* = 7.2 Hz, 4H, Bn), 7.28 (t, *J* = 7.2 Hz, 4H, Bn), 7.22 (t, *J* = 7.2 Hz, 2H, Bn), 4.22 (dd, *J* = 11.2, 7.2 Hz, 1H, *CH₂*O), 4.02 *(dd, J =* 11.2, 5.6 Hz, 1H, *CH₂*O), 3.73 (d, *J* = 14.0 Hz, 2H, N*CH₂*Ph), 3.64 (m, 4H, CH₂*CH₂*N, CH₂CH₂*CH₂*N), 3.54 (d, J= 14.0 Hz, 2H, N*CH₂*Ph), 3.12 (m, 1H, *CH*Me), 2.67 (m, 4H, *CH₂*CH₂N, *CH₂*CH₂CH₂N), 1.80 (m, 2H, CH₂*CH₂*CH₂N), 1.08 (d, J= 4.4 Hz, 3H, Me)
LC/MS (ES⁺) m/z 395.2 (M+H)⁺

### 3) Alkyl derivative preparation

### Preparation of (S)-2-(dibenzylamino)propyl 3-bromopropylcarbamate (Intermediate):

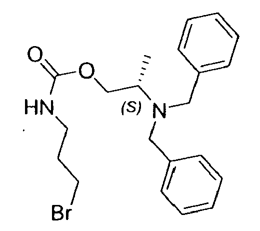

To a stirred solution of (S)-2-(dibenzylamino)propan-1-ol (280 mg, 1.10 mmol) in THF (2.5 mL) at 0°C was added CDI (194 mg, 1.20 mmol) under N₂ atmosphere. The reaction mixture was stirred for 2h at room temperature. The 3-bromopropan-1-amine hydrobromide (219 mg, 1.0 mmol) and triethylamine (209 µL, 1.50 mmol) were added and the reaction mixture was stirred for 72h at room temperature. The solvent was removed on *vacuo* and the crude material was purified by flash chromatography (silica gel, cyclohexane/ethyl acetate 5/5) to afford a pale yellow oil (20 mg, 4%).
¹H NMR (400 MHz, CDCl₃) δ 7.36 (d, *J =* 7.2 Hz, 4H, Bn), 7.29 (t, *J =* 7.2 Hz, 4H, Bn), 7.22 (t, *J =* 7.2 Hz, 2H, Bn), 4.77 (s, 1H, NH), 4.21 (dd, *J*= 11.2, 7.2 Hz, 1H, *CH₂*O), 4.02 (dd, *J =* 11.2, 6.0 Hz, 1H, *CH₂*O), 3.72 (d, *J =* 14.0 Hz, 2H, N*CH₂*Ph), 3.54 (d, *J* = 14.0 Hz, 2H, N*CH₂*Ph), 3.45 (t, *J* = 6.4 Hz, 2H, CH₂*CH₂*N), 3.34 (q, *J* = 6.4 Hz, 2H, *CH₂*CH₂N), 3.06 (m, 1H, *CH*Me), 2.08 (t, *J* = 6.4 Hz, 2H, *CH₂*Br), 1.07 (d, *J =* 6.8 Hz, 3H, Me)
LC/MS (ES⁺) *m*/*z* 419.1-421.0 (M+H)⁺

### Preparation of (S)-2-(dibenzylamino)propyl 3-(1,2-dihydro-2-oxobenzo[d]imidazol-3-yl)propylcarbamate (Compound n°6):

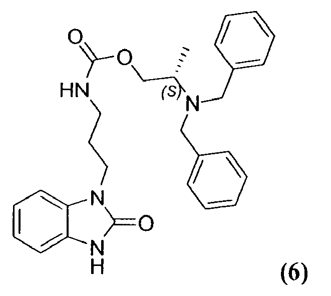

To a stirred solution of benzimidazolone (1 1 mg, 0.08 mmol) in DMF (0.1 mL) at 0°C was added NaH (60% in mineral oil) (3.2 mg, 0.08 mmol) under N₂ atmosphere. The reaction mixture was stirred for 30 min at 0°C. The brominated derivative (30 mg, 0.07 mmol) in DMF (0.1 mL) was added and the reaction mixture was stirred overnight at room temperature. Water (2 mL) was introduced and the mixture was extracted with ethyl acetate (3x5 mL). The combined organic layers were dried over anhydrous Na₂SO₄, filtered and concentrated under vacuum. The crude material was purified by HPLC preparative to afford a white solid (2 mg, 6%).
¹H NMR (400 MHz, CDCl₃) δ 8.45 (s, 1H, NH), 7.36-7.12 (m, 10H, Ar), 7.02 (m, 4H, Ar), 5.72 (s, 1H, NH), 4.31 (m, 1H, *CH₂*O), 4.12 (m, 1H, *CH₂*O), 3.95 (m, 4H, *CH₂*CH₂CH₂NH, CH₂CH₂*CH₂*NH), 3.72 (m, 2H, N*CH₂*Ph), 3.58 (m, 2H, N*CH₂*Ph), 3.18 (m, 1H, *CH*Me), 1.70 (m, 2H, CH₂*CH₂*CH₂NH), 1.09 (m, 3H, Me)
LC/MS (ES⁺) *mlz* 473.2 (M+H)⁺

### 4) Reductive amination

### a. Reductive amination (primary amine):

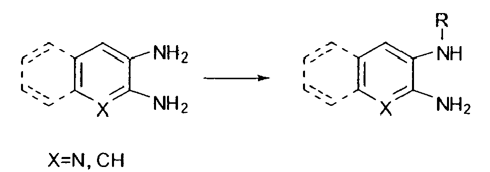

### General procedure:

To a stirred solution of appropriate diamine (3 equiv.) in 1,2-dichloroethane (1 mL/mmol) were added appropriate ketone (1 equiv.), acetic acid (1.7 equiv.) followed by sodium triacetoxyborohydride (1.9 equiv.) under a nitrogen atmosphere. The reaction mixture was stirred for 16h at room temperature. The solvent was removed *on vacuo* and the residue was partitioned between ethyl acetate and 2M aqueous Na₂CO₃ solution (5 mL/mmol). The organic phase was separated and washed by 2M aqueous Na₂CO₃ solution (2 x 5 mL/mmol). The organic layer was dried over anhydrous Na₂SO₄, filtered and concentrated under vacuum. The crude material was purified by flash chromatography on silica gel.

### Preparation of tert-butyl 4-(2-aminophenylamino)piperidine-1-carboxylate (Intermediate):

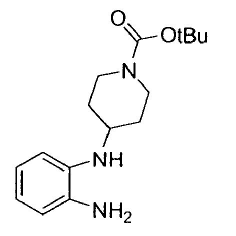

From benzene-1,2-diamine and tert-butyl 4-oxopiperidine-1-carboxylate
Flash chromatography on silica gel (cyclohexane/ethyl acetate 5/5)
Brown solid (88%).
¹H NMR (400 MHz, CDCl₃) δ 6.80 (m, 1H, Ar), 6.75 (m, 3H, Ar), 4.03 (bs, 2H, CHCH₂*CH₂*Nₚᵢₚ), 3.40 (m, 1H, *CH*CH₂CH₂Nₚᵢₚ), 2.92 (bt, 2H, CHCH₂*CH₂*Nₚᵢₚ), 2.04 (m, 2H, CH*CH₂*CH₂Nₚᵢₚ), 1.46 (s, 9H, OC(CH₃)₃), 1.14 (m, 2H, CH*CH₂*CH₂Nₚᵢₚ)
LC/MS (ES⁺) m/z 292.1 (M+H)⁺

### Preparation of (S)-2-(dibenzylamino)propyl 4-(2-aminopyridin-3-ylamino)piperidine-1-carboxylate (Intermediate):

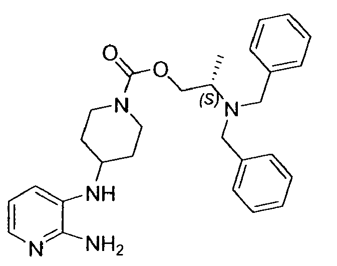

From pyridine-2,3-diamine and (S)-2-(dibenzylamino)propyl 4-oxopiperidine-1-carboxylate
Flash chromatography on silica gel (methylene chloride /ethanol 95/5)
Yellow solid (40%).
LC/MS (ES⁺) m/z 474.2 (M+H)⁺

### Preparation of tert-butyl 3-(2-aminophenylamino)piperidine-1-carboxylate (Intermediate):

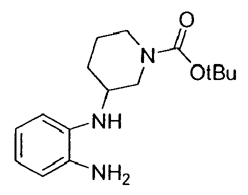

From benzene-1,2-diamine and tert-butyl 3-oxopiperidine-1-carboxylate
Flash chromatography on silica gel (cyclohexane/ethyl acetate 6/4)
Brown oil (50%).
¹H NMR (400 MHz, CDCl₃) δ 6.82-6.70 (m, 4H, Ar), 4.03 (m, 1H, CH*CH₂*N), 3.72 (bd, *J =* 10.8 Hz, 1H, CH*CH₂*N), 3.36 (m, 1H, *CH*CH₂N), 3.20 (bs, 2H, NH₂), 3.08 (m, 1H, CHCH₂*CH₂*N), 2.91 (m, 1H, CHCH₂*CH₂*N), 2.02 (m, 1H, CH*CH₂*CH₂CH₂N), 1.76 (m, 1H, CH*CH₂*CH₂CH₂N), 1.57 (m, 2H, CHCH₂*CH₂*CH₂N), 1.45 (s, 9H, C(CH₃)₃)
LC/MS (ES⁺) m/z 292.1 (M+H)⁺

### Preparation of N-(1-benzylpyrrolidin-3-yl)benzene-1,2-diamine (Intermediate):

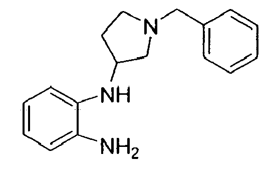

From benzene-1,2-diamine and 1-benzylpyrrolidin-3-one
Flash chromatography on silica gel (ethyl acetate)
Brown oil (40%).
LC/MS (ES⁺) *m*/*z* 268.2 (M+H)⁺

### Preparation of (S)-2-(dibenzylamino)propyl 4-(2-aminonaphthalen-3-ylamino)piperidine-1-carboxylate (Intermediate):

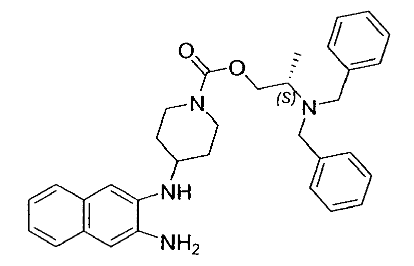

From naphthalene-2,3-diamine and (S)-2-(dibenzylamino)propyl 4-oxopiperidine-1-carboxylate
Flash chromatography on silica gel (cyclohexane/ethyl acetate 7/3)
Brown oil (20%).
LC/MS (ES⁺) *m*/*z* 523.2 (M+H)⁺

### Preparation of (S)-2-(dibenzylamino)propyl 4-(2-aminophenylamino)azepane-1-carboxylate (Intermediate):

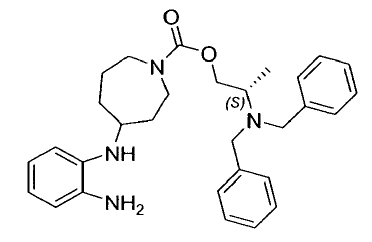

From benzene-1,2-diamine and (S)-2-(dibenzylamino)propyl 4-oxoazepane-1-carboxylate.
Flash chromatography on silica gel (cyclohexane/ethyl acetate 6/4)
Orange oil (58%).
LC/MS (ES⁺) m/z 487.2 (M+H)⁺

### b. Reductive amination (primary amine with electrowithdrawing group on the a position of C=O):

To a stirred solution of appropriate diamine (2 equiv.) in methanol (1.5 mL/mmol) were added appropriate ketone (1 equiv.), acetic acid (1.3 equiv.) followed by supported sodium cyanoborohydride (1 equiv.) under a nitrogen atmosphere. The reaction mixture was stirred for 48h at room temperature and was filtered. The filtrate was concentrated under *vacuo* and the residue was partitioned between ethyl acetate and saturated aqueous Na₂CO₃ solution (5 mL/mmol). The organic phase was separated and washed by saturated aqueous Na₂CO₃ solution (2 x 5 mL/mmol). The organic layer was dried over anhydrous Na₂SO₄, filtered and concentrated under vacuum. The crude material was purified by flash chromatography on silica gel.

### Preparation of ethyl 4-(2-aminophenylamino)-1-benzylpiperidine-3-carboxylate (Intermediate):

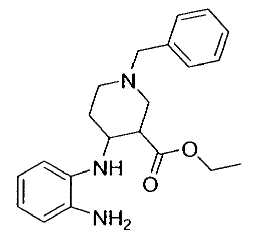

From benzene-1,2-diamine and ethyl 1-benzyl-4-oxopiperidine-3-carboxylate
Flash chromatography on silica gel (cyclohexane/ethyl acetate 5/5)
Brown oil (36%).
LC/MS (ES⁺) m/z 354.2 (M+H)⁺

### c. Reductive amination (secondary amine):

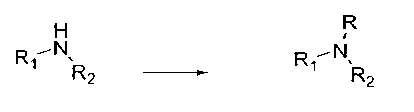

### General procedure:

To a mixture of appropriate amine (1 equiv.) and appropriate ketone or aldehyde (1.5 equiv.) were added acetic acid (1.7 mL/mmol) followed by sodium triacetoxyborohydride (1.5 equiv.) under a nitrogen atmosphere. The reaction mixture was stirred for 1h at room temperature. The solvent was removed *on vacuo* and the residue was dissolved in ethyl acetate. Aqueous 4M NaOH solution was added dropwise to adjust pH to 10. The mixture was extracted with ethyl acetate (3x14 mL/mmol). The combined organic layers were dried over anhydrous Na₂SO₄, filtered and concentrated under vacuum. The crude material was purified by flash chromatography on silica gel.

### Preparation of (S)-2-(dibenzylamino)propyl 4-(indolin-l-yl)piperidine-1-carboxylate (Compound n°7):

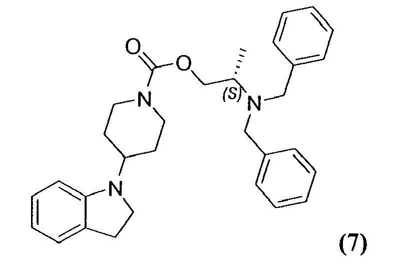

From indoline and (S)-2-(dibenzylamino)propyl 4-oxopiperidine-1-carboxylate
Flash chromatography on silica gel (cyclohexane/ethyl acetate 9/1)
Pale yellow solid (48%).
¹H NMR (400 MHz, CDCl₃) δ 7.37 (m, 4H, Ar), 7.29 (m, 4H, Ar), 7.16 (m, 2H, Ar), 7.07 (m, 2H, Ar), 6.63 (t, *J =* 7.2 Hz, 1H, Ar), 6.44 (d, *J =* 7.6 Hz, 1H, Ar), 4.28 (m, 3H, CHCH₂*CH₂*Nₚᵢₚ, *CH₂*O), 4.04 (m, 1H, *CH₂*O), 3.74 (d, *J* = 14.0 Hz, 2H, NCH₂Ph), 3.58 (m, 3H, N*CH₂*Ph, *CH*CH₂CH₂Nₚᵢₚ), 3.34 (m, 2H, CH₂*CH₂*N_{indoline}), 3.13 (m, 1H, *CH*Me), 2.96 (t, *J* = 8.4 Hz, 2H, *CH*₂CH₂N_{indotine}), 2.86 (m, 2H, CHCH₂*CH₂*Nₚᵢₚ), 1.86 (m, 2H, CH*CH₂*CH₂Nₚᵢₚ), 1.59 (m, 2H, CH*CH*₂CH₂Nₚᵢₚ), 1.09 (d, *J* = 4.0 Hz, 3H, Me).
¹³C NMR (100 MHz, CDCl₃) δ 155.4 (C=O), 150.4 (C_{Ar}), 140.2 (C_{Ar}), 130.1 (C_{Ar}), 128.5 (CH_{Ar}), 128.1 (CH_{Ar}), 127.2 (CH_{Ar}), 126.7 (CH_{Ar}), 124.6 (CH_{Ar}), 117.3 (CH_{Ar}), 106.8 (CH_{Ar}), 66.8 (*CH₂*O), 53.7 *(NCH₂Ph),* 53.1 (CHMe), 51.8 (*CH*CH₂CH₂Nₚᵢₚ), 46.8 (CH₂*CH₂*N_{indoline}), 43.7 (CHCH₂*CH₂*Nₚᵢₚ), 28.2 (*CH₂*CH₂N_{indoline}), 27.6 (CH*CH₂*CH₂Nₚᵢₚ), 11.2 (Me).
LC/MS (ES⁺) *m*/*z* 484.3 (M+H)⁺

### Preparation of (S)-2-(dibenzylamino)propyl 4-(3,4-dihvdroquinolin-1(2H)-yl)piperidine-1-carboxylate (Compound n°8):

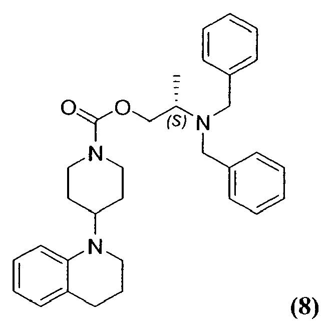

From 1,2,3,4-tetrahydroquinoline and (S)-2-(dibenzylamino)propyl 4-oxopiperidine-1-carboxylate
Flash chromatography on silica gel (methylene chloride)
Yellow oil (5%).
¹H NMR (400 MHz, CDCl₃) δ 7.37 (d, *J* = 7.2 Hz, 4H, Ar), 7.29 (t, *J =* 7.2 Hz, 4H, Ar), 7.19 (t, *J* = 7.2 Hz, 2H, Ar), 7.07 (t, *J* = 7.2 Hz, 1H, Ar), 6.97 (d, *J =* 7.2 Hz, I H, Ar), 6.68 (d, *J* = 8.4 Hz, 1H, Ar), 6.59 (d, *J =* 7.2 Hz, 1H, Ar), 4.32 (m, 2H, CHCH₂*CH₂*Nₚᵢₚ), 4.31 (dd, *J =* 7.2, 11.2 Hz, 1H, *CH₂*O), 4.05 (m, 1H, *CH₂*O)*,* 3.80 (m, 1H, *CH*CH₂CH₂Nₚᵢₚ), 3.75 (d, *J* = 14.0 Hz, 2H, N*CH₂*Ph), 3.55 (d, *J* = 14.0 Hz, 2H, N*CH₂*Ph), 3.15 (m, 3H, *CH*Me, CH₂CH₂*CH₂*N), 2.87 (m, 2H, CHCH₂*CH₂*Nₚᵢₚ), 2.73 (t, *J* = 6.4 Hz, 2H, *CH₂*CH₂CH₂N), 1.90 (m, 2H, CH₂*CH*₂CH₂N), 1.89-1.79 (m, 4H, CH*CH₂*CH₂Nₚᵢₚ), 1.09 (d, *J*= 6.8 Hz, 3H, Me).
¹³C NMR (100 MHz, CDCl₃) δ 155.3 (C=O), 144.9 (C_{Ar}), 140.3 (C_{Ar}), 129.4 (CH_{Ar}), 128.5 (CH_{Ar}), 128.1 (CH_{Ar}), 127.0 (CH_{Ar}), 126.7 (CH_{Ar}), 123.4 (C_{Ar}), 115.7 (CH_{Ar}), 110.5 (CH_{Ar}), 66.6 *(CH₂*O)*,* 54.8 (*CH*CH₂CH₂Nₚᵢₚ), 53.7 (N*CH₂*Ph), 51.8 (*CH*Me), 43.9 (CHCH₂*CH₂*Nₚᵢₚ), 41.9 (CH₂CH₂*CH₂*N), 28.5 (*CH₂*CH₂CH₂N), 28.3 (CH*CH₂*CH₂Nₚᵢₚ), 22.4 (CH₂*CH₂*CH₂N), 11.9 (Me).
LC/MS (ES⁺) *m*/*z* 498.3 (M+H)⁺

### Preparation of 1-(1-(4-(dibenzylamino)butyl)piperidin-4-yl)-1H-benzo[d]imidazol-2(3H)-one (Compound n°9):

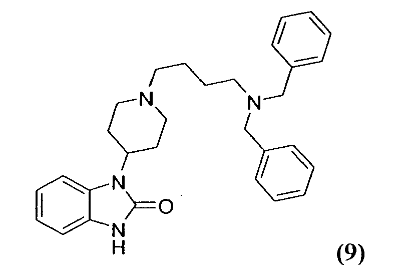

From 4-(2-keto-1-benzimidazolinyl)-piperidine and 4-dibenzylaminobutyraldehyde
Colorless oil (50%).
Flash chromatography on silica gel (cyclohexane/ ethyl acetate 2/8)
¹H NMR (400 MHz, CDCl₃, ppm) δ 9.10 (s, 1H), 7.38 (d, *J* = 7.3 Hz, 4H, *Ph),* 7.32 (t, *J* = 7.4 Hz, 4H, *Ph*), 7.24 (m, 3H, *Ph*), 7.07 (m, 3H, *Ph*), 4.38 (m, 1H, *CH*CH₂CH₂N), 3.57 (s, 4H, N*CH₂*Ph), 3.04 (m, 2H, CHCH₂*CH₂*N), 2.45 (t, *J* = 6.2 Hz, 2H, N*CH₂*(CH₂)₂CH₂NBn₂), 2.32 (m, 2H, CHCH₂*CH₂*N), 2.11 (m, 2H, CH*CH₂*CH₂N), 1.82 (m, 2H, CH*CH₂*CH₂N), 1.68 (m, 2H, NCH₂(CH₂)₂*CH₂*NBn₂), 1.55 (m, 2H, NCH₂(*CH₂*)₂CH₂NBn₂), 1.26 (m, 2H, NCH₂(*CH₂*)₂CH₂NBn₂)
LC/MS (ES⁺) *m*/*z* 469.3 (M+H)⁺

### 5) Indoline oxydation:

### Preparation of (S)-2-(dibenzylamino)propyl 4-(1H-indol-1-yl)piperidine-1-carboxylate (Compound n°10):

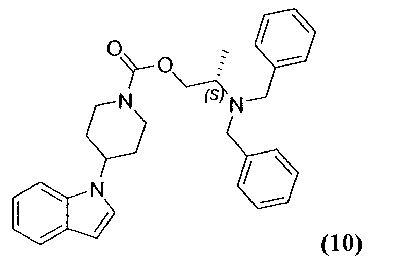

To a stirred solution of (S)-2-(dibenzylamino)propyl 4-(indolin-1-yl)piperidine-1-carboxylate (25 mg, 0.05 mmol) in THF (0.2 mL) cooled to 0°C under N₂ was added DDQ (16 mg, 0.07 mmol) in one portion. The reaction mixture was stirred for 1h30 at 0°C and a saturated aqueous NaHCO₃ solution (1 mL) was added. The mixture was extracted with ethyl acetate (3x5 mL). The combined organic layers were dried over anhydrous Na₂SO₄, filtered and concentrated under vacuum. The crude material was purified by flash chromatography (silica gel, cyclohexane/ethyl acetate 8/2) to afford a brown oil (10 mg, 40%).
¹H NMR (400 MHz, CDCl₃) δ 7.66 (d, *J=* 7.6 Hz, 1H, Ar), 7.37 (m, 4H, Ar), 7.29-7.20 (m, 9H, Ar), 7.11 (m, 1H, Ar), 6.55 (d, *J =* 2.8 Hz, 1H, Ar), 4.42 (m, 3H, CHCH₂*CH₂*Nₚᵢₚ *CH*CH₂CH₂Nₚᵢₚ), 4.25 (dd, *J =* 7.2, 11.2 Hz, 1H, *CH₂*O), 4.08 (m, 1H, *CH₂*O), 3.77 (d, *J =* 14.0 Hz, 2H, N*CH₂*Ph), 3.56 (d, *J* = 14.0 Hz, 2H, N*CH₂*Ph), 3.15 (m, 1H, *CH*Me), 3.00 (m, 2H, CHCH₂*CH₂*Nₚᵢₚ), 2.12 (m, 2H, CH*CH₂*CH₂Nₚᵢₚ), 1.95 (m, 2H, CH*CH₂*CH₂Nₚᵢₚ), 1.11 (d, *J*= 6.8 Hz, 3H, Me).
¹³C NMR (100 MHz, CDCl₃) δ 155.3 (C=O), 148.4 (C_{Ar}), 140.2 (C_{Ar}), 135.8 (C_{Ar}), 128.5 (CH_{Ar}), 128.1 (CH_{Ar}), 126.8 (CH_{Ar}), 123.7 (CH_{Ar}), 121.4 (CH_{Ar}), 121.1 (CH_{Ar}), 119.6 (CH_{Ar}), 109.1 (CH_{Ar}), 101.8 (CH_{Ar}), 66.9 (*CH₂*O), 53.7 (N*CH₂*Ph), 53.2 (*CH*CH₂CH₂N_{piP}), 51.7 (*CH*Me)*,* 43.5 (CHCH₂*CH₂*Nₚᵢₚ), 26.9 (CH*CH₂*CH₂Nₚᵢₚ), 11.0 (Me).
LC/MS (ES⁺) m/z 482.2 (M+H)⁺

### 6) Cyclisation (with carbonyldiimidazole)

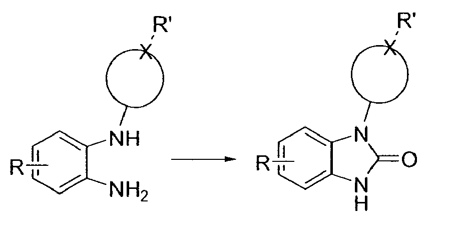

### General procedure:

To a stirred solution of appropriate diamine (1 equiv.) in THF (10 mL/mmol) was added CDI (1.5 equiv) under N₂ atmosphere. The reaction mixture was stirred overnight at room temperature. The solvent was removed *on vacuo* and the residue was purified by flash chromatography on silica gel.

### Preparation of (S)-2-(dibenzylamino)propyl 4-(2,3-dihydro-2-oxoimidazo[4,5-b]pyridin-1-yl)piperidine-1-carboxylate (Compound n°11):

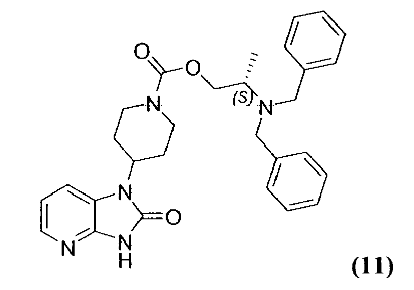

From (S)-2-(dibenzylamino)propyl 4-(2-aminopyridin-3-ylamino)piperidine-1-carboxylate
Stirred for 2 days at room temperature.
Flash chromatography on silica gel (methylene chloride/ acetone 7/3)
Colorless oil (57%).
¹H NMR (400 MHz, CDCl₃) δ 10.34 (bs, 1H, NH), 8.07 (d, *J* = 5.2 Hz, 1H, Ar), 7.37 (m, 4H, Ar), 7.28-7.21 (m, 7H, Ar), 6.95 (t, *J* = 7.6 Hz, 1H, Ar), 4.60 (m, 1H, *CH*CH₂CH₂Nₚᵢₚ), 4.40 (m, 2H, CHCH₂*CH₂*Nₚᵢₚ), 4.27 (m, 1H, *CH₂*O), 4.08 *(dd, J =* 5.2, 10.4 Hz, I H, *CH₂*O), 3.76 (d, *J* = 13.8 Hz, 2H, N*CH₂*Ph), 3.56 (d, *J* = 13.8 Hz, 2H, N*CH₂*Ph), 3.14 (m, 1H, *CH*Me), 2.94 (m, 2H, CHCH₂*CH₂*N_{PiP}), 2.24 (m, 2H, CH*CH₂*CH₂Nₚᵢₚ), 1.92 (m, 2H, CH*CH₂*CH₂N_{Pip}), 1.11 (d, *J* = 6.4 Hz, 3H, Me).
¹³C NMR (100 MHz, CDCl₃) δ 155.1 (C=O), 153.3 (C=O), 143.1 (C_{Ar}), 140.2 (C_{Ar}), 140.1 (CH_{Ar}), 133.1 (C_{Ar}), 128.6 (CH_{Ar}), 128.2 (CH_{Ar}), 126.8 (CH_{Ar}), 116.8 (CH_{Ar}), 115.4 (CH_{Ar}), 66.9 (*CH₂*O), 53.4 (N*CH₂*Ph), 51.6 (*CH*Me), 50.1 (*CH*CH₂CH₂Nₚᵢₚ), 43.1 (CHCH₂*CH₂*Nₚᵢₚ), 28.9 (CH*CH₂*CH₂Nₚᵢₚ), 11.9 (Me).
LC/MS (ES⁺) *m*/*z* 500.2 (M+H)⁺

### Preparation of (S)-2-(dibenzylamino)propyl 4-(1,2-dihydro-2-oxonaphtho[2,3-d]imidazol-3-vl)piperidine-1-carboxylate (Compound n°12):

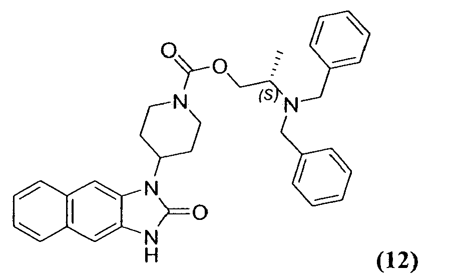

From (S)-2-(dibenzylamino)propyl 4-(2-aminonaphthalen-3-ylamino)piperidine-1-carboxylate
Preparative TLC (silica gel) (cyclohexane/ ethyl acetate 5/5)
Green oil (38%).
¹H NMR (400 MHz, CDCl₃) δ 9.34 (bs, 1H, NH), 7.78 (m, 2H, Ar), 7.40 (m, 8H, Ar), 7.37 (m, 4H, Ar), 7.20 (m, 2H, Ar), 4.58 (m, 1H, *CH*CH₂CH₂Nₚᵢₚ), 4.46 (m, 2H, CHCH₂*CH₂*Nₚᵢₚ), 4.31 (dd, *J* = 7.6, 10.8 Hz, 1H, *CH₂*O), 4.11 (dd, *J =* 5.6, 10.8 Hz, 1H, *CH₂*O), 3.77 (d, *J =* 13.8 Hz, 2H, N*CH₂*Ph), 3.60 (d, *J* = 13.8 Hz, 2H, N*CH₂*Ph), 3.18 (m, 1H, *CH*Me), 3.00 (m, 2H, CHCH₂*CH₂*Nₚᵢₚ), 2.54 (m, 2H, CH*CH₂*CH₂Nₚᵢₚ), 1.92 (m, 2H, CH*CH₂*CH₂Nₚᵢₚ), 1.13 *(d, J =* 6.4 Hz, 3H, Me).
¹³C NMR (100 MHz, CDCl₃) δ 155.6 (C=O), 155.3 (C=O), 140.2 (C_{Ar}), 129.9 (C_{Ar}), 129.4 (C_{Ar}), 128.5 (CH_{Ar}), 128.4 (C_{Ar}), 128.2 (CH_{Ar}), 127.2 (CH_{Ar}), 126.9 (C_{Ar}), 126.8 (2xCH_{Ar}), 124.3 (CH_{Ar}), 124.3 (CH_{Ar}), 105.4 (CH_{Ar}), 105.1 (CH_{Ar}), 66.8 (*CH₂*O), 53.6 (N*CH₂*Ph), 51.9 (*CH*Me), 50.9 (*CH*CH₂CH₂Nₚᵢₚ), 43.6 (CHCH₂*CH₂*N_{PiP}), 28.9 (CH*CH₂*H₂Nₚᵢₚ).
LC/MS (ES⁺) *m*/*z* 549.2 (M+H)⁺

### Preparation of (S)-2-(dibenzylamino)propyl 4-(1,2-dihydro-2-oxobenzo[d]imidazol-3-yl)azepane-1-carboxylate (Compound n°13):

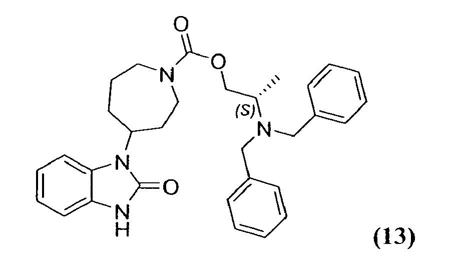

From (S)-2-(dibenzylamino)propyl 4-(2-aminophenylamino)azepane-1-carboxylate.
Flash chromatography on silica gel (cyclohexane/ ethyl acetate 5/5)
Colorless oil (66%).
¹H NMR (400 MHz, CDCl₃) δ 9.37 (bd, 1H, NH), 7.39-7.21 (m, 10H, Ar), 7.04 (m, 4H, Ar), 4.46 (m, 1H, *CH*CH₂CH₂N), 4.28 (m, 1H, *CH₂*O), 4.12 (m, 1H, *CH₂*O), 3.72 (m, 3H, N*CH*₂Ph, CH₂CH₂*CH₂*N), 3.61 (m, 4H, N*CH₂*Ph, CHCH₂*CH₂*N), 3.33 (m, 1H, CH₂CH₂*CH₂*N), 3.14 (m, 1H, *CH*Me), 2.36 (m, 2H, CH*CH₂*CH₂N), 2.05 (m, 3H, *CH₂*CH₂CH₂N, CH₂*CH₂*CH₂N), 1.72 (m, 1H, CH₂*CH₂*CH₂N), 1.10 (m, 3H, Me).
¹³C NMR (100 MHz, CDCl₃) δ 156.2 (C=O), 154.4 (C=O), 140.3 (140.2) (C_{Ar}), 128.9 (128.8) (C_{Ar}), 128.6 (128.5) (CH_{Ar}), 128.1 (CH_{Ar}), 127.9 (127.8) (C_{Ar}), 126.8 (126.7) (CH_{Ar}), 121.2 (CH_{Ar}), 121.1 (121.1) (CH_{Ar}), 109.7 (109.6) (CH_{Ar}), 109.3 (109.2) (CH_{Ar}), 66.8 (*CH₂*O), 53.8 (N*CH*₂Ph), 53.7 (*CH*CH₂CH₂N), 51.9 (*CH*Me), 46.5 (46.6) (CHCH₂*CH₂*N), 43.0 (CH₂CH₂*CH₂*N), 33.0 (32.9) (CH*CH₂*CH₂N), 30.1 (*CH₂*CH₂CH₂N), 26.8 (CH₂*CH₂*CH₂N), 11.6 (Me).
LC/MS (ES⁺) m/z 513.3 (M+H)⁺

### 7) Cyclization (TFA) / NBoc Deprotection / Carbamate preparation:

### Preparation of (S)-2-(dibenzylamino)propyl-4-(2-(trifluoromethyl)-1H-benzo[d]imidazol-1-yl)piperidine-1-carboxylate (Compound n°14):

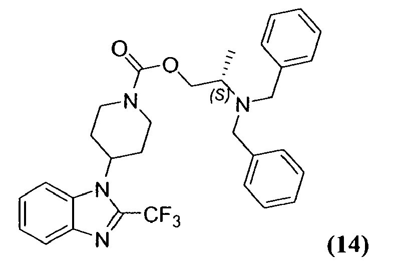

To a solution of tert-butyl 4-(2-aminophenylamino)piperidine-1-carboxylate (70 mg, 0.24 mmol) in CH₂Cl₂ (6 mL) cooled to 0°C under N₂ was added TFA (1.2 mL, 15.6 mmol). The reaction mixture was stirred overnight at room temperature. The solvent was removed *on vacuo* and the residue was triturated with Et₂0 to give a pale red solid. To a stirred solution of (S)-2-(dibenzylamino)propan-l-ol (41 mg, 0.16 mmol) in THF (0.4 mL) cooled at 0°C was added CDI (28 mg, 0.18 mmol) under N₂ atmosphere. The reaction mixture was stirred for 2 h at room temperature. Triethylamine (32 µL, 0.24 mmol) and the I-pipen'din-4-yi-2-trifluoromethyl-1*H*-benzimidazole trifluoroacetate salt were added and the reaction mixture was stirred for 72h at room temperature. The solvent was removed *on vacuo* and the residue was purified by flash chromatography on silica gel (cyclohexane/ethyl acetate 8/2) to afford a colorless oil (44 mg, 55%):
¹H NMR (400 MHz, CDCl₃) δ 7.91 (dd, *J* = 9.2, 4.0 Hz, 1H, Ar), 7.57 (m, 1H, Ar), 7.38 (m, 6H, Ar), 7.29 (m, 4H, Ar), 7.21 (t, *J* = 7.2 Hz, 2H, Ar), 4.62 (m, 1H, *CH*CH₂CH₂Nₚᵢₚ), 4.46 (m, 2H, CHCH₂CH₂Nₚᵢₚ), 4.30 (dd, *J* = 7.2, 10.8 Hz, 1H, *CH₂0),* 4.09 (dd, *J =* 5.6, 10.8 Hz, 1H, *CH₂0),* 3.78 (d, *J* = 14.0 Hz, 2H, N*CH₂*Ph), 3.57 (d, *J* = 14.0 Hz, 2H, N*CH₂*Ph), 3.16 (m, 1H, *CHMe),* 2.95 (m, 2H, CHCH₂*CH*₂Nₚᵢₚ), 2.52 (m, 2H, CH*CH₂*CH₂Nₚᵢₚ), 2.08 (bd, *J* = 11.2 Hz, 2H, CH*CH₂*CH₂Nₚᵢₚ), 1.12 (d, J= 6.8 Hz, 3H, Me).
¹³C NMR (100 MHz, CDC1₃) δ 155.1 (C=O), 140.2 (C_{Bn}), 133.7 (C_{Ar}), 128.5 (CH_{Bn}), 128.1 (CH_{Bn}), 126.8 (CH_{Bn}), 125.2 (CH_{Ar}), 123.6 (CH_{Ar}), 122.3 (CH_{Ar}), 116.2 (C_{Ar}), 113.0 (CH_{Ar}), 67.1 (*CH₂*O), 55.8 (*CH*CH₂CH₂Nₚᵢₚ), 53.7 (N*CH*₂Ph), 51.9 (*CH*Me), 43.4 (CHCH₂*CH₂*Nₚᵢₚ), 30.3 (CH*CH₂*CH₂Nₚᵢₚ), 11.2 (Me).
LC/MS (ES⁺) *m*/*z* 551.1 (M+H)⁺

### 8) Cyclization (AcOH) / NBoc Deprotection / Carbamate preparation:

### Preparation of (S)-2-(dibenzylamino)propyl 4-(2-methyl-1H-benzoldlimidazol-1 - yl)piperidine-1-carboxylate (Compound n°15):

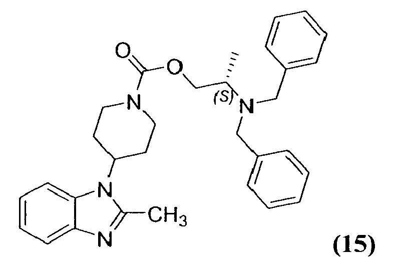

A stirred solution of tert-butyl 4-(2-aminophenylamino)pipen'dine-1-carboxylate (70 mg, 0.24 mmol) in acetic acid (0.8 mL) under N₂ was refluxed overnight. The solvent was removed *on vacuo.* A 4N aqueous NaOH solution was introduced to pH 8 and the mixture was extracted with ethyl acetate (3x10 mL). The combined organic layers were dried over anhydrous Na₂SO₄, filtered and concentrated under vacuum to give a pale yellow oil. A stirred solution of the crude material in 6N HCl (1 mL) was refluxed for 3h. The solvent was removed on *vacuo* and the residue was triturated with acetone to give a pink solid. To a stirred solution of (S)-2-(dibenzylamino)propan-1-ol (56 mg, 0.22 mmol) in THF (1 mL) at 0°C was added CDI (39 mg, 0.24 mmol) under N₂ atmosphere. The reaction mixture was stirred for 2h at room temperature. Triethylamine (67 µL, 0.48 mmol) and the crude salt were added and the reaction mixture was stirred for 72h at room temperature. The solvent was removed on *vacuo* and the residue was purified by flash chromatography on silica gel (ethyl acetate) to afford a pale yellow oil (25 mg, 23%).
¹H NMR (400 MHz, CDCl₃) δ 7.69 (d, *J* = 7.6 Hz, 1H, Ar), 7.38 (m, 5H, Ar), 7.28 (t, *J* = 7.2 Hz, 4H, Ar), 7.20 (m, 4H, Ar), 4.45-4.33 (m, 3H, *CH*CH₂CH₂Nₚᵢₚ, CHCH₂*CH₂*Nₚᵢₚ), 4.29 (dd, *J =* 7.6, 11.2 Hz, 1H, *CH₂*O), 4.08 (dd, *J* = 5.6, 11.2 Hz, 1H, *CH₂*O), 3.78 (d, *J* = 13.8 Hz, 2H, N*CH₂*Ph), 3.56 (d, *J* = 13.8 Hz, 2H, N*CH₂*Ph), 3.42 (m, 1H, *CH*Me), 2.94 (m, 2H, CHCH₂*CH₂*Nₚᵢₚ), 2.65 (s, 3H, Me), 2.46 (m, 2H, CH*CH₂*CH₂Nₚᵢₚ), 1.94 (bd, *J* = 10.8 Hz, 2H, CH*CH₂*CH₂Nₚᵢₚ), 1.07 (d, *J* = 6.8 Hz, 3H, Me).
¹³C NMR (100 MHz, CDCl₃) δ 155.1 (C=O), 150.7 (C=N), 142.9 (C_{Ar}), 140.2 (C_{Bn}), 133.5 (C_{Ar}), 128.5 (CH_{Bn}), 128.1 (CH_{Bn}), 126.8 (CH_{Bn}), 122.1 (CH_{Ar}), 121.8 (CH_{Ar}), 119.4 (CH_{Ar}), 111.1 (CH_{Ar}), 67.0 (*CH₂*O), 54.6 (*CH*CH₂CH₂Nₚᵢₚ), 53.7 (N*CH₂*Ph), 51.9 (*CH*Me), 43.7 (CHCH₂*CH₂*Nₚᵢₚ), 30.2 (CH*CH₂*CH₂Nₚᵢₚ), 15.1 (Me), 11.2 (Me).
LC/MS (ES⁺) m/z 497.3 (M+H)⁺

### 9) Cyclisation (CDI) / NBoc Deprotection / Carbamate preparation:

### General procedure:

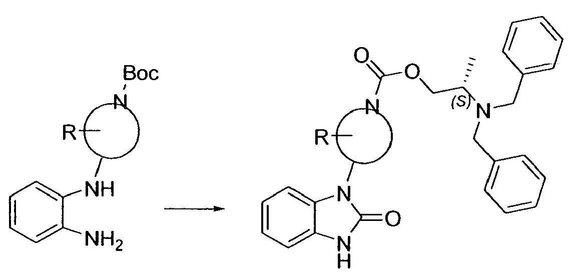

To a stirred solution of appropriate diamine (1 equiv.) in THF (10mL/mmol) was added CDI (1.5 equiv) under N₂ atmosphere. The reaction mixture was stirred overnight at room temperature. The solvent was removed *on vacuo*. Ethyl acetate was added and the organic layer was washed with water, dried over Na₂SO₄ and concentrated *in vacuo*. To a solution of the crude material (1 equiv.) in CH₂Cl₂ (2.5 mL/mmol) cooled to 0°C under N₂ was added TFA (8 equiv.). The reaction mixture was stirred for 2h at room temperature. The solvent was removed *on vacuo* and the residue was triturated with Et₂O to give a solid. To a stirred solution of (S)-2-(dibenzylamino)propan-1-ol (1 equiv.) in THF cooled to 0°C was added CDI (1.1 equiv.) under N₂ atmosphere. The reaction mixture was stirred for 2h at room temperature. Triethylamine (1.6 equiv.) and the crude salt (1 equiv.) were added and the reaction mixture was stirred for 72h at room temperature. The solvent was removed *on vacuo* and the residue was purified by flash chromatography on silica gel.

### Preparation of (S)-2-(dibenzylamino)propyl 3-(1,2-dihydro-2-oxobenzo[d]imidazol-3-yl)piperidine-1-carboxylate (Compound n°16):

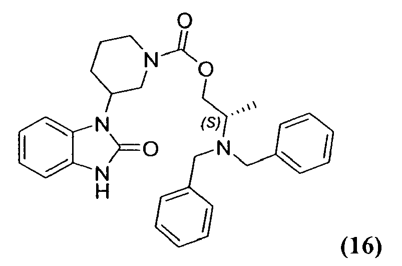

From tert-butyl 3-(2-aminophenylamino)piperidine-1-carboxylate
Flash chromatography on silica gel (cyclohexane/ ethyl acetate 7/3)
Pale yellow solid (77%).
¹H NMR (400 MHz, CDCl₃) δ 10.42-10.31 (bs, 1H, NH), 7.40-7.26 (m, 8H, Ar), 7.22-7.07 (m, 6H, Ar), 4.31 (m, 4H, *CH*CH₂N, CH*CH₂*N, *CH₂*O), 4.07 (m, 2H, *CH₂*O, CH₂CH₂*CH₂*N), 3.74 (m, 2H, N*CH₂*Ph), 3.56 (m, 3H, N*CH₂*Ph, CH₂CH₂*CH₂*N), 3.13 (m, 1H, *CH*Me), 2.86 (bs, 1H, *CH₂*CH₂CH₂N), 2.51 (bs, 1H, *CH₂*CH₂CH₂N), 1.92 (bs, 1H, CH₂*CH₂*CH₂N), 1.86 (m, 1H, CH₂*CH₂*CH₂N), 1.10 (bs, 3H, Me).
¹³C NMR (100 MHz, CDCl₃) δ 155.4 (C=O), 153.2 (C=O), 140.2 (C_{Ar}), 129.1 (C_{Ar}), 128.5 (CH_{Ar}), 128.1 (CH_{Ar}), 128.0 (C_{Ar}), 126.7 (CH_{Ar}), 121.4 (CH_{Ar}), 121.1 (CH_{Ar}), 109.9 (CH_{Ar}), 108.5 (CH_{Ar}), 66.9 (*CH₂*O), 53.7 (N*CH₂*Ph), 51.8 (*CH*Me), 50.2 (*CH*CH₂N), 45.8 (CH*CH₂*N), 43.9 (CH₂CH₂*CH₂*N), 28.0 (*CH₂*CH₂CH₂N), 27.8 (CH₂*CH₂*CH₂N), 11.3 (Me).
LC/MS (ES⁺) *m*/*z* 499.1 (M+H)⁺

### 10) Cyclisation (CDI) / NBn Deprotection / Carbamate preparation:

### General procedure

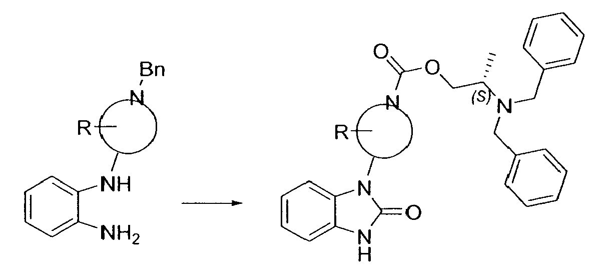

To a stirred solution of appropriate diamine (1 equiv.) in THF (10mL/mmol) was added CDI (1.5 equiv) under N₂ atmosphere. The reaction mixture was stirred overnight at room temperature. The solvent was removed on *vacuo.* Ethyl acetate was added and the organic layer was washed with water, dried over Na₂SO₄ and concentrated in *vacuo.* A solution of the crude material (1 equiv.) in EtOH (10 mL/mmol) was submitted to hydrogenation in the presence of acetic acid (6 equiv.) and 10% Pd/C (100 mg/ mmol) at room pressure and temperature for 24 h. The reaction mixture was filtered through a pad of Celite. The filtrate was concentrated under *vacuo.* To a stirred solution of (S)-2-(dibenzylamino)propan-l-ol (1 equiv.) in THF cooled to 0°C was added CDI (1.1 equiv.) under N₂ atmosphere. The reaction mixture was stirred for 2h at room temperature. Triethylamine (1.6 equiv.) and the crude salt (1 equiv.) were added and the reaction mixture was stirred for 72h at room temperature. The solvent was removed on *vacuo* and the residue was purified by flash chromatography on silica gel.

### Preparation of (S)-2-(dibenzylamino)propyl 3-(1,2-dihydro-2-oxobenzo[d]imidazol-3-yl)pyrrolidine-1-carboxylate (Compound n°17):

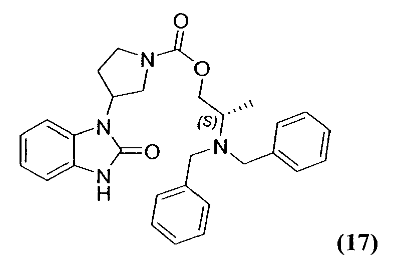

From N-1-(1-benzylpyrroildin-3-yl)bcnzene-1,2-diamine
Flash chromatography on silica gel (cyclohexane/ ethyl acetate 5/5)
Yellow oil (12%).
¹H NMR (400 MHz, CDCl₃) δ 9.07 (bd, 1H, NH), 7.38-7.16 (m, 10H, Ar), 7.09 (m, 4H, Ar), 5.15 (m, 1H, *CH*CH₂N), 4.27 (m, 1H, *CH₂*O), 4.10 (dd, *J* = 5.6, 10.8 Hz, 1H, *CH₂*O), 3.79 (m, 5H, N*CH₂*Ph, CH*CH₂*N, CH₂*CH₂*N), 3.56 (m, 3H, N*CH₂*Ph, CH₂*CH₂*N), 3.10 (m, 1H, *CH*Me), 2.63 (m, 1H, *CH₂*CH₂N), 2.29 (m, 1H, *CH₂*CH₂N), 0.93 (bd, 3H, Me).
LC/MS (ES⁺) m/z 485.2 (M+H)⁺

### Preparation of 1-(S)-2-(dibenzylamino)propyl 3-ethyl 4-(1,2-dihvdro-2-oxobenzo[d]imidazol-3-yl)piperidine-1,3-dicarboxylate (Compound n°18):

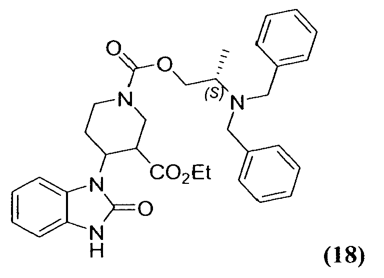

From ethyl 4-(2-aminophcnylainino)-1-bcnzylpipcridinc-3-carboxylate.
Flash chromatography on silica gel (cyclohexane/ ethyl acetate 5/5)
Yellow solid (19%).
¹H NMR (400 MHz, CDCl₃) δ 9.51 (bs, 1H, NH), 7.47 (m, 4H, Ar), 7.21 (m, 7H, Ar), 7.04 (m, 3H, Ar), 4.65 (m, 1H, *CH*CH₂CH₂N), 4.47 (m, 2H, *CH₂*O, CHCH*CH₂*N), 4.31 (m, 2H, *CH₂*O, CHCH₂*CH₂*N), 3.87 (m, 3H, N*CH₂*Ph, O*CH₂*CH₃), 3.74 (m, 3H, N*CH₂*Ph, O*CH₂*CH₃), 3.26 (m, 2H, *CH*Me, CHCH*CH₂*N), 3.06 (m, 3H, CHCH₂*CH₂*N, CH*CH₂*CH₂N), 1.94 (m, 1H, CH*CH*CH₂N), 1.22 (m, 3H, Me), 0.89 (bq, 1.5H, OCH₂*CH₃*), 0.74 (m, 1.5H, OCH₂*CH₃*).
LC/MS (ES⁺) *m*/*z* 571.3 (M+H)⁺

### 11) NBoc Deprotection / Carbamate preparation:

### General procedure

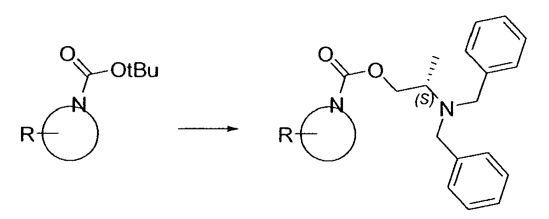

To a stirred solution of appropriate *N*-*tert*-butyloxycarbonyl derivative (1 equiv.) in CH₂Cl₂ (2.5 mL/mmol) cooled to 0°C under N₂ was added TFA (8 equiv.). The reaction mixture was stirred for 2h at room temperature. The solvent was removed on *vacuo* and the residue was triturated with Et₂O to give a solid. To a stirred solution of (S)-2-(dibenzylamino)propan-1-ol (1 equiv.) in THF (2 mL/mmol) at 0°C was added CDI (1.1 equiv.) under N₂ atmosphere. The reaction mixture was stirred for 2h at room temperature. Triethylamine (1.8 equiv.) and the crude salt (1.3 equiv.) were added and the reaction mixture was stirred for 72h at room temperature. The solvent was removed on *vacuo* and the residue was purified by flash chromatography on silica gel.

### Preparation of (S)-2-(dibenzylamino)propyl 4-oxopiperidine-1-carboxylate (Intermediate):

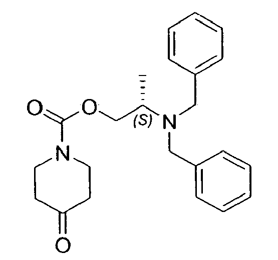

From *N*-*tert*-butyloxycarbonylpiperid-4-one and (S)-2-(dibenzylamino)propan-1-ol.
Flash chromatography on silica gel (methylene chloride/ acetone 97/3).
White solid (31%).
¹H NMR (400 MHz, CDCl₃) δ 7.35 *(d, J =* 7.2 Hz, 4H, Ar), 7.28 (t, *J* = 7.2 Hz, 4H, Ar), 7.18 (t, *J* = 7.2 Hz, 2H, Ar), 4.27 (dd, *J* = 7.6, 11.2 Hz, 1H, *CH₂*O), 4.06 (dd, *J =* 5.6, 11.2 Hz, 1H, *CH₂*O), 3.75 (m, 6H, N*CH₂*Ph, CH₂*CH₂*Nₚᵢₚ), 3.54 (d, *J* = 13.6 Hz, 2H, N*CH₂*Ph), 3.14 (m, 1H, *CH*Me), 2.45 (m, 4H, *CH₂*CH₂Nₚᵢₚ), 1.10 (d, *J* = 6.8 Hz, 3H, Me).
LC/MS (ES⁺) m/z 381.1(M+H)⁺

### Preparation of (S)-2-(dibenzylamino)propyl 4-hydroxypiperidine-1-carboxylate (Intermediate):

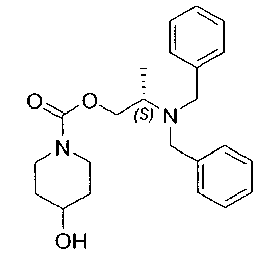

From *N*-*tert*-butyloxy-carbonyl-4-hydroxypiperide and (S)-2-(dibenzylamino)propan-l-ol
Flash chromatography on silica gel (cyclohexane/ethyl acetate: 7/3)
Colorless oil (95%).
¹H NMR (400 MHz, CDCl₃) δ 7.37 *(d, J =* 7.2 Hz, 4H), 7.29 (t, *J* = 7.4 Hz, 4H), 7.23 (m, 2H), 4.22 (dd, *J* = 10.9, 7.5 Hz, 1H, *CH*CH₂CH₂N), 4.03 (dd, *J* = 10.9, 5.5 Hz, 1H, OCH₂), 3.90 (m, 3H, OCH₂, CHCH₂*CH₂*N), 3.74 (d, *J* = 13.9 Hz, 2H, N*CH₂*Ph), 3.56 (d, *J* = 13.9 Hz, 2H, N*CH₂*Ph), 3.15 (m, 3H, *CH*CH₃, CHCH₂*CH₂*N), 1.89 (d, *J* = 9.0 Hz, 2H, CH*CH₂*CH₂N), 1.61-1.44 (m, 2H, CH*CH₂*CH₂N), 1.08 (d, *J* = 6.8 Hz, 3H, CH*CH₃*)
LC/MS (ES⁺) m/z 383.0(M+H)⁺

### Preparation of (S)-2-(dibenzylamino)propyl 4-((1,2-dihydro-2-oxobenzo[d]imidazol-3-yl)methyl)piperidine-1-carboxylate (Compound n°19):

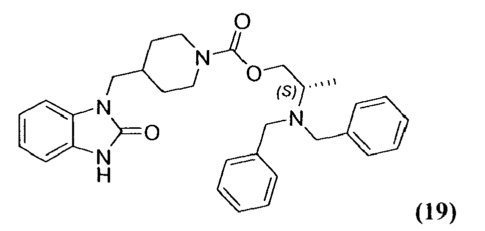

From tert-butyl 4-((1,2-dihydro-2-oxobenzo[d]imidazol-3-yl)methyl)piperidine-1-carboxylate.
THF (10 mL/mmol) was added for the second step to improve solubility.
Flash chromatography on silica gel (cyclohexane/ ethyl acetate 4/6)
Pale yellow oil (10%).
¹H NMR (400 MHz, CDCl₃) δ 9.05 (bs, 1H, NH), 7.36 (d, *J* = 7.2 Hz, 4H, Bn), 7.27 (m, 4H, Bn), 7.18 (m, 2H, Bn), 7.08 (m, 3H, Ar), 6.98 (m, 1H, Ar), 4.21 (m, 3H, CHCH₂*CH₂*Nₚᵢₚ, *CH₂*O), 4.02 (dd, *J=* 5.6, 10.8 Hz, 1H, *CH₂*O), 3.77 (m, 4H, N*CH₂*CH, N*CH₂*Ph), 3.55 (d, *J =* 14.0 Hz, 2H, N*CH₂*Ph), 3.10 (m, 1H, *CH*Me), 2.74 (m, 2H, CHCH₂*CH₂*Nₚᵢₚ), 2.12 (m, 1H, *CH*CH₂CH₂Nₚᵢₚ), 1.74 (m, 2H, CH*CH₂*CH₂Nₚᵢₚ), 1.25 (m, 2H, CH*CH₂*CH₂Nₚᵢₚ), 1.07 (d, *J* = 6.9 Hz, 3H, Me).
LC/MS (ES⁺) *m*/*z* 513.2 (M+H)⁺

### Preparation of (S)-2-(dibenzylamino)propyl 4-(2-(1,2-dihvdro-2-oxobenzo[d]imidazol-3-yl)ethyl)piperidine-1-carboxylate (Compound n°20):

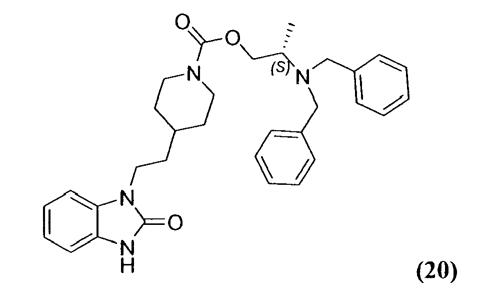

From tert-butyl 4-(2-(1,2-dihydro-2-oxobenzo[d]imidazol-3-yl)ethyl)piperidine-1-carboxylate.
THF (10 mL/mmol) was added for the second step to improve solubility.
Flash chromatography on silica gel (cyclohexane/ ethyl acetate 5/5)
Pale yellow oil (50%).
¹H NMR (400 MHz, CDCl₃) δ 9.79 (bs, 1H, NH), 7.37 (d, *J* = 7.2 Hz, 4H, Bn), 7.36 (t, *J* = 7.2 Hz, 4H, Bn), 7.21 (t, *J* = 7.2 Hz, 2H, Bn), 7.07 (m, 3H, Ar), 6.97 (d, *J* = 7.2 Hz, 1H, Ar), 4.22 (m, 3H, CHCH₂*CH₂*Nₚᵢₚ, *CH₂*O), 4.03 (m, 1H, *CH₂*O), 3.96 (t, *J* = 7.2 Hz, 2H, N*CH₂*CH₂CH), 3.74 (d, *J* = 14.0 Hz, 2H, N*CH₂*Ph), 3.57 (d, *J* = 14.0 Hz, 2H, N*CH₂*Ph), 3.11 (m, 1H, *CH*Me), 2.77 (m, 2H, CHCH₂*CH₂*N_{PiP}), 1.84 (m, 2H, CH*CH₂*CH₂Nₚᵢₚ), 1.75 (m, 2H, NCH₂*CH₂*CH), 1.55 (m, 1H, *CH*CH₂CH₂Nₚᵢₚ), 1.28 (m, 2H, CH*CH₂*CH₂Nₚᵢₚ), 1.08 (d, *J* = 6.8 Hz, 3H).
¹³C NMR (100 MHz, CDCl₃) δ 155.4 (C=O), 155.3 (C=O), 140.3 (C_{Ar}), 130.1 (C_{Ar}), 128.5 (CH_{Ar}), 128.1 (CH_{Ar}), 128.0 (C_{Ar}), 126.8 (CH_{Ar}), 121.6 (CH_{Ar}), 121.3 (CH_{Ar}), 109.6 (CH_{Ar}), 107.7 (CH_{Ar}), 66.5 (*CH₂*O), 53.7 (N*CH₂*Ph), 51.9 (*CH*Me), 44.0 (CHCH₂C*H₂*Nₚᵢₚ), 38.3 (N*CH₂*CH₂CH), 34.8 (NCH₂*CH₂*CH), 33.5 (*CH*CH₂CH₂Nₚᵢₚ), 27.0 (CH*CH*₂CH₂Nₚᵢₚ), 11.3 (Me).
LC/MS (ES⁺) *m*/*z* 527.2 (M+H)⁺

### Preparation of (S)-2-(dibenzylamino)propyl 4-(2-oxoindolin-3-ylidene)piperidine-1-carboxylate (Compound n°21):

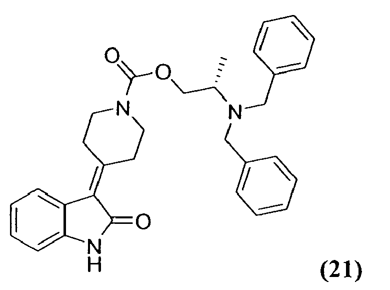

From 4-(2-Oxo-1,2-dihydro-indol-3-ylidene)-N-tert-butyloxycarbonyl piperidine and (S)-2-(dibenzylamino)propan-1-ol.
White solid (7%).
HPLC preparative
¹H NMR (400 MHz, CDCl₃) δ ppm 7.96 (s, 1H, *NH*), 7.51 (d, J = 7.6 Hz, 1H, *Ph*), 7.38 (d, *J* = 7.4 Hz, 4H, *Ph*), 7.30 (m, 4H, *Ph*), 7.21 (m, 3H, *Ph*), 7.03 (t, *J* = 7.6 Hz, 1H, *Ph*), 6.87 (d, *J* = 7.6 Hz, 1H), 4.26 (dd, *J* = 11.0, 7.4 Hz, 1H, O*CH₂*), 4.07 (m, 1H, O*CH₂*), 3.02 (m, 4H, N*CH₂*Ph, C_{q}CH₂*CH₂*N), 3.14 (m, 6H, N*CH₂*Ph, C_{q}CH₂*CH₂*N, C_{q}*CH₂*CH₂N), 3.76 (m, 1H, *CH*CH₃), 3.57 (m, 2H, C_{q}*CH₂*CH₂N), 1.1 1(d, *J* = 6.6 Hz, 3H, CH*CH₃*)
LC/MS (ES⁺) *m*/*z* 496.1 (M+H)+

### Preparation of (S)-2-(dibenzylamino)propyl 4-(2-oxoindolin-3-yl)piperidine-1-carboxylate (Compound n°22):

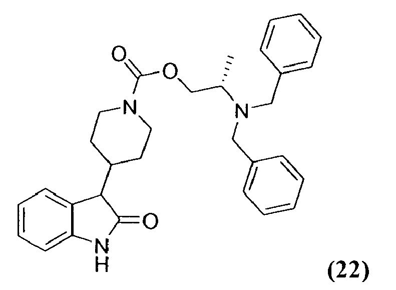

From 4-(2-Oxo-1,2-dihydro-indol-3-yl)-*N-tert*-butyloxycarbonyl piperidine and (S)-2-(dibenzylamino)propan-1-ol
Before "NBoc Deprotection / Carbamate" procedure, 4-(2-Oxo-1,2-dihydro-indol-3-ylidene)-piperidine-1-carboxylic acid tert-butyl ester was hydrogened by treatement of 5%mol of Pd:C (10%), 1.1 equiv of acetic acid, in methyl alcohol (C=0.2 M) on hydrogene atmosphere overnight, filtration and evaporation. The product was not purified anymore before next step.
Colorless oil (45%, 3 steps).
Flash chromatography on silica gel (cyclohexane/ ethyl acetate 7/3)
LC/MS (ES⁺) m/z 497.9 (M+H)⁺

### 12) NBn Deprotection / Carbamate preparation:

### General procedure

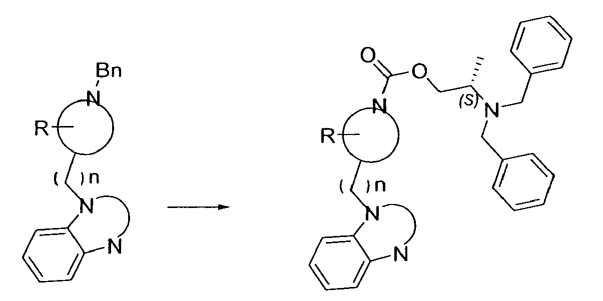

A solution of the N-benzyl derivative (1 equiv.) in EtOH (10 mL/mmol) was submitted to hydrogenation in the presence of acetic acid (6 equiv.) and 10% Pd/C (100 mg/ mmol) at room pressure and temperature for 24 h. The reaction mixture was filtered through a pad of Celite. The filtrate was concentrated under *vacuo.* To a stirred solution of (S)-2-(dibenzylamino)propan-1-ol (1 equiv.) in THF (4 mL/mmol) was added CDI (1.1 equiv.) at 0°C under N₂ atmosphere. The reaction mixture was stirred for 2h at room temperature. Triethylamine (1.6 equiv.) and the crude salt (1.3 equiv.) in THF (4 mL/mmol) were added and the reaction mixture was stirred for 72h at room temperature. The solvent was removed on *vacuo* and the residue was purified by flash chromatography on silica gel.

### Preparation of (S)-2-(dibenzylamino)propyl 2-((1,2-dihydro-2-oxobenzo[d]imidazol-3-yl)methyl)pyrrolidine-1-carboxylate (Compound n°23):

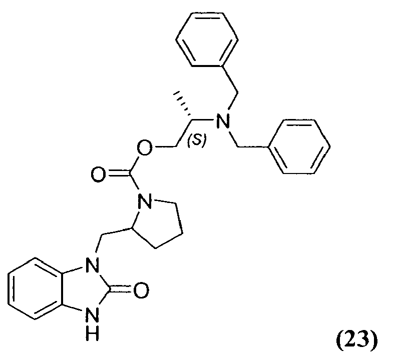

From 1-((1-benzylpyrrolidin-2-yl)methyl)-1H-benzo[d]imidazol-2(3 H)-one
Colorless oil (24%).
Flash chromatography on silica gel (cyclohexane/ ethyl acetate 5/5)
¹H NMR (400 MHz, CDCl₃) δ 9.67 (bs, 1H, NH), 7.39 (m, 5H, Ar), 7.29 (m, 6H, Ar), 7.22 (m, 3H, Ar), 4.25 (m, 2H, *CH₂*O, CH₂*CH*N), 4.12 (m, 1H, *CH₂*O), 4.02 (m, 2H, CH₂*CH₂*N), 3.78 (d, *J* = 13.8 Hz, 2H, N*CH₂*Ph), 3.59 (d, *J* = 13.8 Hz, 2H, N*CH₂*Ph), 3.45 (m, 2H, *CH₂*NCO), 3.15 (m, 1H, *CH*Me), 2.05 (m, 2H, *CH₂*CH₂N), 1.92 (m, 2H, *CH₂*CHN), 1.12 (d, *J=* 6.8 Hz, 3H, Me).
¹³C NMR (100 MHz, CDCI₃) δ 155.8 (C=O), 155.4 (C=O), 140.3 (C_{Bn}), 130.4 (C_{Ar}), 128.6 (CH_{Bn}), 128.1 (CH_{Bn}), 127.7 (C_{Ar}), 126.8 (CH_{Bn}), 121.7 (CH_{Ar}), 121.6 (CH_{Ar}), 109.3 (CH_{Ar}), 108.9 (CH_{Ar}), 66.6 (*CH₂*O), 56.4 (CH₂*CH*N), 53.7 (N*CH₂*Ph), 51.9 (*CH*Me), 46.6 (*CH₂*NCO), 42.2 (CH₂*CH₂*N), 28.3 (*CH₂*CHN), 23.7 (*CH₂*CH₂N), 11.5 (Me).
LC/MS (ES⁺) m/z 499.2 (M+H)⁺

### Preparation of (S)-2-(dibenzylamino)propyl 4-(2-oxoindolin-1-yl)piperidine-1-carboxylate (Compound n°24):

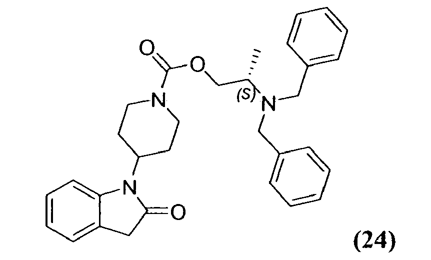

From 1-(1-benzylpiperidin-4-yl)indolin-2-one
Pale yellow oil (1mg, 2%).
Flash chromatography on silica gel (cyclohexane/ ethyl acetate 7/3)
¹H NMR (400 MHz, CDCl₃) δ 7.37 (d, *J* = 7.8 Hz, 4H, Bn), 7.35-7.19 (m, 9H, Ar), 7.00 (t, *J* = 7.6 Hz, 1H, Ar), 4.45-4.32 (m, 3H, CHCH₂*CH₂*Nₚᵢₚ, *CH*CH₂CH₂Nₚᵢₚ), 4.26 (m, 1H, *CH₂*O), 4.06 *(dd, J =* 11.2, 5.6 Hz, 1H, *CH₂*O), 3.75 (d, *J=* 13.6 Hz, 2H, N*CH₂*Ph), 3.56 (d, J= 13.6 Hz, 2H, N*CH₂*Ph), 3.53 (s, 2H, *CH₂*CO), 3.14 (m, 1H, *CH*Me), 2.90 (m, 2H, CHCH₂*CH₂*Nₚᵢₚ), 2.36 (m, 2H, CH*CH₂*CH₂Nₚᵢₚ), 1.79 (m, 2H, CH*CH₂*CH₂Nₚᵢₚ), 1.11 (d, *J* = 6.8 Hz, 3H, Me).
LC/MS (ES⁺) m/z 498.3 (M+H)⁺

### 13) Carbamate preparation (CDI coupling):

### General procedure:

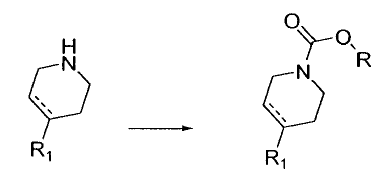

To a stirred solution of the appropriate alcohol (1 equiv.) in THF (2 mL/mmol) at 0°C was added CDI (1.1 equiv.) under N₂ atmosphere. The reaction mixture was stirred for 2h at room temperature. The appropriate amine (1.5 equiv.) in THF (10 mL/mmol) was added and the reaction mixture was stirred for 72h at room temperature. The solvent was removed on *vacuo* and the residue was purified by flash chromatography on silica gel.

### Preparation of (S)-2-(dibenzylamino)propyl 4-(1,2-dihydro-2-oxobenzo[d]imidazol-3-yl)piperidine-1-carboxylate (Compound n°25):

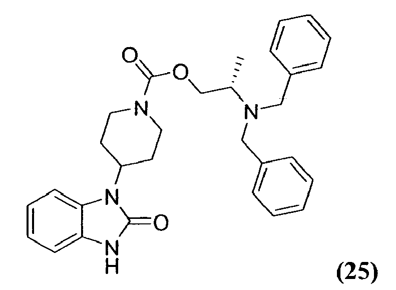

From 4-(2-keto-1-benzimidazolinyl)-piperidine and (S)-2-(dibcnzylamino)propan-1-ol
White solid (100%).
Flash chromatography on preparative TLC (silica gel, cyclohexane/ethyl acetate 7/3)
¹H NMR (400 MHz, CDCl₃, ppm) *δ* 10.21 (s, 1H, *NH*)*,* 7.39 (d, *J* = 7.3 Hz, 4H, *Ph*)*,* 7.28 (dd, *J* = 12.7, 5.1 Hz, 4H, *Ph*), 7.21 (t, *J* = 7.3, 2H, *Ph*), 7.15 (m, 2H, *Ph*)*,* 7.05 (m, 2H, *Ph*)*,* 4.53 (tt, *J* = 12.5, 3.8 Hz, 1H, *CH*CH₂CH₂N), 4.40 (m, 2H, CHCH₂*CH₂*N), 4.28 (dd, *J* = 11.0, 7.4 Hz, 1H, *CH₂*O)*,* 4.08 (dd, *J* = 11.0, 5.6 Hz, 1H, *CH₂*O)*,* 3.78 (d, *J* = 13.9 Hz, 2H, N*CH₂*Ph), 3.58 (d, *J* = 13.9 Hz, 2H, N*CH₂*Ph), 3.16 (sext., *J* = 6.3 Hz, 1H, *CH*CH₃), 2.99 (m, 2H, CHCH₂*CH₂*N), 2.30 (m, 2H, CH*CH₂*CH₂N), 1.90 (m, 2H, CH*CH₂*CH₂N), 1.12 (d, *J* = 6.8 Hz, 3H, CH*CH₃*)
¹³C NMR (100 MHz, CDCl₃, ppm) δ 155.2, 155.0 (C=O), 140.2, 128.8, 128.5, 128.1, 128.0, 126.7, 121.3, 121.1, 109.8, 109.2 (C_{Ph}), 66.8 (*CH₂*O), 53.7 (N*CH₂*Ph), 51.9 (*CH*CH₃), 50.7 (*CH*CH₂CH₂N), 43.6 (CHCH₂*CH₂*N), 29.2 (CH*CH₂*CH₂N), 11,0 (CH*CH₃*)
LC/MS (ES⁺) *m*/*z* 498.9 (M+H)⁺

### Preparation of (S)-2-(dibenzylamino)-3-phenylpropyl 4-(1,2-dihydro-2-oxobenzo[d]imidazol-3-yl)piperidine-1-carboxylate (Compound n°26):

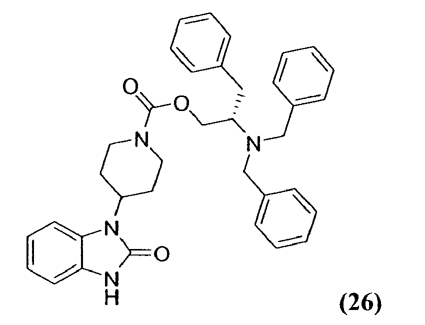

From 4-(2-keto-1-benzimidazolinyl)-piperidine and (S)-2-(dibcnzylamino)-3-phcnylpropan-1-ol
White solid (80%).
Flash chromatography on silica gel (cyclohexane/ ethyl acetate 7/3)
¹H NMR (400 MHz, CDCl₃, ppm) δ 10.49 (s, 1H, *NH*), 7.30 (m, 12H, *Ph*), 7.10 (m, 7H, *Ph),* 4.46 (m, 1H, *CH*CH₂CH₂N), 4.45 (m, 2H, CHCH₂*CH₂*N), 4.38 (m, 1H, *CH₂*O), 4.22 (m, 1H, *CH₂*O), 3.83 (d, *J* = 13.8 Hz, 2H, N*CH*₂Ph), 3.75 (d, *J* = 13.8 Hz, 2H, N*CH₂*Ph), 3.30 (m, 1H, *CH*CH₂Ph), 3.10 (dd, *J* = 13.6, 5.8 Hz, 1H, CH*CH₂*Ph), 2.94 (m, 2H, CHCH₂*CH₂*N), 2.71 (dd, *J* = 13.6, 8.5 Hz, 1H, CH*CH₂*Ph), 2.38 (m, 2H, CH*CH₂*CH₂N), 1.88 (m, 2H, CH*CH₂*CH₂N)
¹³C NMR (100 MHz, CDCl₃, ppm) δ 155.1, 155.1 (C=O), 139.8, 139.5, 135.0, 129.2, 128.9, 128.8, 128.6, 128.3, 128.3, 128.1, 126.9, 126.8, 126.0, 121.4, 121.1, 121.0, 109.9, 109.3 (C*Ph*)*,* 64.7 (*CH₂*O)*,* 58.5 (*CH*CH₂Ph), 54.0 (N*CH₂*Ph), 50.6 (*CH*CH₂CH₂N), 43.6 (CHCH₂*CH₂*N), 34.1 (CH*CH₂*Ph), 29.2 (CH*CH₂*CH₂N)
LC/MS (ES⁺) *m*/*z* 575.1 (M+H)⁺

### Preparation of 2-(N-benzyl-N-methylamino)ethyl 4-(1,2-dihydro-2-oxobenzo[d]imidazol-3-yl)piperidine-1-carboxylate (Compound n°27):

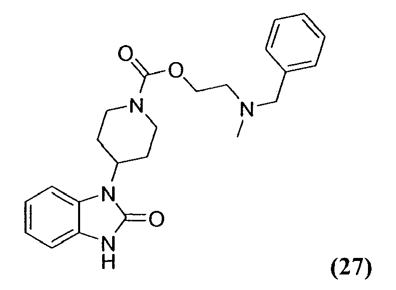

From 4-(2-keto-1-benzimidazolinyl)-piperidine and (S)-2-(*N,N-*benzylmethylamino)propan-1-ol
Pale Yellow oil (52%).
Flash chromatography on preparative TLC (silica gel, dichloromethane/ methyl alcohol 85/15)
¹H NMR (400 MHz, CDCl₃, ppm) δ 10.48 (s, 1H, *NH*), 7.32 (m, 4H, *Ph*), 7.24 (m, 1H, *Ph*), 7.12 (dd, *J* = 7.2, 1.4 Hz, 2H, *Ph*), 7.04 (m, 2H, *Ph*), 4.50 (tt, *J* = 12.4, 3.8 Hz, 1H, *CH*CH₂CH₂N), 4.37 (m, 2H, CHCH₂*CH₂*N), 4.28 (t, *J* = 5.8 Hz, 2H, O*CH₂*CH₂N), 3.59 (s, 2H, N*CH₂*Ph), 2.92 (m, 2H, CHCH₂*CH₂*N), 2.72 (t, *J* = 5.8 Hz, 2H, OCH₂*CH₂*N), 2.35 (m, 5H, CH*CH₂*CH₂N, N*CH₃*), 1.85 (m, 2H, CH*CH₂*CH₂N)
¹³C NMR (100 MHz, CDCl₃, ppm) δ 155.2, 155.1 (C=O), 138.7, 128.9, 128.8, 128.2, 128.1, 127.0, 121.3, 121.0, 109.9, 109.2 (C_{Ph}), 63.4 (O*CH₂*CH₂N), 62.4 (N*CH₂*Ph), 55.6 (OCH₂*CH₂*N), 50.6 (*CH*CH₂CH₂N), 43.6 (CHCH₂*CH*₂N), 42.7 (N*CH₃*), 29.1 (CH*CH*₂CH₂N)
LC/MS (ES⁺) *m*/*z* 409.4 (M+H)⁺

### Preparation of (S)-2-(dibenzylamino)propyl 5,6-dihvdro-4-(1,2-dihvdro-2-oxobenzo[dlimidazol-3-yl)pyridine-1(2H)-carboxylate (Compound n°28):

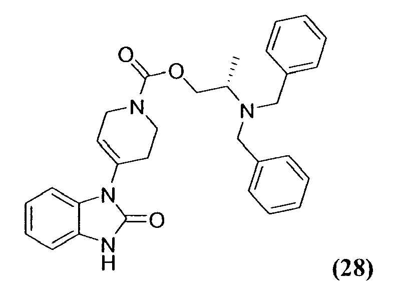

From 1-(1,2,3,6-tetrahydro-pyridin-4-yl)-1,3-dihydro-benzoimidazol-2-one and (S)-2-(dibenzylamino)propan-1-ol
Colorless oil (66%).
Flash chromatography on preparative TLC (silica gel, cyclohexane/ethyl acetate 1/1)
¹H NMR (400 MHz, CDCl₃, ppm) δ 9.97 (s, 1H, *NH*), 7.38 *(d, J* = 7.3 Hz, 4H, *Ph*), 7.3 (m, 4H, *Ph*), 7.20 (m, 2H, *Ph),* 7.05 (m, 4H, *Ph),* 5.97 (s, 1H, NCH₂*CH*=C), 4.26 (m, 3H, *CH₂*O, N*CH₂*CH=C), 4.10 (dd, *J* = 11.2, 5.6 Hz, 1H, *CH₂*O), 3.81 (m, 2H, N*CH₂*CH₂), 3.71 (d, *J* = 13.9 Hz, 2H, N*CH₂*Ph), 3.57 (d, *J* = 13.9 Hz, 2H, N*CH₂*Ph), 3.16 (sext., *J* = 6.6 Hz, 1H, *CH*CH₃), 2.63 (m, 2H, NCH₂*CH₂*), 1.12 (d, *J* = 6.6 Hz, 1H, CH*CH₃*)
¹³C NMR (100 MHz, CDCl₃, ppm) δ 171.2, 154.2 (C=O), 140.2, 129.9, 128.5, 128.1, 126.8, 122.0, 121.5, 109.8, 108.8 (C_{Ph}), 66.9 (*CH₂*O), 53.7 (N*CH₂*Ph), 50.6 (*CH*CH₃), 42,7 (N*CH₂*CH=C), 40,6 (NCH₂*CH₂*C_{q})26.9 (NCH₂*CH₂*C_{q}), 11.2 (CH*CH₃*),
LC/MS (ES⁺) *m*/*z* 496.9 (M+H)⁺

### Preparation of 3-(dibenzylamino)propyl 4-(1,2-dihydro-2-oxobenzo[d]imidazol-3-yl)piperidine-1-carboxylate (Compound n°29):

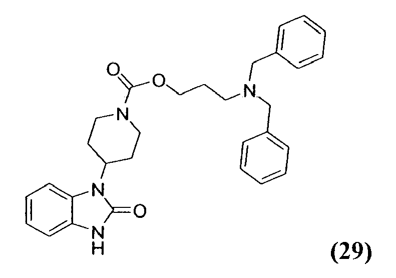

From 4-(2-keto-1-benzimidazolinyl)-piperidine and 3-dibenzylaminopropan-1-ol
White solid (54%).
Flash chromatography on preparative TLC (silica gel, cyclohexane/ethyl acetate 1/1)
¹H NMR (400 MHz, CDCl₃, ppm) δ 10.26 (s, 1H, *NH*)*,* 7.38 (d, *J* = 7.3 Hz, 4H, *Ph*)*,* 7.30 (t, *J* = 7.4 Hz, 4H, *Ph*)*,* 7.23 (m, 2H, *Ph*)*,* 7.10 (m, 4H, *Ph*)*,* 4.47 (tt, *J* = 12.4, 3.8 Hz, 1H, *CH*CH₂CH₂N), 4,39 (m, 1H, CHCH₂*CH₂*N), 4.20 (m, 2H, O*CH₂*CH₂CH₂N), 3.90 (m, 1H, CHCH₂*CH₂*N), 3.58 (s, 4H, N*CH₂*Ph), 2.79 (m, 2H, CHCH₂*CH₂*N), 2.54 (t, *J* = 6.1 Hz, 2H, OCH₂CH₂*CH₂*N), 2.23 (m, 2H, CH*CH₂*CH₂N), 1.82 (m, 4H, CH*CH₂*CH₂N, OCH₂*CH₂*CH₂N)
¹³C NMR (100 MHz, CDCl₃, ppm) δ 155, 155.1 (C=O), 139.6, 128.8, 128.7, 128.1, 128.1, 126.8, 121.4, 121.1, 109.9, 109.3 (C_{Ph}), 63.5 (O*CH₂*CH₂CH₂N), 58.4 (N*CH₂*Ph), 50.6 (*CH*CH₂CH₂N), 49.5 (OCH₂CH₂*CH₂*N), 43.4 (CHCH₂*CH₂*N), 29.1 (CH*CH₂*CH₂N), 26.6 (OCH₂*CH₂*CH₂N)
LC/MS (ES⁺) *m*/*z* 499.1 (M+H)⁺

### Preparation of 2-(dibenzylamino)ethyl 4-(1,2-dihydro-2-oxobenzo[d]imidazol-3-yl)piperidine-1-carboxylate (Compound n°30):

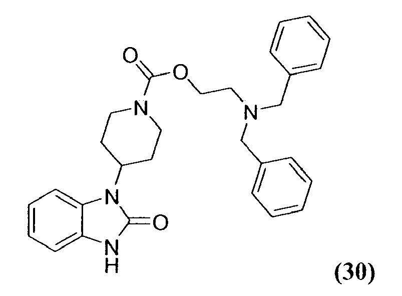

From 4-(2-keto-1-benzimidazolinyl)-piperidine and 2-dibenzylaminoethan-1-ol
White solid (60%).
Flash chromatography on preparative TLC (silica gel, cyclohexane/ethyl acetate 1/1)
¹H NMR (400 MHz, CDCl₃, ppm) δ 10.46 (s, 1H, *NH*), 7.40 (d, *J* = 7.2 Hz, 4H, *Ph*), 7.32 (t, *J* = 7.6 Hz, 4H, *Ph*), 7.24 (m, 2H, *Ph*), 7.15 (m, 2H, *Ph*), 7.06 (m, 2H, *Ph*), 4.54 (m, 1H, *CH*CH₂CH₂N), 4.44 (m, 1H, CHCH₂*CH₂*N), 4.31 (m, 1H, CHCH₂*CH₂*N), 4.26 (t, *J* = 5.6 Hz, 2H, O*CH₂*CH₂N), 3.68 (s, 4H, *CH₂*Ph), 2.92 (t, *J* = 11.2 Hz, 2H, CHCH₂*CH₂*N), 2.79 (t, *J* = 5.6 Hz, 2H, OCH₂*CH₂*N), 2.35 (m, 2H, CH*CH₂*CH₂N), 1.86 (d, *J* = 11.2 Hz, 2H, CH*CH₂*CH₂N)
¹³C NMR (100 MHz, CDCl₃, ppm) δ 155.2, 155.1 (C=O), 139.4, 128.8, 128.8, 128.7, 128.2, 128.1, 127.9, 126.9, 121.3, 121.1, 109.9, 109.3 (C_{Ph}), 63.5(O*CH₂*CH₂N), 58.7 (N*CH₂*Ph), 52.1 (OCH₂*CH₂*N), 50.7 (*CH*CH₂CH₂N), 43.6 (CHCH₂*CH*₂N), 29.2 (CH*CH*₂CH₂N)
LC/MS (ES⁺) *m*/*z* 485.1 (M+H)⁺

### Preparation of 2-(benzyloxy)ethyl 4-(1,2-dihydro-2-oxobenzo[d]imidazol-3-yl)piperidine-1-carboxylate (Compound n°31):

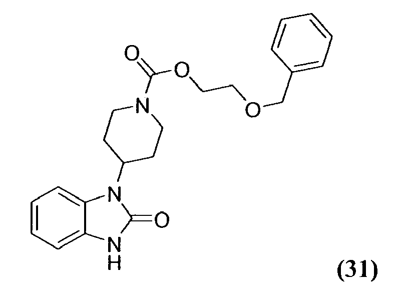

From 4-(2-keto-1-benzimidazolinyl)-piperidine and 2-benzyloxyethan-1-ol.
Colorless oil (36%).
Flash chromatography on preparative TLC (silica gel, dichloromethane/ methyl alcohol 95/5)
'H NMR (400 MHz, CDC1₃, ppm) 8 10.32 (s, 1H, *NH*), 7.35 (m, 4H, *Ph*), 7.30 (m, 1H, *Ph),* 7.13 (m, 2H, *Ph),* 7.05 (m, 2H, *Ph),* 4.60 (s, 2H, O*CH*₂Ph), 4.51 (tt, *J* = 12.3, 4.0 Hz, 1H, *CH*CH₂CH₂N), 4.41 (m, 2H, CHCH₂*CH₂*N), 4.33 (t, *J* = 4.6 Hz, 2H, O*CH₂*CH₂OBn), 3.74 (t, *J* = 4.6 Hz, 2H, OCH₂*CH₂*OBn), 2.96 (m, 2H, CHCH₂*CH₂*N), 2.37 (td, *J* = 12.2, 8.9 Hz, 2H, CH*CH₂*CH₂N), 1.85 (m, 2H, CH*CH₂*CH₂N)
¹³C NMR (100 MHz, CDCl₃, ppm) δ 155.2, 155.1 (C=O), 137.9, 128.8, 128.4, 128.1, 127.7, 127.6, 121.3, 121.1, 109.9, 109.3 (C_{Ph}), 73.0 (O*CH₂*Ph), 68.3 (OCH₂*CH₂*OBn), 64.7 (O*CH₂*CH₂OBn), 50.6 (*CH*CH₂CH₂N), 43.6 (CHCH₂*CH₂*N), 29.1 (CH*CH₂*CH₂N)
LC/MS (ES⁺) *m*/*z* 396.0 (M+H)⁺

### Preparation of (S)-2-(dibenzylamino)propyl 4-benzylpiperidine-1-carboxylate (Compound n°32):

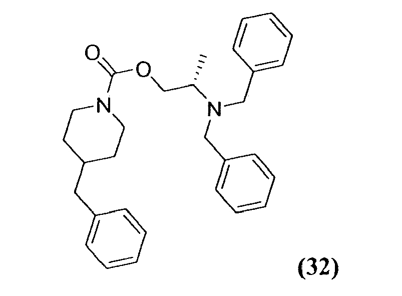

From 4-benzylpiperidine and (S)-2-(dibenzylamino)propan-1-ol
Colorless oil (72%).
Flash chromatography on silica gel (pentane/ ether 9/1)
¹H NMR (400 MHz, CDCl₃, ppm) δ 7.40 (d, *J* = 7.3 Hz, 4H, *Ph),* 7.31 (m, 5H, *Ph),* 7.24 (m, 3H, *Ph),* 7.19 (d, *J* = 7.3 Hz, 2H, *Ph),* 4.22 (m, 3H, *CH₂*O, CHCH₂*CH₂*N), 4.06 (m, 1H, CHCH₂*CH₂*N), 3.77 (d, *J* = 13.9 Hz, 2H, N*CH₂*Ph), 3.58 (d, *J* = 13.9 Hz, 2H, N*CH₂*Ph), 3.10 (m, 1H, *CH*CH₃), 2.74 (m, 2H, CHCH₂*CH₂*N), 2.58 (d, *J* = 5.70 Hz, 2H, CH*CH₂*Ph), 1.81-1.59 (m, 3H, *CHCH₂*CH₂N), 1.32-1.14 (m, 2H, CH*CH₂*CH₂N), 1.13 (t, *J* = 6.8 Hz, 3H, CH*CH₃*)
¹³C NMR (100 MHz, CDCl₃, ppm) δ 155.4 (C=O), 140.3, 140.0, 129.1, 128.5, 128.2, 128.1, 126.7, 126.0 (C_{Ph}), 66.4 (*CH₂*O)*,* 53.7 (N*CH₂*Ph), 51.9 (*CH*CH₃), 44.1 (CHCH₂*CH₂*N), 43.1 (*CH₂*Ph), 38.1 (*CH*CH₂CH₂N), 31.9 (CH*CH₂*CH₂N), 11.4 (CH*CH₃*)
LC/MS (ES⁺) *m*/*z* 457.2 (M+H)⁺

### Preparation of 1-benzylpiperidin-3-yl 4-(1,2-dihydro-2-oxobenzo[d]imidazol-3-yl)piperidine-1-carboxylate (Compound n°33):

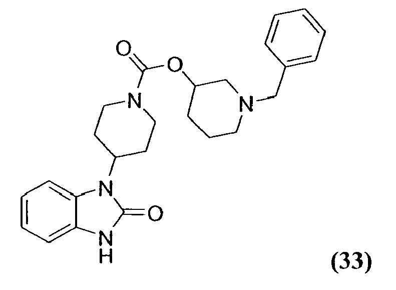

From 4-(2-keto-1-benzimidazolinyl)-piperidine and N-benzyl-3-hydroxypiperidine
White solid (100%).
Flash chromatography on preparative TLC (silica gel, cyclohexane/ethyl acetate 2/8)
¹H NMR (400 MHz, CDCl₃, ppm) δ 10.65 (s, 1H, *NH*), 7.31 (m, 4H, *Ph*), 7.23 (m, 1H, *Ph*), 7.14 (m, 2H, *Ph*), 7.05 (m, 2H, *Ph*), 4.85 (m, 1H, O*CH*), 4.51 (tt, *J* = 12.2, 3.6 Hz, 1H, *CH*CH₂CH₂N), 4.38 (m, 2H, CHCH₂*CH₂*N), 3.55 (s, 2H, *CH₂*Ph), 2.91 (m, 3H, CHCH₂*CH₂*N, OCH*CH₂*N), 2.60 (m, 1H, OCHCH₂CH₂*CH₂*N), 2.35 (m, 2H, CH*CH₂*CH₂N), 2.20 (m, 2H, OCHCH₂CH₂*CH₂*N, OCH*CH₂*N), 1.96 (m, 1H, OCH*CH₂*CH₂CH₂N), 1.85 (d, *J* = 11.8 Hz, 2H, CH*CH₂*CH₂N), 1.78 (m, 1H, OCHCH₂*CH₂*CH₂N), 1.64 (m, 1H, OCHCH₂*CH₂*CH₂N), 1.48 (m, 1H, OCH*CH₂*CH₂CH₂N)
¹³C NMR (100 MHz, CDCl₃, ppm) δ 155.2, 154.7 (C=O), 138.2, 128.8, 128.2, 128.1, 126.9, 121.3, 121.0, 109.9, 109.2 (C_{Ph}), 70.8 (O*CH*), 62.8 (NCH₂Ph), 57.5, 53.1 (OCH*CH₂*N, OCHCH₂CH₂*CH₂*N), 50.6 (*CH*CH₂CH₂N), 43.5 (CHCH₂*CH₂*N), 30.0 (OCH*CH₂*CH₂CH₂N), 29.1 (CH*CH₂*CH₂N), 22.9 (OCHCH₂*CH₂*CH₂N)
LC/MS (ES⁺) *m*/*z* 435.2 (M+H)⁺

### Preparation of 2-(diisopropylamino)ethyl 4-(1,2-dihydro-2-oxobenzo[d]imidazol-3-yl)piperidine-1-carboxylate (Compound n°34):

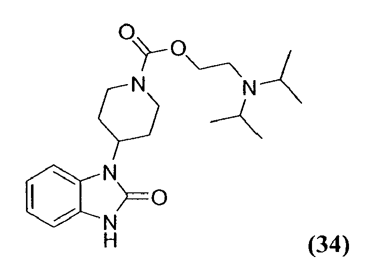

From 4-(2-keto-1-benzimidazolinyl)-piperidine and 2-(diisopropylamino)propan-1-ol
Colorless oil (58%).
Flash chromatography on preparative TLC (silica gel, cyclohexane/ethyl acetate 7/3)
1H NMR (400 MHz, CDCl₃, ppm) δ 9.56 (s, 1H, *NH),* 7.10 (m, 4H, *Ph),* 4.51 (tt, J = 12.4, 3.8 Hz, 1H, *CH*CH₂CH₂N), 4.39 (m, 2H, CHCH₂*CH₂*N), 4.06 (t, *J* = 6.8 Hz, 2H, O*CH₂*CH₂N), 3.00 (m, 2H, *CH*(CH₃)₂), 2.93 (m, 2H, CHCH₂*CH₂*N), 2.69 (t, *J* = 6.8 Hz, 2H, OCH₂*CH₂*N), 2.34 (m, 2H, CH*CH*₂CH₂N), 1.86 (d, *J* = 11.9 Hz, 2H, CH*CH₂*CH₂N), 1.02 (t, *J* = 9.0 Hz, 12H, CH(*CH*₃)₂)
¹³C NMR (100 MHz, CDCl₃, ppm) δ 155.4, 154.8 (C=O), 128.9, 127.9, 121.3, 121.1, 109.7, 109.3 (C_{Ph}), 66.4 (O*CH₂*CH₂N), 50.8, 49.3 (*CH*(CH₃)₂, *CH*CH₂CH₂N), 44.1 (OCH₂*CH*₂N), 43.5 (CHCH₂*CH*₂N), 29.1 (CH*CH*₂CH₂N), 20.8 (CH(*CH*₃)₂)
LC/MS (ES⁺) *m*/*z* 389.3 (M+H)⁺

### Preparation of 2-(dimethylamino)ethyl 4-(1,2-dihydro-2-oxobenzo[d]imidazol-3-yl)piperidine-1-carboxylate (Compound n°35):

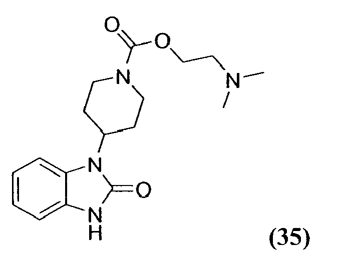

From 4-(2-keto-1-benzimidazolinyl)-piperidine and 2-(dimethylamino)propan-1-ol
Colorless oil (63%).
Flash chromatography on preparative TLC (silica gel, cyclohexane/ethyl acetate 7/3)
¹H NMR (400 MHz, CDCl₃, ppm) δ 9.85 (s, 1H, *NH*), 7.09 (m, 4H, *Ph),* 4.48 (tt, J = 12.5, 4.0 Hz, 1H, *CH*CH₂CH₂N), 4.35 (m, 2H, CHCH₂*CH₂*N), 4.26 (m, 2H, O*CH₂*CH₂N), 2.91 (m, 2H, CHCH₂*CH₂*N), 2.65 (t, *J* = 5.8 Hz, 2H, OCH₂*CH₂*N), 2.31 (m, 8H, CH*CH₂*CH₂N, N*CH₃*)₂), 1.83 (d, *J* = 10.9 Hz, 2H, CH*CH₂*CH₂N)
¹³C NMR (100 MHz, CDCl₃, ppm) δ 155.2, 154.8 (C=O), 128.9, 128.0, 121.3, 121.1, 109.7, 109.3 (C_{Ph}), 63.4 (O*CH₂*CH₂N), 58.2 (OCH₂*CH₂*N), 50.7 (*CH*CH₂CH₂N), 45.7 (N(*CH*₃)₂), 43.6 (CHCH₂*CH₂*N), 29.1 (CH*CH₂*CH₂N)
LC/MS (ES⁺) *m*/*z* 333.2 (M+H)⁺

### Preparation of 3,3-diphenylpropyl 4-(1,2-dihydro-2-oxobenzo[d]imidazol-3-yl)piperidine-1-carboxylate (Compound n°36):

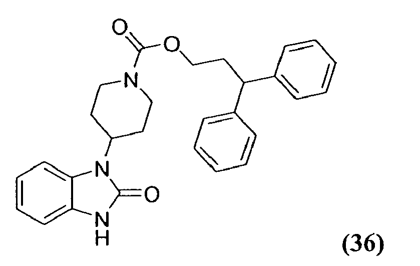

From 4-(2-keto-1-benzimidazolinyl)-piperidine and 3,3-diphenyl-propan-1-ol.
Colorless oil (86%).
Flash chromatography on preparative TLC (silica gel, cyclohexane/ethyl acetate 8/2)
¹H NMR (400 MHz, CDCl₃, ppm) δ 9.83 (s, 1H, *NH),* 7.29 (m, 8H, *Ph),* 7.19 (m, 2H, *Ph*), 7.13 (m, 2H, *Ph*), 7.06 (m, 2H, *Ph*), 4.49 (tt, *J* = 12.4, 3.7 Hz, 1H, *CH*CH₂CH₂N), 4.36 (m, 1H, CHCH₂*CH₂*N), 4.13 (m, 4H, CHCH₂CH₂N, O*CH₂*CH₂*CH*Ph₂), 2.89 (t, *J* = 12.6 Hz, 2H, CHCH₂*CH₂*N), 2.46 *(q, J =* 6.8 Hz, 2H, OCH₂*CH₂*CHPh₂), 2.32 (m, 2H, CH*CH₂*CH₂N), 1.83 (d, *J* = 11.7 Hz, 2H, CH*CH*₂CH₂N)
¹³C NMR (100 MHz, CDCl₃, ppm) δ 155.2, 154.9 (C=O), 144.2, 128.8, 128.5, 127.9, 127.7, 126.3, 121.4, 121.1, 109.8, 109.3 (C_{Ph}), 64.2 (O*CH₂*CH₂CHPh₂), 50.8, (*CH*CH₂CH₂N), 48.0 (OCH₂CH₂*CH*Ph₂), 43.5 (CHCH₂*CH₂*N), 34.7 (OCH₂*CH*₂CHPh₂), 29.2 (CH*CH₂*CH₂N)
LC/MS (ES⁺) m/z 456.1 (M+H)⁺

### Preparation of 2-benzylamino bis [ethyl 4-(1,2-dihydro-2-oxobenzo[d]imidazol-3-yl)piperidine-1-carboxylate (Compound n°37):

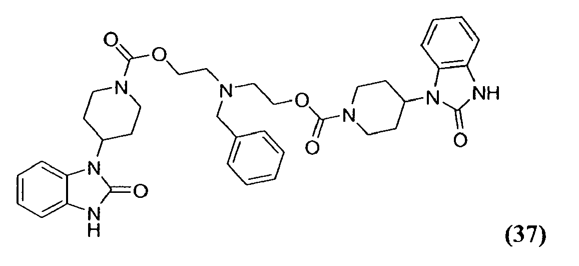

From 4-(2-keto-1-benzimidazolinyl)-piperidine and *N*-benzyl-diethanolamine
White solid (100%).
Flash chromatography on silica gel (dichloromethane/methyl alcohol 95/5)
¹H NMR (400 MHz, CDCl₃, ppm) δ 9.89 (s, 2H, *NH*), 7.37 (d, *J* = 7.4 Hz, 2H, *Ph*), 7.27 (m, 4H, *Ph*), 7.06 (m, 7H, *Ph*), 4.48 (m, 2H, C*H*CH₂CH₂N), 4.37 (m, 2H, CHCH₂C*H*₂N), 4.24 (m, 6H, CHCH₂C*H*₂N, OC*H*₂CH₂N), 3.77 (s, 2H, NC*H*₂Ph), 2.89 (m, 8H, CHCH₂C*H*₂N, OCH₂C*H*₂N), 2.33 (m, 4H, CHC*H*₂CH₂N), 1.84 (m, 4H, CHC*H*₂CH₂N)
¹³C NMR (100 MHz, CDCl₃, ppm) δ 156.6, 155.1, 154.9 (C=O), 139.3, 128.8, 128.5, 128.2, 127.9, 127.8, 127.0, 121.3, 121.1, 109.8, 109.2 (C_{Ph}), 63.7 (O*CH*₂CH₂N), 59.4 (NC*H*₂Ph), 53.4 (OCH₂*CH*₂N), 50.7 (*CH*CH₂CH₂N), 43.6 (CHCH₂*CH*₂N), 29.15 (CH*CH*₂CH₂N)
LC/MS (ES⁺) *m*/*z* 683.2 (M+H)⁺

### Preparation of (S)-2-(dibenzylamino)propyl 4-(6-chloro-1,2-dihydro-2-oxobenzo[d]imidazol-3-yl)piperidine-1-carboxylate (Compound n°38):

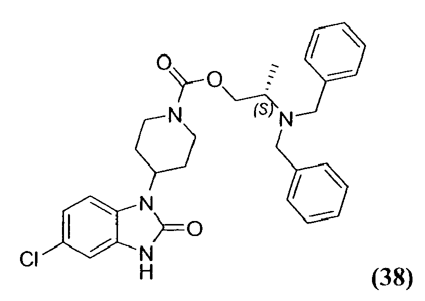

From 5-chloro-1-(piperidin-4-yl)-1H-benzo[d]imidazol-2(3H)-one and (S)-2-(dibenzylamino)propan-1-ol
Colorless oil (79%).
Flash chromatography on silica gel (cyclohexane/ ethyl acetate 6/4)
¹H NMR (400 MHz, CDCl₃) δ 9.05 (bs, 1H, NH), 7.38 (d, *J* = 7.6 Hz, 4H, Bn), 7.29 (t, *J* = 7.2 Hz, 4H, Bn), 7.21 (t, *J* = 7.2 Hz, 2H, Bn), 7.12 (s, 1H, Ar), 7.00 (m, 2H, Ar), 4.49 (m, 3H, CHCH₂*CH₂*Nₚᵢₚ, *CH*CH₂CH₂Nₚᵢₚ), 4.28 (dd, *J* = 5.6, 10.8 Hz, 1H, *CH₂*O)*,* 4.07 (dd, *J =* 5.6, 10.8 Hz, 1H, *CH₂*O)*,* 3.77 (d, *J =* 14.0 Hz, 2H, N*CH₂*Ph), 3.57 (d, J= 14.0 Hz, 2H, N*CH₂*Ph), 3.16 (m, 1H, *CH*Me), 2.95 (m, 2H, CHCH₂*CH₂*Nₚᵢₚ), 2.33 (m, 2H, CH*CH₂*CH₂Nₚᵢₚ), 1.87 (m, 2H, CH*CH₂*CH₂Nₚᵢₚ), 1.11 (d, J= 6.4 Hz, 3H, Me).
¹³C NMR (100 MHz, CDCl₃) δ 155.1 (2xC=O), 140.2 (C_{Bn}), 129.2 (C_{Ar}), 128.5 (CH_{Bn}), 128.1 (CH_{Bn}), 127.4 (C_{Ar}), 127.0 (C_{Ar}), 126.8 (CH_{Bn}), 121.2 (CH_{Ar}), 110.3 (CH_{Ar}), 109.9 (CH_{Ar}), 67.4 (*CH₂*O), 53.7 (N*CH₂*Ph), 51.9 (*CH*Me), 50.9 (*CH*CH₂CH₂Nₚᵢₚ), 43.7 (CHCH₂*CH₂*Nₚᵢₚ), 29.5 (CH*CH₂*CH₂Nₚᵢₚ), 11.2 (Me).
LC/MS (ES⁺) *m*/*z* 533.2 (M+H)⁺

### Preparation of 2-(diethylamino)ethyl 4-(1,2-dihydro-2-oxobenzo[d]imidazol-3-yl)piperidine-1-carboxylate (Compound n°39):

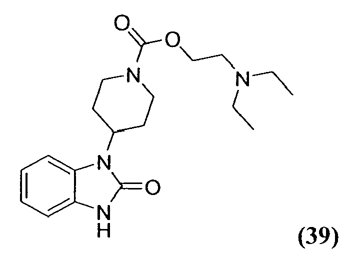

From 1-(piperidin-4-yl)-1H-benzo[d]imidazol-2(3H)-one and 2-(diethylamino)ethanol
White solid (69%).
Flash chromatography on silica gel (methylene chloride/ methanol 85/15)
¹H NMR (400 MHz, CDCl₃) δ 10.0 (bs, 1H, NH), 7.12 (m, 2H, Ar), 7.06 (m, 2H, Ar), 4.51 (m, 1H, *CH*CH₂CH₂Nₚᵢₚ), 4.39 (m, 2H, CHCH₂*CH₂*Nₚᵢₚ), 4.22 (t, *J* = 6.4 Hz, 2H, *CH₂*O), 2.93 (m, 2H, CHCH₂*CH₂*Nₚᵢₚ), 2.78 (t, *J* = 6.4 Hz, 2H, *CH*₂CH₂O), 2.63 (q, *J =* 7.2 Hz, 4H, CH₃*CH₂*N), 2.35 (m, 2H, CH*CH₂*CH₂Nₚᵢₚ), 1.85 (m, 2H, CH*CH₂*CH₂Nₚᵢₚ), 1.07 (t, J= 7.2 Hz, 6H, *CH₃*CH₂N).
¹³C NMR (100 MHz, CDCl₃) δ 155.3 (C=O), 154.9 (C=O), 128.8 (C_{Ar}), 128.0 (C_{Ar}), 121.3 (CH_{Ar}), 121.0 (CH_{Ar}), 109.8 (CH_{Ar}), 109.3 (CH_{Ar}), 63.9 (*CH₂*O), 51.3 (*CH*₂CH₂O), 50.6 (*CH*CH₂CH₂Nₚᵢₚ), 47.7 (CH₃*CH₂*N), 43.6 (CHCH₂*CH₂*Nₚᵢₚ), 29.1 (CH*CH₂*CH₂Nₚᵢₚ), 11.9 (*CH₃*CH₂N).
LC/MS (ES⁺) *m*/*z* 361.2 (M+H)⁺

### Preparation of 2-(1-benzylpiperidin-4-yl)ethyl 4-(1,2-dihydro-2-oxobenzo[d]imidazol-3-yl)piperidine-1-carboxylate (Compound n°40):

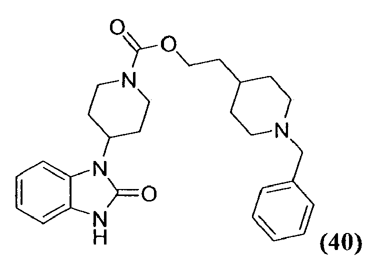

From 4-(2-keto-1-benzimidazolinyl)-piperidine and *N*-benzyl-4-(2-hydroxyethyl)piperidine
White solid (65%).
Flash chromatography on silica gel (methylene chloride/ methanol 9/1)
¹H NMR (400 MHz, CDCl₃) δ 9.88 (bs, 1H, NH), 7.32 (m, 4H, Bn), 7.25 (m, 1H, Bn), 7.13 (dd, *J* = 7.2, 4.0 Hz, 2H, Ar), 7.07 (dd, *J =* 7.2, 4.0 Hz, 2H, Ar), 4.51 (m, 1H, *CH*CH₂CH₂Nₚᵢₚ), 4.35 (m, 2H, CHCH₂*CH₂*Nₚᵢₚ), 4.17 (t, *J* = 6.8 Hz, 2H, CH₂*CH₂*O), 3.51 (s, 2H, NCH₂Ph), 2.90 (m, 4H, CHCH₂*CH*₂Nₚᵢₚ, CHCH₂*CH*₂N_{Bn}), 2.35 (m, 2H, CH*CH*₂CH₂Nₚᵢₚ), 1.98 (m, 2H, CHCH₂*CH*₂N_{Bn}), 1.85 (m, 2H, CH*CH₂*CH₂Nₚᵢₚ), 1.71 (m, 2H, CH*CH*₂CH₂N_{Bn}), 1.62 (dd, *J* = 6.8, 6.4 Hz, 2H, *CH₂*CH₂O), 1.36 (m, 3H, CH*CH₂*CH₂N_{Bn}, *CH*CH₂CH₂N_{Bn}).
¹³C NMR (100 MHz, CDCl₃) δ 155.4 (C=O), 154.9 (C=O), 129.3 (CH_{Ar}), 128.9 (C_{Ar}), 128.1 (CH_{Ar}), 127.9 (2C_{Ar}), 126.9 (CH_{Ar}), 121.3 (CH_{Ar}), 121.1 (CH_{Ar}), 109.8 (CH_{Ar}), 109.3 (CH_{Ar}), 63.8 (CH₂*CH₂*O), 63.5 (N*CH₂*Ph), 53.7 (CHCH₂*CH₂*N_{Bn}), 50.7 (*CH*CH₂CH₂Nₚᵢₚ), 43.6 (CHCH₂*CH*₂Nₚᵢₚ), 35.6 (*CH₂*CH₂O), 32.9 (*CH*CH₂CH₂N_{Bn}), 32.2 (CH*CH₂*CH₂N_{Bn}), 29.2 (CH*CH₂*CH₂Nₚᵢₚ).
LC/MS (ES⁺) *m*/*z* 463.3 (M+H)⁺

### Preparation of 2-(ethyl(phenyl)amino)ethyl 4-(2-oxo-2,3-dihydro-1H-benzo[d]imidazol-1-yl)piperidine-1-carboxylate (Compound n°41):

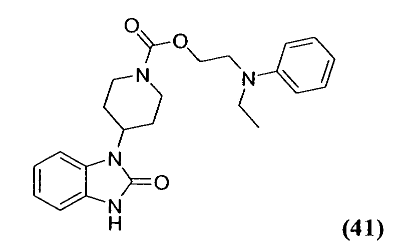

From 1-(piperidin-4-yl)-1H-benzo[d]imidazol-2(3H)-one and 2-(N-ethyl-N-phenylamino)ethanol
White solid (94%).
Flash chromatography on silica gel (cyclohexane/ ethyl acetate 3/7)
¹H NMR (400 MHz, CDCl₃) δ 8.75 (bs, 1H, NH), 7.22 (m, 2H, Ar), 7.06 (m, 4H, Ar), 6.76 (d, *J* = 8.0 Hz, 2H, Ar), 6.68 (t, *J* = 6.8 Hz, 1H, Ar), 4.48 (m, 1H, *CH*CH₂CH₂Nₚᵢₚ), 4.30 (m, 4H, CHCH₂*CH₂*Nₚᵢₚ, CH₂*CH₂*O), 3.61 (t, *J* = 6.4 Hz, 2H, *CH₂*CH₂O), 3.44 (q, *J* = 7.2 Hz, 2H, CH₃*CH₂*N), 2.93 (m, 2H, CHCH₂*CH₂*Nₚᵢₚ), 2.31 (m, 2H, CH*CH₂*CH₂Nₚᵢₚ), 1.85 (m, 2H, CH*CH₂*CH₂Nₚᵢₚ), 1.20 (t, *J* = 7.2 Hz, 3H, *CH₃*CH₂N).
¹³C NMR (100 MHz, CDCl₃) δ 155.1 (C=O), 154.9 (C=O), 147.7 (C_{Ar}), 129.3 (CH_{Ar}), 128.8 (C_{Ar}), 127.9 (C_{Ar}), 121.3 (CH_{Ar}), 121.2 (CH_{Ar}), 116.1 (CH_{Ar}), 111.9 (CH_{Ar}), 109.8 (CH_{Ar}), 109.3 (CH_{Ar}), 62.8 (CH₂*CH₂*O), 50.6 (*CH*CH₂CH₂Nₚᵢₚ), 49.1 (*CH₂*CH₂O), 45.0 (CH₃*CH₂*N), 43.6 (CHCH₂*CH₂*Nₚᵢₚ), 29.1 (CH*CH₂*CH₂Nₚᵢₚ), 12.3 (*CH₃*CH₂N).
LC/MS (ES⁺) *m*/*z* 409.2 (M+H)⁺

### Preparation of ((S)-1-benzylpyrrolidin-2-yl)methyl 4-(1,2-dihydro-2-oxobenzo[d]imidazol-3-yl)piperidine-1-carboxylate (Compound n°42):

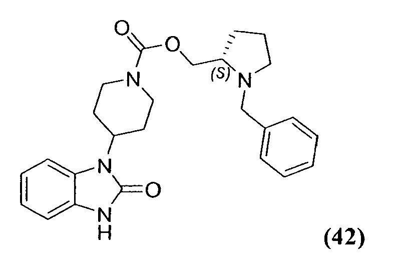

From 1-(piperidin-4-yl)-1H-benzo[d]imidazol-2(3H)-one and ((S)-1-benzylpyrrolidin-2-yl)methanol
Colorless oil (88%).
Flash chromatography on silica gel (methylene chloride/ methanol 95/5)
¹H NMR (400 MHz, CDCl₃) δ 10.33 (bs, 1H, NH), 7.32 (m, 4H, Bn), 7.25 (m, 1H, Bn), 7.12 (m, 1H, Ar), 7.07 (m, 3H, Ar), 4.52 (m, 1H, *CH*CH₂CH₂Nₚᵢₚ), 4.48 (m, 2H, CHCH₂*CH*₂Nₚᵢₚ), 4.17 (m, 3H, *CH₂*O*,* N*CH₂*Ph), 3.42 (d, *J* = 12.8 Hz, 1H, N*CH₂*Ph), 2.91 (m, 3H, CHCH₂*CH₂*Nₚᵢₚ, CH₂*CH*N_{Bn}), 2.35 (m, 2H, CH*CH₂*CH₂Nₚᵢₚ), 2.24 (m, 1H, CH₂*CH₂*N_{Bn}), 1.95 (m, 2H, CH₂*CH₂*N_{Bn}, *CH₂*CHN_{Bn}), 1.85 (m, 2H, CH*CH₂*CH₂Nₚᵢₚ), 1.72 (m, 3H, *CH₂*CH₂N_{Bn}, *CH₂*CHN_{Bn}).
¹³C NMR (100 MHz, CDCl₃) δ 155.4 (C=O), 155.1 (C=O), 139.6 (C_{Ar}), 128.8 (CH_{Ar}), 128.2 (CH_{Ar}), 128.1 (2C_{Ar}), 126.9 (CH_{Ar}), 121.3 (CH_{Ar}), 121.1 (CH_{Ar}), 109.9 (CH_{Ar}), 109.3 (CH_{Ar}), 62.4 (CH₂*CH*N_{Bn}), 59.4 (*CH₂*O)*,* 54.5 (N*CH₂*Ph), 50.6 (*CH*CH₂CH₂Nₚᵢₚ), 43.7 (CHCH₂*CH₂*Nₚᵢₚ), 29.2 (CH*CH₂*CH₂Nₚᵢₚ), 28.4 (*CH₂*CHN_{Bn}), 23.0 (*CH₂*CH₂N_{Bn}).
LC/MS (ES⁺) *m*/*z* 435.1 (M+H)⁺

### Preparation of 2,2-diphenylethyl 4-(1,2-dihydro-2-oxobenzo[d]imidazol-3-yl)piperidine-1-carboxylate (Compound n°43):

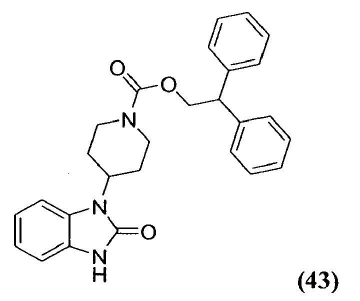

From 1-(piperidin-4-yl)-1H-benzo[d]imidazol-2(3H)-one and 2,2-diphenylethanol
White solid (75%).
Flash chromatography on silica gel (cyclohexane/ ethyl acetate 5/5)
¹H NMR (400 MHz, CDC1₃) δ 10.3 (bs, 1H, NH), 7.31 (m, 8H, Ar), 7.23 (m, 2H, Ar), 7.12 (m, 1H, Ar), 7.06 (m, 2H, Ar), 7.01 (m, 1H, Ar), 4.73 (m, 2H, *CH₂*O), 4.45 (m, 3H, CHCH₂*CH₂*Nₚᵢₚ, *CH*(Ph)₂), 4.06 (m, 1H, *CH*CH₂CH₂Nₚᵢₚ), 2.83 (m, 2H, CHCH₂*CH₂*Nₚᵢₚ), 2.28 (m, 1H, CH*CH₂*CH₂Nₚᵢₚ), 2.06 (m, 1H, CH*CH₂*CH₂Nₚᵢₚ), 1.80-1.72 (m, 2H, CH*CH₂*CH₂Nₚᵢₚ).
¹³C NMR (100 MHz, CDCl₃) δ 155.1 (C=O), 154.9 (C=O), 141.2 (C_{Ar}), 128.7 (C_{Ar}), 128.5 (CH_{Ar}), 128.2 (CH_{Ar}), 128.1 (C_{Ar}), 126.7 (CH_{Ar}), 121.4 (CH_{Ar}), 121.1 (CH_{Ar}), 109.9 (CH_{Ar}), 109.4 (CH_{Ar}), 67.8 (*CH₂*O), 50.5 (*CH*CH₂CH₂Nₚᵢₚ), 50.1 (*CH*(Ph)₂), 43.6 (CHCH₂*CH₂*Nₚᵢₚ), 29.0 (CH*CH₂*CH₂Nₚᵢₚ).
LC/MS (ES⁺) *m*/*z* 442.1 (M+H)⁺

### Preparation of (S)-2-(dibenzylamino)propyl 4-(1H-indol-3-yl)piperidine-1-carboxylate (Compound n°44):

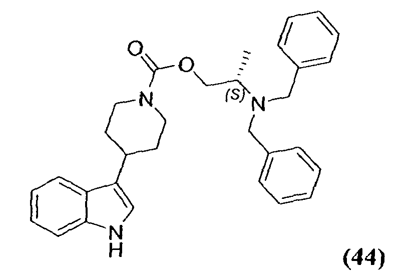

From (S)-2-(dibenzylamino)propan-1-ol and 3-(pipefidin-4-yl)-1H-indole
Colorless oil (55%).
Flash chromatography on silica gel (cyclohexane/ ethyl acetate 75/25)
¹H NMR (400 MHz, CDCl₃) δ 8.10 (bs, 1H, NH), 7.67 (d, *J* = 7.6 Hz, 1H, Ar), 7.41 (d, *J =* 7.6 Hz, 4H, Bn), 7.29 (t, *J =* 7.6 Hz, 4H, Bn), 7.21 (m, 3H, Ar), 7.13 (t, *J* = 7.2 Hz, 2H, Ar), 6.96 (s, 1H, Ar), 4.29 (m, 3H, CHCH₂*CH*₂Nₚᵢₚ, *CH₂*O), 4.09 (m, 1H, *CH₂*O)*,* 3.77 (d, *J =* 14.0 Hz, 2H, N*CH₂*Ph), 3.59 (d, *J* = 14.0 Hz, 2H, N*CH₂*Ph), 3.17 (m, 1H, *CH*Me), 3.02 (m, 3H, CHCH₂*CH₂*Nₚᵢₚ, *CH*CH₂CH₂Nₚᵢₚ), 2.10 (m, 2H, CH*CH₂*CH₂Nₚᵢₚ), 1.73 (m, 2H, CH*CH₂*CH₂Nₚᵢₚ), 1.12 (d, *J =* 6.8 Hz, 3H, Me).
¹³C NMR (100 MHz, CDCl₃) δ 155.5 (C=O), 140.3 (C_{Bn}), 136.4, 128.5 (CH_{Bn}), 128.1 (CH_{Bn}), 126.7 (CH_{Bn}), 126.4, 122.0, 120.7, 119.7, 119.2, 118.9, 111.3, 66.5 (*CH₂*O), 53.7 (N*CH*₂Ph), 51.9 (*CH*Me), 44.6 (CHCH₂*CH₂*Nₚᵢₚ), 33.6 (*CH*CH₂CH₂Nₚᵢₚ), 26.9 (CH*CH*₂CH₂Nₚᵢₚ), 11.2 (Me).
LC/MS (ES⁺) m/z 482.3 (M+H)⁺

### Preparation of (S)-2-(dibenzylamino)propyl 4-(N-phenylpropionamido)piperidine-1-carboxylate (Compound n°45):

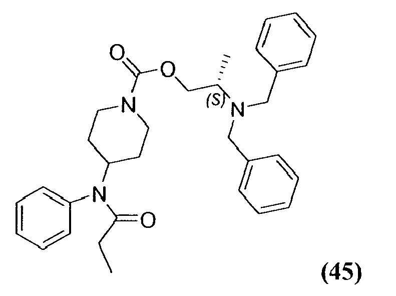

From N-phenyl-N-(piperldin-4-yl)proplonamide hydrochloride salt and (S)-2-(dibcnzylamino)propan-1-ol
Colorless oil (90%).
NEt₃ (2.7 equiv.) was added in the second step.
Flash chromatography on silica gel (cyclohexane/ ethyl acetate 7/3)
¹H NMR (400 MHz, CDCl₃) δ 7.40 (m, 3H, Ar), 7.31 (m, 4H, Ar), 7.21 (m, 6H, Ar), 7.07 (m, 2H, Ar), 4.84 (m, 1H, *CH*CH₂CH₂Nₚᵢₚ), 4.18 (m, 3H, CHCH₂*CH₂*Nₚᵢₚ, *CH₂*O), 3.93 (dd, *J =* 5.6, 10.8 Hz, 1H, *CH₂*O), 3.68 (d, *J =* 14.0 Hz, 2H, N*CH₂*Ph), 3.50 (d, J= 14.0 Hz, 2H, N*CH₂*Ph), 3.04 (m, 1H, *CH*Me), 2.88 (m, 2H, CHCH₂*CH₂*Nₚᵢₚ), 1.93 (q, *J* = 7.2 Hz, 2H, N(C=O)*CH₂*CH₃), 1.81 (m, 2H, CH*CH₂*CH₂Nₚᵢₚ), 1.26 (m, 2H, CH*CH₂*CH₂Nₚᵢₚ), 1.02 (m, 6H, N(C=O)CH₂*CH₃,* Me).
¹³C NMR (100 MHz, CDCl₃) δ 173.5 (C=O), 155.0 (C=O), 140.2 (C_{Bn}), 138.6 (C_{Ar}), 130.2 (CH_{Ar}), 129.4 (CH_{Ar}), 128.5 (CH_{Ar}), 128.4 (CH_{Bn}), 128.0 (CH_{Bn}), 126.7 (CH_{Bn}), 66.5 (*CH₂*O), 53.2 (N*CH₂*Ph), 52.1 (*CH*Me), 51.8 (*CH*CH₂CH₂Nₚᵢₚ), 43.4 (CHCH₂*CH₂*Nₚᵢₚ), 28.4 (CH*CH₂*CH₂Nₚᵢₚ), 26.9 (N(C=O)*CH₂*CH₃), 11.2 (Me), 9.6 (N(C=O)CH₂*CH₃*).
LC/MS (ES⁺) *m*/*z* 514.9 (M+H)⁺

### 14) Dithiocarbonate formation

### General procedure

To a solution of appropriate alcohol (2.5 mmol, 1 equiv.) in 5 ml of anhydrous THF was added 120 mg (3.0 mmol, 1.2 equiv.) of sodium hydride at 0°C and allowed to warm to room temperature for 30'. After addition of 300 µL (5 mmol, 2 equiv.) of carbon disulfure at 0°C, the reaction was continued for 30'. After addition of 280 µL (4.5 mmol, 1.8 equiv.) of methyl iodide at 0°C, the reaction was continued for 30'. The reaction was stopped by addition of ice and the mixture was extracted by dichloromethane. The dichloromethane layer was dried over sodium sulphate, evaporated and used without further purification.

### Preparation of Dithiocarbonic acid O-(2-dibenzylamino-ethyl) ester S-methyl ester (Intermediate):

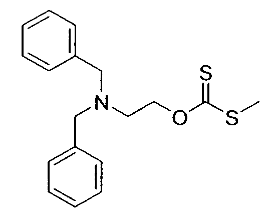

From dibenzylamino-ethan-1-ol
Pale Yellow oil (94%)
¹H NMR (400 MHz, CDCl₃) δ 7.30 (m, 10H, *Ph),* 4.72 (t, *J* = 5.8 Hz, 2H, O*CH₂*CH₂N), 3.69 (s, 4H, N*CH₂*Ph), 2.92 (t, *J* = 5.8 Hz, 2H, O*CH*₂CH₂N), 2.56 (s, 3H, *SCH₃)*
LC/MS (ES⁺) m/z 332.1 (M+H)⁺

### 15) Thiocarbamate formation

### General procedure

To a solution of appropriate dithiocarbonate (1.0 mmol, 1 equiv.) in 1 ml of methyl alcohol was added 260 mg (1.2 mmol, 1.2 equiv.) of 4-(2-keto-1-benzimidazolinyl)-piperidine. The mixture was stirred at 50°C overnight. The solvent was removed on *vacuo* and the residue was purified by flash chromatography on silica gel.

### Preparation of O-2-(dibenzylamino)ethyl 4-(1,2-dihvdro-2-oxobenzoldlimidazol-3-yl)piperidine-l-carbothioate (Compound n°46):

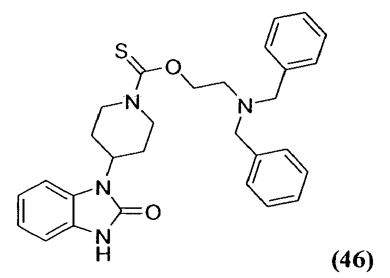

From 4-(2-keto-1-benzimidazolinyl)-piperidine and 2-dibenzylamino-ethan-1-ol.
White solid (59%).
Flash chromatography on silica gel (cyclohexane/ ethyl acetate 7/3)
¹H NMR (400 MHz, CDCl₃, ppm) δ 10.44 (s, 1H, *NH*)*,* 7.39 (d, *J* = 7.4 Hz, 4H, *Ph*)*,* 7.31 (t, *J* = 7.4, 4H, *Ph*)*,* 7.24 (m, 2H, *Ph*)*,* 7.17 (d, *J =* 6.8 Hz, 1H, *Ph),* 7.08 (m, 3H, *Ph),* 5.40 (d, *J* = 13.5 Hz, 1H, CHCH₂*CH₂*N), 4.71 (d, *J* = 13.5 Hz, 1H, CHCH₂*CH₂*N), 4.68-4.57 (m, 3H, *CH*CH₂CH₂N, O*CH₂*CH₂N), 3.68 (s, 4H, N*CH₂*Ph), 3.18 (t, *J* = 12.5 Hz, 1H, CHCH₂*CH₂*N), 2.96 (t, *J* = 12.5 Hz, 1H, CHCH₂*CH₂*N), 2.88 (t, *J* = 5.6 Hz, 2H, OCH₂*CH₂*N), 2.49 (dq, *J* = 12.6, 3.9 Hz, 1H, CH*CH₂*CH₂*N),* 2.31 (dq, *J* = 12.6, 3.9 Hz, 1H, CH*CH₂*CH₂N), 1.96 (d, *J* = 12.6 Hz, 1H, CH*CH₂*CH₂N), 1.89 (d, *J* = 12.6 Hz, 1H, CH*CH₂*CH₂N)
¹³C NMR (100 MHz, CDCl₃, ppm) δ 187.4 (C=S), 155.2 (C=O), 139.3, 128.7, 128.6, 128.2, 128.0, 127.0, 121.5, 121.2, 110.0, 109.3 (C_{Ph}), 69.6 (O*CH₂*CH₂N), 58.7 (N*CH₂*Ph), 51.7 (OCH₂*CH₂*N), 50.4 (*CH*CH₂CH₂N), 49.5, 44.5 (CHCH₂*CH₂*N), 29.1, 28.7 (CH*CH₂*CH₂N)
LC/MS (ES⁺) *m*/*z* 500.8 (M+H)⁺

### Preparation of O-2-(benzyloxy)ethyl 4-(1,2-dihydro-2-oxobenzo[d]imidazol-3-yl)piperidine-1-carbothioate (Compound n°47):

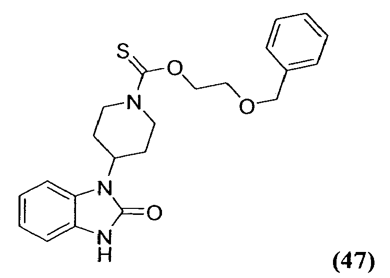

From 4-(2-keto-1-benzimidazolinyl)-piperidine and 2-benzyloxy-ethan-1-ol.
White solid (91%).
Flash chromatography on silica gel (cyclohexane/ ethyl acetate 1/1)
¹H NMR (400 MHz, CDCl₃, ppm) δ 10.42 (s, 1H, *NH*)*,* 7.39-7.24 (m, 5H, *Ph*)*,* 7.20-7.02 (m, 4H, *Ph*)*,* 5.39 (d, *J* = 13.3 Hz, 1H, CHCH₂*CH*₂N), 4.84 (d, *J* = 13.3 Hz, 1H, CHCH₂*CH₂*N), 4.73 (m, 2H, O*CH₂*CH₂OBn), 4.61 (m, 3H, O*CH₂*Ph, *CH*CH₂CH₂N), 3.80 (dd, *J* = 11.1, 6.6 Hz, 2H, OCH₂*CH₂*OBn), 3.19 (t, *J* = 12.5 Hz, 1H, CHCH₂*CH₂*N), 3.02 (t, *J* = 12.5 Hz, 1H, CHCH₂*CH₂*N), 2.50 (dq, *J* = 12.5, 4.0 Hz, 1H, CH*CH₂*CH₂N), 2.36 (dq, *J* = 12.5, 4.0 Hz, 1H, CH*CH₂*CH₂N), 1.86 (m, 2H, CH*CH₂*CH₂N)
¹³C NMR (100 MHz, CDCl₃, ppm) δ 187.3 (C=S), 155.1 (C=O), 137.8, 128.6, 128.4, 128.0, 127.8, 127.7, 121.5, 121.2, 110.0, 109. 3 (Cₚₕ), 73.0 (O*CH₂*Ph), 70.6 (O*CH₂*CH₂OBn), 67.9 (OCH₂*CH₂*OBn), 50.4 (*CH*CH₂CH₂N), 49.6, 44.6 (CHCH₂*CH₂*N), 29.1, 28.6 (CH*CH₂*CH₂N)
LC/MS (ES⁺) *m*/*z* 412.1 (M+H)⁺

### Preparation of O-(S)-2-(dibenzylamino)propyl 4-(1,2-dihydro-2-oxobenzo[d]imidazol-3-yl)piperidine-1-carbothioate (Compound n°48):

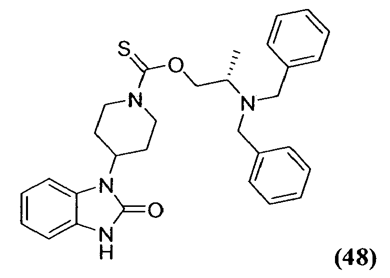

From 4-(2-keto-1-benzimidazolinyl)-piperidine and (S)-2-(dibenzylamino)propan-1-ol
Colorless oil (80%).
Flash chromatography on silica gel (cyclohexane/ ethyl acetate 1/1)
¹H NMR (400 MHz, CDCl₃, ppm) δ 9.19 (m, 1H, *NH*)*,* 7.37 (d, *J =* 7.3 Hz, 4H, *Ph*)*,* 7.32-7.24 (m, 4H, *Ph*)*,* 7.20 (m, 2H, *Ph*)*,* 7.16-7.02 (m, 4H, *Ph*)*,* 5.39 (d, *J* = 13.3 Hz, 1H, CHCH₂*CH₂*N), 4.76 (d, *J* = 13.3 Hz, 1H, CHCH₂*CH₂*N), 4.62 (m, 2H, *CH*CH₂CH₂N, O*CH₂*)*,* 4.40 (m, 1H, O*CH₂*)*,* 3.78 (d, *J* = 13.8 Hz, 2H, N*CH₂*Ph), 3.56 (dd, *J* = 13.8, 3.9 Hz, 2H, N*CH₂*Ph), 3.27 (m, 1H, *CH*CH₃), 3.17 (dd, *J* = 25.5, 14.5 Hz, 1H, CHCH₂*CH₂*N), 3.97 (ddd, *J* = 25.5, 13.2, 12.3 Hz, 1H, CHCH₂*CH₂*N), 2.57-2.32 (m, 1.5H, CH*CH₂*CH₂N), 2.32-2.19 (m, 0.5H, CH*CH₂*CH₂N), 1.96 (d, *J* = 10.9 Hz, 1.5H, CH*CH₂*CH₂N), 1.92-1.82 (d, *J* = 12.5 Hz, 0.5H), 1.15 (dd, *J* = 6.4, 1.7 Hz, 3H, CH*CH₃*)
LC/MS (ES⁺) *m*/*z* 514.8 (M+H)⁺

### 16) "Alkyl link" procedure

To a suspension of 217 mg (1.0 mmol, 1.0 equiv.) of 4-(2-keto-1-benzimidazolinyl)-piperidine, 17 mg (0.1 mmol., 0.1 equiv.) of potassium iodide, 276 mg ( 2.0 mmol., 2 equiv.) of potassium carbonate in 4 ml of anhydrous DMF was added the appropriate bromide derivative (1.0 mmol, 1 equiv.) in 1 ml of anhydrous DMF. The mixture was stirred at 70°C overnight. The solvent was removed in *vacuo* at room temperature and the slurry was partitioned with water and ethyl acetate. The organic layer was dried over sodium sulfate and the solvent was evaporated in *vacuum.* The residue was purified by flash chromatography on silica gel.

### Preparation of 1-(1-(3-(dibenzylamino)propyl)piperidin-4-yl)-1H-benzo[d]imidazol-2(3H)-one (Compound n°49):

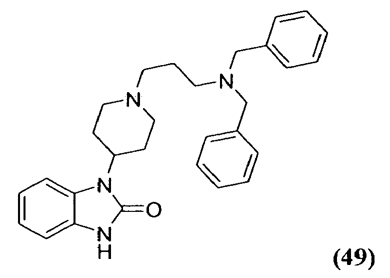

From 4-(2-keto-1-benzimidazolinyl)-piperidine and 3-dibenzylamino-1-bromopropane
Colorless oil (41 %).
Preparative LCMS
1H NMR (400 MHz, CDCI₃, ppm) δ 9.56 (s, 1H, *NH*), 7.31 (m, 11H, *Ph*), 7.08 (m, 3H, *Ph*), 4.58 (tt, *J* = 12.2, 3.8 Hz, 1H, *CH*CH₂CH₂N), 3.67 (s, 4H, N*CH₂*Ph), 3.56 (d, *J* = 11.7 Hz, 2H, CHCH₂*CH₂*N), 2.85 (m, 4H, CHCH₂*CH₂*N, N*CH₂*CH₂CH₂NBn₂), 2.62 (m, 4H, CH*CH₂*CH₂N, NCH₂CH₂*CH₂*NBn₂), 1.93 (m, 4H, CH*CH₂*CH₂N, NCH₂*CH₂*CH₂NBn₂)
¹³C NMR (100 MHz, CDCI₃, ppm) δ 154.7 (C=O), 137.7, 137.6, 129.3, 128.5, 128.0, 127.6, 122.0, 121.9, 110.0, 109.9 (Cpₕ), 58.7 (N*CH₂*Ph), 54.8 (CHCH₂*CH₂*N), 51.7, 50.6 (N*CH₂*CH₂*CH*₂NBn₂), 47.7 (*CH*CH₂CH₂N), 26.0 (CH*CH₂*CH₂N), 21.7 (NCH₂*CH₂*CH₂NBn₂)
LC/MS (ES⁺) *m*/*z* 455.2 (M+H)⁺

### Preparation of 1-(1-((S)-2-(dibenzylamino)propyl)piperidin-4-yl)-1H-benzo[d]imidazol-2(3H)-one (Compound n°50):

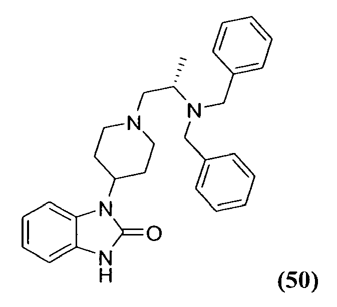

From 4-(2-keto-1-benzimidazolinyl)-piperidine and (S)-2-(dibenzylamino)-1-bromopropane
Yellow oil (6%).
preparative LCMS
LC/MS (ES⁺) *m*/*z* 455.2 (M+H)⁺

### Preparation of 1-(1-((S)-2-(dibenzylamino)-3-phenylpropyl)piperidin-4-yl)-1H-benzo[d]imidazol-2(3H)-one (Compound n°51):

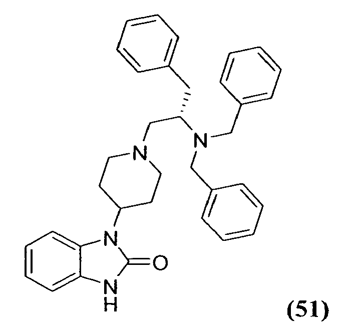

From 4-(2-keto-1-benzimidazolinyl)-piperidine and (S)-2-(dibenzylamino)-3-phenyl-1-bromopropane.
Colorless oil (4%).
preparative LCMS
LC/MS (ES⁺) *m*/*z* 631.1 (M+H)⁺

### Preparation of 1-(1-(2-(dibenzylamino)ethyl)piperidin-4-yl)-1H-benzo[d]imidazol-2(3H)-one (Compound n°52):

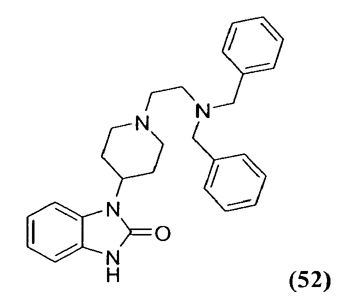

From 4-(2-keto-1-benzimidazolinyl)-piperidine and 2-(dibenzylamino)-1-bromoethane
Colorless oil (20%).
Flash chromatography on TLC preparative silica gel (cyclohexane/ethyl acetate 2/8)
¹H NMR (400 MHz, CDCl₃, ppm) δ 10.32 (s, 1H, *NH*)*,* 7.41 (d, *J* = 7.2 Hz, 4H, *Ph*)*,* 7.33 (m, 4H, *Ph*)*,* 7.25 (m, 3H, *Ph*)*,* 7.12 (m, 1H, *Ph*)*,* 7.08-7.00 (m, 2H, *Ph*)*,* 4.35 (tt, J= 12.4, 4.1 Hz, 1H, *CH*CH₂CH₂N), 3.64 (s, 4H, N*CH₂*Ph), 3.00 (d, *J* = 11.1 Hz, 1H, CHCH₂*CH₂*N), 2.62 (m, 4H, *NCH₂*CH₂NBn₂)*,* 2.43 (m, 2H, CHCH₂*CH₂*N), 2.13 (t, *J* = 11.4, 11.4 Hz, 2H, CH*CH₂*CH₂N), 1.78 (d, *J* = 11.8 Hz, 2H, CH*CH₂*CH₂N)
¹³C NMR (100 MHz, CDCl₃, ppm) δ 154.7 (C=O), 137.7, 137.6, 129.3, 128.5, 128.0, 127.6, 122.0, 121.9, 110.0, 109.9 (C_{Ph}), 58.7 (N*CH₂*Ph), 56.3 (CHCH₂*CH₂*N), 51.0, 53.5 (N*CH₂*CH₂NBn₂), 50.7 (*CH*CH₂CH₂N), 29.3 (CH*CH*₂CH₂N)
LC/MS (ES⁺) *m*/*z* 441.1 (M+H)⁺

### 17) N-sulfonyl- and N-acyl-benzimidazolone preparation:

### General procedure

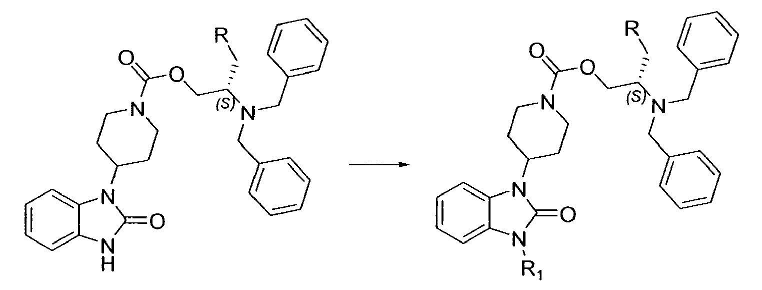

To a stirred solution of the appropriate benzimidazolone derivative (1 equiv.) in THF (20 mL/mmol) at 0°C was added NaH (60% in mineral oil) (1.2 equiv.) under N₂ atmosphere. The reaction mixture was stirred for 1h at room temperature. The appropriate sulfonyl chloride or acyl chloride or *tert-*butyloxycarbonyl anhydride (1.2 equiv.) was added and the reaction mixture was stirred overnight at room temperature. The solvent was removed on *vacuo* and the residue was purified by flash chromatography on silica gel.

### Preparation of (S)-2-(dibenzylamino)-3-phenylpropyl 4-(1,2-dihydro-3-phenylsulfonyl-2-oxobenzo[d]imidazol-1-yl)piperidine-1-carboxylate (Compound n°53):

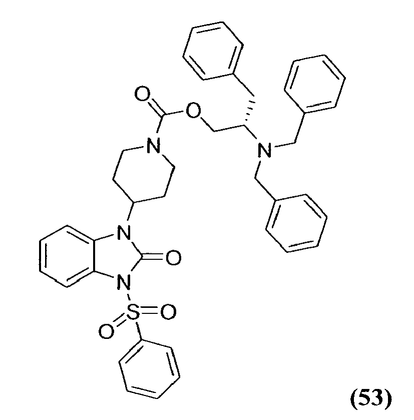

From (S)-2-(dibenzylamino)-3-phenylpropyl 4-(1,2-dihydro-2-oxobenzo[d]imidazol-3-yl)piperidine-1-carboxylate and phenylsulfonyl chloride.
White solid (80%).
Flash chromatography on preparative TLC (silica gel, cyclohexane/ethyl acetate 8/2).
¹H NMR (400 MHz, CDCl₃, ppm) δ 8.16 (d, *J* = 7.6 Hz, 2H, *Ph*), 8.04-7.96 (dd, *J =* 6.9, 2.0, 1H, *Ph*), 7.67 (t, *J* = 7.6 Hz, 1H, *Ph*), 7.55 (t, *J* = 7.6 Hz, 2H, *Ph*), 7.36-7.12 (m, 15H, *Ph*), 7.08 (m, 3H, *Ph*), 4.34 (m, 3H, *CH₂*O, C*H*CH₂CH₂N), 4.13 (dd_{b}, *J* = 11.5, 4.5 Hz, 2H, *CH₂*O, CHCH₂*CH₂*N), 3.76 (2d, *J* = 13.8 Hz, 4H, N*CH₂*Ph), 3.24 (m, 1H, *CH*CH₂Ph), 3.08 (dd, *J* = 13.7, 5.8 Hz, 1H, CH*CH₂*Ph), 2.82 (m, 2H, CHCH₂*CH₂*N), 2.68 (dd, *J* = 13.7, 8.5 Hz, 1H, CH*CH₂*Ph), 2.27 (m, 2H, CH*CH₂*CH₂N), 1.74 (d, *J* = 12.2 Hz, 2H, CH*CH₂*CH₂N)
¹³C NMR (100 MHz, CDCl₃, ppm) δ 155.0, 150.0 (C=O), 139.8, 139.5, 137.9, 134.5, 129.2, 129.1, 128.6, 128.3, 128.1, 128.0, 126.8, 126.1, 126.0, 124.0, 122.4, 113.2, 109.4 (C_{Ph}), 64.8 (*CH₂*O), 58.4 (*CH*CH₂Ph), 53.9 (N*CH₂*Ph), 51.4 (*CH*CH₂CH₂N), 43.4 (CHCH₂*CH₂*N), 34.1 (CH*CH₂*Ph), 28.6 (CH*CH₂*CH₂N)
LC/MS (ES⁺) *m*/*z* 715.1 (M+H)⁺

### Preparation of (S)-2-(dibenzylamino)propyl 4-(2-oxo-3-phenylsulfonyl-2,3-dihydro-1H-benzo[d]imidazol-1-yl)piperidine-1-carboxylate (Compound n°54):

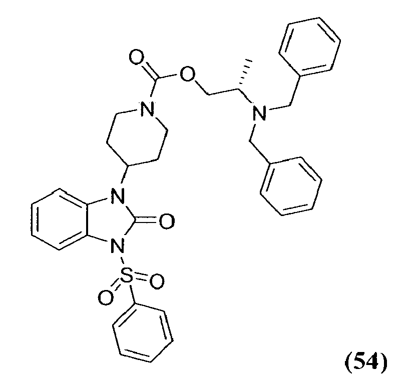

From (S)-2-(dibenzylamino)propyl 4-(1,2-dihydro-2-oxobenzo[d]imidazol-3-yl)piperidine-1-carboxylate and phenylsulfonyl chloride.
White solid (55%).
Flash chromatography on preparative TLC (silica gel, cyclohexane/ethyl acetate 7/3).
¹H NMR (400 MHz, CDCl₃) δ ppm 8.16 (d, *J* = 7.6 Hz, 2H, *Ph*), 8.00 (m, 1H, *Ph*), 7.67 (t, *J* = 7.5 Hz, 1H, *Ph*), 7.55 (t, *J* = 7.8 Hz, 2H, *Ph*), 7.37 (d, *J* = 7.3 Hz, 4H), 7.27 (m, 4H, *Ph*), 7.18 (m, 4H, *Ph*), 7.08 (m, 1H, *Ph*), 4.45-4.19 (m, 4H, *CH*CH₂*CH₂*N, O*CH₂*), 4.04 (dd, *J* = 11.1, 5.7 Hz, 1H, O*CH₂*), 3.75 (d, *J* = 13.9 Hz, 2H, N*CH₂*Ph), 3.56 (d, *J* = 13.9 Hz, 2H, N*CH₂*Ph) 3.12 (sext., *J* = 6.7 Hz, 1H, *CH*CH₃), 2.84 (m, 2H, CHCH₂*CH₂*N), 2.27 (m, 2H, CH*CH₂*CH₂N), 1.71 (m, 2H, CH*CH₂*CH₂N), 1.11 (d, *J* = 6.8 Hz, 3H, CH*CH₃*)
¹³C NMR (100 MHz, CDCl₃, ppm) δ 155.1, 150.0 (C=O), 140.2, 137.9, 134.5, 129.1, 128.5, 128.3, 128.1, 128.1, 126.8, 126.1, 124.1, 122.4, 113.2, 109.4 (C_{Ph}), 66.9 (*CH₂*O), 53.7 (N*CH₂*Ph), 51.9 (*CH*CH₃), 51.5 (*CH*CH₂CH₂N), 43.4 (CHCH₂*CH₂*N), 28.6 (CH*CH₂*CH₂N), 11,2 (CH*CH₃*)
LC/MS (ES⁺) *m*/*z* 638.9 (M+H)⁺

### Preparation of (S)-2-(dibenzylamino)propyl 4-(2-oxo-3-(4-propylphenylsulfonyl)-2,3-dihydro-1H-benzo[d]imidazol-1-yl)piperidine-1-carboxylate (Compound n°55):

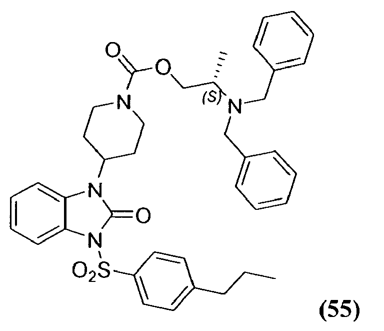

From (S)-2-(dibenzylamino)propyl 4-(1,2-dihydro-2-oxobenzo[d]imidazol-3-yl)piperidine-1-carboxylate and 4-propylphenylsulfonyl chloride.
Colorless oil (70%).
Flash chromatography on silica gel (cyclohexane/ ethyl acetate 8/2)
¹H NMR (400 MHz, CDCl₃) δ 8.04 (d, *J* = 8.4 Hz, 2H, Ar), 8.00 (d, *J =* 6.4 Hz, 1H, Ar), 7.36 (m, 6H, Ar), 7.27 (m, 4H, Ar), 7.20 *(d, J =* 6.8 Hz, 2H, Ar), 7.16 (m, 2H, Ar), 7.07 (m, 1H, Ar), 4.36-4.24 (m, 4H, CH*CH₂*CH₂Nₚᵢₚ, *CH*CH₂CH₂Nₚᵢₚ, *CH₂*O), 4.05 (m, 1H, *CH₂*O), 3.75 (d, *J* = 14.0 Hz, 2H, N*CH*₂Ph), 3.55 (d, *J* = 14.0 Hz, 2H, NCH₂Ph), 3.12 (m, 1H, CHMe), 2.85 (m, 2H, CHCH₂*CH*₂Nₚᵢₚ), 2.65 (t, *J* = 7.6 Hz, 2H, *CH₂*CH₂CH₃), 2.26 (m, 2H, CH*CH₂*CH₂Nₚᵢₚ), 1.77 (m, 2H, CH*CH*₂CH₂Nₚᵢₚ), 1.65 (dd, *J* = 7.2, 7.6 Hz, 2H, CH₂*CH₂*CH₃), 1.09 (d, J= 6.8 Hz, 3H, Me), 0.94 (t, J= 7.6 Hz, 3H, CH₂CH₂*CH₃*).
¹³C NMR (100 MHz, CDCl₃) δ 155.1 (2xC=O), 150.3, 150.0, 140.2, 135.9, 129.2, 128.5, 128.2, 128.1, 126.8, 126.2, 123.9, 122.4, 113.2, 109.4, 66.9 (*CH₂*O), 53.7 (N*CH₂*Ph), 51.9 (*CH*Me), 51.4 (*CH*CH₂CH₂Nₚᵢₚ), 43.4 (CHCH₂*CH₂*Nₚᵢₚ), 38.0 (*CH₂*CH₂CH₃), 28.6 (CH*CH₂*CH₂Nₚᵢₚ), 24.0 (CH₂*CH₂*CH₃), 13.7 (CH₂CH₂*CH₃*), 11.2 (Me).
LC/MS (ES⁺) *m*/*z* 681.3 (M+H)⁺

### Preparation of (S)-2-(dibenzylamino)propyl 4-(3-(4-fluorophenylsulfonyl)-2-oxo-2,3-dihydro-1H-benzo[d]imidazol-1-yl)piperidine-1-carboxylate (Compound n°56):

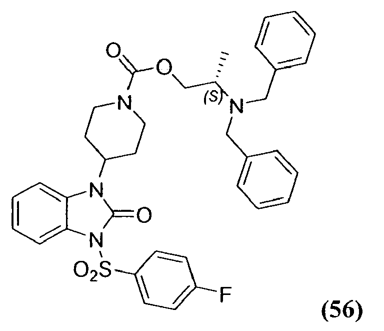

From (S)-2-(dibenzylamino)propyl 4-(1,2-dihydro-2-oxobenzo[d]imidazol-3-yl)piperidine-1-carboxylate and 4-fluorophenylsulfonyl chloride.
White solid (77%).
Flash chromatography on silica gel (cyclohexane/ ethyl acetate 8/2)
¹H NMR (400 MHz, CDCl₃) δ 8.20 (dd, *J* = 8.8, 4.8 Hz, 2H, Ar), 7.98 (m, 1H, Ar), 7.36 (m, 4H, Ar), 7.27 (m, 6H, Ar), 7.18 (m, 4H, Ar), 7.07 (m, 1H, Ar), 4.38-4.26 (m, 4H, CHCH₂*CH*₂Nₚᵢₚ, *CH*CH₂CH₂Nₚᵢₚ, *CH₂*O), 4.05 (dd, *J =* 10.8, 5.6 Hz, 1H, *CH₂*O), 3.74 (d, *J =* 13.6 Hz, 2H, N*CH*₂Ph), 3.55 (d, *J* = 13.6 Hz, 2H, N*CH₂*Ph), 3.12 (m, 1H, *CH*Me), 2.85 (m, 2H, CHCH₂*CH₂*Nₚᵢₚ), 2.27 (m, 2H, CH*CH₂*CH₂Nₚᵢₚ), 1.77 (m, 2H, CH*CH₂*CH₂Nₚᵢₚ), 1.09 (d, *J* = 6.8 Hz, 3H, Me).
¹³C NMR (100 MHz, CDCl₃) δ 166.2 (*J_{C-F}* = 256.2 Hz, C_{Ar}), 155.1 (C=O), 149.9 (C=O), 140.2, 133.8 (*J_{C-F} =* 3.1 Hz, C_{Ar}), 131.2 (*J_{C-F} =* 9.7 Hz, CH_{Ar}), 128.5, 128.3, 128.1, 126.7, 126.0, 124.2, 122.5, 116.5 (*J*_{C-F} = 22.8 Hz, CH_{Ar}), 113.2, 109.5, 66.9 (*CH₂*O), 53.7 (N*CH₂*Ph), 51.9 (*CH*Me), 51.6 (*CH*CH₂CH₂Nₚᵢₚ), 43.4 (CHCH₂*CH₂*Nₚᵢₚ), 28.6 (CH*CH₂*CH₂Nₚᵢₚ), 11.2 (Me).
LC/MS (ES⁺) *m*/*z* 657.2 (M+H)⁺

### Preparation of (S)-2-(dibenzylamino)propyl 4-(3-(4-methoxyphenylsulfonyl)-2-oxo-2,3-dihydro-1H-benzo(dlimidazol-1-yl)piperidine-1-carboxylate (Compound n°57):

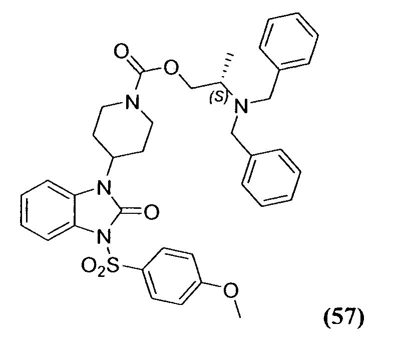

From (S)-2-(dibenzylamino)propyl 4-(1,2-dihydro-2-oxobenzo[d]imidazol-3-yl)piperidine-1-carboxylate and 4-methoxyphenylsulfonyl chloride.
Colorless oil (79%).
Flash chromatography on silica gel (cyclohexane/ ethyl acetate 8/2)
¹H NMR (400 MHz, CDCl₃) δ 8.08 (d, *J =* 9.2 Hz, 2H, Ar), 8.00 (m, 1H, Ar), 7.36 (m, 4H, Ar), 7.27 (m, 4H, Ar), 7.21 (d, *J* = 7.2 Hz, 2H, Ar), 7.16 (m, 2H, Ar), 7.07 (m, 1H, Ar), 6.98 (d, *J* = 9.2 Hz, 2H, Ar), 4.40-4.24 (m, 4H, CHCH₂*CH₂*Nₚᵢₚ, *CH*CH₂CH₂Nₚᵢₚ, *CH₂*O), 4.04 (dd, *J =* 10.8, 5.6 Hz, 1H, *CH₂*O), 3.86 (s, 3H, OMe), 3.74 (d, *J* = 14.0 Hz, 2H, N*CH₂*Ph), 3.55 (d, *J* = 14.0 Hz, 2H, N*CH₂*Ph), 3.12 (m, 1H, *CH*Me), 2.85 (m, 2H, CHCH₂*CH₂*Nₚᵢₚ), 2.26 (m, 2H, CH*CH₂*CH₂Nₚᵢₚ), 1.77 (m, 2H, CH*CH₂*CH₂Nₚᵢₚ), 1.09 (d, *J* = 6.8 Hz, 3H, Me).
¹³C NMR (100 MHz, CDCl₃) δ 164.4 (C_{Ar}), 155.1 (C=O), 150.3 (C=O), 140.2, 130.5, 129.2, 128.5, 128.2, 128.1, 126.8, 126.2, 123.9, 122.4, 114.3, 113.2, 109.4, 66.9 (*CH₂*O), 55.7 (OMe), 53.7 (N*CH₂*Ph), 51.9 (*CHMe*), 51.4 (*CH*CH₂CH₂Nₚᵢₚ), 43.4 (CHCH₂*CH₂*Nₚᵢₚ), 28.6 (CHCH₂*CH₂*Nₚᵢₚ), 11.2 (Me).
LC/MS (ES⁺) *m*/*z* 669.3 (M+H)⁺

### Preparation of (S)-2-(dibenzylamino)propyl 4-(2-oxo-3-(4-(trifluoromethyl)phenylsulfonyl)-2,3-dihydro-1H-benzo[d]imidazol-1-yl)piperidine-1-carboxylate (Compound n°58):

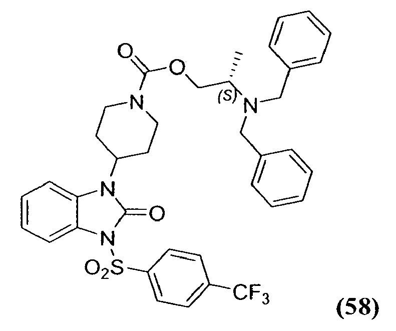

From (S)-2-(dibenzylamino)propyl 4-(1,2-dihydro-2-oxobenzo[d]imidazol-3-yl)piperidine-1-carboxylate and 4-trifluoromethylphenylsulfonyl chloride.
White solid (87%).
Flash chromatography on silica gel (cyclohexane/ ethyl acetate 8/2)
¹H NMR (400 MHz, CDCl₃) δ 8.29 (d, *J* = 8.4 Hz, 2H, Ar), 7.99 (dd, *J* = 5.6, 3.2 Hz, 1H, Ar), 7.81 (d, *J* = 8.4 Hz, 2H, Ar), 7.36 (m, 4H, Ar), 7.26 (m, 4H, Ar), 7.19 (m, 4H, Ar), 7.09 (m, 1H, Ar), 4.38-4.22 (m, 4H, CHCH₂*CH*₂Nₚᵢₚ, *CH*CH₂CH₂Nₚᵢₚ, *CH₂*O), 4.04 (dd, *J* = 10.8, 5.6 Hz, 1H, *CH₂*O), 3.74 (d, *J* = 13.6 Hz, 2H, N*CH₂*Ph), 3.55 (d, *J*= 13.6 Hz, 2H, N*CH₂*Ph), 3.12 (m, 1H, *CH*Me), 2.85 (m, 2H, CHCH₂*CH₂*Nₚᵢₚ), 2.27 (m, 2H, CH*CH₂*CH₂Nₚᵢₚ), 1.77 (m, 2H, CH*CH₂*CH₂Nₚᵢₚ), 1.09 (d, *J=* 6.8 Hz, 3H, Me).
¹³C NMR (100 MHz, CDCl₃) δ 155.1 (C=O), 149.8 (C=O), 141.1, 140.2, 136.0 (*J_{C-F}=* 33.0 Hz, C_{Ar}), 128.8, 128.5, 128.3, 128.1, 126.8, 126.3 (*J_{C-F}* = 3.7 Hz, CH_{Ar}), 125.8, 124.4, 122.7, 113.2, 109.7, 66.9 (*CH₂*O), 53.7 (N*CH₂*Ph), 51.9 (*CH*Me), 51.7 (*CH*CH₂CH₂N_{Pip}), 43.4 (CHCH₂*CH₂*N_{PiP}), 28.6 (CHCH₂*CH₂*N_{PiP}), 11.2 (Me).
LC/MS (ES⁺) *m*/*z* 707.3 (M+H)⁺

### Preparation of (S)-2-(dibenzylamino)propyl 4-(2-oxo-3-(thiophen-2-ylsulfonyl)-2,3-dihydro-1H-benzo[d]imidazol-1-yl)piperidine-1-carboxylate (Compound n°59):

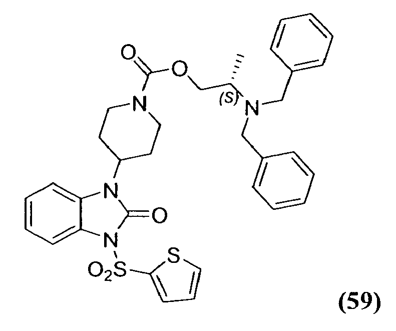

From (S)-2-(dibenzylamino)propyl 4-(1,2-dihydro-2-oxobenzo[d]imidazol-3-yl)piperidine-1-carboxytate and thlophene-2-sulfonyl chloride.
White solid (87%).
Flash chromatography on silica gel (cyclohexane/ ethyl acetate 8/2)
¹H NMR (400 MHz, CDCl₃) δ 8.00 (dd, *J* = 4.0, 1.2 Hz, 1H, Ar_{Thio}), 7.94 (m, 1H, Ar), 7.70 (dd, *J =* 4.8, 1.2 Hz, 1H, Ar_{Thio}), 7.36 (d, *J =* 7.2 Hz, 4H, Bn), 7.27 (t, *J* = 7.2 Hz, 4H, Bn), 7.22-7.14 (m, 4H, Ar), 7.13 (dd, *J =* 4.8, 4.0 Hz, 1H, Ar_{Thio}), 7.09 (m, 1H, Ar), 4.40-4.24 (m, 4H, CHCH₂*CH₂*Nₚᵢₚ, *CH*CH₂CH₂Nₚᵢₚ, *CH₂*O), 4.05 (dd, *J* = 10.8, 5.6 Hz, 1H, *CH₂*O), 3.75 (d, *J* = 14.0 Hz, 2H, N*CH*₂Ph), 3.56 (d, *J* = 14.0 Hz, 2H, N*CH₂*Ph), 3.13 (m, 1H, *CH*Me), 2.87 (m, 2H, CHCH₂*CH*₂Nₚᵢₚ), 2.29 (m, 2H, CH*CH₂*CH₂Nₚᵢₚ), 1.79 (m, 2H, CH*CH₂*CH₂Nₚᵢₚ), 1.10 (d, *J* = 6.8 Hz, 3H, Me).
¹³C NMR (100 MHz, CDCl₃) δ 155.1 (C=O), 149.8 (C=O), 140.2, 137.5, 135.1, 134.7, 128.5, 128.3, 128.1, 127.6, 126.8, 125.7, 124.2, 122.5, 113.2, 109.4, 66.9 (*CH₂*O), 53.7 *(NCH₂Ph),* 51.9 (*CH*Me), 51.6 (*CH*CH₂CH₂Nₚᵢₚ), 43.4 (CHCH₂*CH*₂Nₚᵢₚ), 28.6 (CH*CH₂*CH₂Nₚᵢₚ), 11.2 (Me).
LC/MS (ES⁺) *m*/*z* 645.3 (M+H)⁺

### Preparation of (S)-2-(dibenzvlamino)propyl 4-(2-oxo-3-(guinolin-8-ylsulfonyl)-2,3-dihydro-1H-benzo[d]imidazol-1-yl)piperidine-1-carboxylate (Compound n°60):

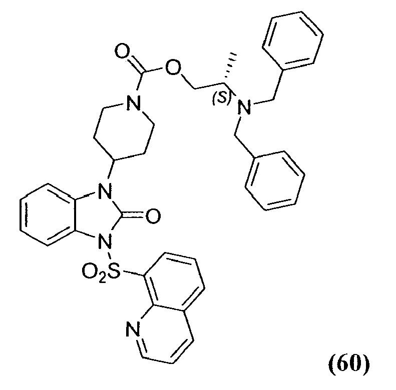

From (S)-2-(dibenzylamino)propyl 4-(1,2-dihydro-2-oxobenzo[d]imidazol-3-yl)piperidine-1-carboxylate and quinoline-8-sulfonyl chloride.
White solid (47%).
Flash chromatography on silica gel (methylene chloride/ acetone 95/5)
¹H NMR (400 MHz, CDCl₃) δ 8.78 (dd, *J* = 7.2, 0.8 Hz, 1H, Quino.), 8.56 (dd, *J=* 4.0, 1.6 Hz, 1H, Quino.), 8.20 (m, 2H, Ar), 8.12 (dd, *J* = 8.0, 0.8 Hz, 1H, Ar), 7.74 (t, *J* = 8.0 Hz, 1H, Ar), 7.39 (m, 1H, Quino.), 7.35 (d, *J* = 7.2 Hz, 4H, Bn), 7.27 (t, *J* = 7.2 Hz, 4H, Bn), 7.22-7.18 (m, 4H, Ar), 7.05 (m, 1H, Ar), 4.40-4.15 (m, 4H, CHCH₂*CH₂*Nₚᵢₚ, *CH*CH₂CH₂Nₚᵢₚ, *CH₂*O), 4.02 (dd, *J =* 10.8, 5.6 Hz, 1H, *CH₂*O), 3.72 (d, *J =* 13.6 Hz, 2H, N*CH₂*Ph), 3.54 (d, *J* = 13.6 Hz, 2H, N*CH₂*Ph), 3.09 (m, 1H, *CH*Me), 2.77 (m, 2H, CHCH₂*CH₂*Nₚᵢₚ), 2.18 (m, 2H, CH*CH₂*CH₂Nₚᵢₚ), 1.62 (bd, 2H, CH*CH₂*CH₂Nₚᵢₚ), 1.08 (d, J= 6.4 Hz, 3H, Me).
¹³C NMR (100 MHz, CDCl₃) δ 155.1 (C=O), 151.3 (C=O), 150.3 (Quino.), 143.8, 140.2, 136.3, 135.0, 134.8, 134.1, 128.5, 128.4, 128.1, 128.0, 127.8, 126.8, 125.5, 123.4, 122.1, 114.9, 109.0, 66.9 (*CH₂*O), 53.7 (N*CH₂*Ph), 51.9 (CHMe), 51.2 (*CH*CH₂CH₂Nₚᵢₚ), 43.4 (CHCH₂*CH₂*Nₚᵢₚ), 28.5 (CH*CH₂*CH₂Nₚᵢₚ), 11.2 (Me).
LC/MS (ES⁺) *m*/*z* 690.3 (M+H)⁺

### Preparation of (S)-2-(dibenzylamino)propyl 4-(3-(naphthalen-1-ylsulfonyl)-2-oxo-2,3-dihydro-1H-benzo[d]imidazol-1-yl)piperidine-1-carboxylate (Compound n°61):

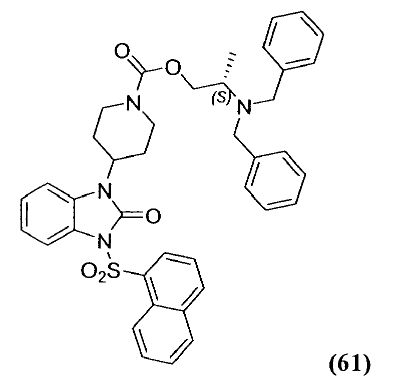

From (S)-2-(dibenzylamino)propyl 4-(1,2-dihydro-2-oxobenzo[d]imidazol-3-yl)piperidine-1-carboxylate and naphthalene-1-sulfonyl chloride.
White solid (56%).
Flash chromatography on silica gel (cyclohexane/ ethyl acetate 8/2)
¹H NMR (400 MHz, CDCl₃) δ 8.66 (d, *J=* 7.2 Hz, 1H, Napht.), 8.56 (m, 1H, Napht.), 8.15 (dd, *J* = 11.6, 8.0 Hz, 2H, Napht.), 7.94 (m, 1H, Ar), 7.66 (t, *J* = 8.0 Hz, 1H, Napht.), 7.39 (m, 2H, Napht.), 7.35 (d, *J =* 7.2 Hz, 4H, Bn), 7.26 (t, *J =* 6.8 Hz, 4H, Bn), 7.20-7.17 (m, 4H, Ar), 7.09 (m, 1H, Ar), 4.28-4.19 (m, 4H, CHCH₂*CH₂*Nₚᵢₚ, *CH*CH₂CH₂Nₚᵢₚ, *CH₂*O), 4.01 *(dd, J =* 10.8, 5.6 Hz, 1H, *CH₂*O), 3.73 (d, *J* = 13.6 Hz, 2H, N*CH₂*Ph), 3.54 (d, *J* = 13.6 Hz, 2H, N*CH₂*Ph), 3.10 (m, 1H, *CH*Me), 2.78 (m, 2H, CHCH₂*CH₂*Nₚᵢₚ), 2.18 (m, 2H, CH*CH₂*CH₂Nₚᵢₚ), 1.67 (bd, 2H, CH*CH₂*CH₂Nₚᵢₚ), 1.08 (d, *J=* 6.8 Hz, 3H, Me).
¹³C NMR (100 MHz, CDCl₃) δ 155.0 (C=O), 149.8 (C=O), 140.2, 136.2, 134.0, 132.9, 132.6, 129.2, 128.9, 128.5, 128.2, 128.1, 126.9, 126.8, 126.5, 124.1, 124.0, 123.4, 122.5, 113.4, 109.6, 66.9 (*CH₂*O), 53.7 (N*CH₂*Ph), 51.9 (*CH*Me), 51.4 (*CH*CH₂CH₂Nₚᵢₚ), 43.3 (CHCH₂*CH₂*Nₚᵢₚ), 28.5 (CH*CH₂*CH₂Nₚᵢₚ), 11.2 (Me).
LC/MS (ES⁺) *m*/*z* 689.3 (M+H)⁺

### Preparation of (S)-2-(dibenzylamino)propyl 4-(2-oxo-3-methylsulfonyl-2,3-dihydro-1H-benzo[d]imidazol-1-yl)piperidine-1-carboxylate (Compound n°62):

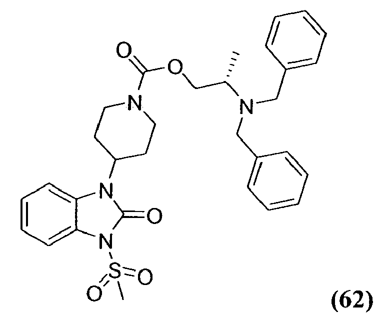

From (S)-2-(dibenzylamino)propyl 4-(1,2-dihydro-2-oxobenzo[d]imidazol-3-yl)piperidine-1-carboxylate and methylsulfonyl chloride.
White solid (45%).
Flash chromatography on preparative TLC (silica gel, cyclohexane/ethyl acetate 1/1)
¹H NMR (400 MHz, CDCl₃, ppm) δ 7.84 (d, *J* = 7.5 Hz, 1H, *Ph*), 7.38 (d, *J* = 7.4 Hz, 4H, *Ph*), 7.28 (m, 4H, *Ph*), 7.16 (m, 5H, *Ph*), 4.45 (tt, *J* = 12.3, 3.7 Hz, 1H, *CH*CH₂CH₂N), 4.39 (m, 2H, CHCH₂*CH₂*N), 4.27 (dd, *J* = 11.1, 7.6 Hz, 1H, O*CH₂*), 4.07 (dd, *J* = 11.1, 5.7 Hz, 1H, O*CH₂*), 3.76 (d, *J* = 13.9 Hz, 2H, N*CH₂*Ph), 3.57 (d, *J* = 13.9 Hz, 2H, N*CH₂*Ph), 3.52 (s, 3H, SO₂*CH₃*), 3.15 (sext., *J =* 6.7 Hz, 1H, *CH*CH₃), 2.93 (m, 2H, CHCH₂*CH₂*N), 2.36 (m, 2H, CHCH₂*CH₂*N), 1.88 (d, *J* = 10.1 Hz, 2H, CH*CH₂*CH₂N), 1.11 (d, *J* = 6.8 Hz, 3H, CH*CH₃*)
¹³C NMR (100 MHz, CDCl₃, ppm) δ 155.1, 150.6 (C=O), 140.2, 128.5, 128.1, 126.8, 126.0, 124.1, 122.5, 113.2, 109.5 (C_{Ph}), 66.9 (*CH₂*O), 53.7 (N*CH₂*Ph), 51.9 (*CH*CH₃), 51.6 (*CH*CH₂CH₂N), 43.4 (CHCH₂*CH₂*N), 41.7 (SO₂*CH₃*), 28.9 (CH*CH₂*CH₂N), 11,2 (CH*CH₃*)
LC/MS (ES⁺) *m*/*z* 576.9 (M+H)⁺

### Preparation of (S)-2-(dibenzylamino)propyl 4-(2-oxo-3-(4-acetamidylphenylsulfonyl)-2,3-dihydro-1H-benzo[d]imidazol-1-yl)piperidine-1-carboxylate (Compound n°63):

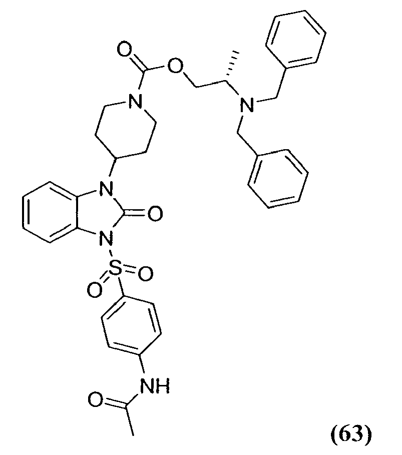

From (S)-2-(dibenzylamino)propyl 4-(1,2-dihydro-2-oxobcnzo[d]imidazol-3-yl)pipcridinc- I -carboxylate and 4-acetylaminophenylsulfonyl chloride.
Yellow oil (56%).
Flash chromatography on preparative TLC (silica gel, cyclohexane/ethyl acetate 1/1)
¹H NMR (400 MHz, CDCl₃, ppm) δ 8.58 (s, 1H, *NH*), 8.02 (d, *J* = 8.8 Hz, 2H, *Ph*), 7.97 (dd, *J* = 6.2, 3.0 Hz, 1H, *Ph*), 7.72 (d, *J =* 8.8 Hz, 2H, *Ph*), 7.36 (d, *J* = 7.3 Hz, 4H, *Ph*), 7.26 (t, *J* = 7.4Hz, 4H, *Ph*), 7.17 (m, 4H, *Ph*), 7.07 (m, 1H, *Ph*), 4.32 (tt_{b}, *J* = 12.3, 4.1 Hz, 2H, *CH*CH₂CH₂N), 4.24 (m, 2H, CHCH₂*CH₂*N, O*CH₂*), 4.02 (dd, *J* = 11.1, 5.7 Hz, 1H, O*CH₂*), 3.74 (d, *J* = 13.9 Hz, 2H, N*CH₂*Ph), 3.54 (d, *J* = 13.9 Hz, 2H, N*CH₂*Ph), 3.12 (sext., *J* = 6.7 Hz, 1H, *CH*CH₃), 2.89 (m, 2H, CHCH₂*CH₂*N), 2.29 (m, 2H, CH*CH₂*CH₂N), 2.18 (s, 3H, CO*CH₃*), 1.75 (m, 2H, CHCH₂*CH₂*N), 1.08 (d, *J* = 6.7 Hz, 3H, CH*CH₃*)
¹³C NMR (100 MHz, CDCl₃, ppm) δ 169.2 (NHC=O), 155.2, 150.1 (C=O), 144.1, 140.1, 131.7, 129.4, 128.5, 128.2, 128.1, 126.8, 126.0, 124.1, 122.6, 119.2, 113.2, 109.4 (C_{Ph}), 66.9 (*CH₂*O), 53.6 (N*CH₂*Ph), 51.8 (*CH*CH₃), 51.5 (*CH*CH₂CH₂N), 43.4 (CHCH₂*CH₂*N), 28.5 (CH*CH₂*CH₂N), 24.2 (NHCO*CH₃*), 11,0 (CH*CH₃*)
LC/MS (ES⁺) *m*/*z* 696.3 (M+H)⁺

### Preparation of (S)-2-(dibenzylamino)propyl 4-(1,2-dihydro-2-oxo-1-(benzoyl)benzo[d]imidazol-3-yl)piperidine-1-carboxylate (Compound n°64):

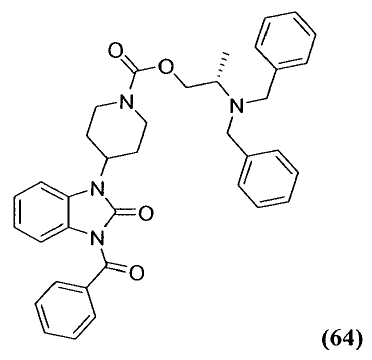

From (S)-2-(dibenzylamino)propyl 4-(1,2-dihydro-2-oxobenzo[d]imidazol-3-yl)piperidine-2-carboxylate and benzoyl chloride.
White solid (67%).
Flash chromatography on TLC preparative, silica gel (cyclohexane/ethyl acetate 7/3)
'H NMR (400 MHz, CDCl₃) δ ppm 8.00 (dd, *J* = 6.4, 2.4 Hz, 1 H, *Ph),* 7.79 (d, *J* = 7.3 Hz, 2H, *Ph),* 7.61 (t, *J* = 7.5 Hz, 1 H, *Ph),* 7.49 (m, 2H, *Ph*), 7.39 (d, *J* = 7.3 Hz, 4H), 7.28 (m, 4H, *Ph),* 7.20 (m, 5H, *Ph),* 4.43 (tt, *J* = 12.2, 3.6 Hz, 1 H, *CH*CH₂CH₂N), 4.34 (m, 2H, CHCH₂*CH₂*N), 4.26 (m, 1H, O*CH₂*), 4.06 (dd, *J =* 11.1, 5.7 Hz, 1 H, O*CH₂*), 3.77 (d, *J* = 13.9 Hz, 2H, N*CH₂*Ph), 3.57 (d, *J* = 13.9 Hz, 2H, N*CH₂*Ph), 3.14 (sext., *J* = 6.7 Hz, 1 H, *CH*CH₃), 2.89 (m, 2H, CHCH₂*CH₂*N), 2.37 (m, 2H, CH*CH₂*CH₂N), 1.87 (d, *J* = 10.7 Hz, 2H, CH*CH₂*CH₂N), 1.12 (d, *J* = 6.8 Hz, 3H, CH*CH₃*)
¹³C NMR (100 MHz, CDC1₃, ppm) δ 168.9, 155.1, 151.4 (C=O), 140.2, 133.8, 132.6, 130.0, 129.2, 129.0, 128.5, 128.4, 128.1, 128.0, 126.9, 126.8, 124.3, 122.4, 114.9, 109.1 (C_{Ph}), 66.9 *(CH₂*O)*,* 53.7 (N*CH₂*Ph), 51.9 (*CH*CH₃), 51.5 (*CH*CH₂CH₂N), 43.5 (CHCH₂*CH₂*N), 28.7 (CH*CH₂*CH₂N), 11,2 *(CHCH₃*)
LC/MS (ES⁺) *m*/*z* 602.9 (M+H)⁺

### Preparation of (S)-2-(dibenzylamino)propyl 4-(1-acetyl-1,2-dihydro-2-oxobenzo[d]imidazol-3-yl)piperidine-1-carboxylate (Compound n°65):

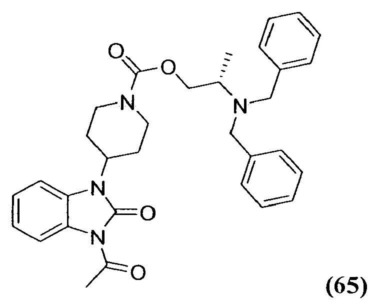

From (S)-2-(dibenzylamino)propyl 4-(1,2-dihydro-2-oxobenzo[d]imidazol-3-yl)piperidine-1-carboxylate and acetyl chloride.
White solid (68%).
Flash chromatography on preparative TLC (silica gel, cyclohexane/ethyl acetate 7/3)
¹H NMR (400 MHz, CDCl₃) δ ppm 8.26 (d, *J* = 7.6 Hz, 1H, *Ph*), 7.39 (d, *J* = 7.4 Hz, 4H, *Ph*), 7.29 (m, 4H, *Ph*), 7.16 (m, 5H, *Ph*), 4.47 (tt, *J* = 12.2, 3.6 Hz, 1H, *CH*CH₂CH₂N), 4.42 (m, 2H, CHCH₂*CH*₂N), 4.27 (dd, *J* = 11.0, 7.5 Hz, 1H, O*CH₂*), 4.08 (dd, *J* = 11.0, 5.7 Hz, 1H, O*CH₂*), 3.77 (d, *J* = 13.9 Hz, 2H, N*CH₂*Ph), 3.57 (d, *J* = 13.9 Hz, 2H, N*CH₂*Ph), 3.15 (sext., *J* = 6.6 Hz, 1H, *CH*CH₃), 2.94 (m, 2H, CHCH₂*CH₂*N), 3.52 (s, 3H, CO*CH₃*), 2.39 (m, 2H, CH*CH₂*CH₂N), 1.87 (d, *J* = 10.6 Hz, 2H, CH*CH₂*CH₂N), 1.12 (d, *J* = 6.8 Hz, 3H, CH*CH₃*)
¹³C NMR (100 MHz, CDCl₃, ppm) δ 170.5 (C=O), 155.1 (C=O), 151.8 (C=O), 140.2, 128.5, 128.1, 126.8, 126.3, 124.4, 122.5, 116.0, 108.7 (C_{Ph}), 66.9 (*CH₂*O), 53.7 (N*CH₂*Ph), 51.9 (*CH*CH₃), 51.3 (*CH*CH₂CH₂N), 43.5 (CHCH₂*CH₂*N), 28.6 (CH*CH₂*CH₂N), 25.8 (CO*CH₃*), 11,2 (CH*CH₃*)
LC/MS (ES⁺) *m*/*z* 540.9 (M+H)⁺

### Preparation of (S)-2-(dibenzylamino)propyl 4-(1,2-dihydro-2-oxo-1-(pivaloyl)benzo[d]imidazol-3-yl)piperidine-1-carboxylate (Compound n°66):

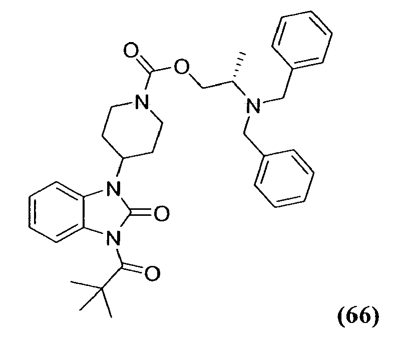

From (S)-2-(dibenzylamino)propyl 4-(1,2-dihydro-2-oxobcnzo[d]imidazol-3-yl)pipcridine-1-carboxylate and pivaloyl chloride.
White solid (6%) instable on silica gel.
HPLC preparative
LC/MS (ES⁺) *m*/*z* 483.3 (M+H)⁺

### Preparation of tert-butyl 3-(1-(((S)-2-(dibenzylamino) propoxy)carbonyl)piperidin-4-yl)-2,3-dihydro-2-oxobenzo[dlimidazole-1-carboxylate (Compound n°67):

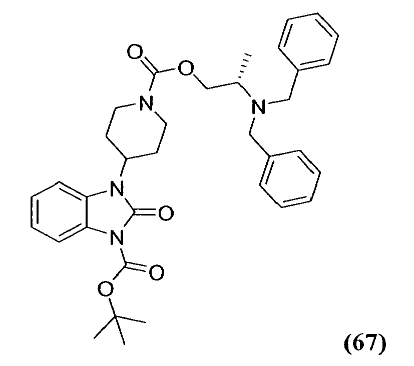

From (S)-2-(dibenzylamino)propyl 4-(1,2-dihydro-2-oxobcnzo[d]imidazol-3-yl)piperidine-1-carboxylate and di-*tert*-butyl-dicarbonate.
White solid (63%).
Flash chromatography on silica gel (cyclohexane/ ethyl acetate 7/3)
¹H NMR (400 MHz, CDCl₃, ppm) δ 7.89 (dd, *J* = 7.2, 1.6 Hz, 1H, *Ph),* 7.38 (d, *J* = 7.3 Hz, 4H, *Ph*)*,* 7.28 (dd, *J* = 12.1, 4.4 Hz, 4H, *Ph*)*,* 7.21 (m, 2H, *Ph*)*,* 7.10 (m, 3H, *Ph*)*,* 4.52 (tt, *J* = 12.6, 3.8 Hz, 1H, *CH*CH₂CH₂N), 4.38 (m, 2H, CHCH₂*CH₂*N), 4.26 (dd, *J* = 11.0, 7.7 Hz, 1H, O*CH₂*)*,* 4.07 *(dd, J* = 11.0, 5.8 Hz, 1H, O*CH₂*)*,* 3.76 (d, *J* = 13.9 Hz, 2H, N*CH₂*Ph), 3.57 (d, *J* = 13.9 Hz, 2H, N*CH₂*Ph), 3.14 (sext., *J* = 6.7 Hz, 1H, *CH*CH₃), 2.92 (m, 2H, CHCH₂*CH₂*N), 2.37 (m, 2H, CH*CH₂*CH₂N), 1.83 (m, 2H, CH*CH₂*CH₂N), 1.69 (s, 9H, *t*Bu), 1.11 (d, *J* = 6.8 Hz, 3H, CH*CH₃*)
¹³C NMR (100 MHz, CDCl₃, ppm) δ 155.1, 150.5 (C=O), 148.8 (C=O), 140.2, 128.5, 128.1, 128.1, 126.8, 126.3, 123.7, 121.9, 114.6, 108.8 (C_{Ph}), 84.7 (C_{q}*t*Bu), 66.8 (*CH₂*O), 53.7 (N*CH₂*Ph), 51.9 (*CH*CH₃), 51.1 (*CH*CH₂CH₂N), 43.5 (CHCH₂*CH₂*N), 28.7 (*t*Bu), 26.9 (CH*CH₂*CH₂N), 11,2 (CH*CH₃*)
LC/MS (ES⁺) *m*/*z* 599.2 (M+H)⁺

### 18) N-alkyl benzimidazolone preparation:

To a stirred solution of (0.48 mmol, 1 equiv.) of benzimidazolone derivative in 1 ml of anhydrous DMF was added at 0°C 21 mg (0.53mmol, 1.1 equiv.) of sodium hydride. The reaction was pursued for I hour. An appropriate bromide (in these cases, 0,1 equiv. of potassium iodide was added) or iodide in 1 ml of DMF was added at 0°C. The reaction was allowed to warm to room temperature overnight. The mixture was evaporated on vacuum at room temperature and purified on silica gel.

### Preparation of (S)-2-(dibenzylamino)-3-phenylpropyl 4-(1,2-dihydro-1-methyl-2-oxobenzo[d]imidazol-3-yl)piperidine-1-carboxylate (Compound n°68):

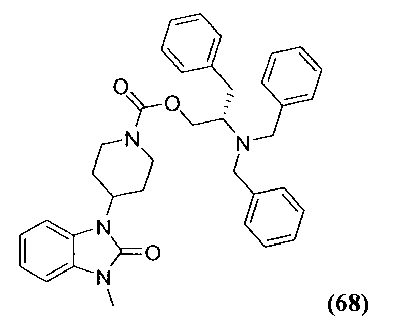

From (S)-2-(dibenzylamino)-3-phenylpropyl 4-(1,2-dihydro-2-oxobenzo[d]imidazol-3-yl)piperidine-1-carboxylate and methyliodide.
White solid (85%).
Flash chromatography on preparative TLC (silica gel, cyclohexane/ethyl acetate 1/1)
¹H NMR (400 MHz, CDCl₃, ppm) δ 7.34-7.17 (m, 13H, *Ph*), 7.15-6.97 (m, 6H, *Ph*)*,* 4.54 (tt, *J* = 12.4, 3.8 Hz, 1H, *CH*CH₂CH₂N), 4.42 (m, 1H, CHCH₂*CH₂*N), 4.36 (dd, *J =* 11.2, 7.0 Hz, 1H, *CH₂*O)*,* 4.26-4.09 (dd_{b}, *J* = 11.2, 4.8 Hz, 2H, *CH₂*O*,* CHCH₂*CH₂*N), 3.78 (2d, *J* = 13.8 Hz, 4H, N*CH₂*Ph), 3.43 (s, 3H, *NCH₃*)*,* 3.28 (m, 1H, *CH*CH₂Ph), 3.09 *(dd, J* = 13.7, 5.9 Hz, 1H, CH*CH₂*Ph), 2.90 (m, 2H, CHCH₂*CH₂*N), 2.70 (dd, *J* = 13.7, 8.5 Hz, 1H, CH*CH₂*Ph), 2.33 (m, 2H, CH*CH₂*CH₂N), 1.85 (d, *J* = 12.6 Hz, 2H, CH*CH₂*CH₂N)
¹³C NMR (100 MHz, CDCl₃, ppm) δ 155.1, 153.8 (C=O), 139.8, 139.5, 130.1, 129.2, 128.6, 128.3, 128.1, 127.9, 126.8, 126.0, 121.1, 121.0, 109.0, 107.6 (C_{Ph}), 64.7 (*CH₂O*), 58.4 (*CH*CH₂Ph), 53.9 (N*CH₂*Ph), 50.9 (*CH*CH₂CH₂N), 43.6 (CHCH₂*CH₂*N), 34.1 (CH*CH₂*Ph), 29.2 (CH*CH₂*CH₂N), 27.1 (N*CH₃*)
LC/MS (ES⁺) *m*/*z* 589.3 (M+H)⁺

### Preparation of (S)-2-(dibenzylamino)propyl 4-(1,2-dihvdro-1-methyl-2-oxobenzo(dlimidazol-3-yl)piperidine-1-carboxylate (Compound n°69):

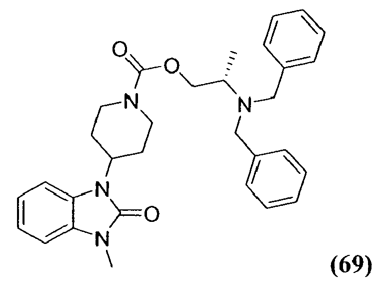

From (S)-2-(dibenzylamino)propyl 4-(1,2-dihydro-2-oxobcnzo[d]imidazol-3-yl)pipcridinc-1-carboxylate and methyliodide.
White solid (95%).
Flash chromatography on silica gel (cyclohexane/ ethyl acetate 1/1)
¹H NMR (400 MHz, CDCl₃, ppm) δ 7.38 (d, *J* = 7.3 Hz, 4H, *Ph),* 7.28 (m, 4H, *Ph),* 7.21 (m, 2H, *Ph),* 7.06 (m, 4H, *Ph),* 4.53 (tt, *J* = 12.4, 3.7 Hz, 1H, CHCH₂CH₂N), 4.49-4.30 (m, 2H, CHCH₂CH₂N), 4.27 (dd, *J* = 11.0, 7.5 Hz, 1H, *CH₂*O)*,* 4.07 *(dd, J* = 11.0, 5.7 Hz, 1H, *CH₂*O)*,* 3.76 (d, *J* = 13.9 Hz, 2H, N*CH*₂Ph), 3.57 (d, *J* = 13.9 Hz, 2H, N*CH₂*Ph), 3.43 (s, 3H, N*CH₃*), 3.14 (sext., *J* = 6.7 Hz, 1H, *CH*CH₃), 2.94 (m, 2H, CHCH₂*CH₂*N), 2.31 (m, 2H, CH*CH₂*CH₂N), 1.86 (d, *J* = 6.8 Hz, 2H, CH*CH₂*CH₂N), 1.12 (d, *J* = 6.8 Hz, 3H, CH*CH₃*)
LC/MS (ES⁺) *m*/*z* 512.9 (M+H)⁺

### Preparation of (S)-2-(dibenzylamino)-3-phenylpropyl 4-(1-benzyl-1,2-dihydro-2-oxobenzo[d]imidazol-3-yl)piperidine-1-carboxylate (Compound n°70):

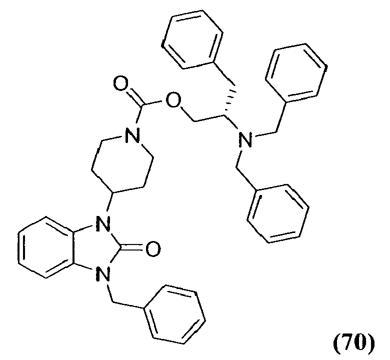

From (S)-2-(dibenzylamino)-3-phenylpropyl 4-(1,2-dihydro-2-oxobenzo[d]imidazol-3-yl)piperidine-1-carboxylate and benzylbromide.
White solid (92%).
Flash chromatography on silica gel (cyclohexane/ ethyl acetate 8/2)
¹H NMR (400 MHz, CDCl₃, ppm) δ 7.37-7.17 (m, 18H, *Ph*), 7.09 (d, *J* = 7.0 Hz, 3H, *Ph*), 7.00 (m, 2H, *Ph*), 6.91 (m, 1H, *Ph*), 5.08 (s, 2H, N*CH₂*Ph), 4.58 (tt, *J =* 12.5, 3.8 Hz, 1H, *CH*CH₂CH₂N), 4.44 (m, 1H, CHCH₂*CH₂*N), 4.35 (dd, *J* = 11.2, 7.1 Hz, 1H, O*CH₂*), 4.20 (m, 2H, *CH₂*O, CHCH₂*CH₂*N), 3.81 (d, *J* = 13.8 Hz, 2H, N*CH₂*Ph), 3.74 (d, *J* = 13.8 Hz, 2H, N*CH₂*Ph), 3.26 (m, 1H, *CH*CH₂Ph), 3.09 (dd, *J =* 13.7, 5.9 Hz, 1H, CH*CH₂*Ph), 2.90 (m, 2H, CHCH₂*CH₂*N), 2.70 (dd, *J* = 13.7, 8.5 Hz, 1H, CH*CH₂*Ph), 2.35 (m, 2H, CH*CH₂*CH₂N), 1.89 (d, *J* = 11.1 Hz, 2H, CH*CH₂*CH₂N)
¹³C NMR (100 MHz, CDCl₃, ppm) δ 155.1, 153.8 (C=O), 139.8, 139.5, 136.1, 129.3, 129.2, 128.7, 128.6, 128.3, 128.1, 128.0, 127.7, 127.4, 126.8, 126.0, 121.1, 121.0, 109.2, 108.5 (C_{Ph}), 64.7 (*CH₂*O), 58.4 (*CH*CH₂Ph), 53.9 (N*CH₂*Ph), 51.0 (*CH*CH₂CH₂N), 44.9 (O=CN*CH₂*Ph), 43.6 (CHCH₂*CH₂*N), 34.1 (CH*CH₂*Ph), 29.2 (CH*CH₂*CH₂N)
LC/MS (ES⁺) *m*/*z* 665.4 (M+H)⁺

### Preparation of (S)-2-(dibenzylamino)propyl 4-(1-benzyl-1,2-dihydro-2-oxobenzoldlimidazol-3-yl)piperidine-1-carboxylate (Compound n°71):

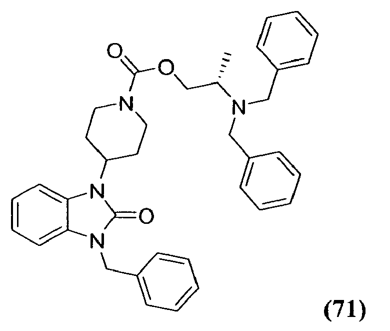

From (S)-2-(dibenzylamino)propyl 4-(1,2-dihydro-2-oxobenzo[d]imidazol-3-yl)piperidine-1-carboxylate and benzylbromide.
Yellow oil (73%).
Flash chromatography on preparative TLC (silica gel, cyclohexane/ethyl acetate 7/3)
¹H NMR (400 MHz, CDCl₃, ppm) δ 7.40 (d, *J* = 7.3 Hz, 4H, *Ph*), 7.31 (m, 9H, *Ph*), 7.22 (t, *J* = 7.2 Hz, 2H, *Ph*), 7.13 (m, 1H, *Ph*), 7.02 (m, 2H, *Ph*), 6.93 (m, 1H, *Ph*), 5.09 (s, 2H, N*CH₂*Ph), 4.60 (tt, *J* = 12.4, 3.8 Hz, 1H, *CH*CH₂CH₂N), 4.39 (m, 2H, CHCH₂*CH₂*N), 4.28 *(dd, J* = 11.0, 7.6 Hz, 1H, O*CH₂*), 4.08 (dd, *J* = 11.0, 5.7 Hz, 1H, O*CH₂*), 3.78 (d, *J* = 13.9 Hz, 2H, N*CH₂*Ph), 3.58 (d, *J* = 13.9 Hz, 2H, N*CH₂*Ph), 3.16 (sext., *J* = 6.7 Hz, 1H, *CH*CH₃), 2.95 (m, 2H, CHCH₂*CH₂*N), 2.38 (m, 2H, CH*CH₂*CH₂N), 1.91 (d, *J* = 10.2 Hz, 2H, CH*CH₂*CH₂N), 1.13 (d, *J* = 6.8 Hz, 3H, CH*CH₃*)
¹³C NMR (100 MHz, CDCl₃, ppm) δ 155.2, 153.8 (C=O), 140.2, 136.2, 129.3, 128.7, 128.5, 128.1, 128.0, 127.7, 127.5, 126.8, 121.1, 121.0, 109.2, 108.5 (C_{Ph}), 64.8 (*CH₂*O), 53.4 (N*CH₂*Ph), 51.9 (*CH*CH₃), 51.1 (*CH*CH₂CH₂N), 44.9 (O=CN*CH₂*Ph), 43.6 (CHCH₂*CH₂*N), 29.2 (CH*CH₂*CH₂N), 11.3 (CH*CH₃*)
LC/MS (ES⁺) *m*/*z* 588.9 (M+H)⁺

### Preparation of (S)-2-(dibenzylamino)propyl 4-(1-(cyclohexylmethyl)-1,2-dihydro-2-oxobenzo[d]imidazol-3-yl)pieridine-1-carboxylate (Compound n°72):

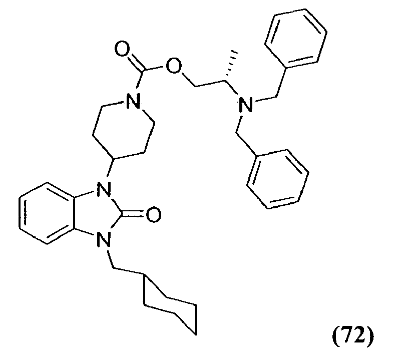

From (S)-2-(dibenzylamino)propyl 4-(1,2-dihydro-2-oxobenzo[d]imidazol-3-yl)piperidine-1-carboxylate and cyclohexylmethylbromide.
Yellow oil (29%).
Flash chromatography on preparative TLC (silica gel, cyclohexane/ethyl acetate 1/1)
¹H NMR (400 MHz, CDCl₃, ppm) δ 7.38 (d, *J* = 7.3 Hz, 4H, *Ph),* 7.28 (t, *J* = 7.2, 4H, *Ph*), 7.21 (t, *J* = 7.2 Hz, 2H, *Ph),* 7.16-6.98 (m, 4H, *Ph),* 4.60 (tt, *J =* 12.4, 3.7 Hz, 1H, *CH*CH₂CH₂N, 1H), 4.39 (m, 2H, CHCH₂*CH*₂N), 4.26 (dd, *J* = 11.0, 7.7 Hz, I H, O*CH₂*), 4.08 (dd, *J* = 11.0, 5.7 Hz, 1H, O*CH₂*), 3.76 (d, *J* = 13.9 Hz, 2H, N*CH*₂Ph), 3.70 (d, *J* = 7.3 Hz, 2H, N*CH₂*C₆H₁₁), 3.57 (d, *J* = 13.9 Hz, 2H, N*CH₂*Ph), 3.14 (sext., *J* = 6.7 Hz, 1H, *CH*CH₃), 2.94 (m, 2H, CHCH₂*CH₂*N), 2.26 (m, 2H, CH*CH₂*CH₂N), 1.87 (m, 3H, CH*CH₂*CH₂N, *C₆H₁₁*), 1.67 (m, 3H, *C₆H₁₁*), 1.23 (m, 4H, *C₆H₁₁*), 1.09 (m, 6H, *C₆H₁₁,* CH*CH₃*)
¹³C NMR (100 MHz, CDCl₃, ppm) δ 155.2, 153.9 (C=O), 140.2, 130.0, 128.5, 128.1, 127.9, 126.8, 120.8, 109.0, 108.1 (C_{Ph}), 66.8 (*CH₂*O), 53.4 (N*CH₂*Ph), 51.9 (*CH*CH₃), 50.8 (*CH*CH₂CH₂N), 47.4 (N*CH₂*C₆H₁₁), 43.6 (CHCH₂*CH₂*N), 37.1 (NCH₂*CH*(CH₂)₅), 30.8 (NCH₂CH(*CH₂*)₅), 29.2 (CH*CH₂*CH₂N), 26.2, 25.7 (NCH₂CH(*CH₂*)₅), 11.2 (CH*CH*₃)
LC/MS (ES⁺) *m*/*z* 595.0 (M+H)⁺

### Preparation of (S)-2-(dibenzyiamino)propyl 4-(3-(2-tert-butyloxycarbonylaminoethyl)-2-oxo-2,3-dihydro-1H-benzo[d]imidazol-1-yl)piperidine-1-carboxylate (Compound n°73):

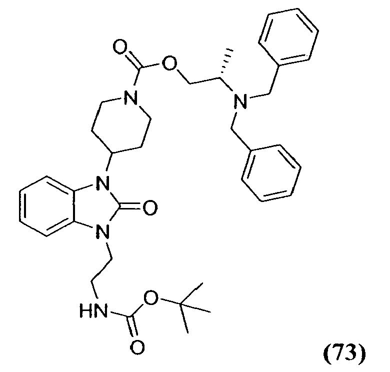

From (S)-2-(dibenzylamino)propyl 4-(1,2-dihydro-2-oxobenzo[d]imidazol-3-yl)piperidine-1-carboxylate and 2-*tert-*butyloxycarbonylamino-1-bromoethane.
Yellow oil (32%).
Flash chromatography on preparative TLC (silica gel, dichloromethane/ methyl alcohol 95/5)
¹H NMR (400 MHz, CDCl₃, ppm) δ 7.38 (d, *J* = 7.3 Hz, 4H, *Ph*), 7.34-7.24 (2d, *J* = 7.2, 7.9 Hz, 4H, *Ph*), 7.20 (t, *J* = 7.2 Hz, 2H, *Ph*), 7.07 (m, 4H, *Ph*), 4.92 (s, 1H, *NH*), 4.52 (tt, *J =* 12.3, 3.7 Hz, 1H, *CH*CH₂CH₂N), 4.37 (m, 2H, CHCH₂*CH₂*N), 4.27 (dd, *J* = 11.0, 7.4 Hz, 1H, *CH₂*O), 4.03 (m, 3H, *CH₂*O, N*CH₂*CH₂NH), 3.77 (d, *J* = 13.9 Hz, 2H, N*CH₂*Ph), 3.57 (d, *J* = 13.9 Hz, 2H, N*CH₂*Ph), 3.47 (dd, *J* = 11.4, 5.6 Hz, 2H NCH₂*CH₂*NH), 3.15 (sext., *J* = 6.8 Hz, 1H, *CH*CH₃), 2.94 (t_{b}, *J* = 12.0 Hz, 2H, CHCH₂*CH₂*N), 2.34 (m, 2H, CH*CH₂*CH₂N), 1.87 (d, *J* = 9.2 Hz, 2H, CH*CH₂*CH₂N), 1.42 (s, 9H, OtBu), 1.11 (d, *J* = 6.8 Hz, 3H, CH*CH₃*)
¹³C NMR (100 MHz, CDCl₃, ppm) δ 155.9, 155.2, 153.8 (C=O), 140.2, 129.7, 128.5, 128.1, 128.0, 127.9, 127.8, 126.8, 121.2, 121.1, 109.1, 108.0 (C_{Ph}), 79.5 (O*C_{q}*(CH₃)₃), 66.8 (*CH₂*O), 53.7 (N*CH₂*Ph), 53.3 (N*CH₂*CH₂NH), 51.9 (*CH*CH₃), 50.9 (*CH*CH₂CH₂N), 43.6 (CHCH₂*CH₂*N), 40.8, 39.6 (NCH₂*CH₂*NH), 29.2 (CH*CH₂*CH₂N), 28.3 (OC_{q}(*CH₃*)₃), 11,3 (CH*CH₃*)
LC/MS (ES⁺) *m*/*z* 642.3 (M+H)⁺

### Preparation of (S)-2-(dibenzylamino)-3-phenylpropyl 4-(3-(2-tert-butyloxycarbonylaminoethyl)-2-oxo-2,3-dihydro-1H-benzo[d]imidazol-1-yl)piperidine-1-carboxylate (Compound n°74):

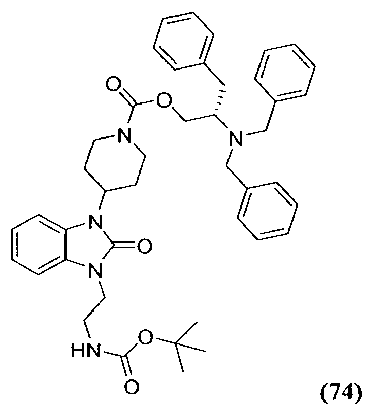

From (S)-2-(dibenzylamino)-3-phenylpropyl 4-(1,2-dihydro-2-oxobenzo[d]imidazol-3-yl)piperidine-l-carboxylate and *2-tert-*butyloxycarbonylamino-1-bromoethane.
White solid (56%, 78% / recovered starting material).
Flash chromatography on silica gel (dichloromethane/ methyl alcohol 98/2)
¹H NMR (400 MHz, CDCl₃, ppm) δ 7.33-7.18 (m, 13H, *Ph*), 7.16-6.99 (m, 6H, *Ph),* 4.96 (s, 1H, *NH*), 4.53 (tt, *J* = 12.4, 3.7 Hz, 1H, *CH*CH₂CH₂N), 4.42 (m, 1H, CHCH₂*CH₂*N), 4.36 (dd, *J* = 11.2, 7.1 Hz, 1H, *OCH₂*), 4.16 (dd_{b}, *J* = 11.2, 4.7 Hz, 2H, O*CH₂*, CHCH₂*CH₂*N), 4.03 (t, *J* = 5.2 Hz, 2H, N*CH₂*CH₂NH), 3.82 (d, *J* = 13.8 Hz, 2H, N*CH₂*Ph), 3.74 (d, *J* = 13.8 Hz, 2H, N*CH₂*Ph), 3.48 (dd, *J* = 10.5, 5.2 Hz, 2H, NCH₂*CH₂*NH), 3.27 (m, 1H, *CH*CH₂Ph), 3.09 (dd, *J* = 13.7, 5.8 Hz, 1H, CH*CH₂*Ph), 2.90 (m, 2H, CHCH₂*CH₂*N), 2.70 (dd, *J* = 13.7, 8.5 Hz, 1H, CH*CH₂*Ph), 2.33 (m, 2H, CH*CH₂*CH₂N), 1.86 (d, *J* = 11.8 Hz, 2H, CH*CH₂*CH₂N), 1.42 (s, 9H, OtBu)
¹³C NMR (100 MHz, CDC1₃, ppm) δ 155.9, 155.1, 153.7 (C=O), 139.8, 139.5, 129.8, 129.2, 128.6, 128.4, 128.3, 128.1, 128.0, 127.8, 126.9, 126.0, 121.2, 121.1, 109.2, 108.0 (C_{Ph}), 79.5 (O*C_{q}*(CH₃)₃), 64.7 (*CH₂*O), 58.4 (*CH*CH₂Ph), 53.9 (N*CH₂*Ph), 53.4 (N*CH₂CH₂*NH), 50.9 (*CH*CH₂CH₂N), 43.6 (CHCH₂*CH₂*N), 40.8, 39.6 (NCH₂*CH₂*NH), 34.1 (CH*CH*₂Ph), 29.3 (CH*CH₂*CH₂N), 28.3 (OC_{q}(*CH₃*)₃)
LC/MS (ES⁺) *m*/*z* 718.4 (M+H)⁺

### 19) N-aryl benzimidazolone preparation:

### Preparation of (S)-2-(dibenzylamino)propyl 4-(1,2-dihydro-2-oxo-1-phenylbenzo(dlimidazol-3-yl)piperidine-1-carboxylate (Compound n°75):

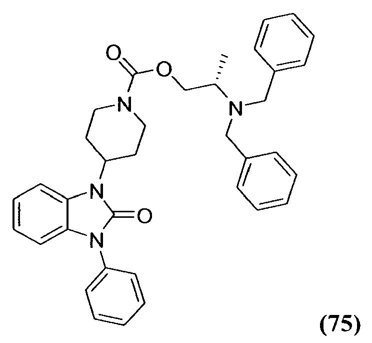

To 20 mg of 4 A° MS, 24 mg (0.2 mmol, 2 equiv.) of phenylboronic acid, 28 µL (0.2 mmol, 2 equiv.) of triethylamine in 500 µL of dry dichloromethane were successively added 50 mg (0.1 mmol, 1 equiv.) of (S)-2-(dibenzylamino)propyl 4-(1,2-dihydro-2-oxo-benzo[d]imidazol-3-yl)piperidine-1-carboxylate, 2 mg (0.01 mmol, 0.1 equiv.) of copper (II) sulfate and 17 mg (0.11 mmol, 1.1 equiv.) of 2,2,2',2'-tetramethylpiperidine-*N*-oxide. The resulting mixture was stirred for 48 hours at room temperature on air. After filtration on celite, the solvant was evaporated on *vacuum* and the residue was purified on silica gel.
White solid (60%).
Flash chromatography on preparative TLC (silica gel, cyclohexane/ethyl acetate 7/3)
¹H NMR (400 MHz, CDCl₃, ppm) δ 7.53 (m, 4H, *Ph*), 7.41 (m, 5H, *Ph*), 7.29 (t, *J* = 7.4 Hz, 4H, *Ph*), 7.21 (t, *J* = 7.2 Hz, 3H, *Ph*), 7.07 (m, 3H, *Ph*), 4.62 (tt, *J =* 12.4, 4.0 Hz, 1H, *CH*CH₂CH₂N, 1H), 4.43 (m, 2H, CHCH₂*CH₂*N), 4.28 (dd, *J* = 10.9, 7.5 Hz, 1H, O*CH₂*), 4.08 (dd, *J* = 10.9, 5.7 Hz, 1H, O*CH₂*), 3.77 (d, *J* = 13.9 Hz, 2H, N*CH₂*Ph), 3.58 (d, *J* = 13.9 Hz, 2H, N*CH₂*Ph), 3.15 (sext., *J* = 6.6 Hz, 1H, *CH*CH₃), 2.97 (m, 2H, CHCH₂*CH₂*N), 2.43 (dq, *J* = 12.5, 4.1 Hz, 2H, CH*CH₂*CH₂N), 1.95 (d, *J* = 10.2 Hz, 2H, CH*CH₂*CH₂N), 1.12 (d, *J* = 6.7 Hz, 3H, CH*CH₃*)
¹³C NMR (100 MHz, CDCl₃, ppm) δ 155.2, 152.7 (C=O), 140.2, 134.5, 129.6, 129.5, 128.5, 128.1, 127.7, 126.8, 126.1, 121.7, 121.2, 114.9, 109.2, 109.0 (C_{Ph}), 66.8 (*CH₂*O), 53.7 (N*CH₂*Ph), 51.9 (*CH*CH₃), 51.0 (*CH*CH₂CH₂N), 43.6 (CHCH₂*CH₂*N), 29.1 (CH*CH₂*CH₂N), 11.2 (CH*CH₃*)
LC/MS (ES⁺) *m*/*z* 575.0 (M+H)⁺

### 20) Preparation of biotinylated derivative

### Preparation of (S)-2-(dibenzylamino)-3-phenylpropyl 4-(3-(2 aminoethyl)-2-oxo-2,3-dihydro-1H-benzo[dlimidazol-1-yl)piperidine-1-carboxylate D-biotin amid (Compound n°76):

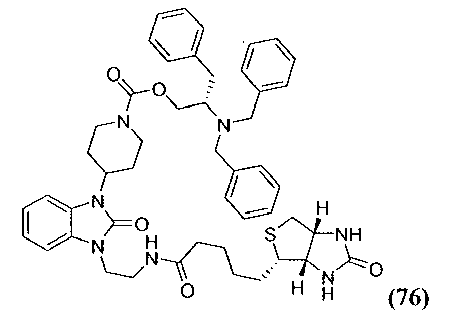

To a stirred solution of (S)-2-(dibenzylamino)-3-phenylpropyl 4-(3-(2-*tert*-butyloxycarbonylaminoethyl)-2-oxo-2,3-dihydro-1H-benzo[d]imidazol-1-yl)piperidine-1-carboxylate at 0°C was treated with 5 ml of a solution of trifluoroacetic acid/ dichloromethane (1/4) and stirred 1h. The acid and solvent were evaporated in vacuum and amine hydrochloride was precipitated in dry diethyl ether and filtered.

To a stirred solution of 48 mg (0.195 mmol, 1.5 equiv.) of D-biotin, 64 mg (0.17 mmol, 1.3 equiv.) of HBTU, 36 µL (0.21 mmol, 1.6 equiv.) of DIEA in 2 ml of dry DMF was added the precedent crude amine salt in 2 ml of dry DMF. After 1 hour, the solvent was evaporated in vacuum at room temperature and the residue purified on silica gel.
White solid (95%).
Flash chromatography on preparative TLC (silica gel, dichloromethane/ methyl alcohol 98/2)
¹H NMR (400 MHz, CDCl₃, ppm) δ 7.36-7.16 (m, 13H, *Ph),* 7.08 (m, 7H, *Ph, NH*), 6.78 (s, 1H, *NH*), 5.84 (s, 1H, *NH*), 4.45 (m, 4H, *CH*CH₂CH₂N, CHCH₂*CH₂*N, O*CH₂*, NHCH*CH*NH), 4.25 (m, 1H, NHCHCHNH), 4.16 (m, 2H, O*CH₂*, CHCH₂*CH₂*N), 4.02 (m, 2H, N*CH₂*CH₂NH), 3.76 (2d, *J* = 13.7 Hz, 4H, N*CH₂*Ph), 3.58 (m, 2H, NCH₂*CH₂*NH), 3.26 (m, 1H, *CH*CH₂Ph), 3.08 (m, 2H, CH*CH₂*Ph, NHCH*CH₂*S), 2.89 (m, 3H, CHCH₂*CH₂*N, NHCH*CH₂*S), 2.67 (m, 2H, CH*CH₂*Ph, NHCH*CH*S), 2.32 (m, 2H, CH*CH₂*CH₂N), 2.15 (m, 2H,*CH₂*CONH), 1.84 (d, *J* = 11.4 Hz, 2H, CH*CH₂*CH₂N), 1.66 (m, 4H, SCH(*CH₂*)₃CH₂CO), 1.38 (m, 2H, SCH(*CH₂*)₃CH₂CO)
¹³C NMR (100 MHz, CDCl₃, ppm) δ 173.8 (NH*C*=O), 164.1 (NHC=ONH), 155.1, 154.0 (C=O), 139.8, 139.5, 129.4, 129.2, 128.6, 128.3, 128.2, 128.1, 127.8, 126.8, 126.7, 126.0, 121.4, 121.3, 109.2, 108.1 (Cₚₕ), 64.7 (*CH₂*O), 61.6, 60.1 (NH*CH*CHCHS), 58.4 (*CH*CH₂Ph), 55.7 (NHCHCHCHS), 53.9 (N*CH₂*Ph), 53.4 (N*CH₂*CH₂NH), 51.0 (*CH*CH₂CH₂N), 43.6 (CHCH₂*CH₂*N), 40.4 (NCH₂*CH₂*NH), 38.4 (NHCH*CH₂*S), 35.8 (*CH₂*CONH), 34.1 (CH*CH₂*Ph), 29.3 (CH*CH₂*CH₂N), 28.1, 28.0, 25.4 (SCH(*CH₂*)₃CH₂CO)
LC/MS (ES⁺) *m*/*z* 844.5 (M+H)⁺

### 21) Alkylation/Hydrolysis/Cyclisation

### Preparation of (S)-2-(dibenzylamino)propyl 4-(3-(4-ethoxy-4-oxobutyl)-2-oxo-2,3-dihydro-1H-benzordlimidazol-1-yl)piperidine-1-carboxylate (Compound n°77):

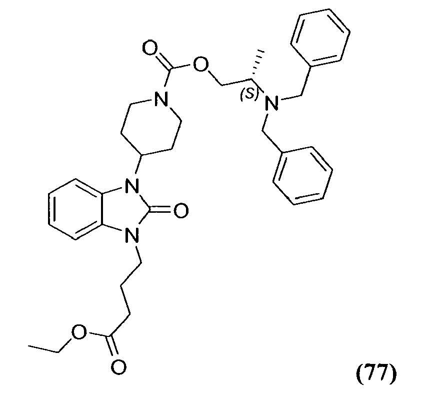

To a stirred solution of (S)-2-(dibenzylamino)propyl 4-(1,2-dihydro-2-oxobenzo[d]imidazol-3-yl)piperidine-1-carboxylate (350 mg, 0.70 mmol) in anhydrous DMF (3 mL) at 0°C was added NaH in mineral oil (60%, 31 mg, 0.77 mmol) under N₂ atmosphere. The reaction mixture was stirred for 1h at room temperature and cooled at 0°C. The ethyl 4-bromobutanoate (115 µL, 0.80 mmol) and potassium iodide (10 mg, 0.07 mmol) were added and the reaction mixture was stirred overnight at room temperature. Cool ice (5 mL) was added and the residue was filtered and dried on *vacuo.* The crude material was purified by flash chromatography (silica gel, cyclohexane/ ethyl acetate 5/5) to afford a white solid (290 mg, 68%).
¹H NMR (400 MHz, CDCl₃) δ 7.38 (d, *J =* 7.2 Hz, 4H, Bn), 7.28 (m, 4H, Bn), 7.20 (d, *J* = 6.8 Hz, 2H, Bn), 7.08 (m, 4H, Ar), 4.53 (m, 1H, *CH*CH₂CH₂Nₚᵢₚ), 4.30 (m, 2H, CHCH₂*CH*₂Nₚᵢₚ), 4.26 (dd, *J* = 10.8, 7.2 Hz, 1H, *CH₂*O), 4.12 (m, 3H, *CH₂*O, CH₃*CH₂*O), 3.94 (t, J= 7.2 Hz, 2H, NCH₂CH₂CH₂), 3.76 (d, *J* = 14.0 Hz, 2H, N*CH₂*Ph), 3.57 (d, *J* = 14.0 Hz, 2H, N*CH₂*Ph), 3.14 (m, 1H, *CH*Me), 2.93 (m, 2H, CHCH₂*CH₂*Nₚᵢₚ), 2.40 (m, 4H, NCH₂CH₂CH₂, CH*CH*₂CH₂Nₚᵢₚ), 2.05 (t, *J =* 7.2 Hz, 2H, NCH₂CH₂CH₂), 1.86 (m, 2H, CH*CH*₂CH₂Nₚᵢₚ), 1.25 *(t, J=* 7.2 Hz, 3H, *CH₃*CH₂O), 1.11 *(d, J=* 6.8 Hz, 3H, Me).
¹³C NMR (100 MHz, CDCl₃) δ 172.7 (C=O_{Ester}), 155.2 (C=O), 153.5 (C=O), 140.2 (C_{Bn}), 129.4 (C_{Ar}), 128.6 (CH_{Bn}), 128.2 (CH_{Bn}), 127.9 (C_{Ar}), 126.8 (CH_{Bn}), 121.0 (CH_{Ar}), 120.9 (CH_{Ar}), 109.1 (CH_{Ar}), 107.9 (CH_{Ar}), 66.9 (*CH₂*O), 60.4 (CH₃*CH₂*O), 53.7 (N*CH₂*Ph), 51.9 (*CH*Me), 50.9 (*CH*CH₂CH₂Nₚᵢₚ), 43.6 (CHCH₂*CH₂*Nₚᵢₚ), 40.3 (N*CH₂*CH₂CH₂), 31.2 (NCH₂CH₂*CH₂*), 29.3 (CH*CH₂*CH₂Nₚᵢₚ), 23.5 (NCH₂*CH₂*CH₂), 14.2 (*CH₃*CH₂O), 11.3 (Me).
LC/MS (ES⁺) *m*/*z* 613.4 (M+H)⁺

### Preparation of (S)-4-(3-(1-((2-(dibenzylamino)propoxy)carbonyl)piperidin-4-yl)-2-oxo-2,3-dihydro-1H-benzo[d]imidazol-1-yl)butanoic acid (Compound n°78):

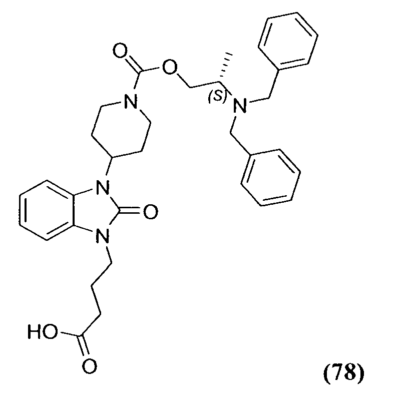

To a stirred solution of the (S)-2-(dibenzylamino)propyl 4-(3-(4-ethoxy-4-oxobutyl)-2-oxo-2,3-dihydro-1H-benzo[d]imidazol-1-yl)piperidine-1-carboxylate (280 mg, 0.46 mmol) in a solution MeOH/H₂O (1:1, 7 mL) and in THF (1 mL) was added NaOH (96 mg, 2.40 mmol). The reaction mixture was stirred for 5h at room temperature. The solvent was removed on *vacuo* and HCl (6N) was added to pH 2. Saturated NaHCO₃ was added to pH 7 and the aqueous phase was extracted with ethyl acetate (3 x 10 mL). The combined organic layers were dried (Na₂SO₄), filtered and concentrated on *vacuo* to afford a white solid (180 mg, 67%).
¹H NMR (400 MHz, CDCl₃) δ 7.37 (d, *J* = 7.2 Hz, 4H, Bn), 7.28 (m, 4H, Bn), 7.21 (d, *J* = 7.2 Hz, 2H, Bn), 7.11-7.06 (m, 4H, Ar), 4.54 (m, 1H, *CH*CH₂CH₂Nₚᵢₚ), 4.40 (m, 2H, CHCH₂*CH*₂Nₚᵢₚ), 4.27 (dd, *J* = 10.8, 7.2 Hz, 1H, *CH₂*O), 4.07 (dd, *J =* 10.8, 6.0 Hz, 1H, *CH₂*O), 3.99 (bt, 2H, N*CH*₂CH₂CH₂), 3.75 (d, *J=* 14.0 Hz, 2H, N*CH*₂Ph), 3.58 (d, *J=* 14.0 Hz, 2H, N*CH*₂Ph), 3.14 (m, 1H, *CH*Me), 2.94 (m, 2H, CHCH₂*CH*₂Nₚᵢₚ), 2.46 (bt, 2H, NCH₂*CH*₂CH₂), 2.25 (m, 2H, CH*CH*₂CH₂Nₚᵢₚ), 2.09 (bt, *J* = 6.0 Hz, 2H, NCH₂CH₂*CH*₂), 1.87 (m, 2H, CH*CH*₂CH₂Nₚᵢₚ), 1.11 (d, *J* = 6.8 Hz, 3H, Me).
¹³C NMR (100 MHz, CDCl₃) δ 176.8 (C=O_{Acide}), 155.3 (C=O), 153.7 (C=O), 140.2 (C_{Bn}), 129.2 (C_{Ar}), 128.6 (CH_{Bn}), 128.2 (CH_{Bn}), 127.9 (C_{Ar}), 126.8 (CH_{Bn}), 121.3 (2xCH_{Ar}), 109.4 (CH_{Ar}), 108.0 (CH_{Ar}), 66.9 (*CH₂*O), 53.7 (N*CH₂*Ph), 51.9 (*CH*Me), 51.0 (*CH*CH₂CH₂Nₚᵢₚ), 43.6 (CHCH₂*CH*₂Nₚᵢₚ), 40.1 (N*CH*₂CH₂CH₂), 31.0 (NCH₂CH₂*CH₂*), 29.2 (CH*CH₂*CH₂Nₚᵢₚ), 23.5 (NCH₂*CH₂*CH₂), 11.2 (Me).
LC/MS (ES⁺) *m*/*z* 585.3 (M+H)⁺

### Preparation of (S)(1-((2-(dibenzylamino)propoxy)carbonyl)piperidin-4-yl)-5,6-dihydro-imidazo[4,5,1,j,k][1]benzazepine-2,7(1H,4H)-dione (Compound n°79):

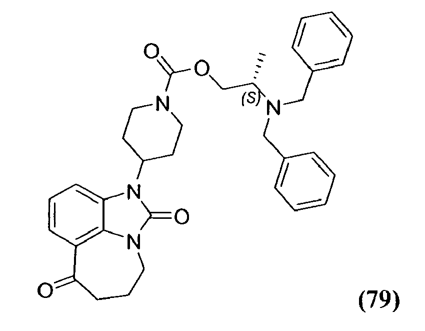

To SOCl₂ (0.2 mL) cooled at 0°C was added (S)-4-(3-(1-((2-(dibenzylamino)propoxy)carbonyl)piperidin-4-yl)-2-oxo-2,3-dihydro-1H-benzo[d]imidazol-1-yl)butanoic acid (90 mg, 0.15 mmol). The reaction mixture was stirred for 1h30 at room temperature and SOCl₂ was removed on *vacuo.* 1,2-dichloroethane (1.5 mL) was added, the mixture was cooled at 0°C and AlCl₃ (104 mg, 0.78 mmol) was added. The reaction mixture was stirred for 3h at room temperature. The solvent was removed on *vacuo* and the residue was purified by flash chromatography (silica gel, cyclohexane/ethyl acetate 3/7) to obtain a pale yellow solid (17 mg, 20%).
¹H NMR (400 MHz, CDCl₃) δ 7.80 (d, *J* = 8.0 Hz, 1H, Ar), 7.38 (d, *J =* 7.2 Hz, 4H, Bn), 7.28 (m, 5H, Ar), 7.21 (t, *J =* 7.2 Hz, 2H, Bn), 7.09 (t, J= 8.0 Hz, 1H, Ar), 4.52 (m, 1H, *CH*CH₂CH₂Nₚᵢₚ), 4.40 (m, 2H, CHCH₂*CH₂*Nₚᵢₚ), 4.25 (m, 1H, *CH₂*O)*,* 4.13-4.06 (m, 3H, *CH₂*O*,* N*CH₂*CH₂CH₂), 3.75 (d, *J* = 14.0 Hz, 2H, N*CH₂*Ph), 3.57 (d, *J* = 14.0 Hz, 2H, N*CH₂*Ph), 3.14 (m, 1H, *CH*Me)*,* 3.08 (m, 2H, NCH₂CH₂*CH₂*), 2.94 (m, 2H, CHCH₂*CH₂*Nₚᵢₚ), 2.38 (m, 2H, CH*CH₂*CH₂Nₚᵢₚ), 2.24 (m, 2H, NCH₂*CH₂*CH₂), 1.87 (m, 2H, CH*CH₂*CH₂Nₚᵢₚ), 1.11 (d, *J* = 6.8 Hz, 3H, Me).
¹³C NMR (100 MHz, CDCl₃) δ 197.1 (C=O_{Cétone}), 155.2 (C=O), 153.0 (C=O), 140.2 (C_{Bn}), 129.2 (C_{Ar}), 129.1 (C_{Ar}), 128.6 (CH_{Bn}), 128.2 (CH_{Bn}), 126.8 (CH_{Bn}), 122.7 (CH_{Ar}), 120.5 (CH_{Ar}), 119.0 (C_{Ar}), 113.0 (CH_{Ar}), 66.9 (*CH₂*O), 53.7 (N*CH₂*Ph), 51.9 (*CH*Me), 51.4 (*CH*CH₂CH₂Nₚᵢₚ), 45.4 (N*CH₂*CH₂CH₂), 44.4 (NCH₂CH₂*CH₂*), 43.6 (CHCH₂*CH₂*Nₚᵢₚ), 29.0 (CH*CH₂*CH₂Nₚᵢₚ), 20.3 (NCH₂*CH₂*CH₂), 11.3 (Me).
LC/MS (ES⁺) *m*/*z* 567.3 (M+H)⁺

### 22) Aniline derivatives preparation (reductive amination):

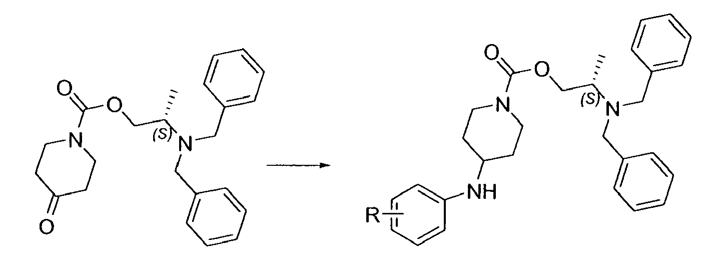

### General procedure:

To a stirred solution of (S)-2-(dibenzylamino)propyl 4-oxopiperidine-1-carboxylate (1 equiv.) in 1,2-dichloroethane (1 mL/mmol) were added appropriate aniline (1 equiv.), acetic acid (1.2 equiv.) followed by sodium triacetoxyborohydride (1.2 equiv.) under a nitrogen atmosphere. The reaction mixture was stirred for 16h at room temperature. The solvent was removed *on vacuo* and the residue was partitioned between ethyl acetate and saturated aqueous Na₂CO₃ solution (5 mL/mmol). The organic phase was separated and washed by saturated aqueous Na₂CO₃ solution (2 x 5 mL/mmol). The organic layer was dried over anhydrous Na₂SO₄, filtered and concentrated under vacuum. The crude material was purified by flash chromatography on silica gel.

### Preparation of (S)-2-(dibenzylamino)propyl 4-(phenylamino)piperidine-1-carboxylate (Compound n°80):

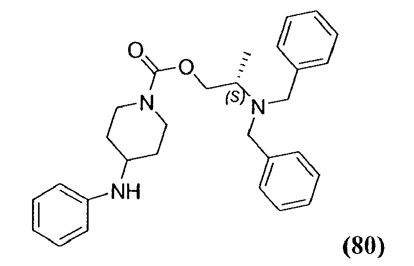

From aniline
Colorless oil (49%).
Flash chromatography on silica gel (pentane/acetone/triethylamine 95/4/1)
¹H NMR (400 MHz, CDCl₃) δ 7.37 (d, *J =* 7.2 Hz, 4H, Bn), 7.26 (m, 4H, Bn), 7.20 (m, 4H, Ar), 6.72 (t, *J* = 7.6 Hz, 1H, Ar), 6.62 (d, *J* = 8.0 Hz, 2H, Ar), 4.23 (dd, *J* = 10.8, 7.6 Hz, 1H, *CH₂*O), 4.11-4.03 (m, 3H, CHCH₂*CH₂*Nₚᵢₚ, *CH₂*O), 3.75 (d, *J=* 14.0 Hz, 2H, N*CH₂*Ph), 3.55 (d, *J*= 14.0 Hz, 2H, N*CH₂*Ph), 3.50 (m, 1H, *CH*CH₂CH₂Nₚᵢₚ), 3.12 (m, 1H, *CH*Me), 3.00 (bt, 2H, CHCH₂*CH₂*Nₚᵢₚ), 2.08 (bd, 2H, CH*CH₂*CH₂Nₚᵢₚ), 1.38 (m, 2H, CH*CH₂*CH₂Nₚᵢₚ), 1.08 (d, *J* = 6.8 Hz, 3H, Me).
¹³C NMR (100 MHz, CDCl₃) δ 155.3 (C=O), 146.7, 140.3, 129.4, 128.5, 128.1, 126.8, 117.5, 113.3, 66.7 (*CH₂*O), 53.7 (N*CH₂*Ph), 51.9 (*CH*Me), 49.9 (*CH*CH₂CH₂Nₚᵢₚ), 42.8 (CHCH₂*CH₂*Nₚᵢₚ), 32.4 (CH*CH₂*CH₂Nₚᵢₚ), 11.3 (Me).
LC/MS (ES⁺) *m*/*z* 458.3 (M+H)⁺

### Preparation of (S)-2-(dibenzylamino)propyl 4-(4-methoxyphenylamino)piperidine-1-carboxylate (Compound n°81):

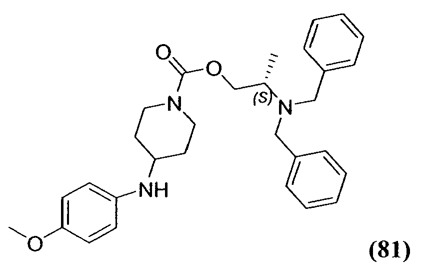

From 4-methoxybenzenamine
Colorless oil (49%).
Flash chromatography on silica gel (pentane/acetone/triethylamine 95/4/1)
¹H NMR (400 MHz, CDCl₃) δ 7.41 (d, J= 7.2 Hz, 4H, Bn), 7.32 (t, *J =* 7.2 Hz, 4H, Bn), 7.24 (t, *J =* 7.2 Hz, 2H, Bn), 6.83 (d, *J* = 9.0 Hz, 2H, Ar), 6.64 (d, *J* = 9.0 Hz, 2H, Ar), 4.27 (dd, *J* = 10.8, 7.2 Hz, 1H, *CH₂*O), 4.17-4.08 (m, 3H, CHCH₂*CH₂*Nₚᵢₚ, *CH₂*O), 3.78 (m, 5H, Ome, N*CH₂*Ph), 3.58 (d, *J* = 14.0 Hz, 2H, N*CH₂*Ph), 3.40 (m, 1H, *CH*CH₂CH₂Nₚᵢₚ), 3.16 (m, 1H, *CH*Me), 3.01 (bt, *J =* 12.0 Hz, 2H, CHCH₂*CH₂*Nₚᵢₚ), 2.10 (bd, *J* = 14.4 Hz, 2H, CH*CH₂*CH₂Nₚᵢₚ), 1.36 (m, 2H, CH*CH₂*CH₂Nₚᵢₚ), 1.11 (d, J= 6.8 Hz, 3H, Me).
¹³C NMR (100 MHz, CDCl₃) δ 155.3 (C=O), 152.3, 140.8, 140.3, 128.5, 128.1, 126.8, 115.1, 115.0, 66.7 (*CH₂*O), 55.8 (Ome), 53.7 (N*CH₂*Ph), 51.9 (*CH*Me), 51.1 (*CH*CH₂CH₂Nₚᵢₚ), 42.8 (CHCH₂*CH₂*Nₚᵢₚ), 32.5 (CH*CH₂*CH₂Nₚᵢₚ), 11.3 (Me).
LC/MS (ES⁺) *m*/*z* 488.3 (M+H)⁺

### Preparation of (S)-2-(dibenzylamino)propyl 4-(2,4-dimethoxyphenylamino)piperidine-1-carboxylate (Compound n°82):

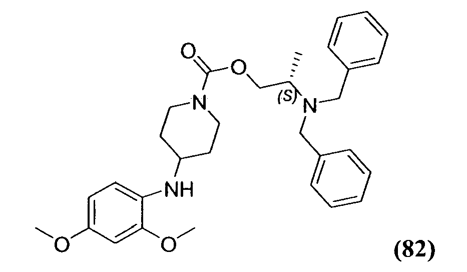

From 2,4-dimethoxybenzenamine
Colorless oil (49%).
Flash chromatography on silica gel (pentane/aeetone/triethylamine 95/4/1)
¹H NMR (400 MHz, CDCl₃) δ 7.39 (d, *J* = 7.2 Hz, 4H, Bn), 7.29 (m, 4H, Bn), 7.22 (m, 2H, Bn), 6.60 (d, *J* = 7.8 Hz, 1H, Ar), 6.50 (s, 1H, Ar), 6.44 (d, *J=* 7.8 Hz, 1H, Ar), 4.24 (m, 1H, *CH₂*O), 4.11-4.03 (m, 3H, CHCH₂*CH₂*Nₚᵢₚ, *CH₂O*), 3.84 (s, 3H, OMe), 3.78 (m, 5H, N*CH₂*Ph, OMe), 3.58 (d, *J*= 14.0 Hz, 2H, N*CH*₂Ph), 3.41 (m, 1H, *CH*CH₂CH₂Nₚᵢₚ), 3.13 (m, 1H, *CH*Me), 3.00 (m, 2H, CHCH₂*CH₂*Nₚᵢₚ), 2.09 (m, 2H, CH*CH*₂CH₂Nₚᵢₚ), 1.38 (m, 2H, CH*CH₂*CH₂Nₚᵢₚ), 1.10 *(d, J=* 6.4 Hz, 3H, Me).
¹³C NMR (100 MHz, CDCl₃) δ 155.3 (C=O), 152.1, 148.3, 140.3, 130.6, 128.5, 128.1, 126.8, 111.4, 103.8, 99.3, 66.6 (*CH₂*O), 55.8 (OMe), 55.4 (OMe), 53.7 (N*CH₂*Ph), 51.8 *(CHMe),* 49.9 (*CH*CH₂CH₂Nₚᵢₚ), 42.8 (CHCH₂*CH₂*Nₚᵢₚ), 32.5 (CH*CH₂*CH₂Nₚᵢₚ), 11.3 (Me).
LC/MS (ES⁺) *m*/*z* 518.3 (M+H)⁺

### Preparation of (S)-2-(dibenzvlamino)propyl 4-(3,4,5-trimethoxyphenylamino)piperidine-1-carboxylate (Compound n°83):

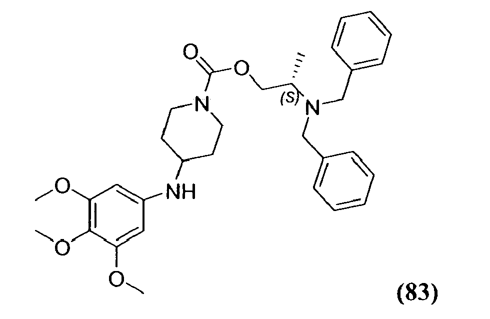

From 3,4,5-trimethoxybenzenamine
Brown oil (30%).
Flash chromatography on silica gel (pentane/acetone/triethylamine 95/4/1)
¹H NMR (400 MHz, CDCl₃) δ 7.38 (d, *J =* 7.2 Hz, 4H, Bn), 7.29 (t, *J =* 7.2 Hz, 4H, Bn), 7.22 (t, *J =* 7.2 Hz, 2H, Bn), 5.87 (s, 2H, Ar), 4.22 (dd, *J* = 10.8, 7.2 Hz, 1H, *CH₂*O), 4.14-4.03 (m, 3H, CHCH₂*CH₂*Nₚᵢₚ, *CH₂*O)*,* 3.84 (s, 6H, OMe), 3.78 (m, 5H, N*CH₂*Ph, OMe), 3.57 (d, *J* = 14.0 Hz, 2H, N*CH₂*Ph), 3.42 (m, 1H, *CH*CH₂CH₂Nₚᵢₚ), 3.13 (m, 1H, *CH*Me)*,* 3.03 (m, 2H, CHCH₂*CH₂*Nₚᵢₚ), 2.05 (m, 2H, CH*CH₂*CH₂Nₚᵢₚ), 1.38 (m, 2H, CH*CH₂*CH₂Nₚᵢₚ), 1.09 (d, *J=* 6.8 Hz, 3H, Me).
¹³C NMR (100 MHz, CDCl₃) δ 155.3 (C=O), 154.0, 143.4, 140.3, 130.3, 128.5, 128.1, 126.8, 91.2, 66.7 (*CH₂*O)*,* 61.1 (OMe), 56.0 (OMe), 53.7 (N*CH₂*Ph)*,* 51.9 (*CH*Me), 50.4 (*CH*CH₂CH₂Nₚᵢₚ), 42.7 (CHCH₂*CH₂*Nₚᵢₚ), 32.5 (CH*CH₂*CH₂Nₚᵢₚ), 11.2 (Me).
LC/MS (ES⁺) *m*/*z* 548.1 (M+H)⁺

### Preparation of (S)-2-(dibenzylamino)propyl 4-(3-tluorophenylamino)piperidine-1-carboxylate (Compound n°84):

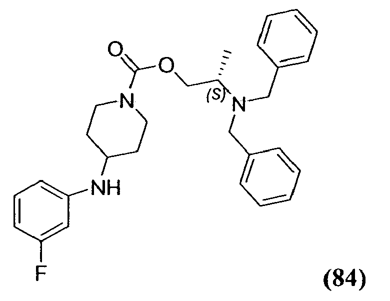

From 3-fluorobenzenamine
Brown oil (90%).
Flash chromatography on silica gel (pentane/acetone/triethylamine 95/4/1)
¹H NMR (400 MHz, CDCl₃) δ 7.38 (d, *J =* 7.2 Hz, 4H, Bn), 7.29 (t, *J =* 7.2 Hz, 4H, Bn), 7.22 (t, *J =* 7.2 Hz, 2H, Bn), 7.10 (q, *J* = 8.0 Hz, 1H, Ar), 6.38 (m, 2H, Ar), 6.62 (dd, *J* = 11.6, 2.0 Hz, 1H, Ar), 4.23 (dd, *J* = 11.2, 7.6 Hz, 1H, *CH₂*O), 4.14-4.03 (m, 3H, CHCH₂*CH₂*Nₚᵢₚ, *CH₂*O), 3.76 (d, *J* = 14.0 Hz, 2H, N*CH₂*Ph), 3.57 (d, *J* = 14.0 Hz, 2H, N*CH₂*Ph), 3.43 (m, 1H, *CH*CH₂CH₂Nₚᵢₚ), 3.13 (m, 1H, *CH*Me), 3.00 (bt, *J* = 12.0 Hz, 2H, CHCH₂*CH₂*Nₚᵢₚ), 2.08 (m, 2H, CH*CH₂*CH₂Nₚᵢₚ), 1.35 (m, 2H, CH*CH₂*CH₂Nₚᵢₚ), 1.09 (d, *J=* 6.8 Hz, 3H, Me).
¹³C NMR (100 MHz, CDCl₃) δ 164.1 (*J_{C-F}* = 241.4 Hz), 155.3 (C=O), 148.4 (*J_{C-F}* = 10.6 Hz), 140.2, 130.4 (*J_{C-F}* = 10.2 Hz), 128.5, 128.1, 126.8, 109.0, 103.9 (*J_{C-F}* = 21.4 Hz), 99.7 (*J_{C-F}* = 25.2 Hz), 66.7 (*CH₂*O), 53.7 (N*CH₂*Ph), 51.9 (*CH*Me), 50.0 (*CH*CH₂CH₂Nₚᵢₚ), 42.7 (CHCH₂*CH₂*Nₚᵢₚ), 32.2 (CH*CH₂*CH₂Nₚᵢₚ), 11.2 (Me).
LC/MS (ES⁺) *m*/*z* 476.3 (M+H)⁺

### Preparation of (S)-2-(dibenzylamino)propyl 4-(4-iodophenylamino)piperidine-1-carboxylate (Compound n°85):

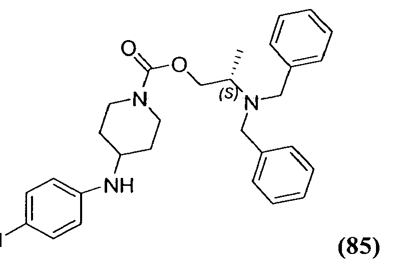

From 4-iodobenzenamine
Colorless oil (30%).
Flash chromatography on silica gel (pentane/acetone/triethylamine 95/4/1)
¹H NMR (400 MHz, CDCl₃) δ 7.42 (d, *J* = 8.4 Hz, 2H, Ar), 7.36 (d, *J =* 7.2 Hz, 4H, Bn), 7.28 (t, *J =* 7.2 Hz ,4H, Bn), 7.20 (t, *J =* 7.2 Hz, 2H, Bn), 6.40 (d, *J* = 8.4 Hz, 2H, Ar), 4.22 (dd, *J* = 11.2, 7.6 Hz, 1H, *CH₂*O)*,* 4.13-4.03 (m, 3H, CHCH₂*CH₂*Nₚᵢₚ, *CH₂*O)*,* 3.74 (d, *J* = 14.0 Hz, 2H, N*CH₂*Ph), 3.55 (d, *J =* 14.0 Hz, 2H, N*CH₂*Ph), 3.41 (m, 1H, *CH*CH₂CH₂Nₚᵢₚ), 3.11 (m, I H, *CH*Me), 2.99 *(bt, J =* 11.2 Hz, 2H, CHCH₂*CH₂*Nₚᵢₚ), 2.05 (bd, *J* = 11.2 Hz, 2H, CH*CH₂*CH₂Nₚᵢₚ), 1.38 (m, 2H, CH*CH₂*CH₂Nₚᵢₚ), 1.08 (d, *J* = 6.8 Hz, 3H, Me).
LC/MS (ES⁺) *m*/*z* 584.2 (M+H)⁺

### Preparation of (S)-2-(dibenzylamino)propyl 4-(3,4-dichlorophenylamino)piperidine-1-carboxylate (Compound n°86):

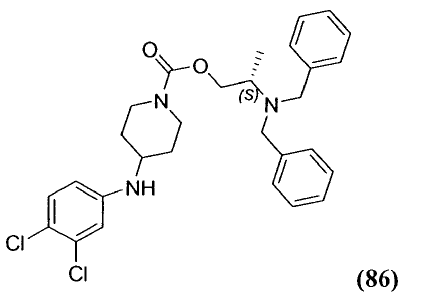

From 3,4-dichlorobenzenamine
Colorless oil (10%).
Flash chromatography on silica gel (pentane/acetone/triethylamine 95/4/1)
¹H NMR (400 MHz, CDCl₃) δ 7.37 (d, *J=* 7.2 Hz, 4H, Bn), 7.29 (t, *J =* 7.2 Hz, 4H, Bn), 7.20 (m, 3H, Ar), 6.67 (d, *J =* 2.8 Hz, 1H, Ar), 6.43 (dd, *J =* 8.8, 2.8 Hz, 1H, Ar), 4.23 (dd, *J* = 10.8, 7.2 Hz, 1H, *CH₂*O), 4.13 (m, 2H, CHCH₂*CH₂*Nₚᵢₚ), 4.03 *(dd, J* = 10.8, 5.6 Hz, 1H, *CH₂*O), 3.75 (d, *J =* 13.8 Hz, 2H, N*CH₂*Ph), 3.56 (d, *J =* 13.8 Hz, 2H, N*CH₂*Ph), 3.39 (m, 1H, *CH*CH₂CH₂Nₚᵢₚ), 3.13 (m, 1H, *CH*Me), 3.00 (bt, *J* = 11.6 Hz, 2H, CHCH₂*CH₂*Nₚᵢₚ), 2.05 (bd, *J* = 12.4 Hz, 2H, CH*CH₂*CH₂Nₚᵢₚ), 1.35 (m, 2H, CH*CH₂*CH₂Nₚᵢₚ), 1.08 (d, *J=* 6.8 Hz, 3H, Me).
¹³C NMR (100 MHz, CDCl₃) δ 155.3 (C=O), 146.1, 140.2, 132.9, 130.7, 128.5, 128.1, 126.8, 119.9, 114.0, 112.9, 66.7 (*CH₂*O), 53.7 (N*CH₂*Ph), 51.9 (*CH*Me)*,* 50.0 (*CH*CH₂CH₂Nₚᵢₚ), 42.5 (CHCH₂*CH₂*Nₚᵢₚ), 32.0 (CH*CH₂*CH₂Nₚᵢₚ), 11.2 (Me).
LC/MS (ES⁺) *m*/*z* 526.2 (M+H)⁺

### Preparation of (S)-2-(dibenzylamino)propyl 4-(2-aminophenylamino)piperidine-1-carboxylate (Compound n°87):

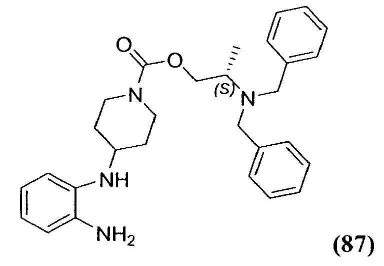

From benzene-1,2-diamine
Brown oil (65%).
Flash chromatography on silica gel (Cyclohexane / Ethyl acetate 1/1)
'H NMR (400 MHz, CDCl₃) δ 7.37 (d, J= 7.2 Hz, 4H, Bn), 7.26 (t, *J =* 7.2 Hz, 4H, Bn), 7.20 (t, *J =* 7.2 Hz, 2H, Bn), 6.72 (t, *J =* 7.2 Hz, 1 H, Ar), 6.62 (m, 3H, Ar), 4.23 (dd, *J=* 10.8, 8.0 Hz, 1H, *CH₂*O), 4.12-4.03 (m, 3H, CHCH₂*CH₂*Nₚᵢₚ, *CH_{z}O),* 3.75 (d, *J =* 13.8 Hz, 2H, *NCH_{z}Ph),* 3.56 (d, *J* = 13.8 Hz, 2H, N*CH₂*Ph), 3.44 (m, 1 H, *CH*CH₂CH₂Nₚᵢₚ), 3. I 3-3.03 (m, 3H, *CH*Me, CHCH₂*CH₂*Nₚᵢₚ), 2.06 (bd, 2H, CH*CH₂*CH₂Nₚᵢₚ), 1.40 (m, 2H, CH*CH₂*CH₂Nₚᵢₚ), 1.08 (d, J= 6.8 Hz, 3H, Me).
¹³C NMR (100 MHz, CDCl₃) δ 155.4 (C=O), 140.3, 135.9, 134.8, 128.5, 128.1, 126.8, 120.6, 119.2, 117.1, 113.4, 66.6 (*CH₂*O), 53.7 (N*CH₂*Ph), 51.9 (*CH*Me), 50.0 (*CH*CH₂CH₂Nₚᵢₚ), 42.6 (CHCH₂*CH₂*Nₚᵢₚ), 32.4 (CH*CH₂*CH₂Nₚᵢₚ), 11.3 (Me).
LC/MS (ES⁺) *m*/*z* 473.3 (M+H)⁺

### 23) Aniline derivatives preparation

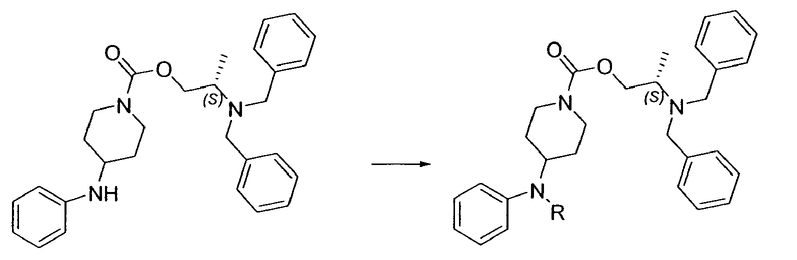

### General procedure:

To a stirred solution of (S)-2-(dibenzylamino)propyl 4-(phenylamino)piperidine-1-carboxylate (1 equiv.) in CH₂Cl₂ (3 mL/mmol) was added NEt₃ (3 equiv.) and appropriate electrophile (2 equiv.) under N₂ atmosphere. The reaction mixture was stirred for 16h at room temperature. The solvent was removed on *vacuo* and the residue was purified by flash chromatography on silica gel.

### Preparation of (S)-2-(dibenzylamino)propyl 4-(1,3-diphenylureido)piperidine-1-carboxylate (Compound n°88):

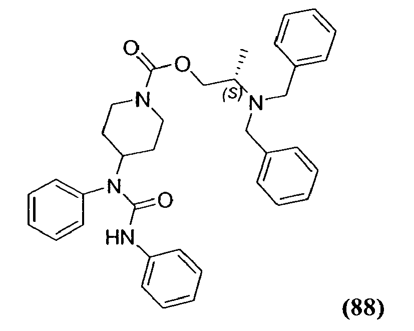

From phenylisocyanate
White solid (79%).
Flash chromatography on silica gel (cyclohexane/ethyl acetate 7/3)
¹H NMR (400 MHz, CDCl₃) δ 7.50 (m, 3H, Ar), 7.32 (d, J= 7.2 Hz, 4H, Bn), 7.26-7.19 (m, 12H, Ar), 7.0 (m, 1H, Ar), 5.87 (s, 1H, NH), 4.74 (m, 1H, *CH*CH₂CH₂Nₚᵢₚ), 4.24-4.14 (m, 3H, CHCH₂*CH₂*Nₚᵢₚ, *CH₂*O), 3.95 (dd, *J =* 11.2, 5.6 Hz, 1H, *CH₂*O), 3.69 (d, *J* = 14.0 Hz, 2H, N*CH₂*Ph), 3.51 (d, *J* = 14.0 Hz, 2H, N*CH₂*Ph), 3.05 (m, 1H, *CH*Me), 2.89 (m, 2H, CHCH₂*CH₂*Nₚᵢₚ), 1.91 (bd, 2H, CH*CH₂*CH₂Nₚᵢₚ), 1.27 (m, 2H, CH*CH₂*CH₂Nₚᵢₚ), 1.03 (d, J= 6.8 Hz, 3H, Me).
¹³C NMR (100 MHz, CDCl₃) δ 155.1 (C=O), 154.0 (C=O), 140.2, 138.6, 137.0, 131.1, 130.2, 129.3, 128.8, 128.4, 128.1, 126.7, 123.0, 119.3, 66.5 (*CH₂*O), 53.6 (N*CH₂*Ph), 52.7 (*CH*Me), 51.8 (*CH*CH₂CH₂N_{Pip}), 43.4 (CHCH₂*CH₂*Nₚᵢₚ), 31.1 (CH*CH₂*CH₂Nₚᵢₚ), 11.1 (Me).
LC/MS (ES⁺) *m*/*z* 577.3 (M+H)⁺

### Preparation of (S)-2-(dibenzylamino)propyl 4-(N-phenylbenzamido)piperidine-1-carboxylate (Compound n°89):

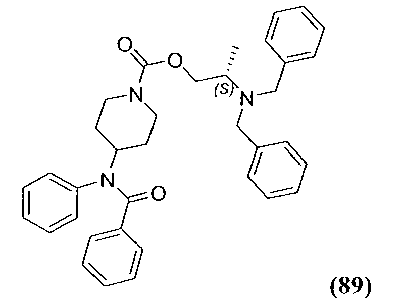

From benzoyl chloride
White solid (64%).
Flash chromatography on silica gel (cyclohexane/ethyl acetate 7/3)
¹H NMR (400 MHz, CDCl₃) δ 7.32 (d, *J =* 7.2 Hz, 4H, Bn), 7.26-7.11 (m, 14H, Ar), 6.99 (d, *J* = 6.4 Hz, 2H, Ar), 4.96 (m, 1H, *CH*CH₂CH₂Nₚᵢₚ), 4.29-4.11 (m, 3H, CHCH₂*CH₂*Nₚᵢₚ, *CH₂*O), 3.96 (dd, *J=* 10.8, 5.6 Hz, 1H, *CH₂*O), 3.69 (d, *J* = 14.0 Hz, 2H, N*CH₂*Ph), 3.52 (d, *J* = 14.0 Hz, 2H, N*CH₂*Ph), 3.06 (m, 1H, *CH*Me), 2.94 (m, 2H, CHCH₂*CH₂*Nₚᵢₚ), 1.97 (bd, 2H, CH*CH₂*CH₂Nₚᵢₚ), 1.45 (m, 2H, CH*CH₂*CH₂Nₚᵢₚ), 1.03 (d, J= 6.8 Hz, 3H, Me).
¹³C NMR (100 MHz, CDCl₃) δ 170.8 (C=O_{Amide}), 155.1 (C=O), 140.2, 139.2, 136.6, 130.6, 129.1, 128.8, 128.4, 128.1 (2C), 127.7, 127.6, 126.7, 66.5 (*CH₂*O), 53.6 (N*CH₂*Ph), 51.8 (*CH*Me, *CH*CH₂CH₂Nₚᵢₚ), 43.4 (CHCH₂*CH₂*Nₚᵢₚ), 30.6 (CH*CH₂*CH₂Nₚᵢₚ), 11.2 (Me).
LC/MS (ES⁺) *m*/*z* 562.3 (M+H)⁺

### Preparation of (S)-2-(dibenzylamino)propyl 4-(N-phenylphenvtsulfonamido)piperidine-1-carboxylate (Compound n°90):

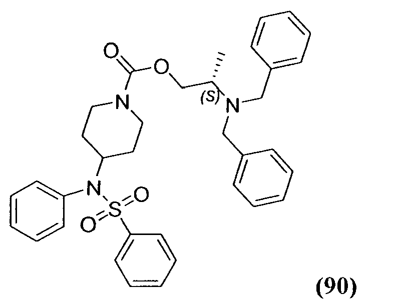

From benzenesulfonyl chloride
White solid (60%).
Flash chromatography on silica gel (cyclohexane/ethyl acetate 8/2)
¹H NMR (400 MHz, CDCl₃) δ 7.74 (d, *J* = 7.2 Hz, 2H, Ar), 7.58 (t, *J* = 7.2 Hz, 1H, Ar), 7.48 (t, *J* = 8.0 Hz, 2H, Ar), 7.36 (m, 2H, Ar), 7.30 (m, 5H, Ar), 7.20 (m, 6H, Ar), 6.99 (d, *J* = 7.2 Hz, 2H, Ar), 4.38 (m, 1H, *CH*CH₂CH₂Nₚᵢₚ), 4.17-4.09 (m, 3H, CHCH₂*CH₂*Nₚᵢₚ, *CH₂O*)*,* 3.91 (dd, *J=* 11.2, 5.6 Hz, 1H, *CH₂*O)*,* 3.66 (d, *J=* 14.0 Hz, 2H, N*CH₂*Ph), 3.49 (d, J= 14.0 Hz, 2H, N*CH₂*Ph)*,* 3.03 (m, 1H, *CH*Me), 2.83 (m, 2H, CHCH₂*CH₂*Nₚᵢₚ), 1.81 (bd, 2H, CH*CH₂*CH₂Nₚᵢₚ), 1.29 (m, 2H, CH*CH₂*CH₂Nₚᵢₚ), 1.00 (d, *J=* 6.8 Hz, 3H, Me).
¹³C NMR (100 MHz, CDCl₃) δ 154.9 (C=O), 141.1, 140.2, 134.9, 132.5, 132.4, 129.0, 128.9, 128.4, 128.0, 127.2 (2C), 126.7, 66.6 (*CH₂*O)*,* 57.1 (*CH*CH₂CH₂Nₚᵢₚ), 53.6 (N*CH₂*Ph), 51.7 (*CH*Me)*,* 43.3 (CHCH₂*CH₂*Nₚᵢₚ), 31.8 (CH*CH₂*CH₂Nₚᵢₚ), 11.1 (Me).
LC/MS (ES⁺) *m*/*z* 598.3 (M+H)⁺

### 24) Thiourea preparation:

### Preparation of N-((S)-2-(dibenzylamino)Propyl)-4-(1,2-dihydro-2-oxobenzo[d]imidazol-3-yl)piperidine-1-carbothioamide (Compound n°91):

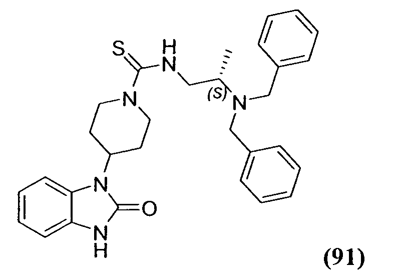

To a stirred solution of EDCI (30 mg, 0.16 mmol) in acetonitrile (0.4 mL) cooled to -10°C under N₂ was added CS₂ (94 µL, 1.57 mmol). A solution of (S)-N,N-dibenzylpropane-1,2-diamine (40 mg, 0.16 mmol) in acetonitrile (0.4 mL) was added dropwise. The reaction mixture was stirred for 3h at room temperature and a solution of 1-(piperidin-4-yl)-1H-benzo[d]imidazol-2(3H)-one (34 mg, 0.16 mmol) in acetonitrile (0.8 mL) was added. The mixture was heated at 55°C overnight and the solvent was removed on *vacuo.* The crude material was purified by flash chromatography (silica gel, cyclohexane/ethyl acetate 6/4) to afford a pale yellow solid (32 mg, 40%).
¹H NMR (400 MHz, CDCl₃) δ 9.09 (bs, 1H, NH), 7.29-7.16 (m, 10H, Ar), 7.09 (m, 4H, Ar), 6.45 (bs, 1H, NH), 4.79-4.56 (m, 3H, CHCH₂*CH₂*Nₚᵢₚ, *CH*CH₂CH₂Nₚᵢₚ), 3.78 (m, 3H, N*CH₂*Ph, *CH₂*NH), 3.33 (m, 3H, N*CH₂*Ph, *CH₂*NH), 3.03 (m, 2H, *CH*Me, CHCH₂*CH₂*Nₚᵢₚ), 2.42-2.27 (m, 2H, CH*CH₂*CH₂Nₚᵢₚ), 1.92 (m, 2H, CH*CH₂*CH₂Nₚᵢₚ), 1.18 (d, J= 6.0 Hz, 3H, Me).
¹³C NMR (100 MHz, CDCl₃) δ 180.3 (C=S), 154.9 (C=O), 139.5 (C_{Bn}), 129.2 (CH_{Bn}), 128.7 (C_{Ar}), 128.5 (CH_{Bn}), 127.9 (C_{Ar}), 127.4 (CH_{Bn}), 121.5 (CH_{Ar}), 121.3 (CH_{Ar}), 109.8 (CH_{Ar}), 109.4 (CH_{Ar}), 53.7 (N*CH₂*Ph), 51.9 (*CH*Me), 50.6 (*CH*CH₂CH₂Nₚᵢₚ), 47.2 (*CH₂*NH), 46.7 (CHCH₂*CH₂*Nₚᵢₚ), 28.9 (CH*CH₂*CH₂Nₚᵢₚ), 9.9 (Me).
LC/MS (ES⁺) *m*/*z* 514.2 (M+H)⁺

### 25) Urea preparation:

### Preparation of N-((S)-2-(dibenzylamino)propyl)-4-(1,2-dihydro-2-oxobenzo[d]imidazol-3-yl)piperidine-1-carboxamide (Compound n°92):

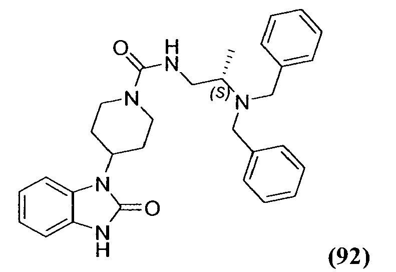

To a stirred solution of the (S)-N,N-dibenzylpropane-1,2-diamine (45 mg, 0.18 mmol) in THF (0.8 mL) at 0°C was added CDI (29 mg, 0.18 mmol) under N₂ atmosphere. The reaction mixture was stirred for 2h at room temperature. The 4-(2-keto-1-benzimidazolinyl)-piperidine (35 mg, 0.16 mmol) in THF (1.5 mL) was added and the reaction mixture was refluxed overnight. The solvent was removed on *vacuo* and the crude material was purified by flash chromatography (silica gel, ethyl acetate) to afford a white solid (30 mg, 38%).
¹H NMR (400 MHz, CDCl₃) δ 9.73 (bs, 1H, NH), 7.28-7.17 (m, 10H, Ar), 7.12 (m, 2H, Ar), 7.06 (m, 2H, Ar), 5.21 (bs, 1H, NH), 4.52 (m, 1H, *CH*CH₂CH₂Nₚᵢₚ), 4.02 (m, 2H, CHCH₂*CH₂*Nₚᵢₚ), 3.79 (d, *J* = 13.2 Hz, 2H, N*CH₂*Ph), 3.33 (m, 3H, N*CH₂*Ph, *CH₂*NH), 3.12 (m, 1H, *CH₂*NH), 2.93-2.85 (m, 2H, *CH*Me, CHCH₂*CH₂*Nₚᵢₚ), 2.37-2.25 (m, 2H, CHCH₂*CH₂*Nₚᵢₚ), 1.88 (bt, 2H, CH*CH₂*CH₂Nₚᵢₚ), 1.12 (d, J= 6.4 Hz, 3H, Me).
¹³C NMR (100 MHz, CDCl₃) δ 157.0 (C=O), 155.0 (C=O), 139.9 (C_{Bn}), 129.0 (CH_{Bn}), 128.8 (C_{Ar}), 128.4 (CH_{Bn}), 128.0 (C_{Ar}), 127.1 (CH_{Bn}), 121.3 (CH_{Ar}), 121.1 (CH_{Ar}), 109.8 (CH_{Ar}), 109.4 (CH_{Ar}), 52.9 (N*CH₂*Ph), 52.4 (*CH*Me), 50.7 (*CH*CH₂CH₂Nₚᵢₚ), 43.6 (*CH₂*NH), 43.4 (CHCH₂*CH₂*Nₚᵢₚ), 29.7 (CH*CH₂*CH₂Nₚᵢₚ), 10.1 (Me).
LC/MS (ES⁺) *m*/*z* 498.4 (M+H)⁺

### 26) Benzimidazol-2-amine derivative preparation

### Preparation of N-(1-benzylpiperidin-4-yl)-1H-benzo[d]imidazol-2-amine (Intermediate):

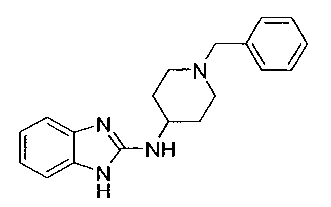

A solution of the 1H-benzo[d]imidazol-2(3H)-one (150 mg, 1.12 mmol) in POCl₃ (0.35 mL, 3.70 mmol) was refluxed overnight. Cool ice (3 mL) was introduced and 4N NaOH solution was added to pH 8. The mixture was extracted with ethyl acetate (3x5 mL). The combined organic layers were dried over anhydrous Na₂SO₄, filtered and concentrated under vacuum. To the crude material was added benzylpiperidin-4-amine (900 µL, 4.48 mmol). The reaction mixture was heated at 130°C for 72h. The residue was purified by flash chromatography (silica gel, ethyl acetate/MeOH/NEt₃ 89/10/1) to afford a pale yellow oil which contains 50% of initial product (270 mg of mixture, 40%).
LC/MS (ES⁺) *m*/*z* 307.2 (M+H)⁺

### Preparation of (S)-2-(Dibenzylamino)propyl 4-(1H-benzoldlimidazol-2-ylamino)piperidine-1-carboxylate (Compound n°93):

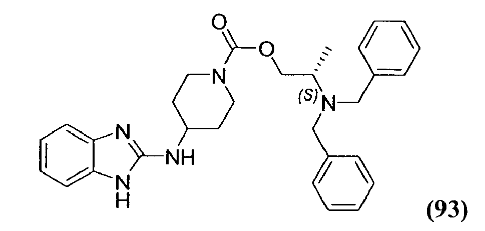

A solution of N-(1-benzylpiperidin-4-yl)-1H-benzo[d]imidazol-2-amine (270 mg, 0.86 mmol) in MeOH (10 mL/mmol) was submitted to hydrogenation in the presence of HCl (4N in dioxane, 215 µL, 0.86 mmol) and 10% Pd/C (46 mg) at room pressure and temperature for 16 h. The reaction mixture was filtered through a pad of Celite. The filtrate was concentrated under *vacuo.* To a stirred solution of (S)-2-(dibenzylamino)propan-1-ol (213 mg, 0.81 mmol) in THF (3.5 mL) cooled at 0°C was added CDI (144 mg, 0.88 mmol) under N₂ atmosphere. The reaction mixture was stirred for 2h at room temperature. Triethylamine (1.0 mL, 7.30 mmol) and the crude salt were added and the reaction mixture was stirred for 72h at room temperature. The crude material was purified by flash chromatography (silica gel, cyclohexane/ethyl acetate 7/3) to afford a colorless oil (90 mg, 22%).
¹H NMR (400 MHz, CDCl₃) δ 7.33 (d, *J* = 7.6 Hz, 4H, Bn), 7.27 (m, 6H, Ar), 7.19 (m, 2H, Ar), 7.04 (dd, *J* = 6.0, 3.2 Hz, 2H, Ar), 5.12 (s, 1H, NH), 4.19 (m, 1H, *CH₂*O), 4.03-3.93 (m, 4H, CHCH₂*CH₂*N_{PiP}, *CH*CH₂CH₂Nₚᵢₚ, *CH₂*O), 3.69 (d, *J*= 14.0 Hz, 2H, N*CH₂*Ph), 3.51 (d, *J=* 14.0 Hz, 2H, N*CH₂*Ph), 3.08 (m, 1H, *CH*Me), 2.86 (m, 2H, CHCH₂*CH₂*Nₚᵢₚ), 2.05 (m, 2H, CH*CH₂*CH₂N_{PiP}), 1.32 (m, 2H, CH*CH₂*CH₂N_{PiP}), 1.03 (d, *J* = 6.4 Hz, 3H, Me).
¹³C NMR (100 MHz, CDCl₃) δ 155.4 (C=O), 153.8 (C=N), 140.1 (C_{Bn}), 137.9 (C_{Ar}), 128.3 (CH_{Bn}), 128.0 (CH_{Bn}), 126.7 (CH_{Bn}), 121.0 (CH_{Ar}), 112.1 (CH_{Ar}), 66.9 (*CH₂*O), 53.6 (N*CH₂*Ph), 51.8 (*CH*Me), 49.8 (*CH*CH₂CH₂Nₚᵢₚ), 42.6 (CHCH₂*CH₂*Nₚᵢₚ), 32.5 (CH*CH₂*CH₂Nₚᵢₚ), 11.0 (Me).
LC/MS (ES⁺) *m*/*z* 498.3 (M+H)⁺

### Preparation of (S)-2-(dibenzylamino)propyl 2-(1-(((S)-2-(dibenzylamino)propoxy)carbonyl)piperidin-4-ylamino)-1H-benzo[d]imidazole-1-carboxylate (Compound n°94):

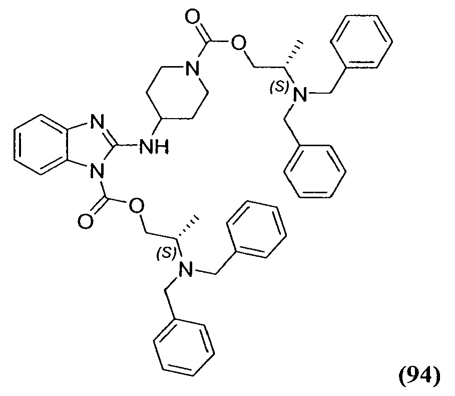

Colorless oil (4 mg, 2%).
¹H NMR (400 MHz, CDCl₃) δ 7.69 (d, *J* = 8.0 Hz, 1H, Ar), 7.45 (m, 1H, Ar), 7.37 (d, *J* = 7.6 Hz, 4H, Ar), 7.32-7.27 (m, 10H, Ar), 7.22 (m, 2H, Ar), 7.17-7.1 5 (m, 6H, Ar), 4.55 (m, 1H, *CH₂*O)*,* 4.27 (m, 1H, *CH₂*O)*,* 4.22 (m, 2H, *CH₂*O)*,* 4.11-4.00 (m, 2H, CHCH₂*CH₂*Nₚᵢₚ, *CH*CH₂CH₂Nₚᵢₚ), 3.84 (d, *J* = 14.0 Hz, 2H, N*CH₂*Ph), 3.75 (d, *J* = 14.0 Hz, 2H, N*CH₂*Ph), 3.56 (d, *J* = 14.0 Hz, 2H, N*CH₂*Ph), 3.49 (d, *J* = 14.0 Hz, 2H, N*CH₂*Ph), 3.37 (m, 1H, *CH*Me), 3.12 (m, 3H, *CH*Me, CHCH₂*CH₂*Nₚᵢₚ), 2.16 (m, 2H, CH*CH₂*CH₂Nₚᵢₚ), 1.59 (m, 2H, CH*CH₂*CH₂Nₚᵢₚ), 1.17 (d, *J* = 6.8 Hz, 3H, Me), 1.09 (d, *J* = 6.8 Hz, 3H, Me).
¹³C NMR (100 MHz, CDCl₃) δ 155.3 (2xC=O), 153.2 (C=N), 140.3, 139.5, 128.6, 128.5, 128.3, 128.2, 128.1, 127.1, 126.9, 126.8, 124.7, 120.7, 116.3, 114.4, 67.8 (*CH₂*O), 66.2 (*CH₂*O), 53.3 (N*CH₂*Ph), 53.2 (N*CH₂*Ph), 51.4 (*CH*Me), 51.2 (*CH*Me), 48.9 (*CH*CH₂CH₂Nₚᵢₚ), 42.1 (CHCH₂*CH₂*Nₚᵢₚ), 31.5 (CH*CH₂*CH₂Nₚᵢₚ), 10.8 (Me), 9.3 (Me).
LC/MS (ES⁺) *m*/*z* 779.5 (M+H)⁺

### 27) Guanidine derivative preparation

### Preparation of N-((S)-2-(dibenzylamino)propyl)-4-(1,2-dihydro-2-oxobenzo[d]intidazol-3-yl)piperidine-1-carboxamidine (Compound n°95):

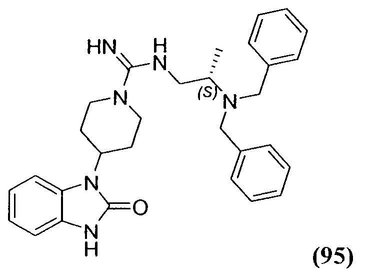

To a stirred solution of the 4-(2-keto-1-benzimidazolinyl)-pipehdine (48 mg, 0.22 mmol) in THF/DMF 4:1 (0.6 mL) was added di(1H-imidazol-1-yl)methanimine (58 mg, 0.36 mmol) under N₂ atmosphere. The reaction mixture was stirred for 72h at room temperature. Water (1 mL) was introduced and the mixture was extracted with ethyl acetate (3x5 mL). The combined organic layers were dried over anhydrous Na₂SO₄, filtered and concentrated under vacuum. To the crude material was added a solution of (S)-N,N-dibenzylpropane-1,2-diamine in THF (0.4 mL). The solution was refluxed for 72h and the solvent was removed on *vacuo.* The residue was purified with preparative HPLC to obtain a colorless oil (4 mg, 4%).
¹H NMR (400 MHz, CDCl₃) δ 10.88 (bs, 1H, NH), 7.29 (m, 6H, Ar), 7.15 (m, 5H, Ar), 7.00 (m, 3H, Ar), 5.97 (s, 1H, NH), 4.21 (m, 1H, *CH*CH₂CH₂Nₚᵢₚ), 3.86 (m, 1H, *CH₂N),* 3.74 (d, *J =* 13.2 Hz, 2H, N*CH*₂Ph), 3.46 (m, 1H, m, *CH₂*N), 3.33 (d, *J* = 13.2 Hz, 2H, N*CH₂*Ph), 3.29-3.05 (m, 2H, CHCH₂*CH₂*Nₚᵢₚ), 3.00-2.91 (m, 3H, *CHMe,* CHCH₂*CH₂*Nₚᵢₚ), 2.58 (m, 1H, CH*CH₂*CH₂Nₚᵢₚ), 2.25 (m, 1H, CH*CH₂*CH₂Nₚᵢₚ), 1.86 (bd, *J* = 10.4 Hz, 1H, CH*CH₂*CH₂Nₚᵢₚ), 1.75 (bd, J= 10.8 Hz, 1H, CH*CH₂*CH₂Nₚᵢₚ), 1.19 (d, J= 5.6 Hz, 3H, Me).
LC/MS (ES⁺) *m*/*z* 497.3 (M+H)⁺

### 28) "Amide link" procedure

### Preparation of 1-[1-(4-Dibenzylamino-butyryl)-piperidin-4-yll-1,3-dihydro-benzoimidazol-2-one (Compound n°96):

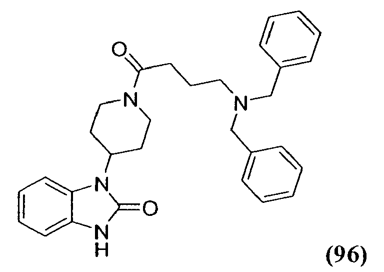

To a solution of 91 mg (0.2 mmol, 1 equiv.) of 4-dibenzylaminobutanoic acid in 1 ml of dry dichloromethane at 0°C was added successively 83 µL (0.6 mmol, 3 eq) of triethylamine, one drop of DMF, 16 µL (0.22 mmol, 1.1 eq) of thionyl chloride. After 30', 56 mg (0.26 mmol, 1.3 equiv.) of 4-(2-keto-1-benzimidazolinyl)-piperidine was added. The reaction was allowed to warm to room temperature overnight. The mixture was diluted by dichloromethane, washed by saturated sodium hydrogenocarbonate. The organic layer was dried over sodium sulphate, the solvent evaporated and the residue purified on silica gel.
Colorless oil (20%).
Flash chromatography on silica gel (cyclohexane/ ethyl acetate 7/3)
¹H NMR (400 MHz, CDCl₃) δ ppm 9.86 (s, 1H, *NH*), 7.37 (d, *J =* 7.3 Hz, 4H, *Ph*)*,* 7.31 (t, *J* = 7.4 Hz, 4H, *Ph),* 7.24 (m, 2H, *Ph*)*,* 7.09 (m, 4H, *Ph*), 4.85 (d, *J* = 13.0 Hz, 1H, CHCH₂*CH₂*N), 4.53 (tt, *J* = 12.3, 3.8 Hz, 1H, *CH*CH₂CH₂N), 3.94 (d, *J* = 13.1 Hz, 1H, CHCH₂CH₂N), 3.61 (s, 4H, N*CH₂*Ph), 3.12 (t, *J* = 12.5 Hz, 1H, CHCH₂*CH₂*N), 2.63 (t, *J* = 12.5 Hz, 1H, CHCH₂*CH₂*N), 2.52 (m, 2H, COCH₂CH₂*CH₂*N), 2.32 (m, 4H, CO*CH₂*CH₂CH₂N, CH*CH₂*CH₂N), 1.89 (m, 4H, COCH₂*CH₂*CH₂N, CH*CH₂*CH₂N)
¹³C NMR (100 MHz, CDCl₃, ppm) δ 171.4, 154.8 (C=O), 128.8, 128.1, 127.9, 126.9, 121.4, 121.2, 109.8, 109.2 (C_{Ph}), 58.4 (N*CH₂*Ph), 52.8 (COCH₂CH₂*CH₂*N), 50.7 (*CH*CH₂CH₂N), 41.3 (CHCH₂*CH₂*N), 31.1 (CO*CH₂*CH₂CH₂N), 29.8 (CH*CH₂*CH₂N), 22.6 (COCH₂*CH₂*CH₂N)
LC/MS (ES⁺) *m*/*z* 483.3 (M+H)⁺

### 29) Preparation of 2-thiobenzimidazolone

### Preparation of (S)-2-(dibenzylamino)propyl 4-(1,2-dihydro-2-thioxobenzo[d]imidazol-3-yl)piperidine-1-carboxylate (Compound n°97):

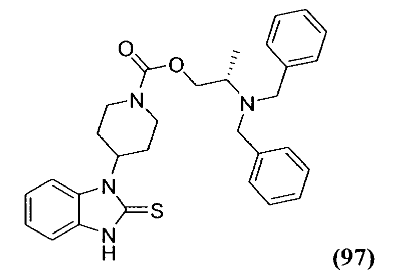

To a solution of 147 mg (0.295 mmol, 1 equiv.) of (S)-2-(dibenzylamino)propyl 4-(1,2-dihydro-2-oxobenzo[d]imidazol-3-yl)piperidine-1-carboxylate in 3 ml of toluene was added 89 mg (0.221 mmol, 0.221 equiv.) of Lawesson reagent. The suspension was refluxed overnight. The solid was filtered and the solvent evaporated in vacuum. The residue was purified on silica gel.
Colorless oil (20%).
Preparative LCMS
¹H NMR (400 MHz, CDCl₃, ppm) δ 11.20 (s, 1H, *NH*), 7.39 (d, *J* = 7.3 Hz, 4H, *Ph*), 7.28 (t, *J* = 7.4 Hz, 6H, *Ph*), 7.21 (t, *J* = 7.3 Hz, 3H, *Ph*), 7.15 (t, *J* = 7.6 Hz, 1H, *Ph*), 5.46 (m, 1H, *CH*CH₂CH₂N), 4.45 (m, 1H), 4.30 (dd, *J* = 10.9, 7.6 Hz, 1H, O*CH₂*), 4.10 (dd, *J* = 12.2, 6.4 Hz, 1H, O*CH₂*), 3.78 (d, *J* = 13.9 Hz, 2H, N*CH₂*Ph), 3.57 (d, *J* = 13.9 Hz, 2H, N*CH₂*Ph), 3.17 (sext., *J* = 6.8 Hz, 1H, *CH*CH₃), 3.02 (m, 2H, CHCH₂*CH₂*N), 2.37 (dd, *J* = 12.3, 3.6 Hz, 2H, CH*CH₂*CH₂N), 1.96 (d, *J* = 8.1 Hz, 2H, CH*CH₂*CH₂N), 1.12 (d, *J* = 6.8 Hz, 3H, CH*CH₃*)
LC/MS(ES⁺) *m*/*z* 515.1 (M+H)⁺

### 30) Amine preparation:

### Preparation of 2-((S)-2-(dibenzylamino)propyl)isoindoline-1,3-dione (Intermediate):

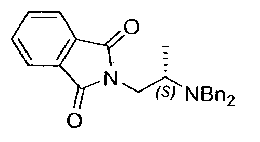

To a stirred suspension of phtalimide (824 mg, 5.60 mmol), (S)-2-(dibenzylamino)propan-1-ol (1.02 g, 4.00 mmol) and PPh₃ (1.47 g, 5.60 mmol) in THF (30 mL) cooled to 0°C under N₂ was added dropwise DEAD (1.2 mL, 7.60 mmol). The resulting mixture was allowed to warm to room temperature and stirred overnight. The solvent was evaporated. The residue was purified by flash chromatography (silica gel, cyclohexane/methylene chloride 5/5) to afford a white solid (1.08 g, 70%).
¹H NMR (400 MHz, CDCl₃) δ 7.81 (dd, *J* = 5.6, 2.8 Hz, 2H, Ar), 7.74 (dd, *J =* 5.6, 3.2 Hz, 2H, Ar), 7.20 (m, 4H, Ar), 7.11 (m, 6H, Ar), 4.00 (dd, *J =* 14.0, 9.2 Hz, 1H, *CH₂*N), 3.81 (d, *J* = 13.6 Hz, 2H, N*CH₂*Ph), 3.38 (m, 3H, *CH₂*N, N*CH₂*Ph), 3.23 (m, 1H, *CH*Me), 1.13 (d, *J =* 6.8 Hz, 3H, Me).
LC/MS (ES⁺) *m*/*z* 385.3 (M+H)⁺

### Preparation of (S)-N,N-dibenzylpropane-1,2-diamine (Intermediate):

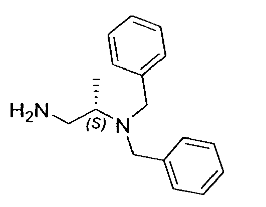

To a stirred solution of 2-((S)-2-(dibenzylamino)propyl)isoindoline-1,3-dione (1.08 g, 2.80 mmol) in ethanol (30 mL) was added hydrazine monohydrate (354 µL, 7.30 mmol) under N₂ atmosphere. The reaction mixture was refluxed for 3h at room temperature. The residue was filtered and washed with ethanol. The filtrate was concentrated under *vacuo* and the crude material was purified by flash chromatography (silica gel, methylene chloride/ethanol/NEt₃ 89/10/1) to afford a colorless oil (700 mg, 98%).
¹H NMR (400 MHz, CDCl₃) δ 7.32 (m, 8H, Ar), 7.23 (m, 2H, Ar), 3.77 *(d, J =* 13.6 Hz, 2H, N*CH₂*Ph), 3.37 (d, *J* = 13.6 Hz, 2H, N*CH₂*Ph), 2.73 (m, 2H, *CH₂*N), 2.47 (m, 1H, *CH*Me), 0.98 (d, *J* = 6.4 Hz, 3H, Me).
LC/MS (ES⁺) *m*/*z* 255.1 (M+H)⁺

### 31) Preparation of aminoalcohols

### a. By dibenzylation:

### Preparation of 2-Dibenzylamino-ethanol (Intermediate):

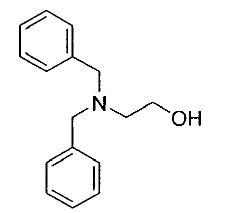

To a vigorously stirred solution of 6.7 ml (1.12 mol, 1 equiv.) ethanolamine in 120 ml of (1/1) MeOH/H₂O was added 7.2 g (1.8 mol., 1.6 equiv.) of sodium hydroxide. The suspension was refluxed for 30 minutes before addition of 24.5 ml (2.3 mol., 2 equiv.) of benzyl chloride. The mixture was refluxed overnight before cooled to room temperature and extracted with 3x160 ml of diethyl ether. The organic solution was dried over sodium sulphate, and evapored. The residue was distilled by Kugel Rohr (100°C, 1 mm Hg).
Colorless oil (54%).
¹H NMR (400 MHz, CDCl₃) δ 7.30 (m, 10H, Ph), 3.68 (s, 4H, N*CH₂*Ph), 3.60 (t, *J* = 5.4 Hz, 2H, O*CH₂*CH₂N), 2.68 (t, *J* = 5.4 Hz, 2H, OCH₂*CH₂*N), 2.36 (s_{b}, 1 H, OH)
LC/MS (ES⁺) *m*/*z* 242.1 (M+H)⁺

### b. By reduction of corresponding acid:

### Preparation of N,N-Dibenzyl-succinamic acid (Intermediate):

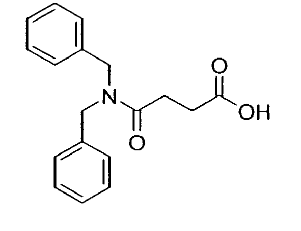

To a stirred solution of dibenzylamine (960 µL, 5mmol, 1 equiv.) was added 1 g (5 mmol, 1 equiv.) of anhydride succinic acid and the resulting mixture heated at 50°C overnight. The white solid obtained was dissolved in 10 ml of sodium hydroxide (1M) and was extracted twice by diethyl ether. The aqueous layer was acidified by HCl (4M) to pH 1 and was extracted by dichloromethane. The dichloromethane layer was dried over sodium sulphate, evaporated and used without further purification.
White solid (91 %)
¹H NMR (400 MHz, CDCl₃) δ 10.00 (s_{b}, 1H, *COOH*), 7.30 (m, 10H, *Ph),* 4.62 (s, 2H, N*CH₂*Ph), 4.48 (s, 2H, N*CH₂*Ph), 2.77 (m, 4H, N*CH₂CH₂*COOH
LC/MS (ES⁺) *m*/*z* 298.1 (M+H)⁺

### Preparation of 4-Dibenzylamino-butan-1-ol (Intermediate):

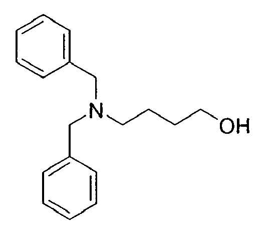

To a stirred suspension lithium aluminium hydride in 4 ml of anhydrous THF was added N,N-Dibenzyl-succinamic acid (185 mg (0.623 mmol, 1 equiv.). The resulting mixture was stirred overnight. Successively 60 µL of water, 60 µL of sodium hydride (2N) and 180 µL of water were added. The precipitate was filtred off and washed with ethyl acetate. The filtrate was dried over sodium sulphate, evaporated and used without further purification.
Yellow oil (75%)
¹H NMR (400 MHz, CDCl₃) δ 7.30 (m, 10H, *Ph*), 4.62 (m, 6H, N*CH₂*Ph, O*CH₂*(CH₂)₂CH₂N), .62 (m, 6H, N*CH₂*Ph, O*CH₂*(CH₂)₂CH₂N), 2.48 (t, *J* = 6.1 Hz, 2H, OCH₂(CH₂)₂*CH₂*N), 1.65 (m, 4H, OCH₂(*CH₂*)₂CH₂N)
LC/MS (ES⁺) *m*/*z* 270.2 (M+H)⁺

### 32) Preparation of bromide from alcohol,

To a stirred solution of (1 mmol., 1 equiv.) appropriate alcohol in 6 ml of anhydrous dichloromethane at 0°C were added successively 365 mg (1.1 mmol., 1.1 equiv.) of carbon tetrabromide and 287 mg (1.1 mmol., 1.1 equiv.) of triphenylphosphine. The reaction was pursued at 0°C for I h. The mixture was evaporated and purified on silica gel as below or was used whithout purification for "one-pot" alkylation procedure.

### Preparation of(2-Bromo-ethyl)-carbamic acid tert-butyl ester (Intermediate):

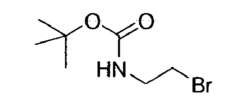

Flash chromatography on silica gel (methylene chloride/methyl alcohol 98/2)
Colorless oil (96%)
¹H NMR (400 MHz, CDCl₃) δ 4.96 (s_{b}, 1H, NH), 3.53 (m, 2H, NCH₂*CH₂*Br), 3.45 (m, 2H, NCH₂*CH₂*Br), 1.45 (s, 9H, *t*Bu)
LC/MS (ES⁺) *m*/*z* 224.1 (M+H)⁺

### 33) Preparation of aldehyde and acid

### Preparation of 4-Dibenzylamino-butyric acid ethyl ester (Intermediate):

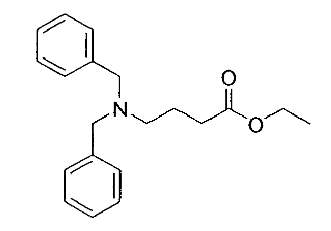

To a solution of dibenzylamine (962 µL, 5 mmol., 1 equiv.) in 2.5 ml of anhydrous DMF was added 715 µL (5 mmol., I equiv.) of ethyl 4-bromobutyrate, 1.38 g (10 mmol., 2 equiv.) of potassium carbonate and 83 mg (0.5 mmol., 0.1 equiv.) of potassium iodide. The resulting mixture was heated at 80°C overnight. The slurry was partitioned with water and ethyl acetate. The aqueous layer was extracted twice with ethyl acetate. The organic layer was dried over sodium sulphate, evaporated and used without further purification.
Yellow oil (98%)
¹H NMR (400 MHz, CDCl₃) δ 7.26 (m, 10H, *Ph*), 4.10 (q, *J* = 7.1 Hz, 2 H, O*CH₂*CH₃), 3.56 (s, 4H, N*CH₂*Ph), 2.45 (t, *J* = 6.8 Hz, 2H, N*CH₂*CH₂CH₂COOEt), 2.32 (t, *J* = 7.5 Hz, 2H, NCH₂CH₂*CH₂*COOEt), 1.83 (m, 2H, NCH₂C*H*₂CH₂COOEt), 1.22 (t, *J* = 7.1 Hz, 3H, OCH₂*CH₃*),
LC/MS (ES⁺) *m*/*z* 313.3 (M+H)⁺

### Preparation of 4-Dibenzylamino-butyraldehyde (Intermediate):

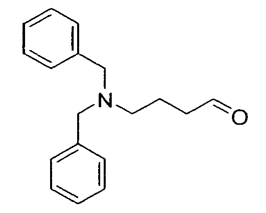

To stirred solution of 297 mg (1 mmol., 1 equiv.) of 4-Dibenzylamino-butyric acid ethyl ester in 2 ml of anhydrous dichloromethane at - 78°C was added dropwise 1.1 ml (1.1 mmol., 1.1 equiv.) of diisobutylaluminium hydride (1 M in dichloromethane). After 40 minutes, 2x40 µL of water was added, and allowed to warm to room temperature. The mixture was diluted with ethyl acetate and filtered. The organic layer was dried over sodium sulphate, evaporated and used without further purification.
Yellow oil (74%)
¹H NMR (400 MHz, CDCl₃) δ 9.70 (s, 1H, *CH*O), 7.26 (m, 10H, *Ph),* 3.54 (s, 4H, N*CH₂*Ph), 2.44 (m, 4H, N*CH₂*CH₂*CH₂*CHO), 1.83 (m, 2H, NCH₂*CH₂*CH₂CHO)
LC/MS (ES⁺) *m*/*z* 268.1 (M+H)⁺

### Preparation of 4-Dibenzylamino-butyric acid (Intermediate):

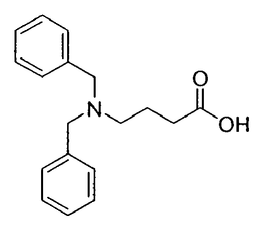

To a stirred solution of 500 mg (1.6 mmol., 1 equiv.) of 4-Dibenzylamino-butyric acid ethyl ester in 1 ml of THF was trailed by 4 ml of HCl 2N for 48 hours. The aqueous layer was extracted twice by ethyl acetate. The pH was adjusted to five with sodium carbonate and extracted by dichloromethane. The dichloromethane layer was dried over sodium sulphate, evaporated and used without further purification.
Yellow oil (40%)
¹H NMR (400 MHz, CDCl₃) δ 12.00 (s_{b}, 1H, *COOH*), 7.30 (m, 10H, *Ph),* 3.75 (s, 4H, N*CH₂*Ph), 2.67 (t, *J* = 6.0 Hz, 2H, N*CH₂*CH₂CH₂COOH), 2.25 (t, *J* = 6.5 Hz, 2H, NCH₂CH₂*CH₂*COOH), 1.80 (m, 2H, NCH₂*CH₂*CH₂COOH)
LC/MS (ES⁺) m/z 284.1 (M+H)⁺

### 34) Preparation of N-Boe-4-hydroxvpiperidine (Intermediate)

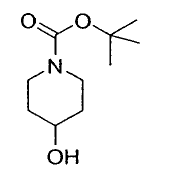

To a stirred solution of 2.5 g (12.5 mmol, 1 equiv.) of *N*-*tert-*buthyloxycarbonyl-4-piperidone in 25 ml of methyl alcohol was added 946 mg (25 mmol, 2 eq) of sodium borohydride at 0°C. The reaction was pursued at 0°C for 2 hours and 2 hours at room temperature. The mixture was diluted by water and brine and extracted by ethyl acetate. The organic layer was dried over sodium sulphate and the solvent was evaporated in vacuum. The residue was used without further purification.
White solide (100%)
¹H NMR (400 MHz, CDCl₃) δ 3.80 (m, 3H, N*CH₂*CH₂CH), 3.03 (t, *J* = 10.4 Hz, 2H, N*CH₂*CH₂CH), 3.75 (s, 4H, N*CH₂*Ph), 2.67 (t, *J* = 6.0 Hz, 2H, N*CH₂*CH₂CH₂COOH), 2.25 (t, *J* = 6.5 Hz, 2H, NCH₂CH₂*CH₂*COOH), 1.80 (m, 2H, NCH₂*CH₂*CH₂COOH)
LC/MS (ES⁺) *m*/*z* 284.1 (M+H)⁺

### 35) Preparation of building blocks by alkylation

### Preparation of 4-(2-Oxo-1,2-dihydro-indol-3-ylidene)-piperidine-1-carboxylic acid tert-butyl ester (Intermediate):

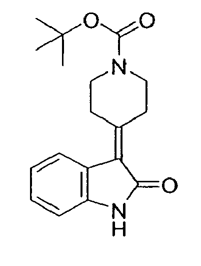

To a stirred solution of indolinone (666 mg, 5 mmol, 1 equiv.) of was solubilized in 10 ml of anhydrous THF. To this solution was added by portion 440 mg (11 mmol, 2.2 equiv.) of sodium hydride at 0°C. After 30 minutes, 996 mg (5 mmol, 1 equiv.) of *N*-*t*-butylcarbonylpiperidin-4-one was added. The reaction was allowed to warm to room temperature overnight and stopped by addition of satured solution of ammonium chloride. The mixture was extracted by ethyl acetate. The organic layer was dried over sodium sulphate, evaporated and purified on silica gel.
Flash chromatography on silica gel (cyclohexane/ethyl acetate 8/2 to 1/1)
Pale Yellow oil (50%)
¹H NMR (400 MHz, CDCl₃) δ 7.95 (s, 1H, NH), 7.51 (d, *J* = 7.8 Hz, 1H, *Ph),* 7.23 (dd, *J =* 10.5, 18.1 Hz, 1H, *Ph),* 7.02 (t, *J* = 7.6 Hz, 1H, *Ph*), 6.87 (d, *J* = 7.7 Hz, 1H, *Ph),* 3.61 (t, *J* = 5.7 Hz, 2H, CqCH₂*CH₂*N), 3.56 (m, 4H, Cq*CH₂CH₂*N), 3.01 (t, *J* = 5.9 Hz, 2H, Cq*CH₂*CH₂N), 1.49 (s, 9H, *t*-Bu)
¹³C NMR (100 MHz, CDCl₃) δ 169.6 (tBuO*C*=*O*), 156.3 (C=O), 139.7 (Cq), 128.1, 123.8 (CH_{Ph}), 123.5, 122.4 (Cq), 121.8 (CH_{Ph}), 114.9 (Cq), 109.7 (CH_{Ph}), 79.8 ((CH₃)₃*C_{q}*O), 32.0, 30.1 (CqCH₂*CH₂*N), 28.4 (Cq*CH₂*CH₂N), 28.3 ((*CH₃*)₃C_{q}O)
LC/MS (ES⁺) *m*/*z* 315.1 (M+H)⁺

### 36) Preparation of hydrochloride salt

### Preparation of (S)-2-(dibenzylamino)-3-phenylpropyl 4-(1-benzyl-1,2-dihydro-2-oxobenzo[d]imidazol-3-yl)piperidine-1-carboxylate, hydrochloride salt (Compound n°99)

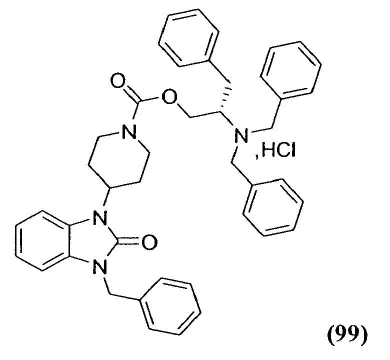

The hydrochloride salt was prepared from (S)-2-(dibenzylamino)-3-phenylpropyl 4-(1-benzyl-1,2-dihydro-2-oxobenzo[d]imidazol-3-yl)piperidine-1-carboxylate after treatment by HCl/AcOEt (1M). The solid was purified by washing with dry diethyl ether.
White solid (100%).
LC/MS (ES⁺) *m*/*z* 665.4 (M+H)⁺

### 37) Solid phase synthesis

### a. Synthesis of N-Benzyl-N-methyl polystyrene (Intermediate)

To a suspension of *Merrified resin* (3.1 mmol, 1 equiv., 1.97 mmol/g) in 50 ml of dry DMF was successively added 1.4 g (9.4 mmol, 3 equiv.) of sodium iodide, 1 g (4.7 mmol, 1.5 equiv.) of 1,8-bis(dimethylamine)naphtalene and 1.7 ml (15.6 mmol, 5 equiv.) of benzylamine. The suspension was heated for 30 hours at 90°C. The resin was filtered off and washed with hot DMF (2x20 ml), water (2x20 ml), and consecutive wash of methyl alcohol and dichloromethane (4x10 ml). Resin was dried in *vacuo* after washing with diethyl ether (1x10 ml)
White resin
Loading: 1.36 mmol/g
IR (KBr): u = 3446, 3022, 2922, 2308, 1944, 1872, 1798, 1746, 1720, 1652, 1601, 1509, 1492, 1452, 1360, 1181, 1102, 1022, 964, 903, 820, 738, 695, 522 cm⁻¹
Anal. Calcd N= 2.42%; Found N=1.90 %

### b. Synthesis of (4-(3-Benzyl-3-methylpolystyryl-1-triazenyl)-phenyl)-methanol (Intermediate)

To a solution of 893 mg (7.25 mmol, 5 equiv.) of 4-aminobenzylalcohol, 1.8 ml (14.5 mmol, 10 equiv.) of boron trifluoride diethyl ether complex in 10 ml of dry THF was added 1.7 ml (14.5 mmol, 10 equiv.) of *t-*butylnitrite at -10°C. The mixture was stirred 1 hours and the diazo suspension was solubiliezd by addition 10 ml of a solution of dried DMF/pyridine (1/1). 1 g (1.41 mmol, 1 equiv.) of N-Benzyl-N-methyl polystyrene was added and stirred for 1 hour. Resin was filtered off and washed by successively (9/1) DMF/pyridine (3x10 ml), (9/1) THF/NEt₃ (3x10 ml), (9/1) MeOH/NEt₃ (3x10 ml), MeOH (1x10 ml). This procedure was repeted for optimal loading. Resin was dried in *vacuo* after washing with diethyl ether (1x10 ml).
Orange resin
Loading: 0.92 mmol/g
IR (KBr): u = 3439, 3052, 3025, 2918, 1943, 1802, 1601, 1493, 1450, 1346, 1143, 1073, 1026, 904, 841, 753, 697, 537 cm⁻¹
Anal. Calcd N= 5.89 %; Found: N= 3.85 %

### c. Synthesis of (4-(3-Benzyl-3-methylpolystyryl-1-triazenyl)-phenyl)-formaldehyde (Intermediate)

To a suspension of N-Benzyl-N-methyl-N-(4-methanolphenyl) polystyrene (100 mg, 0.143 mmol, 1 equiv.) in 5 ml of dry dichloromethane was added 112 mg (0.28 mmol, 2 equiv.) of Dess Martin reagent. The resin was shaken overnight, filtered off and washed by CH₂Cl₂ (4x5 ml) and consecutive wash of methyl alcohol and dichloromethane (4x10 ml). Resin was dried in *vacuo* after washing with diethyl ether (1x10 ml).
Orange resin
IR (KBr): u = 3056, 3025, 2918, 2860, 2732, 2339, 1945, 1879, 1800, 1693, 1597, 1492, 1448, 1402, 1338, 1138, 1109, 838, 747, 696, 535 cm⁻¹

### d. Typical procedure for the preparation of aminoalcohol resins, first reductive amination

To a suspension of 100 mg (0.14 mmol, 1 equiv.) of N-Benzyl-N-methyl-N-(4-formylphenyl) polystyrene in 2 ml of a solution of dry dichloromethane/acetic acid (2,5% v/v) was added 1.4 mmol (10 equiv.) of appropriate aminoalcohol (typicaly ethanolamine) and shaken for 2 hours. 424 mg (2 mmol, 15 equiv.) of sodium triacetylborohydride was added and the suspension was shaken overnight. The excess of sodium triacetylborohydride was destroyed by carefully addition of methyl alcohol, resine was filtered off and wash by MeOH (1x5 ml), (9/1) THF/NEt₃ (3x5 ml x 15 minutes), (1/1) THF/water (2x5 ml) and consecutive wash of methyl alcohol and dichloromethane (4x5 ml). Resin was dried in *vacuo* after washing with diethyl ether (1 x5 ml).

### e. Typical procedure for the preparation of aminoalcohol resins, second reductive amination

To a suspension of 100 mg (0.14 mmol, 1 equiv.) of N-alkylaminoalcohol resin in 2 ml of dry trimethyl orthoformate was added 22.5 mg (1.4 mmol, 10 equiv.) of the corresponding aldehyde and the suspension was shaken overnight. The resin was filtered off in N₂ atmosphere and washed by dry DMF (2x2 ml), and dried in *vacuo.* Resin was suspended in 4 ml of dry dichloromethane before addition of 315 mg (1.4 mmol, 10 equiv.) of sodium triacetylborohydride and shaken overnight. Resine was filtered off and washed by MeOH (1x5 ml), (9/1) THF/NEt₃ (3x5 ml x 15 minutes), (1/1) THF/water (2x5 ml) and consecutive wash of methyl alcohol and dichloromethane (4x5 ml). Resin was dried in *vacuo* after washing with diethyl ether (1 x5 ml).

### f. Typical "CDI procedure" on solid phase

To a suspension of 95 mg (0.136 mmol, 1 equiv.) of N,N-dialkylaminoalcohol resin in 5 ml of dry THF was added 220 mg (1.36 mmol, 10 equiv.) of carbonyldiimidazole. The resin was shaken overnight, filtered off and washed by dry THF (3x5 mlx 5 minutes). Resin was suspended in 10 ml of dry THF and 147 mg (0.68 mmol, 5 equiv.) of 4-(2-keto-1-benzimidazolinyl)-piperidine was added. The resin was shaken for 72 hours, filtered off, washed by DMF (3x4 ml) and consecutively by methyl alcohol and dichloromethane (4x10 ml). Resin was dried in *vacuo* after washing with diethyl ether (1x5 ml).

### g. Typical cleavage procedure

To a suspension of 53 mg (0.076 mmol, 1 equiv.) of triazen resin in 2ml of dry dichloromethane was added at room temperature 30 µL (0.30 mmol, 4 equiv.) of trichlorosilane. The suspension was shaken for 12 hours and the excess of trichlorosilane was destroyed by addition of silice until no gaz appeared and directely eluted by 5 ml of methyl alcohol. The filtrate was concentrated in *vacuo* and purified on aminopropyl silica gel (eluant dichloromethane/methyl alcohol 9/1) afforded desired pure product. In some case a preparative HPLC purification was needed.

### Preparation of 2-(N-(4-nitrobenzyl)-N-benzylamino)ethyl 4-(1,2-dihydro-2-oxobenzo[d]imidazol-3-yl)piperidine-1-carboxylate (Compound n°98):

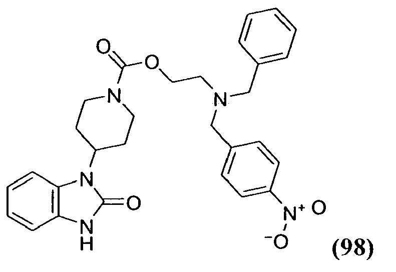

Colorless oil (16% for six steps).
Preparative HPLC
¹H NMR (400 MHz, CDCl₃, ppm) δ 9.11 (s, 1H, *NH*)*,* 8.17 (d, *J* = 8.4 Hz, 2H, *Ph),* 7.56 (d, *J* = 8.4 Hz, 2H, *Ph),* 7.32 (t, *J* = 7.6 Hz, 5H, *Ph),* 7.06 (m, 4H, *Ph),* 4.50 (m, 2H, *CH*CH₂*CH₂*N), 4.26 (m, 3H, *CHCH₂CH₂N,* O*CH₂*CH₂N), 3.76 (s, 2H, *CH₂*Ph), 3.68 (s, 2H, *CH₂Ph),* 2.93 (m, 2H, CHCH₂*CH*₂N), 2.79 (t, *J* = 5.6 Hz, 2H, OCH*₂CH*₂N), 2.35 (m, 2H, CH*CH₂*CH₂N), 1.86 (d, *J* = 11.6 Hz, 2H, CH*CH₂*CH₂N)
LC/MS (ES⁺) *m*/*z* 530.3 (M+H)⁺

### EXAMPLE 2: IN VIVO DEMONSTRATION OF THE ANTIPARASITIC ACTIVITY (ANTI-MALARIA) OF COMPOUNDS OF FORMULA (1)

In this example, we have tested the activity of different compounds of formula (I) on the proliferation of *Plasmodium falciparum.*

### 1) Materials and method

***Plasmodium falciparum* culture.** Prior to the experiments, parasites were maintained in culture according to the method described by Trager and Jensen (Trager, W. et al., Science, 1976, 193, 673-675).

### Labeled hypoxanthine incorporation assay:

*In vitro* anti-malarial activity was measured on asynchronous 3D7 chloroquine-sensitive *P. falciparum* infected red blood cells (RBC). Suspensions of infected RBC (1.5% final hematocrite, 0.6% parasitemia) were maintained in complete medium (RPMI 1640 complemented with 0.5% type I Albumax) either in absence of the compound of formula (I) (controls) or in contact with a range of molecular concentrations of compounds of formula (I) during 48 h (length of the parasite's cycle) according to the method described by Desjardins et al., Antimicrob Agents Chemother., 1979, 16(6), 710-718.

This range consists in serial dilutions of a high concentrated (10 mM) initial preparation of the different tested compounds of formula (I) or comparative prior art compounds in DMSO, diluted in complete medium at the first expected concentration. After 48 h incubation, 0.5 µCi [3H]-hypoxanthine (radiolabelled precursor of the nucleic acids metabolism) were added to each well of the microtiter plate. After an 18h-incubation, hypoxanthine incorporation reactions were stopped by a -80°C freezing. Parasite macromolecules, including radiolabeled nucleic acids were collected from the cell lysates on glass-fiber filters. Amount of radiolabeled uptake was measured after addition of scintillation cocktail in a liquid scintillation counter. Radioactive background was measured from uninfected red blood cells and subtracted from each corresponding well. Parasitic viability was expressed as the ability of compounds-treated parasites to synthesize nucleic acids from the radiolabelld hypoxanthine in comparison to control parasites treated in the absence of drugs. Parasite viability is expressed as a percentage of the control. Analyses of dose-effect curves were performed with the Graphpad Prism analytical software. IC₅₀, corresponding to the drug concentration leading to 50% parasite growth inhibition, were graphically determined from two independent experiments (different cell cultures, different drug dilution stocks) performed in duplicates.

Prior art comparative molecules for inhibition of *Plasmodium* proliferation are chloroquine, artesunate, artemisinin and triclosan.

### 2) Results

The results obtained are presented in table 1 below:

**TABLE 1**

| **Number of the tested compound** | **NAME** | **IC₅₀** assessed **on *Plasmodium Falciparum*** |
|---|---|---|
| | **Chloroquine** | 0.0103 µM |
| | **Artesunate** | 0.0097 µM |
| | **Artemisinine** | 0.0287 µM |
| | **Triclosan** | 1.55 µM |
| **25** | (S)-2-(dibenzylamino)propyl4-(1,2-dihydro-2-oxobenzo[d]imidazol-3-yl)piperidine-1-carboxylate | 4.9 µM |
| **26** | (S)-2-(dibenzylamino)-3-phenylpropyl 4-(1,2-dihydro-2-oxobenzo[d]imidazol-3-yl)piperidine-1-carboxytate | 1.4 µM |
| **30** | 2-(dibenzylamino)ethy 4-(1,2-dihydro-2-oxobenzo[d]imidazol-3-yl)piperidine-1-carboxylate | 6.9 µM |
| **29** | 3-(dibenzylamino)propyl4-(1,2-dihydro-2-oxobenzo[d]imidazol-3-yl)piperidine-1-carboxylate | 3.6 µM |
| **28** | (S)-2-(dibenzylamino)propyl 5,6-dihydro-4-(1,2-dihydro-2-oxobenzo[d]imidazol-3-yl)pyridine-1(2H)-carboxylate | 4.1 µM |
| **32** | (S)-2-(dibenzylamino)propyl 4-benzylpiperidine-1-carboxylate | 4.3 µM |
| **97** | (S)-2-(dibenzylamino)propyl 4-(1,2-dihydro-2-thioxobenzo[d]imidazol-3-yl)piperidine-1-carboxylate | 1.06 µM |
| **1** | ethyl2-(1-(((S)-2-(dibenzylamino)propoxy)carbonyl)piperidin-4-yloxy)-1H-indole-1-carboxylate | 2.14 µM |
| **3** | (S)-2-(dibenzylamino)propyl4-(1H-benzo[d]imidazol-1-yl)piperidine-1-carboxylate | 1.81 µM |
| **5** | (S)-2-(dibenzylamino)propyl 4-(1-methyl-1H-benzo[d]imidazol-2-yloxy)piperidine-1-carboxylate | 6.16 µM |
| **67** | tert-butyl 3-(1-(((S)-2-(dibenzylamino)propoxy)carbonyl)piperidin-4-yl)-2,3-dihydro-2-oxobenzo[d]imidazole-1-carboxylate | 0.785 µM |
| **49** | 1-(1-(3-(dibenzylamino)propyl)piperidin-4-yl)-1H-benzo[d]imidazol-2(3H)-one | 1.27 µM |
| **69** | (S)-2-(dibenzylamino)propyl 4-(1,2-dihydro-1-methyl-2-oxobenzo[d]imidazol-3-yl)piperidine-1-carboxylate | 1.54 µM |
| **52** | 1-(1-(2-(dibenzylamino)ethyl)piperidin-4-yl)-1H-benzo[d]imidazol-2(3H)-one | 1.57 µM |
| **4** | (S)-2-(dibenzylamino)propyl4-(1H-benzo[d][1,2,3]triazol-1-yl)piperidine-1-carboxylate | 5.95 µM |
| **51** | 1-(1-((S)-2-(dibenzylamino)-3-phenylpropyl)piperidin-4-yl)-1H-benzo[d]imidazol-2(3H)-one | 2.11 µM |
| **50** | 1-(1-((S)-2-(dibenzylamino)propyl)piperidin-4-yl)-1H-benzo[d]imidazol-2(3H)-one | 3.34 µM |
| **2** | (S)-2-(dibenzylamino)propyl 4-(2-oxobenzo[d]oxazol-3(2H)-yl)piperidine-1-carboxylate | 14.8 µM |
| **46** | O-2-(dibenzylamino)ethyl4-(1,2-dihydro-2-oxobenzo[d]imidazol-3-yl)piperidine-1-carbothioate | 2.84 µM |
| **21** | (S)-2-(dibenzylamino)propyl 4-(2-oxoindolin-3-ylidene)piperidine-1-carboxylate | 3.12 µM |
| **47** | O-2-(benzyloxy)ethyl 4-(1,2-dihydro-2-oxobenzo[d]imidazol-3-yl)piperidine-1-carbothioate | 23.2 µM |
| **48** | O-(S)-2-(dibenzylamino)propyl4-(1,2-dihydro-2-oxobenzo[d]imidazol-3-yl)piperidine-1-carbothioate | 2.95 µM |
| **19** | (S)-2-(dibenzylamino)propyl 4-((1,2-dihydro-2-oxobenzo[d]imidazol-3-yl)methyl)piperidine-1-carboxylate | 5.79 µM |
| **14** | (S)-2-(dibenzylamino)propyl4-(2-(trifluoromethyl)-1H-benzo[d]imidazol-1-yl)piperidine-1-carboxylate | 0.83 µM |
| **6** | (S)-2-(dibenzylamino)propyl 3-(1,2-dihydro-2-oxobenzo[d]imidazol-3-yl)propylcarbamate | 16.6 µM |
| **11** | (S)-2-(dibenzylamino)propyl 4-(2,3-dihydro-2-oxoimidazo[4,5-b]pyridin-1-yl)piperidine-1-carboxylate | 3.84 µM |
| **16** | (S)-2-(dibenzylamino)propyl 3-(1,2-dihydro-2-oxobenzo[d]lmidazol-3-yl)piperidine-1-carboxylate | 2.81 µM |
| **22** | (S)-2-(dibenzylamino)propyl 4-(2-oxoindolin-3-yl)piperidine-1-carboxylate | 3.87 µM |
| **71** | (S)-2-(dibenzylamino)propyl 4-(1-benzyl-1,2-dihydro-2-oxobenzo[d]imidazol-3-yl)piperidine-1-carboxylate | 0.77 µM |
| **72** | (S)-2-(dibenzylamino)propyl4-(1-(cyclohexylmethyl)-1,2-dihydro-2-oxobenzo[d]imidazol-3-yl)piperidine-1-carboxylate | 1.03 µM |
| **62** | (S)-2-(dibenzylamino)propyl 4-(2-oxo-3-methylsulfonyl-2,3-dihydro-1H-benzo[d]imidazol-1-yl)piperidine-1-carboxylate | 1.60 µM |
| **10** | (S)-2-(dibenzylamino)propyl 4-(1H-indol-1-yl)piperidine-1-carboxylate | 3.14 µM |
| **15** | (S)-2-(dibenzylamino)propyl 4-(2-methyl-1H-benzo[d]imidazol-1-yl)piperidine-1-carboxylate | 2.42 µM |
| **18** | 1-(S)-2-(dibenzylamino)propyl 3-ethyl 4-(1,2-dihydro-2-oxobenzo[d]imidazol-3-yl)piperidine- 1,3-dicarboxylate | 1.52 µM |
| **54** | (S)-2-(dibenzylamino)propyl 4-(2-oxo-3-phenylsulfonyl-2,3-dihydro-1H-benzo[d]imidazol-1-yl)piperidine-1-carboxylate | 0.23 µM |
| **64** | (S)-2-(dibenzylamino)propyl4-(1,2-dihydro-2-oxo-1-(benzoyl)benzo[d]imidazol-3-yl)piperidine-1-carboxylate | 2.88 µM |
| **65** | (S)-2-(dibenzylamino)propyl 4-(1-acetyl-1,2-dihydro-2-oxobenzo[d]imidazol-3-yl)piperidine-1-carboxylate | 2.94 µM |
| **7** | (S)-2-(dibenzylamino)propyl 4-(indolin-1-yl)piperidine-1-carboxylate | 2.83 µM |
| **38** | (S)-2-(dibenzylamino)propyl 4-(6-chloro-1,2-dihydro-2-oxobenzo[d]imidazol-3-yl)piperidine-1-carboxylate | 4.68 µM |
| **8** | (S)-2-(dibenzylamino)propyl4-(3,4-dihydroquinolin-1(2H)-yl)piperidine-1-carboxylate | 2.81 µM |
| **13** | (S)-2-(dibenzylamino)propyl 4-(1,2-dihydro-2-oxobenzo[d]imidazol-3-yl)azepane-1-carboxylate | 10.7 µM |
| **40** | 2-(1-benzylpiperidin-4-yl)ethyl 4-(1,2-dihydro-2-oxobenzo[d]imidazol-3-yl)piperidine-1-carboxylate | 8.16 µM |
| **41** | 2-(ethyl(phenyl)amino)ethyl 4-(2-oxo-2,3-dihydro-1H-benzo[d]imidazol-1-yl)piperidine-1-carboxylate | 24.4 µM |
| **42** | ((S)-1-benzylpyrrolidin-2-yl)methyl4-(1,2-dihydro-2-oxobenzo[d]imidazol-3-yl)piperidine-1-carboxylate | 18.8 µM |
| **36** | 3,3-diphenylpropyl4-(1,2-dihydro-2-oxobenzo[d]imidazol-3-yl)piperidine-1-carboxylate | 8.54 µM |
| **37** | 2-benzylamino bis [ethyl 4-(1,2-dihydro-2-oxobenzo[d]imidazol-3-yl)piperidine-1-carboxylate | 1.27 µM |
| **17** | (S)-2-(dibenzylamino)propyl 3-(1,2-dihydro-2-oxobenzo[d]imidazol-3-yl)pyrrolidine-1-carboxylate | 7.18 µM |
| **23** | (S)-2-(dibenzylamino)propyl 2-((1,2-dihydro-2-oxobenzo[d]imidazol-3-yl)methyl)pyrrolidine- 1-carboxylate | 7.27 µM |
| **20** | (S)-2-(dibenzylamino)propyl 4-(2-(1,2-dihydro-2-oxobenzo[d]imidazol-3-yl)ethyl)piperidine-1-carboxylate | 6.84 µM |
| **12** | (S)-2-(dibenzylamino)propyl 4-(1,2-dihydro-2-oxonaphtho[2,3-d]imidazol-3-yl)piperidine-1-carboxylate | 7.20 µM |
| **44** | (S)-2-(dibenzylamino)propyl4-(1H-indol-3-yl)piperidine-1-carboxylate | 5.21 µM |
| **45** | (S)-2-(dibenzylamino)propyl 4-(N-phenylpropionamido)piperidine-1-carboxylate | 7.20 µM |
| **96** | 1-[1-(4-dibenzylamino-butyryl)-piperidin-4-yl]-1,3-dihydrobenzoimidazol-2-one | 11.3 µM |
| **75** | (S)-2-(dibenzylamino)propyl 4-(1,2-dihydro-2-oxo-1-phenylbenzo[d]imidazol-3-yl)piperidine-1-carboxylate | 2.29 µM |
| **9** | 1-(1-(4-(dibenzylamino)butyl)piperidin-4-yl)-1H-benzo[d]imidazol-2(3H)-one | 2.95 µM |
| **68** | (S)-2-(dibenzylamino)-3-phenylpropyl 4-(1,2-dihydro-1-methyl-2-oxobenzo[d]imidazol-3-yl)piperidine-1-carboxylate | 0.452 µM |
| **53** | (S)-2-(dibenzylamino)-3-phenylpropyl4-(1,2-dihydro-3-phenylsulfonyl-2-oxobenzo[d]imidazol-1-yl)piperidine-1-carboxylate | 1.94 µM |
| **92** | N-((S)-2-(dibenzylamino)propyl)-4-(1,2-dihydro-2-oxobenzo[d]imidazol-3-yl)piperidine-1-carboxamide | 3.47 µM |
| **91** | N-((S)-2-(dibenzylamino)propyl)-4-(1,2-dihydro-2-oxobenzo[d]imidazol-3-yl)piperidine-1-carbothioamide | 2.71 µM |
| **24** | (S)-2-(dibenzylamino)propyl4-(2-oxoindolin-1-yl)piperidine-1-carboxylate | 3.64 µM |
| **77** | (S)-2-(dibenzylamino)propyl 4-(3-(4-ethoxy-4-oxobutyl)-2-oxo-2,3-dihydro-1 H-benzo[d]imidazol-1-yl)piperidine-1-carboxylate | 0.60 µM |
| **78** | (S)-4-(3-(1-((2-(dibenzylamino)propoxy)carbonyl)piperidin-4-yl)-2-oxo-2,3-dihydro-1H-benzo[d]imidazol-1-yl)butanoic acid | 6.29 µM |
| **79** | (S)(1-((2-(dibenzylamino)propoxy)carbonyl)piperidin-4-yl)-5,6-dihydro-imidazo[4,5,1j,k][1]benzazepine-2,7(1H,4H)-dione | 3.12 µM |
| **43** | 2,2-diphenylethyl4-(1,2-dihydro-2-oxobenzo[d]imidazol-3-yl)piperidine-1-carboxylate | 8.33 µM |
| **73** | (S)-2-(dibenzylamino)propyl 4-(3-(2-tert-butyloxycarbonylaminoethyl)-2-oxo-2,3-dihydro-1H-benzo[d]imidazol-1-yl)piperidine-1-carboxylate | 1.42 µM |
| **74** | (S)-2-(dibenzylamino)-3-phenylpropyl 4-(3-(2-tert-butyloxycarbonylaminoethyl)-2-oxo-2,3-dihydro-1H-benzo[d]imidazol-1-yl)piperidine-1-carboxylate | 0.384 µM |
| **76** | (S)-2-(dibenzylamino)-3-phenylpropyl 4-(3-(2 aminoethyl)-2-oxo-2,3-dihydro-1H-benzo[d]imidazol-1-yl)piperidine-1-carboxylate D-biotin amid | 0.48 µM |
| **55** | (S)-2-(dibenzylamino)propyl 4-(2-oxo-3-(4-propylphenylsulfonyl)-2,3-dihydro-1H-benzo[d]imidazol-1-yl)piperidine-1-carboxylate | 9.1 µM |
| **56** | (S)-2-(dibenzylamino)propyl 4-(3-(4-fluorophenylsulfonyl)-2-oxo-2,3-dihydro-1H-benzo[d]imidazo)-1-yl)piperidine-1-carboxylate | 0.33 µM |
| **57** | (S)-2-(dibenzylamino)propyl 4-(3-(4-methoxyphenylsulfonyl)-2-oxo-2,3-dihydro-1H-benzo[d]imidazol-1-yl)piperidine-1carboxylate | 0.41 µM |
| **58** | (S)-2-(dibenzylamino)propyl 4-(2-oxo-3-(4-(trifluoromethyl)phenylsulfonyl)-2,3-dihydro-1 H-benzo[d]imidazol-1-yl)piperidine-1-carboxylate | 0.42 µM |
| **59** | (S)-2-(dibenzylamino)propyl 4-(2-oxo-3-(thiophen-2-ylsulfonyl)-2,3-dihydro-1H-benzo[d]imidazol-1-yl)piperidine-1-carboxylate | 0.47 µM |
| **60** | (S)-2-(dibenzylamino)propyl 4-(2-oxo-3-(quinolin-8-ylsulfonyl)-2,3-dihydro-1H-benzo[d]imidazol-1-yl)piperidine-1-carboxylate | 0.78 µM |
| **61** | (S)-2-(dibenzylamino)propyl 4-(3-(naphthalen-1-ylsulfonyl)-2-oxo-2,3-dihydro-1H-benzo[d]imidazol-1-yl)piperidine-1-carboxylate | 0.95 µM |
| **94** | (S)-2-(dibenzylamino)propyl 2-(1-(((S)-2-(dibenzylamino)propoxy)carbonyl)piperidin-4-ylamino)-1H-benzo[d]imidazole-1-carboxylate | 2.62 µM |
| **93** | (S)-2-(Dibenzylamino)propyl 4-(1H-benzo[d]imidazol-2-ylamino)piperidine-1-carboxylate | 0.180 pM |
| **80** | (S)-2-(dibenzylamino)propyl 4-(phenylamino)piperidine-1-carboxylate | 3.41 µM |
| **81** | (S)-2-(dibenzylamino)propyl 4-(4-methoxyphenylamino)piperidine-1-carboxylate | 8.42 µM |
| **82** | (S)-2-(dibenzylamino)propyl 4-(2,4-dimethoxyphenylamino)piperidine-1-carboxylate | 2.38 µM |
| **83** | (S)-2-(dibenzylamino)propyl 4-(3,4,5-trimethoxyphenylamino)piperidine-1-carboxylate | 3.09 µM |
| **85** | (S)-2-(dibenzylamino)propyl 4-(4-iodophenylamino)piperidine-1-carboxylate | 8.09 µM |
| **98** | 2-(N-(4-nitrobenzyl)-N-benzylamino)ethyl 4-(1,2-dihydro-2-oxobenzo[d]imidazol-3-yl)piperidine-1-carboxylate | 3.04 µM |
| **63** | (S)-2-(dibenzylamino)propyl 4-(2-oxo-3-(4-acetamidylphenylsulfonyl)-2,3-dihydro-1H-benzo[d]imidazol-1yl)piperidine-1-carboxylate | 1.54 µM |
| **70** | (S)-2-(dibenzylamino)-3-phenylpropyl 4-(1-benzyl-1,2-dihydro-2-oxobenzo[d]imidazol-3-yl)piperidine- 1-carboxylate | 0.953 µM |
| **99** | (S)-2-(dibenzylamino)-3-phenylpropyl4-(1-benzyl-1,2-dihydro-2-oxobenzo[d]imidazol-3-yl)piperidine-1-carboxylate, hydrochloride salt | 0.873 µM |
| **95** | N-((S)-2-(dibenzylamino)propyl)-4-(1,2-dihydro-2-oxobenzo[d]imidazol-3-yl)piperidine-1-carboxamidine | 0.511 µM |
| **88** | (S)-2-(dibenzylamino)propyl 4-(1,3-diphenylureido)piperidine-1-carboxylate | 4.12 µM |
| **89** | (S)-2-(dibenzylamino)propyl 4-(N-phenylbenzamido)piperidine-1-carboxylate | 1.46 µM |
| **90** | (S)-2-(dibenzylamino)propyl 4-(N-phenylphenylsulfonamido)piperidine-1-carboxylate | 0.525 µM |
| **84** | (S)-2-(dibenzylamino)propyl 4-(3-fluorophenylamino)piperidine-1-carboxylate | 6.74 µM |
| **86** | (S)-2-(dibenzylamino)propyl 4-(3,4-dichlorophenylamino)piperidine-1-carboxylate | 7.25 µM |
| **87** | (S)-2-(dibenzylamino)propyl 4-(2-aminophenylamino)piperidine-1-carboxylate | 10.3 µM |

### EXAMPLE 3: IN VIVO DEMONSTRATION OF THE ANTIPARASITIC ACTIVITY (ANTI-TOXOPLASMA PROPERTIES) OF COMPOUNDS OF FORMULA (I)

In this example, we have tested the activity of different compounds of formula (I) on the proliferation of *Toxoplasma gondii.*

### 1) Materials and method

***Toxoplasma gondii* culture.** *Toxoplasma gondii* RH-β1 strain was maintained by serial passage in confluent human foreskin fibroblast (HFF) monolayer as described by Morisaki, J. H., et al., (J. Cell. Sci., 1995, 108, 2457-2464). RH-β1 strain carries the *Escherichia coli lacZ* (β-galactosidase) gene under the control of SAG1 promoter (Seeber and Boothroyd, Gene169, 1996, 39-45). Cultures were maintained in Dulbecco's Modified Eagle Medium (DMEM) containing L-glutamine, supplemented with 10% fetal bovine serum (FBS) and antibiotics (10000 U/ml Penicillin and 10 mg/ml Streptomycin) (Gibco, Invitrogen Corporation, UK) at 37°C with 5% CO₂. *Toxoplasma gondii* cells used in IC₅₀ assay were prepared as follows. A culture of RH-β1 which had completely lysed on HFF monolayer, was forced through a 27-gauge needle twice and was then filtered through a 3-µm-pore-size filter to remove host cell debris and parasite aggregates. The filters were rinsed with 10 ml phosphate-buffered saline (PBS). The combined flow-through was centrifuged at 250 x g for 10 min to collect the parasite. Then, they were washed by re-suspension in 5 ml PBS and repeated centrifugation. The resulting parasites were re-suspended, counted, and diluted in medium to a concentration of 10⁴ per 200 µl per well.

**Colorimetric microtiter assay.** Microtiter plates (96 wells per plate, 32-mm² wells; Coming, N.Y.) were seeded with HFF cells and allowed to grow to confluence in DMEM containing L-glutamine, 10% fetal bovine serum (FBS) and antibiotics (10000 U/ml Penicillin and 10 mg/ml Streptomycin) (Gibco, invitrogen corporation, UK) at 37°C with 5% C0₂. The DMEM used in this assay lacked phenol red since the readout is dependent on the generation and detection of a derivative of phenol red. HFF cell monolayers grown in the 96-well microtiter plates were infected with 10⁴ parasites per well. Invasion was allowed for 15 min at 37°C with 5% CO₂ after 15 min of parasite sedimentation on ice. Wells were then rinsed three times with PBS. Increasing doses of molecules were added. Controls consisted of DMEM lacking phenol red, with or without DMSO. Each assay was carried out in triplicates. Plates were then incubated at 37°C with 5% CO₂ for 48-72 hr to allow the formation of parasitophorous vacuoles (Conseil et al., Antimicrob Agents Chemother, 1999, 43, 1358-1361). β-galactosidase activity was measured as described by Seeber and Boothroyd, 1996. Briefly, plates were rinsed once with PBS and 100 µl of lysis buffer (100 mM HEPES pH8, 1 mM MgSO₄-7H₂O, 1% Triton X-100 and 5 mM DTT) was added, then plates were incubated at 50°C for 30 min. 110 µl of β-gal buffer (100 mM β-Mercaptoethanol, 9 mM MgCl₂ in 100 mM phosphate buffer pH7.3) was added and subsequently incubated for 5 min at 37°C. A final concentration of 1 mM chlorophenol red-beta-D-galactopyranoside (CPRG), diluted in phosphate buffer, was added. The plates were incubated from 30 min to 16 hours at 37°C, depending on the magnitude of β-galactosidase activity (i.e. color revelation). The plates were read at 570 and 630 nm on a Bio-tek microtiter plate reader. Results are presented as the mean ± 2 standard errors. Triplicates of the standard curve experiments were carried out in parallel, by infecting another 96-well plate with serial dilutions of the corresponding parasite strain (0-10⁷ parasite/well).

### 2) Results

The results obtained are tabulated in table 2 below:

**TABLE 2**

| **Number of the tested compound** | **NAME OF THE TESTED COMPOUND** | **IC₅₀** assessed **on Toxoplama Gondii** |
|---|---|---|
| | **Triclosan** | 0.320 µM |
| **25** | (S)-2-(dibenzylamino)propyl4-(1,2-dihydro-2-oxobenzo[d]imidazol-3-yl)piperidine-1-carboxylate | 14 µM |
| **26** | (S)-2-(dibenzylamino)-3-phenylpropyl4-(1,2-dihydro-2-oxobenzo[d]imidazol-3-yl)piperidine-1-carboxylate | 4.8 µM |
| **30** | 2-(dibenzylamino)ethyl 4-(1,2-dihydro-2-oxobenzo[d]imidazol-3-yl)piperidine-1-carboxylate | 7.5 µM |
| **29** | 3-(dibenzylamino)propyl 4-(1,2-dihydro-2-oxobenzo[d]imidazol-3-yl)piperidine-1-carboxylate | 2.5 µM |
| **27** | 2-(N-benzyl-N-methylamino)ethyl4-(1,2-dihydro-2-oxobenzo[d)imidazol-3-yl)piperidine-1-carboxylate | 2 µM |
| **28** | (S)-2-(dibenzylamino)propyl 5,6-dihydro-4-(1,2-dihydro-2-oxobenzo[d]imidazol-3-yl)pyridine-1(2H)-carboxylate | 3.5 µM |
| **31** | 2-(benzyloxy)ethyl 4-(1,2-dihydro-2-oxobenzo[d]imidazol-3-yl)piperidine-1-carboxylate | 0.5 µM |
| **97** | (S)-2-(dibenzylamino)propyl 4-(1,2-dihydro-2-thioxobenzo[d]imidazol-3-yl)piperidine-1-carboxylate | 3.5 µM |
| **1** | ethyl 2-(1-(((S)-2-(dibenzylamino)propoxy)carbonyl)piperidin-4-yloxy)-1H-indote-1-carboxylate | 5.2 µM |
| **3** | (S)-2-(dibenzylamino)propyl4-(1H-benzo[d]imidazol-1-yl)piperidine-1-carboxylate | 8 µM |
| **5** | (S)-2-(dibenzylamino)propyl 4-(1-methyl-1H-benzo[d]imidazol-2-yloxy)piperidine-1-carboxylate | 2.5 µM |
| **67** | tert-butyl 3-(1-(((S)-2-(dibenzylamino)propoxy)carbonyl)piperidin-4-yl)-2,3-dihydro-2-oxobenzo[d]imidazole-1-carboxylate | 2 µM |
| **49** | 1-(1-(3-(dibenzylamino)propyl)piperidin-4-yl)-1H-benzo[d]imidazol-2(3H)-one | 0.8 µM |
| **69** | (S)-2-(dibenzylamino)propyl4-(1,2-dihydro-1-methyl-2-oxobenzo[d]imidazol-3-yl)piperidine-1-carboxylate | 0.2 µM |
| **52** | 1-(1-(2-(dibenzylamino)ethyl)piperidin-4-yl)-1H-benzo[d]imidazol-2(3H)-one | 2 µM |
| **4** | (S)-2-(dibenzylamino)propyl 4-(1H-benzo[d][1,2,3]triazol-1-yl)piperidine-1-carboxylate | 4 µM |
| **51** | 1-(1-((S)-2-(dibenzylamino)-3-phenylpropyl)piperidin-4-yl)-1H-benzo[d]imidazol-2(3H)-one | 4 µM |
| **50** | 1-(1-((S)-2-(dibenzylamino)propyl)piperidin-4-yl)-1H-benzo[d)imidazol-2(3H)-one | 7 µM |
| **2** | (S)-2-(dibenzylamino)propyl4-(2-oxobenzo[d]oxazol-3(2H)-yl)piperidine-1-carboxylate | 0.8 µM |
| **46** | O-2-(dibenzylamino)ethyl4-(1,2-dihydro-2-oxobenzo[d]imidazol-3-yl)piperidine-1-carbothioate | 0.181 µM |
| **21** | (S)-2-(dibenzylamino)propyl 4-(2-oxoindolin-3-ylidene)piperidine-1-carboxylate | 1.36 µM |
| **47** | O-2-(benzyloxy)ethyl 4-(1,2-dihydro-2-oxobenzo[d]imidazol-3-yl)piperidine-1-carbothioate | 1.13 µM |
| **48** | O-(S)-2-(dibenzylamino)propyl 4-(1,2-dihydro-2-oxobenzo[d]imidazol-3-yl)piperidine-1-carbothioate | 1.18 µM |
| **19** | (S)-2-(dibenzylamino)propyl 4-((1,2-dihydro-2-oxobenzo[d]imidazol-3-yl)methyl)piperidine-1-carboxylate | 3.41 µM |
| **14** | (S)-2-(dibenzylamino)propyl 4-(2-(trifluoromethyl)-1H-benzo[d]imidazol-1-yl)piperidine-1-carboxylate | 2 µM |
| **6** | (S)-2-(dibenzylamino)propyl 3-(1,2-dihydro-2-oxobenzo[d]imidazol-3-yl)propylcarbamate | 0.381 µM |
| **11** | (S)-2-(dibenzylamino)propyl 4-(2,3-dihydro-2-oxoimidazo[4,5-b]pyridin-1-yl)piperidine-1-carboxylate | 2.5 µM |
| **33** | 1-benzylpiperidin-3-yl 4-(1,2-dihydro-2-oxobenzo[d]imidazol-3-yl)piperidine-1-carboxylate | 0.450 µM |
| **16** | (S)-2-(dibenzylamino)propyl 3-(1,2-dihydro-2-oxobenzo[d]imidazol-3-yl)piperidine-1-carboxylate | 2.35 µM |
| **22** | (S)-2-(dibenzylamino)propyl 4-(2-oxoindolin-3-yl)piperidine-1-carboxylate | 5.57 µM |
| **71** | (S)-2-(dibenzylamino)propyl 4-(1-benzyl-1,2-dihydro-2-oxobenzo[d]imidazol-3-yl)piperidine-1-carboxylate | 1.76 µM |
| **72** | (S)-2-(dibenzylamino)propyl 4-(1-(cyclohexylmethyl)-1,2-dihydro-2-oxobenzo[d]imidazol-3-yl)piperidine-1-carboxylate | 1.57 µM |
| **62** | (S)-2-(dibenzylamino)propyl 4-(2-oxo-3-methylsulfonyl-2,3-dihydro-1H-benzo[d]imidazol-1-yl)piperidine-1-carboxylate | 1.98 µM |
| **10** | (S)-2-(dibenzylamino)propyl 4-(1H-indol-1-yl)piperidine-1-carboxylate | 0.248 µM |
| **15** | (S)-2-(dibenzylamino)propyl 4-(2-methyl-1H-benzo[d]imidazol-1-yl)piperidine-1-carboxylate | 3.41 µM |
| **18** | 1-(S)-2-(dibenzylamino)propyl 3-ethyl4-(1,2-dihydro-2-oxobenzo[d]imidazol-3-yl)piperidine-1,3-dicarboxylate | 2.81 µM |
| **54** | (S)-2-(dibenzylamino)propyl 4-(2-oxo-3-phenylsulfonyl-2,3-dihydro-1 H-benzo[d]imidazol-1-yl)piperidine-1-carboxylate | 1.68 µM |
| **64** | (S)-2-(dibenzylamino)propyl 4-(1,2-dihydro-2-oxo-1-(benzoyl)benzo[d]imidazol-3-yl)piperidine-1-carboxylate | 0.96 µM |
| **65** | (S)-2-(dibenzylamino)propyl 4-(1-acetyl-1,2-dihydro-2-oxobenzo[d]imidazol-3-yl)piperidine-1-carboxylate | 0.99 µM |
| **7** | (S)-2-(dibenzylamino)propyl 4-(indolin-1-yl)piperidine-1-carboxylate | 1.98 µM |
| **38** | (S)-2-(dibenzylamino)propyl4-(6-chloro-1,2-dihydro-2-oxobenzo[d]imidazol-3-yl)piperidine-1-carboxylate | 1 µM |
| **8** | (S)-2-(dibenzylamino)propyl 4-(3,4-dihydroquinolin-1(2H)-yl)piperidine-1-carboxylate | 25 µM |
| **13** | (S)-2-(dibenzylamino)propyl 4-(1,2-dihydro-2-oxobenzo[d]imidazol-3-yl)azepane-1-carboxylate | 5 µM |
| **39** | 2-(diethylamino)ethyl4-(1,2-dihydro-2-oxobenzo[d]imidazol-3-yl)piperidine-1-carboxylate | 12 µM |
| **66** | (S)-2-(dibenzylamino)propyl 4-(1,2-dihydro-2-oxo-1-(pivaloyl)benzo[d]imidazol-3-yl)piperidine-1-carboxylate | 2.80 µM |
| **34** | 2-(diisopropylamino)ethyl4-(1,2-dihydro-2-oxobenzo[d]imidazol-3-yl)piperidine-1-carboxylate | 27 µM |
| **40** | 2-(1-benzylpiperidin-4-yl)ethyl4-(1,2-dihydro-2-oxobenzo[d]imidazol-3-yl)piperidine-1-carboxylate | 0.98 µM |
| **41** | 2-(ethyl(phenyl)amino)ethyl 4-(2-oxo-2,3-dihydro-1H-benzo[d]imidazol-1-yl)piperidine-1-carboxylate | 1.8 µM |
| **42** | ((S)-1-benzylpyrrolidin-2-yl)methyl 4-(1,2-dihydro-2-oxobenzo[d]imidazol-3-yl)piperidine-1-carboxylate | 5 µM |
| **35** | 2-(dimethylamino)ethyl 4-(1,2-dihydro-2-oxobenzo[d]imidazol-3-yl)piperidine-1-carboxylate | 4 µM |
| **36** | 3,3-diphenylpropyl4-(1,2-dihydro-2-oxobenzo[d]imidazol-3-yl)piperidine-1-carboxylate | 1.2 µM |
| **37** | 2-benzylamino bis [ethyl 4-(1,2-dihydro-2-oxobenzo[d]imidazol-3-yl)piperidine-1-carboxylate | 1.01 µM |
| **17** | (S)-2-(dibenzylamino)propyl 3-(1,2-dihydro-2-oxobenzo[d]imidazol-3-yl)pyrrolidine-1-carboxylate | 3.3 µM |
| **23** | (S)-2-(dibenzylamino)propyl 2-((1,2-dihydro-2-oxobenzo[d]imidazol-3-yl)methyl)pyrrolidine-1-carboxylate | 3.1 µM |
| **20** | (S)-2-(dibenzylamino)propyl 4-(2-(1,2-dihydro-2-oxobenzo[d]imidazol-3-yl)ethyl)piperidine-1-carboxylate | 4.2 µM |
| **12** | (S)-2-(dibenzylamino)propyl 4-(1,2-dihydro-2-oxonaphtho[2,3-d]imidazol-3-yl)piperidine-1-carboxylate | 0.75 µM |
| **44** | (S)-2-(dibenzylamino)propyl 4-(1H-indol-3-yl)piperidine-1-carboxylate | 1.85 µM |
| **45** | (S)-2-(dibenzylamino)propyl 4-(N-phenylpropionamido)piperidine-1-carboxylate | 1.3 µM |
| **96** | 1-[1-(4-dibenzylamino-butyryl)-piperidin-4-yl]-1,3-dihydro-benzoimidazol-2-one | 6.3 µM |
| **75** | (S)-2-(dibenzylamino)propyl 4-(1,2-dihydro-2-oxo-1-phenylbenzo[d]imidazol-3-yl)piperidine-1-carboxylate | 3.5 µM |
| **9** | 1-(1-(4-(dibenzylamino)butyl)piperidin-4-yl)-1H-benzo[d]imidazol-2(3H)-one | 1.5 µM |
| **68** | (S)-2-(dibenzylamino)-3-phenylpropyl 4-(1,2-dihydro-1-methyl-2-oxobenzo[d] imidazol-3-yl)piperidine- 1-carboxylate | 0.800 µM |
| **53** | (S)-2-(dibenzylamino)-3-phenylpropyl4-(1,2-dihydro-3-phenylsulfonyl-2-oxobenzo[d]imidazol-1-yl)piperidine-1-carboxylate | 22 µM |
| **92** | N-((S)-2-(dibenzylamino)propyl)-4-(1,2-dihydro-2-oxobenzo[d] imidazol-3-yl)piperidine-1-carboxamide | 15 µM |
| **91** | N-((S)-2-(dibenzylamino)propyl)-4-(1,2-dihydro-2-oxobenzo[d]imidazol-3-yl)piperidine-1-carbothioamide | 7.5 µM |
| **24** | (S)-2-(dibenzylamino)propyl 4-(2-oxoindolin-1-yl)piperidine-1-carboxylate | 11 µM |
| **77** | (S)-2-(dibenzylamino)propyl 4-(3-(4-ethoxy-4-oxobutyl)-2-oxo-2,3-dihydro-1H-benzo[d]imidazol-1-yl)piperidine-1-carboxylate | 5.7 µM |
| **78** | (S)-4-(3-(1-((2-(dibenzylamino)propoxy)carbonyl)piperidin-4-yl)-2-oxo-2,3-dihydro-1H-benzo[d]imidazol-1-yl)butanoic acid | 1.7 µM |
| **79** | (S)(1-((2-(dibenzylamino)propoxy)carbonyl)piperidin-4-yl)-5,6-dihydro-imidazo[4,5,1,j,k][1]benzazepine-2,7(1H,4H)-dione | 11.5 µM |
| **43** | 2,2-diphenylethyl4-(1,2-dihydro-2-oxobenzo[d]imidazol-3-yl)piperidine-1-carboxylate | 12 µM |
| **73** | (S)-2-(dibenzylamino)propyl 4-(3-(2-tert-butyloxycarbonylaminoethyl)-2-oxo-2,3-dihydro-1Hbenzo[d]imidazol-1-yl)piperidine-1-carboxylate | 3.0 µM |
| **74** | (S)-2-(dibenzylamino)-3-phenylpropyl 4-(3-(2-tert-butyloxycarbonylaminoethyl)-2-oxo-2,3-dihydro-1Hbenzo[d]imidazol-1-yl)piperidine-1-carboxylate | 3.2 µM |
| **76** | (S)-2-(dibenzylamino)-3-phenylpropyl 4-(3-(2 aminoethyl)-2-oxo-2,3-dihydro-1H-benzo[d]imidazol-1-yl)piperidine-1-carboxylate D-biotin amid | 2.6 µM |
| **55** | (S)-2-(dibenzylamino)propyl 4-(2-oxo-3-(4-propylphenylsulfonyl)-2,3-dihydro-1H-benzo[d]imidazol-1-yl)piperidine-1-carboxylate | 5.5 µM |
| **56** | (S)-2-(dibenzylamino)propyl 4-(3-(4-fluorophenylsulfonyl)-2-oxo-2,3-dihydro-1H-benzo[d]imidazol-1-yl)piperidine-1-carboxylate | 1.5 µM |
| **57** | (S)-2-(dibenzylamino)propyl 4-(3-(4-methoxyphenylsulfonyl)-2-oxo-2,3-dihydro-1H-benzo[d]imidazol-1-yl)piperidine-1-carboxylate | 1.75 µM |
| **58** | (S)-2-(dibenzylamino)propyl 4-(2-oxo-3-(4-(trifluoromethyl)phenylsulfonyl)-2,3-dihydro-1H-benzo[d]imidazol-1-yl)piperidine-1-carboxylate | 8.5 µM |
| **59** | (S)-2-(dibenzylamino)propyl 4-(2-oxo-3-(thiophen-2-ylsulfonyl)-2,3-dihydro-1H-benzo[d]imidazol-1-yl)piperidine-1-carboxylate | 3.8 µM |
| **61** | (S)-2-(dibenzylamino)propyl 4-(3-(naphthalen-1-ylsulfonyl)-2-oxo-2,3-dihydro-1 H-benzo[d]imidazol-1-yl)piperidine-1-carboxylate | 2.3 µM |
| **94** | (S)-2-(dibenzylamino)propyl 2-(1-(((S)-2-(dibenzylamino)propoxy)carbonyl)piperidin-4-ylamino)-1H-benzo[d]imidazole-1-carboxylate | 9 µM |
| **93** | (S)-2-(Dibenzylamino)propyl 4-(1H-benzo[d]imidazol-2-ylamino)piperidine-1-carboxylate | 0.98 µM |
| **80** | (S)-2-(dibenzylamino)propyl 4-(phenylamino)piperidine-1-carboxylate | 9.5 µM |
| **81** | (S)-2-(dibenzylamino)propyl 4-(4-methoxyphenylamino)piperidine-1-carboxylate | 10 µM |
| **82** | (S)-2-(dibenzylamino)propyl 4-(2,4-dimethoxyphenylamino)piperidine-1-carboxylate | 7.3 µM |
| **83** | (S)-2-(dibenzylamino)propyl 4-(3,4,5-trimethoxyphenylamino)piperidine-1-carboxylate | 16 µM |
| **85** | (S)-2-(dibenzylamino)propyl 4-(4-iodophenylamino)piperidine-1-carboxylate | 7.65 µM |
| **98** | 2-(N-(4-nitrobenzyl)-N-benzylamino)ethyl4-(1,2-dihydro-2-oxobenzo[d]imidazol-3-yl)piperidine-1-carboxylate | 3.33 µM |
| **63** | (S)-2-(dibenzylamino)propyl 4-(2-oxo-3-(4-acetamidylphenylsulfonyl)-2,3-dihydro-l H-benzo[d]imidazol-1yl)piperidine-1-carboxylate | 3 µM |
| **70** | (S)-2-(dibenzylamino)-3-phenylpropyl4-(1-benzyl-1,2-dihydro-2-oxobenzo[d]imidazol-3-yl)piperidine-1-carboxylate | 3.6 µM |
| **99** | (S)-2-(dibenzylamino)-3-phenylpropyl 4-(l 1-benzyl-1,2-dihydro-2-oxobenzo[d]imidazol-3-yl)piperidine-1-carboxylate, hydrochloride salt | 1.8 µM |
| **95** | N-((S)-2-(dibenzylamino)propyl)-4-(1,2-dihydro-2-oxobenzo[d]imidazol-3-yl)piperidine-1-carboxamidine | 11 µM |
| **88** | (S)-2-(dibenzylamino)propyl 4-(1,3-diphenylureido)piperidine-1-carboxylate | 2.7 µM |
| **89** | (S)-2-(dibenzylamino)propyl 4-(N-phenylbenzamido)piperidine-1-carboxylate | 2.7 µM |
| **90** | (S)-2-(dibenzylamino)propyl 4-(N-phenylphenylsulfonamido)piperidine-1-carboxylate | 1.75µM |
| **84** | (S)-2-(dibenzylamino)propyl 4-(3-fluorophenylamino)piperidine-1-carboxylate | 5.5 µM |
| **86** | (S)-2-(dibenzylamino)propyl 4-(3,4-dichlorophenylamino)piperidine-1-carboxylate | 3.75 µM |
| **87** | (S)-2-(dibenzylamino)propyl 4-(2-aminophenylamino)piperidine-1-carboxylate | 12.9 µM |

## Claims

1. A compound of general formula (I) below: 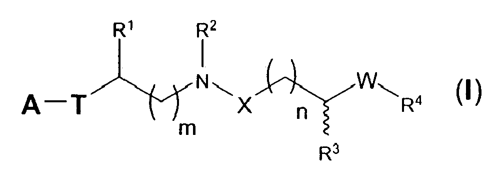 or a pharmaceutically acceptable salt thereof, wherein:
■ T is selected from the group consisting of -CH₂-, -CH₂CH₂-, -O-, -N-, a simple bond and a double bond;
■ m and n, independently, are an integer equal to 0, 1, 2, 3 or 4;
■ R¹ and R² simultaneously represent an hydrogen atom or R¹ and R² form together an alkyl chain having from 2 or 3 carbon atoms with possibly on insaturation;
■ R³ represents hydrogen or a group selected among alkyl, alkenyl, cycloalkyl, aryl, arylalkyl, heteroalkyl, heteroaryl and heteroarylalkyl groups wherein said groups defined for R³ are optionnaly substituted with one or more groups independently selected from halogen, trifluoromethyl, difluoromethyl, azido, alkyl, alkoxy, cyano, nitro and carbethoxy;
■ R⁴ represents hydrogen or a group selected among alkyl, alkenyl, cycloalkyl, aryl, arylalkyl, heteroaryl, heteroarylalkyl and heteroalkyl, wherein said groups defined for R⁴ are optionnaly substituted with one or more groups independently selected from halogen, trifluoromethyl, difluoromethyl, azido, alkyl, alkoxy, cyano, nitro, carboxy and carbethoxy;
■ X represents a single bond, -CH₂- or a functional group chosen among the groups of formulas (X-1) to (X-6) below: 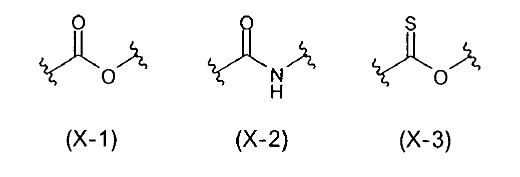 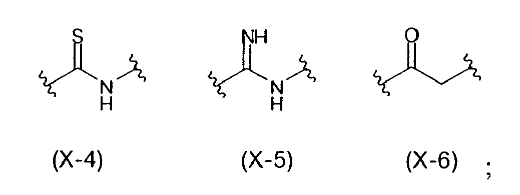
■ A represents a cyclic moiety selected from the group consisting of:
i) a moiety of formula (A-1) below: 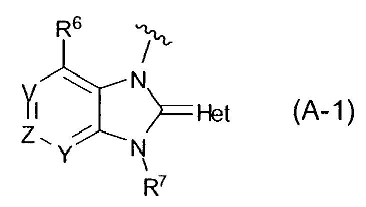 in which:
- V, Y and Z, identical or different, represent -CH-, C-R⁶ or N, it being understood that V can also form, together with R⁶ or Z, a fused aryl or heteroaryl ring depending of the nature of R⁶ or Z;
- Het represents an oxygen atom or a sulphur atom;
- R⁶ represents hydrogen, halogen, a group selected from the group consisting in alkoxy, alkyl, alkenyl, alkynyl, cycloalkyl, aryl, arylalkyl, heteroaryl and heteroalkyl, wherein said alkoxy, alkyl, alkenyl, alkynyl cycloalkyl, aryl, arylalkyl, heteroaryl and heteroalkyl groups are optionnaly substituted with one or more groups independently selected from halogen, trifluoromethyl, difluoromethyl, azido, alkyl, alkoxy, cyano and nitro;
- R⁷ represents a hydrogen atom, a linear or branched alkyl or alkenyl group; a heteroaryl group, a cycloalkyl group, an aryl group and an arylalkyl group, wherein said groups are optionnaly substituted with one or more groups independently selected from halogen, trifluoromethyl, difluoromethyl, azido, alkyl, alkoxy, cyano, nitro; a SO₂R⁸, a COR⁸ group or a COOR⁸ group wherein R⁸ represents a group chosen among alkyl, alkenyl, cycloalkyl, aryl, heteroaryl, arylalkyl and heteroalkyl, wherein said groups mentioned for R⁸ are optionnaly substituted with one or more groups independently selected from halogen, trifluoromethyl, difluoromethyl, azido, alkyl, alkoxy, cyano, nitro, carboxy and carbethoxy; a group of formula-alkylC(O)R⁹ in which R⁹ represents hydrogen or an alkoxy group; a group of formula alkyl-NHR¹⁰ in which R¹⁰ represents a alkoxycarbonyl group or a biotine moiety; R⁷ may also form, together with Y when Y is a carbon atom, a saturated heterocarbonated ring comprising 3 or 4 carbon atoms in addition to the carbon atom ofY, said ring being optionally substituted with an oxo functional group;
ii) a moiety of formula (A-2) below: 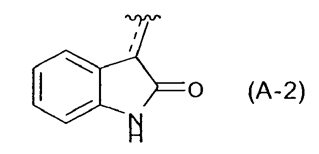 in which the dashed line represents an optional double bond;
iii) a moiety of formula (A-3) below: 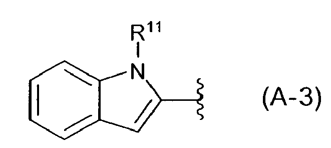 in which R¹¹ represents an ethylformate group or an alkylaryl group;
iv) a moiety of formula (A-4) below: 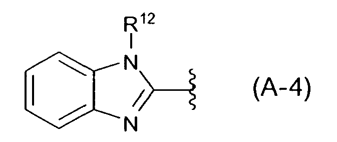 in which R¹² represents a hydrogen atom, an alkyl radical or a group of formula -NC(O)-OCH₂-CH(CH₃)-N-(Bz)₂ in which Bz stands for benzyl;
v) a moiety of formula (A-5) below: 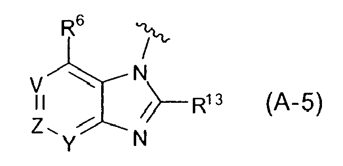 in which:
- V, Y, Z and R⁶ are as defined for the moiety of formula (A-1) above;
- R¹³ represents a hydrogen atom or a group chosen among, an alkyl, alkoxy, alkyl, alkenyl, aryl, arylalkyl, heteroaryl and heteroalkyl groups, wherein said groups defined for R¹³ are optionnaly substituted with one or more groups independently selected from halogen, trifluoromethyl, difluoromethyl, azido, alkyl, alkoxy, cyano and nitro;
vi) a moiety of formula (A-6) below: 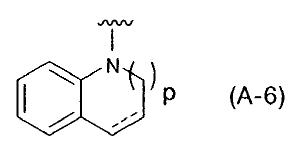 in which:
- p is an integer equal to 0, 1 or 2; and
- the dashed line represents an optional double bond;
vii) a moiety of formulas (A-7) or (A-7') below: 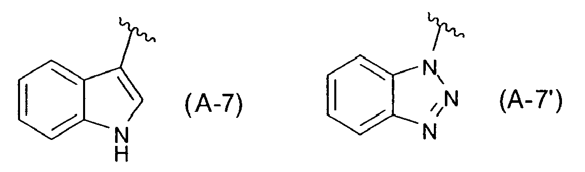
viii) a moiety of formula (A-8) below: 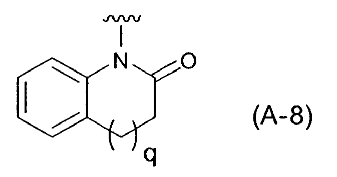 in which q is an integer equal to 0, 1 or 2;
ix) a moiety of formula (A-9) below: 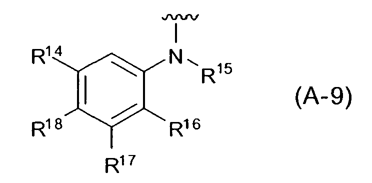 in which:
- R¹⁴, R¹⁶, R¹⁷ and R¹⁸, which may be identical or different, represent a hydrogen atom, a halogen atom, or a group selected among alkyl, nitro, alkoxy, aminoalkyl, aryl and heteroaryl groups;
- R¹⁵ represents a hydrogen atom, an alkyl group, an alkenyl group, an aryl group, a heteroaryl group or a group selected from the groups having the following formulas -COOR¹⁹, -C(O)NR¹⁹R²⁰, -C(S)NR¹⁹R²⁰, -C(O)NHR¹⁹, -C(O)R¹⁹,
- SO₂R¹⁹ in which R¹⁹ and R²⁰, which may be identical or different, represent a hydrogen atom, an alkoxy group, an amino group, an alkyl group, an alkenyl group, an aryl group and a heteroaryl group;
x) a moiety of formula (A-10) or (A-11) below: 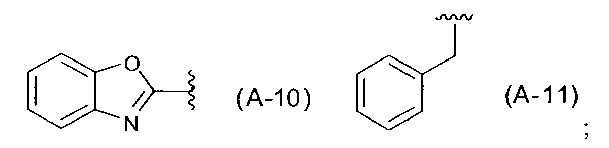
■ W represents C-R²¹, N-R²¹ or O-R²¹ wherein R²¹ represents a group selected among alkyl, alkenyl, cycloalkyl, aryl, arylalkyl heteroaryl and heteroalkyl, wherein said groups defined for R²¹ are optionnaly substituted with one or more groups independently selected from halogen, trifluoromethyl, difluoromethyl, azido, alkyl, alkoxy, cyano, nitro, carboxy and carbethoxy; W may also represents a moiety having the formula W-1 below: 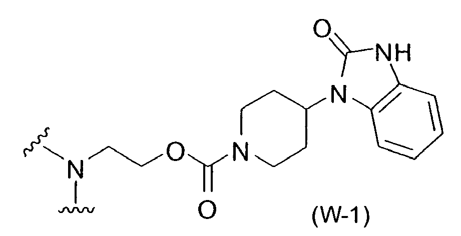 or a N-containing ring selected among moieties having the formulas W-2, W-3 and W-4 below:

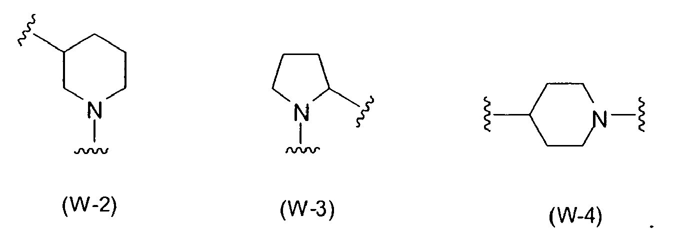

2. The compound of claim 1, wherein R¹ and R², together with the carbon atom (for R¹), the nitrogen atom (for R²) and the chain bonding the carbon atom bearing R¹ and the nitrogen atom bearing R² form a moiety selected from the group consisting of the following moieties: 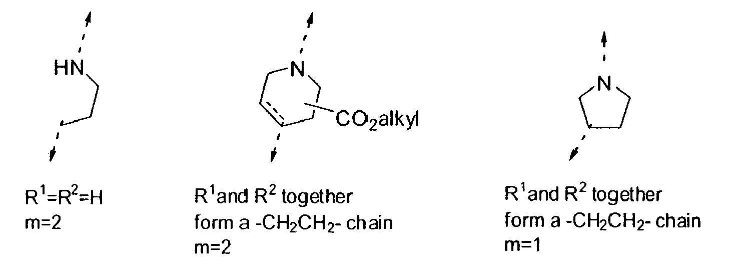 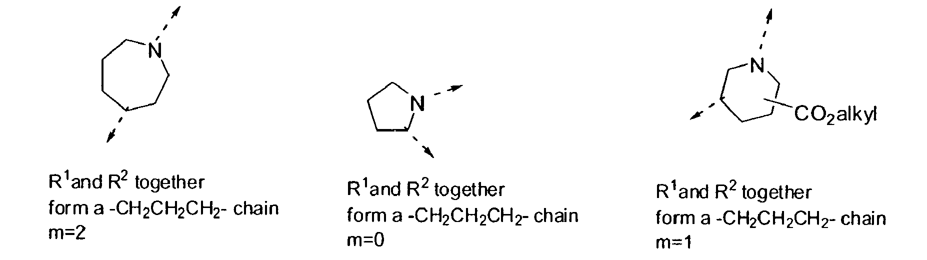 wherein the dashed arrows represent the attachment point of these moieties to T and X respectively via a covalent bond.

3. The compound of claim 1 or 2, wherein linear and branched alkyl groups are chosen among (C₁-C₄)alkyl groups and wherein the linear and branched alkoxy groups are chosen among (C₁-C₄)alkoxy groups.

4. The compounds of claim 3, wherein (C₁-C₄)alkyl groups are chosen among methyl, ethyl, n-propyl, isopropyl, n-butyl, tert-butyl and isobutyl radicals; whereas (C₁-C₄)alkoxy groups are chosen among methoxy, ethoxy, n-propyloxy, iso-propyloxy, n-butyloxy, *tert*-butyloxy and isobutyloxy radicals.

5. The compound of any one of the preceding claims, wherein halogen atoms are chosen among bromine, chlorine, fluorine and iodine.

6. The compound of any one of the preceding claims, wherein heteroaryl groups represents furan, pyridine, pyrrole, thiophene, imidazole, pyrazole, oxazole, isoxazole, thiazole, benzene, pyridine, pyrazine, pyrimidine, pyridazine, benzylcyclobutene, pentalene, benzofurane, isobenzofurane, indole, isoindole, benzothiophene, benzo[c]thiophene, benzimidazole, indazole, benzoxazole, benzisoxazole, benzothiazole, naphthalene, quinoline, isoquinoline, quinoxaline, quinazoline, cinnoline, purine, anthracene or acridine.

7. The compound of any one of the preceding claims, wherein said compounds is selected from the group consisting of:
- (S)-2-(dibenzylamino)propyl 4-(1,2-dihydro-2-oxobenzo[d]imidazol-3-yl)piperidine-1-carboxylate;
- (S)-2-(dibenzylamino)-3-phenylpropyl 4-(1,2-dihydro-2-oxobenzo[d]imidazo)-3-yl)piperidine-1-carboxylate;
- 2-(dibenzylamino)ethyl 4-(1,2-dihydro-2-oxobenzo[d]imidazol-3-yl)piperidine-1-carboxylate;
- 3-(dibenzylamino)propyl 4-(1,2-dihydro-2-oxobenzo[d]imidazol-3-yl)piperidine-1-carboxylate;
- 2-(N-benzyl-N-methylamino)ethyl 4-(1,2-dihydro-2-oxobcnzo[d]imidazol-3-yl)piperidine-1-carboxylate;
- (S)-2-(dibenzylamino)propyl 5,6-dihydro-4-(1,2-dihydro-2-oxobenzo[d]imidazol-3-yl)pyridine-1 (2H)-carboxylate;
- (S)-2-(dibenzylamino)propyl 4-benzylpiperidine-1-carboxylate;
- 2-(benzyloxy)ethyl 4-(1,2-dihydro-2-oxobenzo[d]imidazol-3-yl)piperidine-1-carboxylate;
- (S)-2-(dibenzylamino)propyl 4-(1,2-dihydro-2-thioxobenzo[d]imidazol-3-yl)piperidine-1-carboxylate;
- Ethyl 2-(1-(((S)-2-(dibenzylamino)propoxy)carbonyl)piperidin-4-yloxy)-1 H-indole-1-carboxylate;
- (S)-2-(dibenzylamino)propyl 4-(2-oxobenzo[d]oxazol-3(2H)-yl)piperidine-1-carboxylate;
- (S)-2-(dibenzylamino)propyl 4-(1H-benzo[d]imidazol-1-yl)piperidine-1-carboxylate;
- (S)-2-(dibenzylamino)propyl 4-(1-methyl-1H-benzo[d]imidazol-2-yloxy)piperidine-1-carboxylate;
- (S)-2-(dibenzylamino)propyl 3-(1,2-dihydro-2-oxobenzo[d]imidazol-3-yl) pyrrolidine-1-carboxylate;
- *tert*-butyl 3-(1-(((S)-2-(dibenzylamino)propoxy)carbonyl) piperidin-4-yl)-2,3-dihydro-2-oxobenzo[d]imidazole-1-carboxylate;
- 1-(1-(3-(dibenzylamino)propyl)plperidin-4-yl)-1H-benzo[d]-imidazol-2(3H)-one;
- (S)-2-(dibenzylamino)propyl 4-(1,2-dihydro-1-methyl-2-oxobenzo-[d]imidazol-3-yl)piperidine-1-carboxylate;
- 1-(1-(2-(dibenzylamino)ethyl)piperidin-4-yl)-1H-benzo[d]imidazol-2(3H)-one;
- (S)-2-(dibenzylamino)propyl 4-(1H-benzo[d][1,2,3]triazol-1-yl)piperidine-1-carboxylate;
- 1-(1-((S)-2-(dibenzylamino)-3-phenylpropyl)piperidin-4-yl)-1H-benzo[d]imidazol-2(3H)-one;
- 1-(1-((S)-2-(dibenzylamino)propyl)piperidin-4-yl)-1H-benzo[d]imidazol-2(3H)-one;
- O-2-(dibenzylamino)ethyl 4-(1,2-dihydro-2-oxobenzo[d]imidazol-3-yl)piperidine-1-carbothioate;
- (S)-2-(dibenzylamino)propyl 4-(2-oxoindolin-3-ylidene) piperidine-1-carboxylate;
- O-2-(benzyloxy)ethyl 4-(1,2-dihydro-2-oxobenzo[d]imidazol-3-yl)piperidine-1-carbothioate;
- O-(S)-2-(dibenzylamino)propyl 4-(1,2-dihydro-2-oxobenzo[d]imidazol-3-yl)piperidine-1-carbothioate;
- (S)-2-(dibenzylamino)propyl 4-((1,2-dihydro-2-oxobenzo[d]imidazol-3-yl)methyl)plperidine-1-carboxylate;
- (S)-2-(dibenzylamino)propyl 4-(2-(trifluoromethyl)-1H-benzo[d]imidazol-1-yl)piperidtne-1-carboxylate;
- (S)-2-(dibenzylamino)propyl 3-(1,2-dihydro-2-oxobenzo[d]imidazol-3-yl)propylcarbamate;
- (S)-2-(dibenzylamino)propyl 4-(2,3-dihydro-2-oxoimidazo[4,5-b]pyridin-1-yl)piperidine-1-carboxylate;
- 1-benzylpiperidin-3-yl 4-(1,2-dihydro-2-oxobenzo[d]imidazol-3-yl)piperidine-1-carboxylate;
- (S)-2-(dibenzylamino)propyl 3-(1,2-dihydro-2-oxobenzo[d]imidazol-3-yl)pipendine-1-carboxylate;
- (S)-2-(dibenzylamino)propyl 4-(2-oxoindolin-3-yl)piperidine-1-carboxylate;
- (S)-2-(dibenzylamino)propyl 4-(1-benzyl-1,2-dihydro-2-oxobenzo[d]imidazol-3-yl) piperidine-1-carboxylate;
- (S)-2-(dibenzylamino)propyl 4-(1-(cyclohexylmethyl)-1,2-dihydro-2-oxobenzo[d]lmldazol-3-yl)pipen'dine- I -carboxylate;
- (S)-2-(dibenzylamino)propyl 4-(2-oxo-3-methylsulfonyl-2,3-dihydro-1 H-benzo[d]imidazot-1-yl)piperidine-1-carboxylate;
- (S)-2-(dibenzylamino)propyl 4-(1H-indol-1-yl)piperidine-1-carboxylate;
- (S)-2-(dibenzylamino)propyl 4-(2-methyl-1H-benzo[d]imidazol-1-yl)piperidine-1-carboxylate;
- 1-(S)-2-(dibenzylamino)propyl 3-ethyl 4-(1,2-dihydro-2-oxobenzo[d]imidazol-3-yl)piperidine-1,3-dicarboxylate;
- (S)-2-(dibenzylamino)propyl 4-(2-oxo-3-phenylsulfonyl-2,3-dihydro-1H-benzo[d]imidazol-1-yl)piperidine-1-carboxylate;
- (S)-2-(dibenzylamino)propyl 4-(1,2-dihydro-2-oxo-1-(benzoyl)benzo[d]imidazol-3-yl)piperidine-1-carboxylate;
- (S)-2-(dibenzylamino)propyl 4-(indolin-1-yl)piperidine-1-carboxylate;
- (S)-2-(dibenzylamino)propyl 4-(6-chloro-1,2-dihydro-2-oxobenzo[d]lmidazol-3-yl)piperidine-1-carboxylate;
- (S)-2-(dibenzylamino)propyl 4-(3,4-dihydroquinolin-1(2H)-yl)piperidine-1-carboxylate;
- (S)-2-(dibenzylamino)propyl 4-(1,2-dihydro-2-oxobcnzo[d]imidazol-3-yl)azcpanc-1-carboxylatc;
- 2-(diethylamino)ethyl 4-(1,2-dihydro-2-oxobenzo[d]imidazol-3-yl)piperidine-1-carboxylate;
- (S)-2-(dibenzylamino)propyl 4-(1,2-dihydro-2-oxo-1-(pivaloyl)bcnzo[d]imidazol-3-yl)piperidine-1-carboxylate;
- 2-(diisopropylamino)ethyl 4-(1,2-dihydro-2-oxobenzo[d]imidazol-3-yl)piperidine-1-carboxylate;
- 2-(1-benzylpiperidin-4-yl)ethyl 4-(1,2-dihydro-2-oxobenzo[d]imidazol-3-yl)piperidine-1-carboxylate;
- 2-(ethyl(phenyl)amino)ethyl 4-(2-oxo-2,3-dihydro-1H-benzo[d]imidazol-1-yl)piperidine-1-carboxylate;
- ((S)-1-benzylpyrrolidin-2-yl)methyl 4-(1,2-dihydro-2-oxobenzo[d]imidazol-3-yl)piperidine-1-carboxylate;
- 2-(dimethylamino)ethyl 4-(1,2-dihydro-2-oxobenzo[d]imidazol-3-yl)piperidine-1-carboxylate;
- 3,3-diphenylpropyl 4-(1,2-dihydro-2-oxobenzo[d]imidazol-3-yl)piperidine-1-carboxylate;
- (S)-2-(dibenzylamino)propyl 2-((1,2-dihydro-2-oxobenzo[d]imidazol-3-yl)methyl)pyrrolidine-1-carboxylate;
- (S)-2-(dibenzylamino)propyl 4-(2-(1,2-dihydro-2-oxobenzo[d]imidazol-3-yl)ethyl)piperidine-1-carboxylate;
- (S)-2-(dibenzylamino)propyl 4-(1,2-dihydro-2-oxonaphtho[2,3-d]imidazol-3-yl)piperidine-1-carboxylate;
- (S)-2-(dibenzylamino)propyl 4-(1H-indol-3-yl)piperidine-1-carboxylate;
- (S)-2-(dibenzylamino)propyl 4-(N-phenylproplonamido) piperidine-1-carboxylate;
- (S)-2-(dibenzylamino)propyl 4-(1,2-dihydro-2-oxo-1-phenylbenzo[d]imidazol-3-yl)piperidine-1-carboxylate;
- 1-(1-(4-(dibenzylamino)butyl)piperidin-4-yl)-1H-benzo[d]imidazol-2(3H)-one;
- (S)-2-(dibenzylamino)-3-phenylpropyl 4-(1,2-dlhydro-1-methyl-2-oxobenzo[d]imidazol-3-yl)piperidine-1-carboxylate;
- (S)-2-(dibenzylamino)-3-phenylpropyl 4-(1,2-dihydro-3-phenylsulfonyl-2-oxobenzo[d]imidazol-1-yl)piperidine-1-carboxylate;
- N-((S)-2-(dibenzylamino)propyl)-4-(1,2-dihydro-2-oxobenzo[d]imidazol-3-yl)piperidine-1-carboxamide;
- N-((S)-2-(dibenzylamino)propyl)-4-(1,2-dihydro-2-oxobenzo[d]imidazol-3-yl)plperidine-1-carbothloamide;
- (S)-2-(dibenzylamino)propyl 4-(2-oxoindolin-1-yl)piperidine-1-carboxylate;
- (S)-2-(dibenzylamino)propyl 4-(3-(4-ethoxy-4-oxobutyl)-2-oxo-2,3-dihydro-1H-berizo[d]imidazol-1-yl)piperidine-1-carboxylate;
- (S)-4-(3-(1-((2-(dibenzylamino)propoxy)carbonyl)plperidin-4-yl)-2-oxo-2,3-dihydro-1H-benzo[d]imidazol-1-yl)butanoic acid;
- 2,2-diphenylethyl 4-(1,2-dihydro-2-oxobenzo[d]imidazol-3-yl)piperidine-1-carboxylate;
- (S)-2-(dibenzylamino)propyl 4-(2-oxo-3-(4-propylphenylsulfonyl)-2,3-dihydro-1H-benzo[d]imidazol-1-yl)piperidine-1-carboxylate;
- (S)-2-(dibenzylamino)propyl 4-(3-(4-fluorophenylsulfonyl)-2-oxo-2,3-dihydro-1 H-benzo[d]imidazol-1-yl)piperidine-1-carboxylate;
- (S)-2-(dibenzylamino)propyl 4-(3-(4-methoxyphenylsulfonyl)-2-oxo-2,3-dihydro-1 H-benzo[d]imidazot-1-yl)piperidme-1-carboxylate;
- (S)-2-(dibenzylamino)propyl 4-(2-oxo-3-(4-(trifluoromethyl) phenylsulfonyl)-2,3-dihydro-1 H-benzo[d]imidazol-1-yl)piperidine-1-carboxylate;
- (S)-2-(dibenzylamino)propyl 4-(2-oxo-3-(thiophen-2-ylsulfonyl)-2,3-dihydro-1 H-benzo[d]imidazot-1-yl)piperidine-1-carboxytate;
- (S)-2-(dibenzylamino)propyl 4-(2-oxo-3-(quinolin-8-ylsulfonyl)-2,3-dihydro-1 H-benzo[d]imidazol-1-yl)piperidine-1-carboxylate;
- (S)-2-(dibenzylamino)propyl 4-(3-(naphthalen-1-ylsulfonyl)-2-oxo-2,3-dihydro-1H-benzo[d]imidazol-1-yl)piperidine-1-carboxylate;
- (S)-2-(dibenzylamino)propyl 2-(1-(((S)-2-(dibenzylamino) propoxy)carbonyl)piperidin-4-ylamino)-1H-benzo[d]imidazole-1-carboxylate;
- (S)-2-(dibenzylamino)propyl 4-(1H-benzo[d]imidazol-2-ylamino)piperidine-1-carboxylate;
- (S)-2-(dibenzylamino)propyl 4-(phenylamino)piperidine-1-carboxylate;
- (S)-2-(dibenzylamino)propyl 4-(4-methoxyphenylamino) piperidine-1-carboxylate;
- (S)-2-(dibenzylamino)propyl 4-(2,4-dimethoxyphenylamino) piperidine-1-carboxylate;
- (S)-2-(dibenzylamino)propyl 4-(3,4,5-trimethoxyphenylamino) piperidine-1-carboxylate;
- (S)-2-(dibenzylamino)propyl 4-(4-iodophenylamino)piperidine-1-carboxylate;
- 2-(N-(4-nitrobenzyl)-N-benzylamino)ethyl 4-(1,2-dihydro-2-oxobenzo[d]imidazol-3-yl)piperidine-1-carboxylate;
- (S)-2-(dibenzylamino)propyl 4-(2-oxo-3-(4-acetamidylphenylsulfonyl)-2,3-dihydro-1 H-benzo[d]imidazol-1-yl)piperidine-1-carboxylate;
- (S)-2-(dibenzylamino)-3-phenylpropyl 4-(1-benzyl-1,2-dihydro-2-oxobenzo[d]imidazol-3-yl)piperidine-1-carboxylate;
- N-((S)-2-(dibenzylamino)propyl)-4-(1,2-dihydro-2-oxobenzo[d]imidazol-3-yl)piperidine-1-carboxamidine;
- (S)-2-(dibenzyiamino)propyl 4-(1,3-diphenylureldo)piperidine-1-carboxylate;
- (S)-2-(dibenzylamino)propyl 4-(N-phenylbenzamido)piperidine-1-carboxylate;
- (S)-2-(dibenzylamino)propyl 4-(N-phenylphenylsulfonamido) piperidine-1-carboxylate;
- (S)-2-(dibenzylamino)propyl 4-(3-fluorophenylamino)piperidine-1-carboxylate;
- (S)-2-(dibenzylamino)propyl 4-(3,4-dichlorophenylamino) piperidine-1-carboxylate;
- (S)-2-(dibenzyiamino)propyl 4-(2-aminophenylamino)piperidine-1-carboxylate;
- (S)-2-(dibenzylamino)propyl 4-(1-acetyl-1,2-dihydro-2-oxobenzo[d]imidazol-3-yl)piperidine-1-carboxylate;
- (S)(1-((2-(dibenzylamino)propoxy)carbonyl)piperidin-4-yl)-5,6-dihydro-imidazo[4,5, 1,j,k][1]benzazepine-2,7( 1H,4H)-dione;
- 2-benzylamino bis [ethyl 4-(1,2-dihydro-2-oxobenzo[d]imidazol-3-yl)piperidine-1-carboxylate;
- (S)-2-(dibenzylamino)propyl 4-(3-(2-tert-butyloxycarbonylaminoethyl)-2-oxo-2,3-dihydro-1H-benzo[d]imidazol-1-yl)piperidine-1-carboxylate;
- (S)-2-(dibenzylamino)-3-phenylpropyl 4-(3-(2-tert-butyloxycarbonylaminoethyl)-2-oxo-2,3-dihydro-1H-benzo[d]imidazol-1-yl)piperidine-1-carboxylate;
- (S)-2-(dibenzylamino)-3-phenylpropyl 4-(3-(2 aminoethyl)-2-oxo-2,3-dihydro-1H-benzo[d]imidazol-1-yl)piperidine-1-carboxylate D-biotin amid;
- (S)-2-(dibenzylamino)propyl 3-(1,2-dihydro-2-oxobenzo[d]imidazol-3-yl)pyrrolidine-1-carboxylate;
- 1-[1-(4-dibenzylamino-butyryl)-piperidin-4-yl]-1,3-dihydro-benzoimidazol-2-one; and pharmaceutically acceptable salts thereof.

8. The compound as claimed is any one of the preceding claims, wherein said pharmaceutically acceptable salts are acid addition salts formed with pharmaceutically acceptable acids.

9. The compound of claim 8, wherein said acid addition salts are hydrochloride, hydrobromide, sulphate or bisulphate, phosphate or hydrogenophosphate, acetate, benzoate, succinate, fumarate, maleate, lactate, citrate, tartrate, gluconate, methanesulphonate, benzene-sulphonate, paratoluene-sulphonate salts.

10. The compounds of formula (I) as claimed in any one of the preceding claims for a use as a drug.

11. The compounds of formula (I) as claimed in any one of the preceding claims for a use as a drug for the prevention and/or the treatment of parasitic diseases involving apicomplexan parasites.

12. The compounds of formula (I) according to claim 10 or 11, wherein when the drug is intended for the prevention and/or treatment of malaria, compounds of formula (I) are selected in the group consisting of:
- (S)-2-(dibenzylamino)propyl 4-(1H-benzo[d]imidazol-2-ylamino)piperidine-1-carboxylate;
- (S)-2-(dibenzylamino)propyl 4-(2-oxo-3-phenylsulfonyl-2,3-dihydro-1H-benzo[d]imidazol-1-yl)piperidine-1-carboxylate;
- (S)-2-(dibenzylamino)propyl 4-(3-{4-fluorophenylsulfonyl)-2-oxo-2,3-dihydro-1H-benzo[d]imidazo)-1-yl)piperidine-1-carboxylate;
- (S)-2-(dibenzylamino)propyl 4-(3-(4-methoxyphenylsulfonyl)-2-oxo-2,3-dihydro-1H-benzo[d]imidazol-1-yl)piperidine-1-carboxylate;
- (S)-2-(dibenzylamino)propyl 4-(2-oxo-3-(4-(trifluoromethyl)phcnylsulfonyl)-2,3-dihydro-l H-bcnzo[d]imidazol-I-yl)pipcridinc-I-carboxylate;
- (S)-2-(dibenzylamino)-3-phenylpropyl 4-(1,2-dihydro-1-methyl-2-oxobenzo[d]imidazol-3-yl)piperidine-1-carboxylate;
- (S)-2-(dibenzylamino)propyl 4-(2-oxo-3-(thiophen-2-ylsulfonyl)-2,3-dihydro-1H-benzo[d]imidazol-1-yl)piperidine-1-carboxylate;
- N-((S)-2-(dibenzylamino)propyl)-4-(1,2-dihydro-2-oxobenzo[d]imidazol-3-yl)pipendine-1-carboxamidine,
- (S)-2-(dibenzylamino)propyl 4-(N-phenylphenylsulfonamido)piperidine-1-carboxylate;
- (S)-2-(dibenzylamino)propyl 4-(3-(4-ethoxy-4-oxobutyl)-2-oxo-2,3-dihydro-1H-benzo[d]imidazol-1-yl)piperidine-1-carboxylate;
- (S)-2-(dibenzylamino)propyl 4-(1-benzyl-1,2-dihydro-2-oxobenzo[d]1midazol-3-yl)pipendine-1-carboxylate;
- (S)-2-(dibenzylamino)propyl 4-(2-oxo-3-(quinolin-8-ylsulfonyl)-2,3-dihydro-1H-benzo[d]imidazol-1-yl)piperidine-1-carboxylate;
- tert-butyl 3-(1-(((S)-2-(dibenzylamino)propoxy)carbonyl)piperidin-4-yl)-2,3-dihydro-2-oxobenzo[d]imidazole-1-carboxylate;
- (S)-2-(dibenzylamino)propyl 4-(2-(trifluoromethyl)-1H-benzo[d]imidazol-1-yl)piperidine-l-carboxylate;
- (S)-2-(dibenzylamino)-3-phenylpropyl 4-(1-benzyl-1,2-dihydro-2-oxobenzo[d]imidazol-3-yl)piperidine-1-carboxylate;
- (S)-2-(dibenzylamino)propyl 4-(3-(naphthalen-1-ylsulfonyl)-2-oxo-2,3-dihydro- H-benzo[d]imidazol-1-yl)piperidine-1-carboxylate;
- (S)-2-(dibenzylamino)propyl 4-(1-(cyclohexylmethyl)-1,2-dihydro-2-oxobenzo[d]imidazol-3-yl)piperidine-1-carboxylate;
- (S)-2-(dibenzylamino)propyl 4-(1,2-dihydro-2-thioxobcnzo[d]imidazol-3-yl)pipcridinc-I-carboxylatc;
- 1-(1-(3-(dibenzylamino)propyl)piperidin-4-yl)-1 H-benzo[d]imidazol-2(3H)-one;
- (S)-2-(dibenzylamino)-3-phenylpropyl 4-(1,2-dihydro-2-oxobenzo[d]imidazol-3-yl)piperidine-1-carboxylate;
- (S)-2-(dibenzylamino)propyl 4-(N-phenylbenzamido)piperidine-1-carboxylate;
- 1-(S)-2-(dibenzylamino)propyl 3-ethyl 4-(1,2-dihydro-2-oxobenzo[d]imidazol-3-yl)piperidine-1,3-dicarboxylate;
- (S)-2-(dibenzylamino)propyl 4-(1,2-dihydro-1-methyl-2-oxobenzo[d]imidazol-3-yl)plperldine-1-carboxylate;
- (S)-2-(dibenzylamino)propyl 4-(2-oxo-3-(4-acetamidylphenylsulfonyl)-2,3-dihydro-1H-benzo[d]imidazol-1-yl)piperidine-1-carboxylate-,
- 1-(1-(2-(dibenzylamino)ethyl)piperidin-4-yl)-1 H-benzo[d]imidazol-2(3H)-one;
- (S)-2-(dibenzylamino)propyl 4-(2-oxo-3-methylsulfonyl-2,3-dihydro-1 H-benzo[d]imidazol-1-yl)piperidine-1-carboxylate;
- (S)-2-(dibenzylamino)propyl 4-(1H-benzo[d]imidazol-1-yl)piperidine-1-carboxylate;
- (S)-2-(dibenzylamino)-3-phenylpropyl 4-(1,2-dihydro-3-phenylsulfonyl-2-oxobenzo[d]imidazol-1-yl)piperidine-1-carboxylate;
- 2-benzylamino bis [ethyl 4-(1,2-dihydro-2-oxobenzo[d]imidazol-3-yl)piperidine-1-carboxylate;
- (S)-2-(dibenzylamino)propyl 4-(3-(2-tert-butyloxycarbonylaminoethyl)-2-oxo-2,3-dihydro-1H-benzo[d]imidazol-1-yl)piperidine-1-carboxylate;
- (S)-2-(dibenzylamino)-3-phenylpropyl 4-(3-(2-tert-butyloxycarbonylaminoethyl)-2-oxo-2,3-dihydro-1H-benzo[d] imidazol-1-yl)piperidine-1-carboxylate;
- (S)-2-(dibenzylamino)-3-phenylpropyl 4-(3-(2 aminoethyl)-2-oxo-2,3-dihydro-1H-benzo[d]imidazol-1-yl)piperidine-1-carboxylate D-biotin amid and pharmaceutically acceptable salts thereof.

13. The compounds of formula (I) according to claim 10 or 11, wherein when the drug is intended for the prevention and/or treatment of toxoplasmose, compounds of formula (I) are selected in the group consisting of:
- O-2-(dibenzylamino)ethyl 4-(1,2-dihydro-2-oxobenzo[d]imidazol-3-yl)plperidine-1-carbothioate;
- (S)-2-(dibenzylamino)propyl 4-(1,2-dihydro-1-methyl-2-oxobenzo[d]imidazol-3-yl)piperidine-1-carboxylate;
- (S)-2-(dibenzylamino)propyl 4-(1H-indol-1-yl)piperidine-1-carboxylate;
- (S)-2-(dibenzylamino)propyl 3-(1,2-dihydro-2-oxobenzo[d]imidazol-3-yl)propylcarbamate;
- 1-benzylpiperidin-3-yl 4-(1,2-dihydro-2-oxobenzo[d]imidazol-3-yl)piperidine-1-carboxylate;
- 2-(benzyloxy)ethyl 4-(1,2-dihydro-2-oxobenzo[d]imidazol-3-yl)piperidine-1-carboxylate;
- (S)-2-(dibenzylamino)propy) 4-(1,2-dihydro-2-oxonaphtho[2,3-d]imidazol-3-yl)piperidine-1-carboxylate;
- 1-(1-(3-(dibenzylamino)propyl)piperidin-4-yl)-1H-benzo[d]imidazol-2(3H)-one;
- (S)-2-(dibenzylamino)propyl 4-(2-oxobenzo[d]oxazol-3(2H)-yl)piperidine-1-carboxylate;
- (S)-2-(dibenzylamino)-3-phenylpropyl 4-(1,2-dihydro-1-methyl-2-oxobenzo[d]imidazol-3-yl)piperidine-1-carboxylate;
- (S)-2-(dibenzylamino)propyl 4-(1,2-dihydro-2-oxo-1-(benzoyl)benzo[d]imidazol-3-yl)piperidine-1-carboxylate;
- 2-(1-benzylpiperidin-4-yl)ethyl 4-(1,2-dihydro-2-oxobenzo[d]imidazol-3-yl)piperidine-1-carboxylate;
- (S)-2-(dibenzylamino)propyl 4-(1H-benzo[d]imidazol-2-ylamino)piperidine-1-carboxylate;
- (S)-2-(dibenzylamino)propyl 4-(lacetyl-1,2-dihydro-2-oxobenzo[d]imidazol-3-yl)piperidine-1-carboxylate;
- (S)-2-(dibenzylamino)propyl 4-(6-chloro-1,2-dihydro-2-oxobenzo[d]imidazol-3-yl)piperidine-1-carboxylate;
- O-2-(benzyloxy)ethyl 4-(1,2-dihydro-2-oxobenzo[d]imidazol-3-yl)piperidine-1-carbothioate
- O-(S)-2-(dibenzylamino)propyl 4-(1,2-dihydro-2-oxobenzo[d]imidazol-3-yl)piperidine-1-carbothioate;
- 3,3-diphenylpropyl 4-(1,2-dihydro-2-oxobenzo[d]imidazol-3-yl)piperidine-1-carboxylate;
- (S)-2-(dibenzylamino)propyl 4-(N-phenylpropionamido)piperidine-1-carboxylate;
- (S)-2-(dibenzylamino)propyl 4-(2-oxoindolin-3-ylidene)piperidine-1-carboxylate;
- 1-(1-(4-(dibenzylamino)butyl)piperidin-4-yl)-1H-benzo[d]imidazol-2(3H)-one;
- (S)-2-(dibenzylamino)propyl 4-(3-(4-fluorophenylsulfonyl)-2-oxo-2,3-dihydro-1H-benzo[d]imidazol-1-yl)plperidine-1-carboxylate;
- (S)-2-(dibenzylamino)propyl 4-(1-(cyclohexylmcthyl)-1,2-dihydro-2-oxobenzo[d]imidazol-3-yl)piperidine-1-carboxylate;
- (S)-2-(dibenzylamino)propyl 4-(2-oxo-3-phenylsulfonyl-2,3-dihydro-1H-benzo[d]imidazol-1-yl)piperidine-1-carboxylate;
- (S)-4-(3-(1-((2-(dibenzylamino)propoxy)carbonyl)piperidin-4-yl)-2-oxo-2,3-dihydro-1H-benzo[d]imidazol-1-yl)butanoic acid;
- (S)-2-(dibenzylamino)propyl 4-(3-(4-methoxyphenylsulfonyl)-2-oxo-2,3-dihydro-1H-benzo[d]imidazol-1-yl)piperidine-1-carboxylate;
- (S)-2-(dibenzylamino)propyl 4-(N-phenylphenylsulfonamido)piperidine-1-carboxylate;
- (S)-2-(dibenzylamino)propyl 4-(1-benzyl-1,2-dihydro-2-oxobenzo[d]imidazol-3-yl)piperidine-1-carboxylate;
- 2-(ethyl(phenyl)amino)ethyl 4-(2-oxo-2,3-dihydro-1H-benzo[d]imidazol-1-yl)piperidine-1-carboxylate;
- (S)-2-(dibenzylamino)-3-phenylpropyl 4-(1-benzyl-1,2-dihydro-2-oxobenzo[d]imidazol-3-yl)piperidine-1-carboxylate;
- (S)-2-(dibenzylamino)propyl 4-(1H-indol-3-yl)piperidine-1-carboxylate;
- (S)-2-(dibenzylamino)propyl 4-(2-oxo-3-methylsulfonyl-2,3-dihydro-1H-benzo[d]imidazo)-1-yl)piperidine-1-carboxylate;
- (S)-2-(dibenzylamino)propyl 4-(indolin-1-yl)piperidine-1-carboxylate;
- 2-benzylamino bis [ethyl 4-(1,2-dihydro-2-oxobenzo[d]imidazol-3-yl)piperidine-1-carboxylate and pharmaceutically acceptable salts thereof.

14. A pharmaceutical composition comprising, as an active principle, at least one compound of formula (I) as defined in any one of claims 1 to 9, and at least one pharmaceutically acceptable excipient.

15. The pharmaceutical composition of claim 14, wherein it comprises, in addition to the at least one compound of formula (I), one or more additional antiparasitic active principle.
